# EUROPEAN PATENT SPECIFICATION

(11) **EP 3 880 238 B1**
(45) Date of publication and mention of the grant of the patent: **24.06.2026**
(21) Application number: 19832221.6
(22) Date of filing: 15.11.2019
(51) Int. Cl.: A61K 40/00, A61K 40/11, A61K 40/31, A61K 40/42, A61K 39/00, C07K 14/70, C07K 16/00, C12N 5/0783, A61P 35/00, A61P 35/02

(54) **METHODS OF DOSING ENGINEERED T CELLS FOR THE TREATMENT OF B CELL MALIGNANCIES**
VERFAHREN ZUR DOSIERUNG VON MANIPULIERTEN T-ZELLEN ZUR BEHANDLUNG VON B-ZELL-MALIGNITÄTEN
MÉTHODES DE POSOLOGIE POUR CELLULES T MODIFIÉES POUR LE TRAITEMENT DE CANCERS À CELLULES B

(30) Priority: 16.11.2018 US 201862768844 P; 11.10.2019 US 201962914303 P
(43) Date of publication of application: 22.09.2021
(73) Proprietor: Juno Therapeutics, Inc., Seattle, WA 98109 (US)
(72) Inventor: TREDE, Nikolaus Sebastian, Seattle, WA 98109 (US); ALBERTSON, Tina, Seattle, WA 98109 (US); CHRISTIN, Brian, Seattle, WA 98109 (US); YOST, Rachel K., Seattle, WA 98109 (US); KANG, Michelle, Seattle, WA 98109 (US); LARSON, Ryan P., Seattle, WA 98109 (US); TEOH, Jeffrey, Seattle, WA 98109 (US)
(74) Representative: Carpmaels & Ransford LLP
(86) International application number: PCT/US2019/061876
(87) International publication number: WO 2020/102770

(56) References cited:
- DANIEL W LEE ET AL: "T cells expressing CD19 chimeric antigen receptors for acute lymphoblastic leukaemia in children and young adults: a phase 1 dose-escalation trial", THE LANCET, vol. 385, no. 9967, 1 February 2015 (2015-02-01), AMSTERDAM, NL, pages 517 - 528, XP055388598, ISSN: 0140-6736, DOI: 10.1016/S0140-6736(14)61403-3
- QASIM WASEEM ET AL: "612. Preliminary Results of UCART19, an Allogeneic Anti-CD19 CAR T-Cell Product in a First-in-Human Trial (PALL) in Pediatric Patients with CD19+ Relapsed/Refractory B-Cell Acute Lymphoblastic Leukemia", BLOOD, 7 December 2017 (2017-12-07), pages 1 - 4, XP093094064, Retrieved from the Internet <URL:https://ashpublications.org/blood/article/130/Supplement%201/1271/116214/Preliminary-Results-of-UCART19-an-Allogeneic-Anti> [retrieved on 20231023]
- QASIM WASEEM ET AL: "Preliminary Results of UCART19, an Allogeneic Anti-CD19 CAR T-Cell Product in a First-in-Human Trial (PALL) in Pediatric Patients with CD19+ Relapsed/Refractory B-Cell Acute Lymphoblastic Leukemia", BLOOD, 8 December 2017 (2017-12-08), pages 1271 - 1271, XP093086673, Retrieved from the Internet <URL:https://www.cellectis.com/uploads/files/Poster_PALL_ASH.pdf> [retrieved on 20230928], DOI: 10.1182/blood.V130.Suppl_1.1271.1271
- CARLOS A. RAMOS ET AL: "In�Vivo Fate and Activity of Second- versus Third-Generation CD19-Specific CAR-T Cells in B Cell Non-Hodgkin?s Lymphomas", MOLECULAR THERAPY, vol. 26, no. 12, 1 December 2018 (2018-12-01), US, pages 2727 - 2737, XP055758437, ISSN: 1525-0016, DOI: 10.1016/j.ymthe.2018.09.009
- SHINICHI MAKITA ET AL: "Clinical development of anti-CD19 chimeric antigen receptor T-cell therapy for B-cell non-Hodgkin lymphoma", CANCER SCIENCE, vol. 108, no. 6, 25 May 2017 (2017-05-25), JP, pages 1109 - 1118, XP055765261, ISSN: 1347-9032, DOI: 10.1111/cas.13239
- THERAPEUTICS JUNO: "Juno Advances CAR T-cell CAR017 and Halts CAR015 in Non-Hodgkin Lymphoma", 8 March 2017 (2017-03-08), XP093086029, Retrieved from the Internet <URL:https://blogs.shu.edu/cancer/2017/03/08/juno-advances-car-t-cell-car017-and-halts-car015-in-non-hodgkin-lymphoma/> [retrieved on 20230926]
- SHANNON L. MAUDE ET AL: "Tisagenlecleucel in Children and Young Adults with B-Cell Lymphoblastic Leukemia", THE NEW ENGLAND JOURNAL OF MEDICINE, - NEJM -, vol. 378, no. 5, 1 February 2018 (2018-02-01), US, pages 439 - 448, XP055665831, ISSN: 0028-4793, DOI: 10.1056/NEJMoa1709866
- REBECCA A. GARDNER ET AL: "Intent to treat leukemia remission by CD19CAR T cells of defined formulation and dose in children and young adults", BLOOD, vol. 129, no. 25, 13 April 2017 (2017-04-13), pages 3322 - 3331, XP055509718, ISSN: 0006-4971, DOI: 10.1182/blood-2017-02-769208
- N. SINGH ET AL: "Early memory phenotypes drive T cell proliferation in patients with pediatric malignancies", SCIENCE TRANSLATIONAL MEDICINE, vol. 8, no. 320, 6 January 2016 (2016-01-06), US, pages 320ra3 - 320ra3, XP055281945, ISSN: 1946-6234, DOI: 10.1126/scitranslmed.aad5222

## Description

### Field

The present disclosure relates in some aspects to uses involving the administration of doses of engineered T cells for treating subjects with disease and conditions such as certain B cell malignancies. The engineered cells express anti-CD19 chimeric antigen receptors (CARs). In some embodiments, the disease or condition is acute lymphoblastic leukemia (ALL) or non-Hodgkin lymphoma (NHL). The subject is within a particular range of age, i.e. the subjects are 25 years or less of age, such as pediatric subjects.

### Background

Various immunotherapy and/or cell therapy methods are available for treating diseases and conditions. For example, adoptive cell therapies (including those involving the administration of cells expressing recombinant receptors specific for a disease or disorder of interest, such as chimeric antigen receptors (CARs) and/or other recombinant receptors, as well as other adoptive immune cell and adoptive T cell therapies) can be beneficial in the treatment of diseases or disorders, such as B cell malignancies or hematological malignancies. Improved approaches are needed. Provided are methods and uses that meet such needs.
Maude et al, The New England Journal of Medicine (NEJM), 2018, vol. 378, no. 5, pages 439 - 448 describes a study of tisagenlecleucel in children and young adults with B-cell lymphoblastic leukemia. Gardner et al, Blood, 2017, vol. 129, no. 25, pages 3322 - 3331 describes intent to treat leukemia remission by CD19 CAR T cells of defined formulation and dose in children and young adults. Fraietta et al, Nature Medicine, 2018, vol. 24, no. 5, pages 563-571 describes determinants of response and resistance to CD19 chimeric antigen receptor (CAR) T cell therapy of chronic lymphocytic leukemia.
Daniel W Lee et al, The Lancet, 2015, vol. 385, no. 9967, pages 517-528 describes a clinical phase I trial, registered with the ClinicalTrials.gov, number NCT01593696, analysing the feasibility, toxicity, the maximum tolerated dose of anti-CD19 CART cells administered to children and young adults aged between 1-30 years with refractory B-cell malignancies, wherein the administration of CAR+ T cells is based only on the weight of the subjects.

### Summary

The invention is defined by the appended claims.

The invention provides a T cell composition for use in a method of treating a B cell malignancy in a subject at or younger than 25 years of age, wherein the T cell composition comprises T cells expressing an anti-CD19 chimeric antigen receptor (CAR),
wherein the T cells are autologous to the subject;
wherein the CAR comprises an scFv specific for a human CD19, a transmembrane domain, a cytoplasmic signaling domain derived from a costimulatory molecule, and a cytoplasmic signaling domain derived from a primary signaling ITAM-containing molecule, which is or comprises a human CD3zeta signaling domain;
the method comprising:
   a) determining the dose of CAR+ T cells based on the age of the subject; and
   b) administering, to the subject, the CAR + T cell composition, wherein :
      (i) if the subject is younger than 18 years of age, from at or about 0.05 x 10⁶ CAR+ T cells/kg body weight of the subject to at or about 1.5 x 10⁶ CAR+ T cells/kg body weight of the subject, but that does not exceed at or about 1.5 x 10⁸ CAR+ T cells; and
      (ii) if the subject is between 18 and 25 years of age, inclusive, from at or about 0.05 x 10⁸ CAR+ T cells to at or about 1.5 x 10⁸ CAR+ T cells.

In some embodiments, the T cell composition for use is administered to a subject at or younger than 25 years of age, wherein the T cell composition comprises T cells expressing an anti-CD19 chimeric antigen receptor (CAR), wherein the composition is administered in an amount selected from: (i) if the subject is younger than 18 years of age from at or about 0.5 x 10⁶ CAR+ T cells/kg body weight of the subject to at or about 1.5 x 10⁶ CAR+ T cells/kg body weight of the subject, but that does not exceed at or about 1.5 x 10⁸ total CAR+ T cells; or (ii) if the subject is between 18 and 25 years of age, inclusive, from at or about 0.5 x 10⁸ CAR+ T cells to at or about 1.5 x 10⁸ CAR+ T cells.

In some of any embodiments,: (i) if the subject is younger than 18 years of age, the T cell composition is administered in an amount from at or about 0.05 x 10⁶ CAR+ T cells/kg body weight of the subject to at or about 0.75 x 10⁶ CAR+ T cells/kg body weight of the subject, but that does not exceed at or about 0.75 x 10⁸ total CAR+ T cells; and (ii) if the subject is between 18 and 25 years of age, inclusive, the T cell composition is administered in an amount from at or about 0.05 x 10⁸ CAR+ T cells to at or about 0.75 x 10⁸ CAR+ T cells.

In some of any embodiments, if the subject is younger than 18 years of age the T cell composition is administered in an amount from at or about 0.05 x 10⁶ CAR+ T cells/kg body weight of the subject to at or about 1.0 x 10⁶ CAR+ T cells/kg body weight of the subject, but that does not exceed at or about 1.0 x 10⁸ total CAR+ T cells.

In some of any embodiments, if the subject is younger than 18 years of age the T cell composition is administered in an amount from at or about 0.5 x 10⁶ CAR+ T cells/kg body weight of the subject to at or about 1.0 x 10⁶ CAR+ T cells/kg body weight of the subject, but that does not exceed at or about 1.0 x 10⁸ total CAR+ T cells.

**In** some of any embodiments, if the subject is younger than 18 years of age, the T cell composition is administered in an amount that is at least at or about 0.05 x 10⁶ CAR+ T cells/kg body weight of the subject but that does not exceed at or about 0.05 x 10⁸ total CAR+ T cells. In some of any embodiments, if the subject exhibits no response and does not develop a toxicity at or about 21, 22, 23, 24, 25, 26, 27, 28, 29, 30, 31, 32, 33, 34 or 35 days after administration of the T cell composition; further comprising administering to the subject an additional dose of the T cell composition wherein, if the subject is younger than 18 years of age, the additional dose of the T cell composition is administered in an amount that is at least at or about 0.1 x 10⁶ CAR+ T cells/kg body weight of the subject but that does not exceed at or about 0.1 x 10⁸ total CAR+ T cells. In some of any embodiments, if the subject is younger than 18 years of age, the T cell composition is administered in an amount that is at least at or about 0.15 x 10⁶ CAR+ T cells/kg body weight of the subject but that does not exceed at or about 0.15 x 10⁸ total CAR+ T cells. In some of any embodiments, if the subject is younger than 18 years of age, the T cell composition is administered in an amount that is at least at or about 0.3 x 10⁶ CAR+ T cells/kg body weight of the subject but that does not exceed at or about 0.3 x 10⁸ total CAR+ T cells. In some of any embodiments, if the subject is younger than 18 years of age, the T cell composition is administered in an amount that is at least at or about 0.5 x 10⁶ CAR+ T cells/kg body weight of the subject but that does not exceed at or about 0.5 x 10⁸ total CAR+ T cells. In some of any embodiments, if the subject is younger than 18 years of age, the T cell composition is administered in an amount that is at least at or about 0.75 x 10⁶ CAR+ T cells/kg body weight of the subject but that does not exceed at or about 0.75 x 10⁸ total CAR+ T cells. In some of any embodiments, if the subject is younger than 18 years of age, the T cell composition is administered in an amount that is at least at or about 1.0 x 10⁶ CAR+ T cells/kg body weight of the subject but that does not exceed at or about 1.0 x 10⁸ total CAR+ T cells.

**In** some of any embodiments, if the subject is between 18 and 25 years of age, inclusive, the T cell composition is administered in an amount from at or about 0.05 x 10⁸ CAR+ T cells to at or about 1.0 x 10⁸ CAR+ T cells. In some of any embodiments, if the subject is between 18 and 25 years of age, inclusive, the T cell composition is administered in an amount from at or about 0.5 x 10⁸ CAR+ T cells to at or about 1.0 x 10⁸ CAR+ T cells. In some of any embodiments, if the subject is between 18 and 25 years of age, inclusive, the T cell composition is administered in an amount that is at or about 0.05 x 10⁸ CAR+ T cells. In some of any embodiments, if the subject is between 18 and 25 years of age, inclusive, the T cell composition is administered in an amount that is at or about 0.15 x 10⁸ CAR+ T cells. In some of any embodiments, if the subject is between 18 and 25 years of age, inclusive, the T cell composition is administered in an amount that is at or about 0.3 x 10⁸ CAR+ T cells. In some of any embodiments, if the subject is between 18 and 25 years of age, inclusive, the T cell composition is administered in an amount that is at or about 0.5 x 10⁸ CAR+ T cells. In some of any embodiments, if the subject is between 18 and 25 years of age, inclusive, the T cell composition is administered in an amount that is at or about 0.75 x 10⁸ CAR+ T cells. In some of any embodiments, if the subject is between 18 and 25 years of age, inclusive, the T cell composition is administered in an amount that is at or about 1.0 x 10⁸ CAR+ T cells.

**In** some of any of the provided T cell compositions for use in a method of treating a subject at or younger than 25 years of age having or suspected of having a B-cell malignancy, the subject is at least at or about 6 kg in body weight. In some of any of the provided T cell compositions for use, the subject is at least at or about 12 kg in body weight.

**In** some of any embodiments, the T cell composition for use is administered to a subject at or younger than 25 years of age and weighing 12 kg or more, wherein the T cell composition comprises T cells expressing an anti-CD19 chimeric antigen receptor (CAR), wherein the T cell composition is administered in an amount selected from: (i) if the subject is younger than 18 years of age, at or about 0.05 x 10⁶ CAR+ T cells/kg body weight of the subject, but not exceeding at or about 0.05 x 10⁸ total CAR+ T cells; and (ii) if the subject is between 18 and 25 years of age, inclusive, at or about 0.05 x 10⁸ CAR+ T cells.

**In** some of any embodiments, the T cell composition for use is administered to a subject at or younger than 25 years of age and weighing 12 kg or more, wherein the T cell composition comprises T cells expressing an anti-CD19 chimeric antigen receptor (CAR), wherein the T cell composition is administered in an amount selected from: (i) if the subject is younger than 18 years of age, at or about 0.15 x 10⁶ CAR+ T cells/kg body weight of the subject, but not exceeding at or about 0.15 x 10⁸ total CAR+ T cells; and (ii) if the subject is between 18 and 25 years of age, inclusive, at or about 0.15 x 10⁸ CAR+ T cells.

**In** some of any embodiments, the T cell composition for use is administered to a subject at or younger than 25 years of age and weighing 12 kg or more, wherein the T cell composition comprises T cells expressing an anti-CD19 chimeric antigen receptor (CAR), wherein the T cell composition is administered in an amount selected from: (i) if the subject is younger than 18 years of age, at or about 0.3 x 10⁶ CAR+ T cells/kg body weight of the subject, but not exceeding at or about 0.3 x 10⁸ total CAR+ T cells; and (ii) if the subject is between 18 and 25 years of age, inclusive, at or about 0.3 x 10⁸ CAR+ T cells.

**In** some of any embodiments, the T cell composition for use is administered to a subject at or younger than 25 years of age and weighing 12 kg or more, wherein the T cell composition comprises T cells expressing an anti-CD19 chimeric antigen receptor (CAR), wherein the T cell composition is administered in an amount selected from: (i) if the subject is younger than 18 years of age, at or about 0.5 x 10⁶ CAR+ T cells/kg body weight of the subject, but not exceeding at or about 0.5 x 10⁸ total CAR+ T cells; and (ii) if the subject is between 18 and 25 years of age, inclusive, at or about 0.5 x 10⁸ CAR+ T cells.

**In** some of any embodiments, the T cell composition for use is administered to a subject at or younger than 25 years of age and weighing 12 kg or more, wherein the T cell composition comprises T cells expressing an anti-CD19 chimeric antigen receptor (CAR), wherein the T cell composition is administered in an amount selected from: (i) if the subject is younger than 18 years of age, at or about 0.75 x 10⁶ CAR+ T cells/kg body weight of the subject, but not exceeding at or about 0.75 x 10⁸ total CAR+ T cells; and (ii) if the subject is between 18 and 25 years of age, inclusive, at or about 0.75 x 10⁸ CAR+ T cells.

**In** some of any embodiments, the T cell composition for use is administered to a subject at or younger than 25 years of age and weighing 12 kg or more, wherein the T cell composition comprises T cells expressing an anti-CD19 chimeric antigen receptor (CAR), wherein the T cell composition is administered in an amount selected from: (i) if the subject is younger than 18 years of age, at or about 1 x 10⁶ CAR+ T cells/kg body weight of the subject, but not exceeding at or about 1 x 10⁸ total CAR+ T cells; and (ii) if the subject is between 18 and 25 years of age, inclusive, at or about 1 x 10⁸ CAR+ T cells.

**In** some of any embodiments, the T cell composition for use is administered to a subject at or younger than 25 years of age and weighing 6 kg or more, wherein the T cell composition comprises T cells expressing an anti-CD19 chimeric antigen receptor (CAR), wherein the T cell composition is administered in an amount selected from (i) if the subject is younger than 18 years of age at or about 1 x 10⁶ CAR+ T cells/kg body weight of the subject, but not exceeding at or about 1 x 10⁸ total CAR+ T cells; and (ii) if the subject is between 18 and 25 years of age, inclusive, at or about 1.0 x 10⁸ CAR+ T cells.

**In** some of any embodiments, the T cell composition for use is administered to a subject younger than 18 years of age and weighing 12 kg or more, wherein the composition comprises T cells expressing an anti-CD19 chimeric antigen receptor (CAR), wherein the composition is administered in an amount that is at or about 0.05 x 10⁶ CAR+ T cells/kg body weight of the subject to at or about 1.5 x 10⁶ CAR+ T cells/kg body weight of the subject, but that does not exceed at or about 1.5 x 10⁸ total CAR+ T cells. In some of any embodiments, the composition is administered in an amount from at or about 0.5 x 10⁶ CAR+ T cells/kg body weight of the subject to at or about 1.0 x 10⁶ CAR+ T cells/kg body weight of the subject, but that does not exceed at or about 1.0 x 10⁸ total CAR+ T cells.

**In** some of any embodiments, the T cell composition for use is administered to a subject younger than 18 years of age and weighing 12 kg or more, wherein the composition comprises T cells expressing an anti-CD19 chimeric antigen receptor (CAR), wherein the composition is administered in an amount that is at or about 0.5 x 10⁶ CAR+ T cells/kg body weight of the subject to at or about 1.5 x 10⁶ CAR+ T cells/kg body weight of the subject, but that does not exceed at or about 1.5 x 10⁸ total CAR+ T cells. In some of any embodiments, the composition is administered in an amount from at or about 0.5 x 10⁶ CAR+ T cells/kg body weight of the subject to at or about 1.0 x 10⁶ CAR+ T cells/kg body weight of the subject, but that does not exceed at or about 1.0 x 10⁸ total CAR+ T cells.

**In** some of any embodiments, the composition is administered in an amount that is at least at or about 0.05 x 10⁶ CAR+ T cells/kg body weight of the subject but that does not exceed at or about 0.05 x 10⁸ total CAR+ T cells. In some of any embodiments, the composition is administered in an amount that is at or about 0.05 x 10⁶ CAR+ T cells/kg body weight of the subject to at or about 0.75 x 10⁶ CAR+ T cells/kg body weight of the subject, but that does not exceed at or about 0.75 x 10⁸ total CAR+ T cells. In some of any embodiments, the composition is administered in an amount that is at least at or about 0.15 x 10⁶ CAR+ T cells/kg body weight of the subject but that does not exceed at or about 0.15 x 10⁸ total CAR+ T cells. In some of any embodiments, the composition is administered in an amount that is at least at or about 0.3 x 10⁶ CAR+ T cells/kg body weight of the subject but that does not exceed at or about 0.3 x 10⁸ total CAR+ T cells. In some of any embodiments, the composition is administered in an amount that is at least at or about 0.5 x 10⁶ CAR+ T cells/kg body weight of the subject but that does not exceed at or about 0.5 x 10⁸ total CAR+ T cells. In some of any embodiments, the composition is administered in an amount that is at least at or about 0.75 x 10⁶ CAR+ T cells/kg body weight of the subject but that does not exceed at or about 0.75 x 10⁸ total CAR+ T cells. In some of any embodiments, the composition is administered in an amount that is at least at or about 1.0 x 10⁶ CAR+ T cells/kg body weight of the subject but that does not exceed at or about 1.0 x 10⁸ total CAR+ T cells.

In some of any embodiments, a total volume of at least 0.05 mL at a concentration of at or greater than 2.5 x 10⁶ cells/mL of the T cell composition is administered. In some of any embodiments, a total volume of at least at or about 0.1 mL at a concentration of at or greater than 2.5 x 10⁶ cells/mL of the T cell composition is administered. In some of any of the provided T cell compositions for use , a total volume of at least at or about 0.5 mL at a concentration of at or greater than 2.5 x 10⁶ cells/mL of the T cell composition is administered.

In some of any embodiments, the T cell composition for use is administered to a subject at or younger than 25 years of age, wherein the T cell composition comprises T cells expressing an anti-CD19 chimeric antigen receptor (CAR), wherein the T cell composition is administered in an amount selected from: (i) if the subject is younger than 18 years of age, an amount not exceeding at or about 1.5 x 10⁸ total CAR+ T cells in a volume of at least 0.1 mL; and (ii) if the subject is between 18 and 25 years of age, inclusive, less than at or about 1.5 x 10⁸ CAR+ T cells.

In some of any embodiments, the T cell composition for use is administered to a subject at or younger than 25 years of age, wherein the T cell composition comprises T cells expressing an anti-CD19 chimeric antigen receptor (CAR), wherein the T cell composition is administered in an amount selected from: (i) if the subject is younger than 18 years of age, an amount not exceeding at or about 0.05 x 10⁸ total CAR+ T cells in a volume of at least 0.1 mL; and (ii) if the subject is between 18 and 25 years of age, inclusive, at or about 0.05 x 10⁸ CAR+ T cells.

In some of any embodiments, the T cell composition for use is administered to a subject at or younger than 25 years of age, wherein the T cell composition comprises T cells expressing an anti-CD19 chimeric antigen receptor (CAR), wherein the T cell composition is administered in an amount selected from: (i) if the subject is younger than 18 years of age, an amount not exceeding at or about 0.15 x 10⁸ total CAR+ T cells in a volume of at least 0.1 mL; and (ii) if the subject is between 18 and 25 years of age, inclusive, at or about 0.15 x 10⁸ CAR+ T cells.

In some of any embodiments, the T cell composition for use is administered to a subject at or younger than 25 years of age, wherein the T cell composition comprises T cells expressing an anti-CD19 chimeric antigen receptor (CAR), wherein the T cell composition is administered in an amount selected from: (i) if the subject is younger than 18 years of age, an amount not exceeding at or about 0.3 x 10⁸ total CAR+ T cells in a volume of at least 0.1 mL; and (ii) if the subject is between 18 and 25 years of age, inclusive, at or about 0.3 x 10⁸ CAR+ T cells.

In some of any embodiments, the T cell composition for use is administered to a subject at or younger than 25 years of age, wherein the T cell composition comprises T cells expressing an anti-CD19 chimeric antigen receptor (CAR), wherein the T cell composition is administered in an amount selected from: (i) if the subject is younger than 18 years of age, an amount not exceeding at or about 0.5 x 10⁸ total CAR+ T cells in a volume of at least 0.1 mL; and (ii) if the subject is between 18 and 25 years of age, inclusive, at or about 0.5 x 10⁸ CAR+ T cells.

Provided herein are methods of treating a subject having or suspected of having a B cell In some of any embodiments, the T cell composition for use is administered to a subject at or younger than 25 years of age, wherein the T cell composition comprises T cells expressing an anti-CD19 chimeric antigen receptor (CAR), wherein the T cell composition is administered in an amount selected from: (i) if the subject is younger than 18 years of age, an amount not exceeding at or about 0.75 x 10⁸ total CAR+ T cells in a volume of at least 0.1 mL; and (ii) if the subject is between 18 and 25 years of age, inclusive, at or about 0.75 x 10⁸ CAR+ T cells.

In some of any embodiments, the concentration of the T cells is at or greater than 2.5 x 10⁶ cells/mL.

In some of any embodiments, the T cell composition for use is administered to a subject at or younger than 25 years of age, wherein the T cell composition comprises T cells expressing an anti-CD19 chimeric antigen receptor (CAR) at a concentration of at or greater than 2.5 x 10⁶ cells/mL, wherein the T cell composition is administered in an amount selected from: (i) if the subject is younger than 18 years of age, an amount not exceeding at or about 0.05 x 10⁸ total CAR+ T cells in a volume of at least 0.05 mL; and (ii) if the subject is between 18 and 25 years of age, inclusive, at or about 0.05 x 10⁸ CAR+ T cells.

In some of any embodiments, the T cell composition for use is administered to a subject at or younger than 25 years of age, wherein the T cell composition comprises T cells expressing an anti-CD19 chimeric antigen receptor (CAR) at a concentration of at or greater than 2.5 x 10⁶ cells/mL, wherein the T cell composition is administered in an amount selected from: (i) if the subject is younger than 18 years of age, an amount not exceeding at or about 0.15 x 10⁸ total CAR+ T cells in a volume of at least 0.15 mL; and (ii) if the subject is between 18 and 25 years of age, inclusive, at or about 0.15 x 10⁸ CAR+ T cells.

In some of any embodiments, the T cell composition for use is administered to a subject at or younger than 25 years of age, wherein the T cell composition comprises T cells expressing an anti-CD19 chimeric antigen receptor (CAR) at a concentration of at or greater than 2.5 x 10⁶ cells/mL, wherein the T cell composition is administered in an amount selected from: (i) if the subject is younger than 18 years of age, an amount not exceeding at or about 0.3 x 10⁸ total CAR+ T cells in a volume of at least 0.3 mL; and (ii) if the subject is between 18 and 25 years of age, inclusive, at or about 0.3 x 10⁸ CAR+ T cells.

In some of any embodiments, the T cell composition for use is administered to a subject at or younger than 25 years of age, wherein the T cell composition comprises T cells expressing an anti-CD19 chimeric antigen receptor (CAR) at a concentration of at or greater than 2.5 x 10⁶ cells/mL, wherein the T cell composition is administered in an amount selected from (i) if the subject is younger than 18 years of age, an amount not exceeding at or about 0.5 x 10⁸ total CAR+ T cells in a volume of at least 0.5 mL; and (ii) if the subject is between 18 and 25 years of age, inclusive, at or about 0.5 x 10⁸ CAR+ T cells.

In some of any embodiments, the T cell composition for use is administered to a subject at or younger than 25 years of age, wherein the T cell composition comprises T cells expressing an anti-CD19 chimeric antigen receptor (CAR) at a concentration of at or greater than 2.5 x 10⁶ cells/mL, wherein the T cell composition is administered in an amount selected from: (i) if the subject is younger than 18 years of age, an amount not exceeding at or about 0.75 x 10⁸ total CAR+ T cells in a volume of at least 0.75 mL; and (ii) if the subject is between 18 and 25 years of age, inclusive, at or about 0.75 x 10⁸ CAR+ T cells.

In some of any embodiments, the T cell composition for use is administered to a subject at or younger than 25 years of age, wherein the T cell composition comprises T cells expressing an anti-CD19 chimeric antigen receptor (CAR) at a concentration of at or greater than 2.5 x 10⁶ cells/mL, wherein the T cell composition is administered in an amount selected from: (i) if the subject is younger than 18 years of age, an amount not exceeding at or about 1 x 10⁸ total CAR+ T cells in a volume of at least 0.5 mL; and (ii) if the subject is between 18 and 25 years of age, inclusive, at or about 1.0 x 10⁸ CAR+ T cells. In some of any embodiments, a total volume of at least at or about 0.05 mL of the T cell composition is administered. In some of any embodiments, a total volume of at least at or about 0.1 mL of the T cell composition is administered. In some of any embodiments, a total volume of at least at or about 0.5 mL of the T cell composition is administered. In some of any embodiments, a total volume of at least at or about 1.0 mL of the T cell composition is administered.

In some of any embodiments, the total volume of the T cell composition administered is at least 0.05 mL. In some of any embodiments, the total volume of the T cell composition administered is at least 0.1 mL. In some of any of the provided embodiments, the total volume of the T cell composition administered is at least 1.0 mL.

In some of any of the provided embodiments, the concentration of the T cell composition is greater than at or about 5 x 10⁶ cells/mL or is or is about 5 x 10⁶ cells/mL In some of any of the provided embodiments, the concentration of the T cell composition is greater than at or about 10 x 10⁶ cells/mL or is or is about 10 x 10⁶ cells/mL. In some of any of the provided embodiments, the concentration of the T cell composition is greater than or greater than about 15 x 10⁶ cells/mL or is or is about 15 x 10⁶ cells/mL.

In some of any of the provided embodiments, the T cell composition for use comprises CD4⁺ and CD8⁺ CAR+ T cells. In some of any embodiments, the composition comprises a first composition comprising one of the CD4+ T cells and the CD8+ T cells and a second composition comprising the other of the CD4+ T cells and the CD8+ T cells. In some of any embodiments, the first composition and the second composition are administered separately. In some of any embodiments, the first composition and the second composition are administered simultaneously. In some of any embodiments, the first composition and the second composition are administered sequentially, in either order. In some aspects, the first composition comprises CD4⁺ CAR+ T cells and the second composition comprises CD8+ T cells. In other aspects, the first composition comprises CD8⁺ CAR+ T cells and the second composition comprises CD4+ CAR+ T cell.

In some of any of the provided embodiments, the amount of the T cell composition administered comprises a defined ratio of CD4⁺ CAR+ T cells to CD8⁺ CAR+ T cells and/or of CD4⁺ T cells to CD8⁺ T cells, that is or is approximately 1:1 or is between approximately 1:3 and approximately 3:1. In some of any embodiments, such as involving administration of a first and second composition, the CD4⁺ CAR+ T cells in the one of the first and second compositions and the CD8⁺ CAR+ T cells in the other of the first and second compositions are present at a defined ratio that is or is approximately 1:1 or is between approximately 1:3 and approximately 3:1. In some of any embodiments, such as involving administration of a first and second composition, the CD4⁺ CAR+ T cells and the CD8⁺ CAR+ T cells administered in the first and second compositions are present at a defined ratio, which ratio is or is approximately 1:1 or is between approximately 1:3 and approximately 3:1. In some of any of the provided embodiments, the defined ratio is or is approximately 1:1.

In some of any of the provided T cell compositions for use, the B cell malignancy is a lymphoma or a leukemia. In some of any embodiments, the B cell malignancy is relapsed and/or refractory.

In some of any of the provided T cell compositions for use, the B cell malignancy is a B-cell acute lymphoblastic leukemia (B-ALL). In some of any embodiments, the B cell malignancy is a B-cell acute lymphoblastic leukemia (B-ALL), optionally CD19+ B-ALL. In some of any embodiments, the B cell malignancy is relapsed or refractory (r/r) B-cell Acute Lymphoblastic Leukemia (B-ALL). In some of any embodiments, subject with r/r B-ALL has morphological evidence of disease in bone marrow, optionally wherein the subject has 5% or greater lymphoblast by morphology. In some of any of the embodiments, the subject has a B-ALL that is any of the following: first or greater marrow relapse, any marrow relapse after allogeneic hematopoietic stem cell transplantation (HSCT); primary refractory, optionally following 2 or more separate induction regimens; chemo-refractory, optionally after 1 cycle of chemotherapy for relapsed leukemia; or is ineligible for allogeneic HSCT. In some of any of the embodiments, the B-ALL is relapsed and/or refractory. In some of any embodiments, the subject has a B-ALL comprising any of the following: first or greater marrow relapse, any marrow relapse after allogeneic hematopoietic stem cell transplantation (HSCT); primary refractory, optionally not achieving a complete response (CR) or complete response with incomplete blood count recovery (CRi), optionally following 2 or more separate induction regimens; chemo-refractory, optionally not achieving CR/Cri, optionally after 1 cycle of chemotherapy for relapsed leukemia; or is ineligible for allogeneic HSCT.

In some of any embodiments, the B-ALL is minimum residual disease positive (MRD+).. In some of any embodiments, subjects with B-ALL has less than 5% lymphoblast by morphology and/or minimum residual disease positive (MRD+) disease as detected by a validated assay at a frequency of 1 x10⁻⁴ or greater in bone marrow cells after two lines of therapy.

In some of any embodiments, the subject has Philadelphia chromosome positive ALL and is intolerant to or have failed one or more lines of tyrosine-kinase inhibitor (TKI) therapy, or TKI therapy is contraindicated.

In some of any of the provided T cell compositions for use, the B cell malignancy is a B-cell non-Hodgkin lymphoma (B-NHL). In some of any embodiments, the B cell malignancy is a B-cell non-Hodgkin lymphoma (B-NHL), optionally CD19+ B-NHL. In some of any embodiments, the B cell malignancy is relapsed or refractory (r/r) B-cell non-Hodgkin lymphoma (B-NHL).

In some of any embodiments, the B-NHL is diffuse large B cell lymphoma (DLBCL), primary mediastinal large B cell lymphoma (PMBCL), or Burkitt's lymphoma. In some of any of the embodiments, the subject has a B-NHL in which there is measurable disease after 1 or more lines of chemotherapy, has failed HSCT, or is ineligible for HSCT. In some of any of the embodiments, the B-NHL is relapsed and/or refractory.

In some of any of the provided T cell compositions for use, prior to the administration, the subject has been preconditioned with a lymphodepleting therapy comprising the administration of fludarabine and/or cyclophosphamide. In some of any of the provided T cell compositions for use in methods of treating a subject at or younger than 25 years of age having or suspected of having a B-cell malignancy, the method further comprises, immediately prior to the administration, administering a lymphodepleting therapy to the subject comprising the administration of fludarabine and/or cyclophosphamide. In some of any embodiments, the lymphodepleting therapy comprises administration of cyclophosphamide at about 200-400 mg/m², optionally at or about 300 mg/m², inclusive, and/or fludarabine at about 20-40 mg/m², optionally 30 mg/m², daily for 2-4 days, optionally for 3 days. In some of any embodiments, the lymphodepleting therapy comprises administration of cyclophosphamide at about 300 mg/m² daily for 3 days and fludarabine at about 30 mg/m² daily for 3 days. In some of any embodiments, the cyclophosphamide and fludarabine are administered concurrently. In some of any embodiments, the cyclophosphamide and fludarabine are administered intravenously. In some of any embodiments, the lymphodepleting therapy is administered 2-7 days prior to the administration of the T cell composition.

In any of the provided T cell compositions for use, the subject is a human.

In any of the provided T cell compositions for use, the CAR comprises an scFv specific for human CD19, a transmembrane domain, a cytoplasmic signaling domain derived from a costimulatory molecule, and a cytoplasmic signaling domain derived from a primary signaling ITAM-containing molecule. In some of any embodiments, the CAR comprises, in order, an scFv specific for human CD19, a transmembrane domain, a cytoplasmic signaling domain derived from a costimulatory molecule, and a cytoplasmic signaling domain derived from a primary signaling ITAM-containing molecule. In some of any such embodiments, the cytoplasmic domain derived from a costimulatory molecule is or comprises a human 4-1BB. In some of any such embodiments, the cytoplasmic signaling domain derived from a primary signaling ITAM-containing molecule is or comprises a human CD3zeta signaling domain. In some of any such embodiments, the CAR optionally further comprises a spacer between the transmembrane domain and the scFv.

In any of the provided embodiments, the CAR comprises, in order, an scFv specific for human CD19, a spacer, a transmembrane domain, a cytoplasmic signaling domain derived from a costimulatory molecule, and a cytoplasmic signaling domain derived from a primary signaling ITAM-containing molecule. In some of any such embodiments, the cytoplasmic domain derived from a costimulatory molecule is or comprises a human 4-1BB. In some of any such embodiments, the cytoplasmic signaling domain derived from a primary signaling ITAM-containing molecule is or comprises a human CD3zeta signaling domain.

In some of any of the provided embodiments, the spacer is a polypeptide spacer that comprises or consists of all or a portion of an immunoglobulin hinge or a modified version thereof, optionally an IgG4 hinge, or a modified version thereof. In some of any of the provided embodiments, the spacer is about 15 amino acids or less, and does not comprise a CD28 extracellular region or a CD8 extracellular region. In some of any of the provided embodiments, the spacer is at or about 12 amino acids in length. In some of any of the provided embodiments, the spacer has or consists of the sequence of SEQ ID NO: 1, a sequence encoded by SEQ ID NO: 2, SEQ ID NO: 30, SEQ ID NO: 31, SEQ ID NO: 32, SEQ ID NO: 33, SEQ ID O:N 34, or a variant of any of the foregoing having at least 85%, 86%, 87%, 88%, 89%, 90%, 91%, 92%, 93%, 94%, 95%, 96%, 97%, 98%, 99% or more sequence identity thereto. In some of any of the provided embodiments, the spacer comprises or consists of the formula X₁PPX₂P, where X₁ is glycine, cysteine or arginine and X₂ is cysteine or threonine.

In some of any of the provided embodiments, the cytoplasmic signaling domain derived from a costimulatory molecule, i.e., costimulatory domain, comprises SEQ ID NO: 12 or a variant thereof having at least 85%, 86%, 87%, 88%, 89%, 90%, 91%, 92%, 93%, 94%, 95%, 96%, 97%, 98%, 99% or more sequence identity thereto.

In some of any of the provided embodiments, the cytoplasmic signaling domain derived from a primary signaling ITAM-containing molecule, i.e., primary signaling domain, comprises SEQ ID NO: 13, 14 or 15 having at least 85%, 86%, 87%, 88%, 89%, 90%, 91%, 92%, 93%, 94%, 95%, 96%, 97%, 98%, 99% or more sequence identity thereto.

In some of any of the provided embodiments, the scFv comprises a variable light (V_{L}) chain comprising a CDRL1 sequence of RASQDISKYLN (SEQ ID NO: 35), a CDRL2 sequence of SRLHSGV (SEQ ID NO: 36), and a CDRL3 sequence of GNTLPYTFG (SEQ ID NO: 37), and a variable heavy (V_{H}) chain comprising a CDRH1 sequence of DYGVS (SEQ ID NO: 38), a CDRH2 sequence of VIWGSETTYYNSALKS (SEQ ID NO: 39), and a CDRH3 sequence of YAMDYWG (SEQ ID NO: 40). In some of any of the provided embodiments, the scFv comprises a V_{L} comprising a CDRL1 sequence of FMC63, a CDRL2 sequence of FMC63, a CDRL3 sequence of FMC63, and a VH comprising a CDRH1 sequence of FMC63, a CDRH2 sequence of FMC63, and a CDRH3 sequence of FMC63. In some of any of the provided embodiments, the scFv comprises a V_{H} set forth in SEQ ID NO:41 and a V_{L} set forth in SEQ ID NO: 42. In some of any of the provided embodiments, the V_{H} and V_{L} are separated by a flexible linker. In some of any of the provided embodiments, the flexible linker is or comprises the sequence set forth in SEQ ID NO:24. In some of any of the provided embodiments, the scFv is or comprises the sequence set forth in SEQ ID NO:43.

In some of any of the provided embodiments, the T cells are primary T cells obtained from a subject. In some of any of the provide embodiments, the T cells are autologous to the subject.

In some of any of the provided embodiments, prior to administering the composition, the subject is or has been identified as having cells expressing CD19. In some of any of the provided embodiments, the expression of CD19 is detected by flow cytometry in the peripheral blood or bone marrow, and/or by immunohistochemistry of a bone marrow biopsy. In some of any embodiments, the expression of CD19 is detected by flow cytometry, optionally in the peripheral blood or bone marrow, and/or by immunohistochemistry, optionally of a bone marrow biopsy.

In some of any embodiments, the subject has not received prior cell therapy that comprises administration of a T cell composition comprising T cells expressing a CAR. In some of any embodiments, the subject has received a prior cell therapy that comprises administration of a T cell composition comprising T cells expressing a CAR. In some of any embodiments, the subject has received a prior therapy targeting CD19, optionally wherein the subject is or has been identified as having cells expressing CD19 or having a CD19-positive disease after completion of the prior therapy targeting CD19.

In some of any embodiments, at least at or about 35%, 40%, 50%, 60%, 70%, 75%, 80%, 85%, 90%, 91%, 92%, 93%, 94%, 95%, 96%, 97%, 98%, 99% or more of the CAR-expressing T cells, CAR-expressing CD4+ T cells or CAR-expressing CD8+ T cells in the composition, are surface positive for CCR7, CD27, CD45RA and/or CD28.In some of any embodiments, at least at or about 70%, 75%, 80%, 85%, 90%, 91%, 92%, 93%, 94%, 95%, 96%, 97%, 98%, 99% or more of the CAR-expressing T, CAR-expressing CD4+ T cells or CAR-expressing CD8+ T cells in the composition, are surface positive for CCR7. In some of any embodiments, at least at or about 35%, 40%, 50%, 60%, 70%, 75%, 80%, 85%, 90%, 91%, 92%, 93%, 94%, 95%, 96%, 97%, 98%, 99% o or more of the CAR-expressing T cells, CAR-expressing CD4+ T cells or CAR-expressing CD8+ T cells in the composition, are surface positive for CCR7 and CD45RA.

In some of any embodiments, at least at or about 70%, 75%, 80%, 85%, 90%, 91%, 92%, 93%, 94%, 95%, 96%, 97%, 98%, 99% or more of the CAR-expressing T, CAR-expressing CD4+ T cells or CAR-expressing CD8+ T cells in the composition, are surface positive for CD27. In some of any embodiments, at least at or about 60%, 70%, 75%, 80%, 85%, 90%, 91%, 92%, 93%, 94%, 95%, 96%, 97%, 98%, 99% o or more of the CAR-expressing T cells, CAR-expressing CD4+ T cells or CAR-expressing CD8+ T cells in the composition, are surface positive for CD27 and CD28. In some of any embodiments, at least at or about 75%, 80%, 85%, 90%, 91%, 92%, 93%, 94%, 95%, 96%, 97%, 98%, 99% or more of the CAR-expressing T cells, CAR-expressing CD4+ T cells or CAR-expressing CD8+ T cells in the composition, are negative for active Caspase 3.

### Detailed Description

Embodiments of the invention fall within some of the instances disclosed below. Any references herein to methods of treatment are intended to refer to products for use in said methods.

Disclosed herein are methods and uses, such as therapeutic methods or uses, that involve administration of engineered cells (e.g., T cells) and/or compositions thereof, for the treatment of subjects having a disease or condition, which generally is or includes a B cell malignancy or a hematological malignancy. In some disclosures, the B cell malignancy is a B-cell acute lymphoblastic leukemia (B-ALL) or a B-cell non-Hodgkin lymphoma (B-NHL). In some disclosures, the engineered cells express a recombinant receptor, such as a chimeric antigen receptor (CAR), that can target, bind and/or recognize an antigen that is associated with the B cell malignancy. In some disclosures, the subjects include subjects who are at or younger than 25 years of age, such as pediatric subjects or young adult subjects. In some disclosures, the methods involve determining the amount, number or dose of engineered cells for administration for treatment in a particular group of subjects, such as subjects who are at or younger than 25 years of age, such as pediatric subjects or young adult subjects. In some disclosures, the subjects are pediatric subjects. In some disclosures, the subjects are young adults. Also disclosed herein are compositions for use in cell therapy, for example, in accordance with the methods and uses described herein. Also disclosed herein are articles of manufacture and kits, e.g., for use in the methods or uses disclosed herein. In some disclosures, the articles of manufacture and kits optionally contain instructions for using, according to the methods or uses disclosed herein.

Adoptive cell therapies (including those involving the administration of cells expressing chimeric receptors such as chimeric antigen receptors (CARs) and/or other recombinant antigen receptors, specific for a disease or disorder of interest, as well as other adoptive immune cell and adoptive T cell therapies) can be effective in the treatment of hematological malignancies or B cell malignancies, and other diseases and disorders, such as other cancers. In certain contexts, available approaches to adoptive cell therapy may not always be entirely satisfactory. In some contexts, optimal response to therapy can depend on the ability of the administered cells to recognize and bind to a target, e.g., target antigen, to traffic, localize to and successfully enter appropriate sites within the subject, tumors, and environments thereof, to become activated, expand, to exert various effector functions, including cytotoxic killing and secretion of various factors such as cytokines, to persist, including long-term, to differentiate, transition or engage in reprogramming into certain phenotypic states, to provide effective and robust recall responses following clearance and re-exposure to target ligand or antigen, and avoid or reduce exhaustion, anergy, terminal differentiation and/or differentiation into a suppressive state.

In some disclosures, the therapeutic effect of adoptive cell therapy may be limited by risk and/or the development of toxicity in the subject to whom such cells are administered, which toxicity in some cases can be severe, at certain doses or exposure of administered cells. In some cases, while a higher dose of such cells can increase the therapeutic effect, for example, by increasing exposure to the cells such as by promoting expansion and/or persistence, they may also result in an even greater risk of developing a toxicity or a more severe toxicity. Also, in some cases, subjects with a higher disease burden also may be at a greater risk for developing a toxicity or a more severe toxicity. Further, among factors that may increase the risk, relative risk and/or probability of developing a toxicity following administration of a dose of cells of a cell therapy (e.g. CAR-T cell therapy), include the number of cells administered to a subject due to weight-based dosing of cells, e.g. more cells administered to subjects with a greater weight. Certain available methods for dosing subjects cell therapy may not always be entirely satisfactory. Increasing a dose of cells or promoting expansion or proliferation of administered cells in the subject can be related to higher response rates, but also an increase in development of toxicity.

In some disclosures, particular group of subjects, such as pediatric subjects or young adult subjects, can exhibit differences in response to the adoptive cell therapy and/or development of toxicity after administration, due to the differences in body size, volume of circulation and/or activity or function of the administered cells. In some disclosures, determining appropriate doses for particular group of subjects, where the dose results in a high or specified desired degree of likelihood of a treatment outcome such as a favorable outcome or response and/or a durable response or outcome, and also a relatively low or minimized or desired degree of likelihood of risk of developing a toxic outcome or toxicity following administration to the subject of the cell therapy, can be difficult. Disclosed herein are methods and uses that can achieve such results.

Thus, in some disclosures, the methods, uses, articles of manufacture and/or compositions, can offer advantages over other available methods or solutions or approaches for treatment such as for adoptive cell therapy. In particular, among the disclosures are those that include methods of administering a T cell therapy containing T cells (CD4 and/or CD8 T cells) expressing a CAR, such as an anti-CD 19 CAR, to subjects at or younger than 25 years of age, such as pediatric subjects or young adult subjects, with a B cell malignancy, such as a B-ALL or a B-NHL. The disclosed methods and uses permit dosing of cells that can achieve or can be associated with an increased likelihood of response, and a decreased likelihood of risk for developing a toxicity.

The section headings used herein are for organizational purposes only and are not to be construed as limiting the subject matter described.

### I. METHODS AND USES IN CELL THERAPY WITH GENETICALLY ENGINEERED T CELLS

Disclosed herein are methods and uses, such as therapeutic methods and uses, involving administration of engineered cells (e.g., T cells) and/or compositions thereof, for the treatment of subjects having a disease or condition, which generally is or includes a B cell malignancy or a hematologic malignancy. In some disclosures, the B cell malignancy is a B-cell acute lymphoblastic leukemia (B-ALL) or a B-cell non-Hodgkin lymphoma (B-NHL). In some disclosures, the methods and uses are for treatment of a particular group of subjects, such as subjects who are at or younger than 25 years of age and/or pediatric subjects or young adult subjects.

In some disclosures, the methods and uses include administering to the subject cells expressing genetically engineered (recombinant) cell surface receptors in adoptive cell therapy, which generally include chimeric receptors such as chimeric antigen receptors (CARs), binding or recognizing an antigen expressed by, associated with and/or specific to the B cell malignancy, such as B-ALL or B-NHL, and/or cell type from which it is derived. The cells are generally administered in a composition formulated for administration. In some disclosures, engineered cells or compositions comprising the engineered cells are administered to a subject having the B cell malignancy, e.g., via adoptive cell therapy, such as adoptive T cell therapy. In some disclosures, the methods involve treating a subject having a B cell malignancy with a dose of antigen receptor-expressing cells (e.g. CAR-expressing cells).

In some disclosures, the methods involve administering a particular amount, such as one or more doses of the cells to the subject, which amount or dose(s) may include a particular number or relative number of cells or of the engineered cells, and/or a defined ratio or compositions of two or more sub-types within the composition, such as CD4 vs.CD8 T cells, or a particular number or relative number of cells per body weight of the subject (e.g., per kilogram (kg) body weight). In some disclosures, if the subject exceeds a certain maximum body weight, a particular number or relative number of cells are administered. In some disclosures, the methods involve administering an amount or dose to a subject depending on the age and/or body weight of the subject.

In some disclosures, the methods and uses involve treating a specific group or subset of subjects, e.g., subjects that are of a particular age, such as subjects who are at or younger than 25 years of age. In some disclosures, the methods and uses involve treating subjects that are of a particular age, such as subjects who are younger than 18 years of age. In some disclosures, the methods and uses involve treating pediatric subjects or young adult subjects.

In some disclosures, the methods, uses and articles of manufacture involve, or are used for treatment of subjects involving, selecting or identifying a particular group or subset of subjects, e.g., based on age, specific types of disease, diagnostic criteria, prior treatments and/or response to prior treatments. In some disclosures, the methods involve treating a subject having relapsed following remission after treatment with, or become refractory to, one or more prior therapies; or a subject that has relapsed or is refractory (R/R) to one or more prior therapies, e.g., one or more lines of standard therapy. In some disclosures, the methods and uses involve treating a specific group or subset of subjects, such as pediatric subjects and/or young adult subjects identified as having a B cell malignancy that has R/R to standard therapy. In some disclosures, the subjects can have a high-risk disease, such as a B cell malignancy that is aggressive and/or has a poor prognosis or that has R/R to standard therapy. In some disclosures, the methods involve treating subjects having a R/R B-ALL, a R/R B-NHL, or a B-ALL that exhibits minimum residual disease (MRD+ B-ALL). In some disclosures, the NHL can include a diffuse large B-cell lymphoma (DLBCL), a Burkitt's lymphoma (BL) or a primary mediastinal B-cell lymphoma (PMBCL). In some disclosures, the subject has a R/R DLBCL, a R/R BL or a R/R PMBCL.

In some disclosures, the antigen receptor (e.g. CAR) specifically binds to a target antigen associated with the disease, disorder or condition, such as those associated with a B cell malignancy, such as B-ALL or B-NHL. In some disclosures, the antigen associated with the disease or disorder is CD19, CD20, CD22, ROR1, CD45, CD21, CD5, CD33, Igkappa, Iglambda, CD79a, CD79b or CD30. In some disclosures, the antigen associated with the disease or disorder is selected from CD19.

In some instances disclosed herein are compositions, methods and uses for administration of a defined composition of the cell therapy, at particular doses, that are associated with a high response rate and/or high durability of response, and low levels and/or incidence of toxicity. In some disclosures, the composition or dose administered, for some subjects, in a body weight-based dose, such as a particular amount or number of cells per kilogram (kg) body weight of the subject. In some disclosures, the composition or dose administered, for some subjects, in a flat and/or fixed dose, such as a precise flat dose, of cells and/or of one or more cells having a particular phenotype, such as a particular number of such cells or a number that is within a particular range and/or degree of variability or variance as compared to a target number.

In some instances disclosed herein are methods of treating a subject having or suspected of having a B cell malignancy, such as a B-ALL or a B-NHL, including a malignancy thereof that is relapsed and/or refractory, the method comprising administering, to a subject at or younger than 25 years of age, or in some cases younger than 18 years of age, a T cell composition comprising T cells expressing an anti-CD19 chimeric antigen receptor (CAR). Such methods and uses include therapeutic methods and uses, for example, involving administration of the anti-CD19 CAR-expressing T cell compositions in an effective amount to effect treatment of the disease or disorder. Uses include uses of such anti-CD19 CAR-expressing T cells or compositions containing such cells, in such methods and treatments, and in the preparation of a medicament in order to carry out such therapeutic methods. Also disclosed herein are compositions, such as pharmaceutical compositions, for example containing such anti-CD19 CAR-expressing T cells, in accordance with the methods disclosed herein. In some disclosures, the methods and uses thereby treat the disease or condition or disorder in the subject.

**In** some disclosures, the methods and uses involve administering, to a subject at or younger than 25 years of age, a T cell composition comprising T cells expressing an anti-CD19 chimeric antigen receptor (CAR), wherein the composition is administered in an amount selected from: (i) if the subject is less than 100 kilograms (kg) in body weight and is younger than 18 years of age, from at or about 0.05 x 10⁶ CAR+ T cells/kg body weight of the subject to at or about 1.5 x 10⁶ CAR+ T cells/kg body weight of the subject; and (ii) if the subject is at or greater than 100 kilograms (kg) in body weight or is between 18 and 25 years of age, inclusive, from at or about 0.05 x 10⁸ CAR+ T cells to at or about 1.5 x 10⁸ CAR+ T cells. In some of any disclosures, if the subject is between 18 and 25 years of age, inclusive, the T cell composition is administered in an amount from at or about 0.05 x 10⁸ CAR+ T cells to at or about 1.5 x 10⁸ CAR+ T cells.

**In** some instances, disclosed herein are methods of treating a subject having or suspected of having a B cell malignancy, the method comprising administering, to a subject at or younger than 25 years of age, a T cell composition comprising T cells expressing an anti-CD19 chimeric antigen receptor (CAR), wherein the composition is administered in an amount selected from: (i) if the subject is less than 100 kilograms (kg) in body weight and is younger than 18 years of age, from at or about 0.5 x 10⁶ CAR+ T cells/kg body weight of the subject to at or about 1.5 x 10⁶ CAR+ T cells/kg body weight of the subject; and (ii) if the subject is at or greater than 100 kilograms (kg) in body weight or is between 18 and 25 years of age, inclusive, from at or about 0.5 x 10⁸ CAR+ T cells to at or about 1.5 x 10⁸ CAR+ T cells. In some disclosures, if the subject is between 18 and 25 years of age, inclusive, the T cell composition is administered in an amount from at or about 0.5 x 10⁸ CAR+ T cells to at or about 1.5 x 10⁸ CAR+ T cells.

**In** some disclosures, the methods and uses involve, to a subject that is younger than 18 years of age, a T cell composition comprising T cells expressing an anti-CD19 chimeric antigen receptor (CAR), wherein the T cell composition is administered in an amount selected from: (i) if the subject is less than 100 kilograms (kg) in body weight, from at or about 0.05 x 10⁶ CAR+ T cells/kg body weight of the subject to at or about 1.5 x 10⁶ CAR+ T cells/kg body weight of the subject; and (ii) if the subject is at or greater than 100 kilograms (kg) in body weight, from at or about 0.05 x 10⁸ CAR+ T cells to at or about 1.5 x 10⁸ CAR+ T cells.

**In** some instances, disclosed herein are methods of treating a subject having or suspected of having a B cell malignancy, the method comprising administering, to a subject that is younger than 18 years of age, a T cell composition comprising T cells expressing an anti-CD19 chimeric antigen receptor (CAR), wherein the T cell composition is administered in an amount selected from: (i) if the subject is less than 100 kilograms (kg) in body weight, from at or about 0.5 x 10⁶ CAR+ T cells/kg body weight of the subject to at or about 1.5 x 10⁶ CAR+ T cells/kg body weight of the subject; and (ii) if the subject is at or greater than 100 kilograms (kg) in body weight, from at or about 0.5 x 10⁸ CAR+ T cells to at or about 1.5 x 10⁸ CAR+ T cells. In some disclosures, if the subject is less than 100 kilograms (kg) in body weight the T cell composition is administered in an amount from at or about 0.5 x 10⁶ CAR+ T cells/kg body weight of the subject to at or about 1 x 10⁶ CAR+ T cells/kg body weight of the subject. In some disclosures, if the subject is at or greater than 100 kilograms (kg) in body weight the T cell composition is administered in an amount from at or about 0.5 x 10⁸ CAR+ T cells to at or about 1.5 x 10⁸ CAR+ T cells.

In some of any disclosures, if the subject is less than 100 kilograms (kg) in body weight the T cell composition is administered in an amount from at or about 0.05 x 10⁶ CAR+ T cells/kg body weight of the subject to at or about 1 x 10⁶ CAR+ T cells/kg body weight of the subject.

In some instances, disclosed herein are methods of treating a subjecting having or suspected of having a B cell malignancy, the method comprising administering, to a subject at or younger than 25 years of age, a T cell composition comprising T cells expressing an anti-CD19 chimeric antigen receptor (CAR), wherein the composition is administered in an amount selected from: (i) if the subject is younger than 18 years of age from at or about 0.5 x 10⁶ CAR+ T cells/kg body weight of the subject to at or about 1.5 x 10⁶ CAR+ T cells/kg body weight of the subject, but that does not exceed at or about 1.5 x 10⁸ total CAR+ T cells; or (ii) if the subject is between 18 and 25 years of age, inclusive, from at or about 0.5 x 10⁸ CAR+ T cells to at or about 1.5 x 10⁸ CAR+ T cells.

In some disclosures, if the subject is younger than 18 years of age the T cell composition is administered in an amount from at or about 0.5 x 10⁶ CAR+ T cells/kg body weight of the subject to at or about 1.0 x 10⁶ CAR+ T cells/kg body weight of the subject, but that does not exceed at or about 1.0 x 10⁸ total CAR+ T cells. In some disclosures, if the subject is younger than 18 years of age, the T cell composition is administered in an amount that is at least at or about 0.5 x 10⁶ CAR+ T cells/kg body weight of the subject but that does not exceed at or about 0.5 x 10⁸ total CAR+ T cells. In some disclosures, if the subject is younger than 18 years of age, the T cell composition is administered in an amount that is at least at or about 1.0 x 10⁶ CAR+ T cells/kg body weight of the subject but that does not exceed at or about 1.0 x 10⁸ total CAR+ T cells. In some disclosures, if the subject is between 18 and 25 years of age, inclusive, the T cell composition is administered in an amount from at or about 0.5 x 10⁸ CAR+ T cells to at or about 1.0 x 10⁸ CAR+ T cells. In some disclosures, if the subject is between 18 and 25 years of age, inclusive, the T cell composition is administered in an amount that is at or about 0.5 x 10⁸ CAR+ T cells. In some disclosures, if the subject is between 18 and 25 years of age, inclusive, the T cell composition is administered in an amount that is at or about 1.0 x 10⁸ CAR+ T cells.

In some of the disclosed methods or uses, the subject is at least at or about 6 kg in body weight. In some of the disclosed methods or uses, the subject is at least at or about 12 kg in body weight. In some of the disclosed methods or uses, the subject less than at or about 100 kg in body weight.

In some instances, disclosed herein are methods of treating a subject having or suspected of having a B cell malignancy, the method comprising administering, to a subject at or younger than 25 years of age and weighing 12 kg or more, a T cell composition comprising T cells expressing an anti-CD19 chimeric antigen receptor (CAR), wherein the T cell composition is administered in an amount selected from: (i) if the subject is younger than 18 years of age, at or about 0.5 x 10⁶ CAR+ T cells/kg body weight of the subject, but not exceeding at or about 0.5 x 10⁸ total CAR+ T cells; and (ii) if the subject is between 18 and 25 years of age, inclusive, at or about 0.5 x 10⁸ CAR+ T cells.

In some instances, disclosed herein are methods of treating a subject having or suspected of having a B cell malignancy, the method comprising administering, to a subject at or younger than 25 years of age and weighing 6 kg or more, a T cell composition comprising T cells expressing an anti-CD19 chimeric antigen receptor (CAR), wherein the T cell composition is administered in an amount selected from (i) if the subject is younger than 18 years of age at or about 1 x 10⁶ CAR+ T cells/kg body weight of the subject, but not exceeding at or about 1 x 10⁸ total CAR+ T cells; and (ii) if the subject is between 18 and 25 years of age, inclusive, at or about 1.0 x 10⁸ CAR+ T cells.

In some instances, disclosed herein are methods of treating a subject having or suspected of having a B cell malignancy, the method comprising administering, to a subject younger than 18 years of age and weighing 12 kg or more, a composition comprising T cells expressing an anti-CD19 chimeric antigen receptor (CAR), wherein the composition is administered in an amount that is at or about 0.5 x 10⁶ CAR+ T cells/kg body weight of the subject to at or about 1.5 x 10⁶ CAR+ T cells/kg body weight of the subject, but that does not exceed at or about 1.5 x 10⁸ total CAR+ T cells. In some disclosures, the composition is administered in an amount from at or about 0.5 x 10⁶ CAR+ T cells/kg body weight of the subject to at or about 1.0 x 10⁶ CAR+ T cells/kg body weight of the subject, but that does not exceed at or about 1.0 x 10⁸ total CAR+ T cells. In some disclosures, the composition is administered in an amount that is at least at or about 0.5 x 10⁶ CAR+ T cells/kg body weight of the subject but that does not exceed at or about 0.5 x 10⁸ total CAR+ T cells.

In some disclosures, the methods involve administering, to a subject at or younger than 25 years of age, a T cell composition comprising T cells expressing an anti-CD19 chimeric antigen receptor (CAR), wherein the composition is administered in an amount selected from: (i) if the subject is younger than 18 years of age from at or about 0.05 x 10⁶ CAR+ T cells/kg body weight of the subject to at or about 1.5 x 10⁶ CAR+ T cells/kg body weight of the subject, but that does not exceed at or about 1.5 x 10⁸ total CAR+ T cells; or (ii) if the subject is between 18 and 25 years of age, inclusive, from at or about 0.05 x 10⁸ CAR+ T cells to at or about 1.5 x 10⁸ CAR+ T cells.

In some of the disclosed methods or uses, a total volume of at least 0.05 mL at a concentration of at or greater than 2.5 x 10⁶ cells/mL of the T cell composition is administered. In some of the disclosed methods or uses, a total volume of at least 0.5 mL at a concentration of at or greater than 2.5 x 10⁶ cells/mL of the T cell composition is administered.

**In** some of any disclosures, if the subject is younger than 18 years of age, the T cell composition is administered in an amount that is at or about 0.05 x 10⁶ CAR+ T cells/kg body weight of the subject to at or about 1.5 x 10⁶ CAR+ T cells/kg body weight of the subject, but that does not exceed at or about 1.5 x 10⁸ total CAR+ T cells.

**In** some of any disclosures, if the subject is younger than 18 years of age, the T cell composition is administered in an amount that is at least at or about 0.05 x 10⁶ CAR+ T cells/kg body weight of the subject but that does not exceed at or about 0.05 x 10⁸ total CAR+ T cells. In some of any disclosures, if the subject is younger than 18 years of age, the T cell composition is administered in an amount that is at least at or about 0.15 x 10⁶ CAR+ T cells/kg body weight of the subject but that does not exceed at or about 0.15 x 10⁸ total CAR+ T cells. In some of any disclosures, if the subject is younger than 18 years of age, the T cell composition is administered in an amount that is at least at or about 0.3 x 10⁶ CAR+ T cells/kg body weight of the subject but that does not exceed at or about 0.3 x 10⁸ total CAR+ T cells. In some of any disclosures, if the subject is younger than 18 years of age, the T cell composition is administered in an amount that is at least at or about 0.5 x 10⁶ CAR+ T cells/kg body weight of the subject but that does not exceed at or about 0.5 x 10⁸ total CAR+ T cells. In some of any disclosures, if the subject is younger than 18 years of age, the T cell composition is administered in an amount that is at least at or about 0.75 x 10⁶ CAR+ T cells/kg body weight of the subject but that does not exceed at or about 0.75 x 10⁸ total CAR+ T cells. In some of any disclosures, if the subject is younger than 18 years of age, the T cell composition is administered in an amount that is at least at or about 1.0 x 10⁶ CAR+ T cells/kg body weight of the subject but that does not exceed at or about 1.0 x 10⁸ total CAR+ T cells.

**In** some of any disclosures, if the subject is between 18 and 25 years of age, inclusive, the T cell composition is administered in an amount from at or about 0.05 x 10⁸ CAR+ T cells to at or about 1.0 x 10⁸ CAR+ T cells. In some of any disclosures, if the subject is between 18 and 25 years of age, inclusive, the T cell composition is administered in an amount that is at or about 0.05 x 10⁸ CAR+ T cells. In some of any disclosures, if the subject is between 18 and 25 years of age, inclusive, the T cell composition is administered in an amount that is at or about 0.15 x 10⁸ CAR+ T cells. In some of any disclosures, if the subject is between 18 and 25 years of age, inclusive, the T cell composition is administered in an amount that is at or about 0.3 x 10⁸ CAR+ T cells. In some of any disclosures, if the subject is between 18 and 25 years of age, inclusive, the T cell composition is administered in an amount that is at or about 0.5 x 10⁸ CAR+ T cells. In some of any disclosures, if the subject is between 18 and 25 years of age, inclusive, the T cell composition is administered in an amount that is at or about 0.75 x 10⁸ CAR+ T cells. In some of any disclosures, if the subject is between 18 and 25 years of age, inclusive, the T cell composition is administered in an amount that is at or about 1.0 x 10⁸ CAR+ T cells.

**In** some instances, disclosed herein are methods of treating a subject having or suspected of having a B cell malignancy, the method comprising administering, to a subject at or younger than 25 years of age, a T cell composition comprising T cells expressing an anti-CD19 chimeric antigen receptor (CAR) at a concentration of at or greater than 2.5 x 10⁶ cells/mL, wherein the T cell composition is administered in an amount selected from (i) if the subject is younger than 18 years of age, an amount not exceeding at or about 0.5 x 10⁸ total CAR+ T cells in a volume of at least 0.5 mL; and (ii) if the subject is between 18 and 25 years of age, inclusive, at or about 0.5 x 10⁸ CAR+ T cells.

In some instances, disclosed herein are methods of treating a subject having or suspected of having a B cell malignancy, the method comprising administering, to a subject at or younger than 25 years of age, a T cell composition comprising T cells expressing an anti-CD19 chimeric antigen receptor (CAR) at a concentration of at or greater than 2.5 x 10⁶ cells/mL, wherein the T cell composition is administered in an amount selected from: (i) if the subject is younger than 18 years of age, an amount not exceeding at or about 1 x 10⁸ total CAR+ T cells in a volume of at least 0.5 mL; and (ii) if the subject is between 18 and 25 years of age, inclusive, at or about 1.0 x 10⁸ CAR+ T cells.

In some of any disclosures, the total volume of the T cell composition administered is at least 0.05 mL. In some of any disclosures, the total volume of the T cell composition administered is at least 0.1 mL. In some of the disclosures, the total volume of the T cell composition administered is at least 1.0 mL. In some of the disclosures, the concentration of the T cell composition is greater than at or about 5 x 10⁶ cells/mL or is or is about 5 x 10⁶ cells/mL In some of the disclosures, the concentration of the T cell composition is greater than at or about 10 x 10⁶ cells/mL or is or is about 10 x 10⁶ cells/mL. In some of the disclosures, the concentration of the T cell composition is greater than or greater than about 15 x 10⁶ cells/mL or is or is about 15 x 10⁶ cells/mL.

In some disclosures, the composition or dose administered contains a defined ratio of CD4⁺ and CD8⁺ cells (e.g., 1:1 ratio of CD4⁺:CD8⁺ CAR⁻ T cells) and/or contains a ratio that is within a certain degree of variability from such ratio, such as no more than + 10%, such as no more than + 8%, such as a degree of variability or variance of no more than + 10%, such as no more than + 8%. In some disclosures, the CD4⁺ and CD8⁺ cells are individually formulated and administered. In some disclosures, the administered cells exhibit consistent activity and/or function, e.g., cytokine production, apoptosis and/or expansion. In some disclosures, the compositions exhibit highly consistent and defined activity, and low variability between cells, e.g., in terms of cell number, cell function and/or cell activity, in the composition or between preparations. In some disclosures, the consistency in activity and/or function, e.g., low variability between preparations of compositions, allows improved efficacy and/or safety. In some disclosures, administration of the defined compositions resulted in low product variability and low toxicity, e.g., CRS or neurotoxicity, compared to administration of cell compositions with high heterogeneity. In some disclosures, the defined, consistent composition also exhibits consistent cell expansion. Such consistency can facilitate the identification of dose, therapeutic window, evaluation of dose response and identification of factors of the subject that may correlate with safety or toxicity outcomes.

In some disclosures, in a certain cohort of subjects receiving a single infusion of a particular dose level, a durable response rate after 6 months of greater than 60% can be achieved. In some disclosures, the subjects in some cohorts can achieve an overall response rate (ORR, in some cases also known as objective response rate) of more than 80%, a complete response (CR) rate of more than 60% and/or a high durable CR rate at 6 months. In some disclosures, subjects receiving a defined dose show improved safety outcomes, e.g., more than two-thirds of the subjects that do not exhibit any CRS or NT. In some disclosures, the rate of severe CRS or severe NT is low. In some disclosures, a higher exposure (e.g., Cₘₐₓ and AUC₀₋₂₈) observed with a particular defined dose, does not associate with increased toxicity, e.g., CRS or NT. In some disclosures, particular factors of the subject, e.g., certain biomarkers, can be used to predict the risk of toxicity. In some instances, the disclosures can be used to achieve high response rate with low risk of toxicity.

In some disclosures, no more than 25%, no more than 20%, no more than 15%, no more than 10% or no more than 5% of subjects treated using the disclosed compositions, articles of manufacture, kits, methods and uses are administered an agent (e.g. tocilizumab and/or dexamethasone) to ameliorate, treat or prevent a toxicity, either prior to or subsequent to administration of the cell therapy. In some disclosures, the subject is not administered any prophylaxis treatment prior to receiving the engineered cells (e.g. CAR-T cells).

In some disclosures, the methods, cells and compositions can provide high rate of durable response to subjects across a range of patient characteristics and/or tumor burden. In some disclosures, the methods, cells and compositions can provide high rate of durable response to high risk patients with poor prognosis, with a reduced risk of adverse effects or toxicities. In some disclosures, the methods and uses provide for or achieve a higher response rate and/or more durable responses or efficacy and/or a reduced risk of toxicity or other side effects that can be associated with cell therapy, such as neurotoxicity (NT) or cytokine release syndrome (CRS). In some disclosures, the provided observations indicated a low rate of severe NT (sNT) or severe CRS (sCRS), and a high rate of patients without any toxicities, e.g., NT or CRS.

### A. Method of Treatment

The methods disclosed herein are methods of treatment that involve administering engineered cells or compositions containing engineered cells, such as engineered T cells. Also disclosed are methods and uses, such as therapeutic and prophylactic uses, of engineered cells (e.g., T cells) and/or compositions thereof, including methods or uses for the treatment of subjects having a disease, disorder or condition, such as a B cell malignancy or hematological malignancy, that involves administration of the engineered cells and/or compositions thereof. Such methods and uses include therapeutic methods and uses, for example, involving administration of engineered cells, or compositions containing the same, to a subject having a B cell malignancy or hematological malignancy, such as B-cell acute lymphoblastic leukemia (B-ALL) or a B-cell non-Hodgkin lymphoma (B-NHL). In some disclosures, the molecule, cell, and/or composition is/are administered in an effective amount to effect treatment of the disease, disorder or condition.

In some disclosures, the methods involve administering engineered cells or compositions containing engineered cells, such as engineered T cells, to a subject, such as a subject that has a hematological malignancy or a B cell malignancy. In some instances, also disclosed herein are uses of engineered cells or compositions containing engineered cells, such as engineered T cells for treatment of a B cell malignancy or hematological malignancy, in some disclosures, in accord with any of the methods described herein. In some instances, also disclosed herein are uses of engineered cells or compositions containing engineered cells, such as engineered T cells for the manufacture of a medicament for the treatment of a B cell malignancy, for example, in order to carry out such therapeutic methods. In some instances, also disclosed herein are methods of administering engineered cells or compositions containing engineered cells, such as engineered T cells, for use in treatment of a B cell malignancy, or for administration to a subject having a B cell malignancy. In some disclosures, the methods are carried out by administering the engineered cells or compositions comprising the same, to the subject having, having had, or suspected of having a B cell malignancy. In some disclosures, the methods thereby treat the B cell malignancy in the subject. Also disclosed herein are of use of any of the compositions, such as pharmaceutical compositions disclosed herein, for the treatment of a B cell malignancy, such as use in a treatment regimen.

In some disclosures, the methods and uses can achieve improved response and/or more durable responses or efficacy and/or a reduced risk of toxicity or other side effects, e.g., in particular groups of subjects treated, as compared to certain alternative methods.

General methods for administration of cells for adoptive cell therapy are known and may be used in connection with the disclosed methods and compositions. For example, adoptive T cell therapy methods are described, e.g., in US Patent App. Pub. No. 2003/0170238; US Patent No. 4,690,915; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

In some disclosures, the methods and uses involve treatment of subjects involving, selecting or identifying a particular group or subset of subjects, e.g., based on age, specific types of disease, diagnostic criteria, prior treatments and/or response to prior treatments.

In some disclosures, the B cell malignancy or hematological malignancy to be treated according to the methods and uses disclosed herein can be any in which expression of an antigen that is associated with and/or involved in the etiology of the B cell malignancy or hematological malignancy, e.g. causes, exacerbates or otherwise is involved in the B cell malignancy or hematological malignancy. In some disclosures, the B cell malignancy is associated with transformation of cells (e.g. cancer). In particular disclosures, the recombinant receptor, e.g., CAR, specifically binds to an antigen associated with the B cell malignancy or hematological malignancy.

In some disclosures, the B cell malignancy or hematological malignancy is associated with a tumor, a cancer, a neoplasm, or other proliferative disease or disorder. In some disclosures, such diseases include but are not limited to leukemia, lymphoma, e.g., acute myeloid (or myelogenous) leukemia (AML), chronic myeloid (or myelogenous) leukemia (CML), acute lymphocytic (or lymphoblastic) leukemia (ALL), chronic lymphocytic leukemia (CLL), hairy cell leukemia (HCL), small lymphocytic lymphoma (SLL), Mantle cell lymphoma (MCL), Marginal zone lymphoma, Burkitt's lymphoma, Hodgkin lymphoma (HL), non-Hodgkin lymphoma (NHL), Anaplastic large cell lymphoma (ALCL), follicular lymphoma, refractory follicular lymphoma, diffuse large B-cell lymphoma (DLBCL) and multiple myeloma (MM). In some disclosures, disease or condition is a B cell malignancy selected from among acute lymphoblastic leukemia (ALL), adult ALL, chronic lymphoblastic leukemia (CLL), non-Hodgkin lymphoma (NHL), and Diffuse Large B-Cell Lymphoma (DLBCL). In some disclosures, the disease or condition is NHL and the NHL is selected from the group consisting of aggressive NHL, diffuse large B cell lymphoma (DLBCL), NOS (de novo or transformed from indolent), primary mediastinal large B cell lymphoma (PMBCL), T cell/histocyte-rich large B cell lymphoma (TCHRBCL), Burkitt's lymphoma (BL), mantle cell lymphoma (MCL), follicular lymphoma (FL), and/or follicular lymphoma Grade 3B (FL3B). Among the B cell malignancy are B-cell acute lymphoblastic leukemia (B-ALL) and a B-cell non-Hodgkin lymphoma (B-NHL). In some disclosures, the NHL can include diffuse large B-cell lymphoma (DLBCL), Burkitt's lymphoma (BL) or primary mediastinal B-cell lymphoma (PMBCL).

In some disclosures, the B cell malignancy is an acute lymphoblastic leukemia (ALL), such as a B-cell acute lymphoblastic leukemia (B-ALL). In some disclosures, ALL, an aggressive cancer of the blood and the bone marrow (BM), is classified by the World Health Organization (WHO) as a precursor lymphoid neoplasm of primarily the B-cell type, with only about 10% to 15% of cases involving T cells. In some disclosures, precursor B-cell ALL (B-ALL), in some cases expressing CD79a, CD19, human leukocyte antigen - antigen D related (HLA-DR), and other B-cell antigens, accounts for 80% to 85% of childhood ALL and about 30% of all childhood cancers. ALL is the most prevalent cancer among children and adolescents in the United States (US), representing 20% of all cancers diagnosed in persons aged less than 20 years. In some disclosures, the disease, disorder or condition can be referred to as lymphoblastic lymphoma (LBL) if the infiltration of bone marrow (BM) is below 25%, and if the BM involvement is above 25% the disease, disorder or condition can be referred to as a leukemia.

In some disclosures, immunophenotyping (for example, by flow cytometry) and/or cytogenetics can be used to differentiate between B and T precursor ALL, and reveals the differentiation status of the malignant B-cells (for example, pro-B, common, pre-B, and mature B-cell). In some disclosures, the subject to be treated according to the methods and uses disclosed herein has ALL, and can be identified as having an immunophenotypic or a cytogenetic feature associated with different types of ALL, as described in described herein, for example, in Table 2. In some cases, the determination of disease type and differentiation status is essential for treatment selection. In some disclosures, ALL is the most common form of precursor B-cell neoplasm and is characterized by the proliferation and accumulation of malignant, transformed, and immature hematopoietic cells ("blasts") that accumulate in blood and in the bone marrow (see, e.g., Gokbuget et al., Blood. 2012 Aug 30;120(9):1868-76). In general, bone marrow analysis is required for the diagnosis of ALL. In some disclosures, bone marrow analysis of children with ALL show that the majority of children with ALL exhibit a massive leukemic infiltration of more than 50% blast cells by light microscopy. In some cases, other lymphatic organs, such as lymph nodes and spleen, can also be affected, as well as non-lymphatic organs, notably the central nervous system (CNS). In some disclosures, less than 10% of pediatric subjects have symptomatic CNS involvement, but the frequency is higher in subjects with mature B-ALL (Faderl et al., Cancer. 2010 Mar 01;116(5):1165-76.). In general, risk factors for developing ALL include age, exposure to chemotherapy or radiation therapy, and genetic disorders, including Down's syndrome. In some disclosures, the risk for developing ALL is highest in children younger than 5 years of age; the risk declines slowly until the mid-20s, and begins to rise again after the age of 50. In some cases, approximately 75% of patients with B-ALL have recurrent chromosomal translocations or somatic aneuploidy, some of which can be used for disease prognosis. In some disclosures, approximately half of patients with childhood ALL have chromosomal translocations. In some cases, these chromosomal translocations are undetectable by conventional cytogenetic analysis, but detected using fluorescence in situ hybridization (FISH) or polymerase chain reaction (PCR). In general, the most common chromosomal translocation for ALL is the t(12;21) translocation, resulting in an ETV6-runt-related transcription factor 1 fusion that occurs in 20% to 25% of childhood National Cancer Institute (NCI) standard-risk B-ALL. In some cases, children with this genetic alteration can exhibit up to a > 95% overall survival (OS) rate.

In some disclosures, multidrug chemotherapy is a treatment option for childhood ALL, and in some cases, is used as first-line therapy. In some disclosures, first-line induction therapy, regardless of presenting features, includes vincristine, dexamethasone (or prednisone), asparaginase, and doxorubicin (in some cases referred to as the Berlin- Frankfurt-Münster (BFM) regimen). In some cases, more than 95% of children receiving first-line therapy can achieve a complete response (CR) within the first 4 weeks of treatment, with a 5-year overall survival (OS) rate near 90% (Pui et al., J Clin Oncol. 2015 Sep 20;33(27):2938-48). In some cases, subjects presenting with central nervous system (CNS) disease, intrathecal triple therapy (administering methotrexate (MTX), cytarabine, and prednisolone) can be used during induction therapy together with intravenous (IV) high-dose MTX to reduce the risk of systemic relapse. In some cases, radiation therapy can be used in combination with intrathecal MTX in limited situations where there is a particularly high-risk of CNS relapse, but due to the risk of delayed neurocognitive impairment, radiation therapy is rarely used as a first-line therapy. In some disclosures, once a subject achieves a complete response (CR), post-induction treatment can vary depending on risk group assignment. In general, all subjects receive an intensification therapy after CR and before beginning maintenance therapy. In some disclosures, commonly used therapy includes cyclophosphamide, low-dose cytarabine, and a thiopurine. In some cases, maintenance therapy can include mercaptopurine, low-dose MTX, and in some cases, vincristine/steroid pulses. In some disclosures, therapeutic regimen can depend on the risk category of the patient. Due to the risk of late toxicity, in some cases, exposure to anthracyclines and alkylating agents can be limited in children with standard-risk ALL. In some cases, anthracyclines and alkylating agents can be used if the subject presents minimal residual disease (MRD). In some disclosures, for ALL, increased levels of MRD can be associated with worsening outcomes. In some cases, persistence of MRD at 12 weeks can be a very poor prognostic factor, with 5-year disease-free survival (DFS) of approximately 40% (Borowitz et al., Blood. 2015 Aug 20;126(8):964-71). In some cases, in subjects with intermediate risk BM relapse of ALL, low MRD after induction can be associated with good long-term prognosis with conventional chemo-/radiotherapy. In contrast, in some cases, subjects with insufficient response have an extremely poor prognosis. In some disclosures, MRD levels after induction can be a strong independent prognostic factor for long-term outcome in children with intermediate risk BM relapse of ALL.

In some disclosures, ALL can be further classified based on the World Health Organization classification of acute lymphoblastic leukemia. In some disclosures, the classification can be based on genetic, immunophenotype, molecular, and morphological features found through cytogenetic and molecular diagnostics tests. (see, e.g., Arber et al., Blood. 127(20): 2391-2405; Mrozek et al., Hematol Oncol Clin North Am. 2009 October; 23(5): 991-v). In some disclosures, the classification of B-cell lymphoblastic leukemiallymphoma include the following categories: with recurrent genetic abnormalities; with t(9;22)(q34.1;q11.2),BCR-ABL1; with t(v;11q23.3),KMT2A rearranged; with t(12;21)(p13.2;q22.1), ETV6-RUNX1; with t(5;14)(q3 1.1;q32.3),IL3-IGH; with t(1;19)(q23;p13.3),TCF3-PBX1; with hyperdiploidy; with hypodiploidy; and not otherwise specified (NOS). In some disclosures, ALL can include T-lymphoblastic leukemiallymphoma. In some disclosures, ALL can include acute leukemias of ambiguous lineage, categorized as follows: Acute undifferentiated leukemia; Mixed phenotype acute leukemia (MPAL) with t(9:22)(q34.1.q11.2),BCR-ABL1; MPAL with t(v;1 1q23.3),KMT2A rearranged; MPAL, B/myeloid, NOS; and MPAL, T/myeloid, NOS.

In some disclosures, B-ALL can be subdivided into the following based on phenotypes of the cells: early pre B-ALL (TdT+, CD19+, CD10-); common ALL (CD19+, CD10+/CALLA+); pre B ALL (CD10+/-, CD19+, HLA Dr+, cytoplasmic IgM+); and mature B ALL (CD10+, CD19+, CD20+, CD22+, surface IgM+).

In some disclosures, ALL can be classified based on the French-American-British (FAB) system, as follows: ALL-L1 (T cell or pre-B cell; with small and homogeneous (uniform) cells); ALL-L2 (T cell or pre-B cell; with large and heterogeneous (varied) cells); and ALL-L3 (B cell; with large and varied cells with vacuoles). In some disclosures, mature B-cell ALL can also be referred to as Burkitt leukemia.

In some disclosures, the ALL is a Philadelphia chromosome positive (Ph+) subtype of ALL. This subtype is characterized, in part, by poor outcomes with treatments of standard chemotherapy. The Philadelphia chromosome is present in 3-4% of pediatric acute lymphoblastic leukemia (Ph⁺ ALL), and about 25% of adult ALL cases. In certain disclosures, the Philadelphia chromosome is contains a translocation, t(9;22)(q34;ql 1), that results in a novel chimeric gene and protein which fuses the BCR gene on chromosome 22 with the gene encoding the Abelson tyrosine kinase (ABL1) on chromosome 9. The resulting BCR-ABL1 fusion transcript and protein is a constitutively activated tyrosine kinase which activates various signaling pathways to promote leukemic transformation in hematopoietic stem cells. In some disclosures, subjects having the Ph+ subtype of ALL have one or more cells that have a Philadelphia chromosome, such as bone marrow cells. In some disclosures, the ALL is a Philadelphia-like (Ph-like) subtype of ALL. In some disclosures, the Ph-like subtype is characterized by related gene expression signatures variously referred to as "cluster group R8," "Philadelphia Chromosome (Ph)-like, "Ph-like," "BCR-ABL1-like," or an "activated tyrosine kinase gene expression signature." These gene expression signatures have been shown to be highly similar to gene expression profiles measured in Ph+ ALL subjects, despite the fact that, in some disclosures, Ph-like subjects to not have the Philadelphia chromosome translocation or the BCR-ABL1 fusion transcript. The prevalence of the Ph-like subtype is approximately 12% in children, 21% in adolescents (16-20 years of age), and 20% to 24% in adults older than 40 years, with a peak (27%) in young adults 21 to 39 years old. In some cases, it occurs more often in male individuals and patients with Down syndrome. Ph-like ALL is overrepresented in those with Hispanic ethnicity and is associated with inherited genetic variants in GATA3 (rs3824662). In some disclosures, Ph-like ALL a clinically and biologically heterogeneous subtype of B-ALL.

In some disclosures, non-Hodgkin lymphoma (NHL) is a heterogeneous group of lymphoproliferative malignancies with differing clinical courses and responses to treatment. In some cases, the WHO Lymphoma Classification scheme is used to define specific subtypes of lymphoma and subdivides them based on cell of origin (B, T or natural killer (NK)) and the differentiation status of the lymphocytes. In some disclosures, eighty percent to 90% of NHL are of B-cell origin and express CD19. In some cases, the prognosis of NHL depends on the histologic type (indolent versus aggressive), stage, age, and treatment. In some disclosures, the NHL subtypes that occur in adult and pediatric populations show both similarities as well as differences. In some disclosures, childhood NHL displays an aggressive clinical course, while, in some disclosures, adult NHLs show both indolent and aggressive histological forms. In some disclosures, indolent subtypes of NHL that occur in adults include follicular lymphoma (FL), marginal zone lymphoma (MZL), and chronic lymphocytic leukemia (CLL). In some disclosures, aggressive B-cell subtypes include diffuse large B-cell lymphoma (DLBCL) and mantle cell lymphoma (MCL).

In some disclosures, many of the NHL types, including FL, CLL, and MCL, that are relatively common in adults occur rarely, if at all, in children. In some cases, pediatric B-cell non-Hodgkin's lymphoma (B-NHL), comprised of 3 main histological subtypes, are classified as aggressive: Burkitt lymphoma (BL), DLBCL, and primary mediastinal large B-cell lymphoma (PMBCL), in descending order of overall incidence. In some disclosures, LBL cases are T cell lineage with the remainder having a pre-B or mature B-cell immunophenotyped.

In some disclosures, the occurrence of NHL in infants is rare, and the incidence in adults increases with age. In some disclosures, adolescents have a higher mortality rate compared to children. In some disclosures, prevalent forms of pediatric B-NHL comprise about 60% of all childhood NHL. In some disclosures, approximately 25% to 30% of children will relapse or experience refractory disease with cure rates less than 30% (Jourdain et al., Haematologica. 2015 Jun;100(6):810-7).

In some cases, for both aggressive and indolent subtypes, r/r pediatric B-NHL represents a treatment challenge. In some cases, prognosis after relapse remains relatively poor. In some disclosures, salvage treatment consists of high-dose chemotherapy followed by an intensification phase with either autologous or allogenic hematopoietic SCT (HSCT).

In some disclosures, diffuse large B-cell lymphoma (DLBCL) accounts for 10% to 20% of B-cell lymphoma in children and occurs more frequently in adolescents. In some disclosures, diffuse large B-cell lymphoma is a prevalent form of NHL in children and adolescents, occurring with greater frequency in adolescents, and comprising up to 37% of all NHL in the 15 to 19 year age range.

In some disclosures, BL is the most common NHL in children of 14 years of age or less, comprising 30% to 40% of all NHL in North America and Europe. In some disclosures, NHL can arise in the abdomen and/or head and neck region and presents as advanced-stage disease involving the bone marrow and/or CNS in approximately 20% to 25% of patients. In some disclosures, the incidence of BL remains relatively constant throughout life, in contrast to other NHL subtypes including DLBCL, which can increase with age. In some disclosures, BL has a distinct epidemiological pattern because of its association with Epstein-Barr virus (EBV) and malaria infections that are endemic in sub-Saharan Africa.

In some disclosures, until recently, PMBCL was considered as a form of DLBCL, comprising 10% or fewer of all cases. In some cases, PMBCL is now recognized as a distinct entity in the WHO classification because, in some disclosures, PMBCL has a distinct immunophenotype, gene expression profile, and clinical presentation compared to other histologies. In some disclosures, the incidence of PMBCL peaks in the third or fourth decade of life and is more common in women.

In some disclosures, NHL can be staged based on the Lugano classification (see, e.g., Cheson et al., (2014) JCO 32(27):3059-3067; Cheson, B.D. (2015) Chin Clin Oncol 4(1):5). In some cases, the stages are described by Roman numerals I through IV (1-4), and limited stage (I or II) lymphomas that affect an organ outside the lymph system (an extranodal organ) are indicated by an E. Stage I represents involvement in one node or a group of adjacent nodes, or a single extranodal lesions without nodal involvement (IE). Stage 2 represents involvement in two or more nodal groups on the same side of the diaphragm or stage I or II by nodal extent with limited contiguous extranodal involvement (IIE). Stage III represents involvement in nodes on both sides of the diaphragm or nodes above the diaphragm with spleen involvement. Stage IV represents involvement in additional non-contiguous extralymphatic involvement. In addition, "bulky disease" can be used to describe large tumors in the chest, in particular for stage II. The extent of disease is determined by positron emission tomography (PET)-computed tomography (CT) for avid lymphomas, and CT for non-avid histologies.

In some disclosures, the subject to be treated according to the methods and uses disclosed herein has NHL, and has or has been identified as having an immunophenotypic or a cytogenetic feature as described in described herein, for example, in Table 2. In some disclosures, the subject to be treated according to the methods and uses disclosed herein has NHL, and has or has been identified as having as having a double/triple hit lymphoma or a lymphoma of the double/triple hit molecular subtypes. In some disclosures, the lymphoma is a double hit lymphoma characterized by the presence of MYC (myelocytomatosis oncogene), BCL2 (B-cell lymphoma 2), and/or BCL6 (B-cell lymphoma 6) gene rearrangements (e.g., translocations). In some disclosures, the gene rearrangement affects the MYC/8q24 locus in combination with another gene rearrangement. For example, the other gene rearrangement includes t(14;18)(q32;q21) involving BCL2. In some disclosures, the gene rearrangements affect the MYC/8q24 locus in combination with BCL6/3q27. In some disclosures, the lymphoma is a triple hit lymphoma characterized by the presence of MYC, BCL2, and BCL6 gene rearrangements; see, e.g., Aukema et al., (2011) Blood 117:2319-2331. In some disclosures of such disclosures the subject is ECOG 0-1 or does not have or is not suspected or characterized as having DLBCL transformed from MZL or CLL. In disclosures, the therapy is indicated for such subjects and/or the instructions indicate administration to a subject within such population. In some disclosures, based on the 2016 WHO criteria (Swerdlow et al., (2016) Blood 127(20):2375-2390), double/triple hit lymphoma can be considered high-grade B-cell lymphoma, with MYC and BCL2 and/or BCL6 rearrangements with DLBCL histology (double/triple hit).

In some disclosures, the type of B cell malignancy or hematological malignancy can be identified by assessing and/or analyzing the chromosomal structures for abnormalities, e.g., based on the cytogenetic features described herein, for example, in Table 2. For example, in some disclosures, the chromosomes are analyzed by karyotyping, e.g., a G-banding technique. G-banding produces an individual's karyotype, whereby Giemsa stain is used to produce a series of dark and light bands, with each chromosome displaying a unique banding pattern under light microscope. Each chromosome can be further distinguished by the position of its centromere (metacentric, submetacentric, acrocentric), dividing it into a shorter arm, the p (petite) arm and a longer arm, called the q arm. Chromosomes are then arranged with pairs side by side to detect abnormalities including deletions, duplications, or other structural rearrangements. This technique is relatively inexpensive and is a good first-line test for anomalies, but a limitation of this technique is the inability to detect small deletions or rearrangements.

In some disclosures, other techniques that can be used to determine the types of B cell malignancy or hematological malignancy include fluorescent in situ hybridization (FISH) and multicolor FISH which uses a fluorescently-labeled probes to detect the presence or absence of a particular chromosome segment or gene. FISH and multicolor FISH can detect small deletions, duplications and/or subtle chromosomal rearrangements. FISH and multicolor FISH analysis can be performed on the same specimens obtained for chromosome analysis. In some disclosures, the type of B cell malignancy is identified and/or detected by FISH and/or multicolor FISH.

In some disclosures, the methods involve treating a subject having relapsed following remission after treatment with, or become refractory to, one or more prior therapies; or a subject that has relapsed or is refractory (R/R) to one or more prior therapies, e.g., one or more lines of standard therapy. In some disclosures, the methods and uses involve treating a specific group or subset of subjects, such as pediatric subjects identified as having a B cell malignancy that has R/R to standard therapy. In some disclosures, the subjects can have a high-risk disease, such as a B cell malignancy that is aggressive and/or has a poor prognosis or that has R/R to standard therapy. In some disclosures, the methods involve treating subjects having a R/R B-ALL, a R/R B-NHL, or a B-ALL that exhibits minimum residual disease (MRD+ B-ALL). In some disclosures, the subject has a R/R DLBCL, a R/R BL or a R/R PMBCL.

In some disclosures, the methods and uses involve treating a specific group or subset of subjects, e.g., subjects that are of a particular age, such as subjects who are at or younger than 25 years of age. In some disclosures, the subject is at or younger than 25, 24, 23, 22, 21, 20, 19, 18, 17, 16 or 15 of age. In some disclosures, the methods and uses involve treating subjects that are of a particular age, such as subjects who are at or younger than 25 years of age. In some disclosures, the methods and uses involve treating subjects that are of a particular age, such as subjects who are younger than 18 years of age. In some disclosures, the methods and uses involve treating pediatric subjects or young adult subjects.

In some disclosures, the methods and uses involve administering CAR-expressing cells to a subject that is at or greater than at or about 6 kg in body weight. In some disclosures, the methods and uses involve administering CAR-expressing cells to a subject that is at or greater than at or about 6, 7, 8, 9, 10, 11, 12, 13, 14, 15, 16, 17, 18, 19 or 20 kg in body weight. In some disclosures, the methods and uses involve administering CAR-expressing cells to a subject that is at or greater than at or about 12 kg in body weight. In some disclosures, the subject is less than at or about 100 kg in weight.

In some disclosures, the subject is younger than 18 years of age, and is at or greater than at or about 12 kg in body weight. In some disclosures, the subject is younger than 18 years of age, and is at or greater than at or about 6 kg in body weight. In some disclosures, the subject is 25 years of age or younger, and is at or greater than at or about 12 kg in body weight. In some disclosures, the subject is 25 years of age or younger, and is at or greater than at or about 6 kg in body weight.

In some disclosures, prior to administering the cells or composition, the subject is or has been identified as having cells expressing the antigen targeted by the recombinant receptor (e.g., CD19). In some disclosures, the subject or a biological sample from the subject exhibits evidence of CD19 expression or contains CD19-expressing cells, as determined via flow cytometry (e.g., from peripheral blood or bone marrow samples) or immunohistochemistry (e.g., bone marrow biopsy). In some disclosures, the expression of the antigen (e.g., CD19) is detected by flow cytometry in the peripheral blood or bone marrow, and/or by immunohistochemistry of a bone marrow biopsy.

In some disclosures, prior to administering the cells or composition, the subject is or has been identified as having a Karnofsky score of 50 or higher if the subject is 16 years of age or older, or a Lansky score of 50 or higher if the subject is less than 16 years of age.

In some disclosures, the methods include administration of cells to a subject selected or identified as having a certain prognosis or risk of an ALL or an NHL. In some cases, subjects with an ALL or an NHL may be classified into groups that may inform disease prognosis and/or recommended treatment strategy. In some cases, these groups may be "low risk," "intermediate risk," "high risk," and/or "very high risk" and patients may be classified as such depending on a number of factors including, but not limited to, genetic abnormalities and/or morphological or physical characteristics. In some disclosures, subjects treated in accord with the methods, and/or with the articles of manufacture or compositions, are classified or identified based on the risk of an ALL or an NHL. In some disclosures, the subject is one that has a high risk ALL or a high risk NHL.

In some disclosures, the subject has been previously treated with a therapy or a therapeutic agent targeting the disease or condition, e.g., an ALL or an NHL, prior to administration of the cells expressing the recombinant receptor. In some disclosures, the subject has been previously treated with a hematopoietic stem cell transplantation (HSCT), e.g., allogeneic HSCT or autologous HSCT. In some disclosures, the subject has had poor prognosis after treatment with standard therapy and/or has failed one or more lines of previous therapy. In some disclosures, the subject has been treated or has previously received at least or about at least or about 1, 2, 3, or 4 other therapies for treating the ALL or NHL other than a lymphodepleting therapy and/or the dose of cells expressing the antigen receptor. In some disclosures, the subject has been previously treated with chemotherapy or radiation therapy. In some disclosures, the subject is refractory or non-responsive to the other therapy or therapeutic agent. In some disclosures, the subject has persistent or relapsed disease, e.g., following treatment with another therapy or therapeutic intervention, including chemotherapy or radiation.

In some disclosures, the subject is one that is eligible for a transplant, such as is eligible for a hematopoietic stem cell transplantation (HSCT), e.g., allogeneic HSCT. In some such disclosures, the subject has not previously received a transplant, despite being eligible, prior to administration of the engineered cells (e.g. CAR-T cells) or a composition containing the cells to the subject as disclosed herein.

In some disclosures, the subject is one that is not eligible for a transplant, such as is not eligible for a hematopoietic stem cell transplantation (HSCT), e.g., allogeneic HSCT. In some disclosures, such a subject is administered the engineered cells (e.g. CAR-T cells) or a composition containing the cells according to the disclosures herein.

In some disclosures, prior to administering the cells or composition, the subject has a relapsed and/or refractory (R/R) B-ALL. In some disclosures, the subject has a R/R B-ALL, which can be characterized as morphological evidence of disease in the bone marrow, e.g., 5% or greater lymphoblast by morphology, and any of the following: (a) first or greater marrow relapse, or (b) any marrow relapse after an allogeneic hematopoietic stem cell transplantation (HSCT), or (c) primary refractory defined as not achieving a complete response (CR) or a complete response with incomplete blood count recovery (CRi) after 2 or more separate induction regimens (or chemo-refractory as not achieving CR/CRi after 1 cycle of standard chemotherapy for relapsed leukemia), or (d) ineligible for allogeneic HSCT.

In some disclosures, prior to administering the cells or composition, the subject has minimal residual disease positive (MRD+) B-ALL. In some disclosures, the subject has MRD+ B-ALL, which can be characterized as having less than 5% lymphoblasts by morphology, and/or MRD can be detected by a validated assay at a frequency of 1 x10⁻⁴ or greater in BM cells after two lines of therapy.

In some disclosures, prior to administering the cells or composition, the subject has a B-NHL. In some disclosures, the subject has a B-NHL, which can be characterized as having measurable disease after 1 or more lines of chemotherapy and/or having failed HSCT or being ineligible for HSCT.

In some disclosures, prior to administering the cells or composition, the subject has a Philadelphia chromosome positive ALL, that is intolerant to or have failed one or more lines of tyrosine-kinase inhibitor (TKI) therapy, or TKI therapy has been contraindicated.

In some disclosures, prior to administering the cells or composition, the subject is shown to have adequate organ function, such as adequate bone marrow function, adequate kidney function, adequate pulmonary function and/or adequate cardiac function. In some disclosures, adequate bone marrow function can be characterized as adequate bone marrow function to receive lymphodepleting therapy. In some disclosures, adequate kidney function can be characterized as creatinine clearance calculated using the Schwartz formula, or radioisotope glomerular filtration rate (GFR) > 70 mL/min/1.73 m². In some disclosures, adequate pulmonary function can be characterized as ≤ Grade 1 dyspnea according to Common Toxicity Criteria for Adverse Events (CTCAE) and oxygen saturation (SaO₂) ≥ 92% on room air. In some disclosures, adequate cardiac function can be characterized as left ventricular ejection fraction (LVEF) ≥ 40% as assessed by echocardiogram (ECHO) or multi-gated acquisition scan (MUGA) within 4 weeks prior to leukapheresis.

In some disclosures, prior to administering the cells or composition, the subject is shown to have adequate vascular access for leukapheresis procedure.

In some disclosures, prior to and during the treatment regimen with cells or composition, the subject uses effective contraception.

In some cases, certain subjects are ineligible for treatment or are excluded from treatment according to the disclosed methods and uses. In some disclosures, subjects that are not eligible or are excluded from treatment according to the disclosed methods and uses, can include the presence of any, some or all of the following: subject has any significant medical condition, laboratory abnormality, or psychiatric illness that would prevent the subject from receiving the treatment regimen; subject has any condition including the presence of laboratory abnormalities that can places the subject at an unacceptable risk if treated according to the methods and uses; subject has any condition that confounds the ability to interpret the results from the treatment; subject with a history of another primary malignancy that has not been in remission for at least 2 years prior to administration of the cells; subjects who have received prior therapies targeting the same antigen targeted by the recombinant receptor expressed by the cell (e.g., CD19), if shown to not express the antigen (e.g., CD19) or exhibits a CD19-negative disease after completing the prior therapy; subjects that have received a prior therapy that includes CAR T cell or other genetically-modified T cell therapy; subject with a previous history of or active hepatitis B, hepatitis C, or human immunodeficiency virus (HIV) infection; subjects with uncontrolled systemic fungal, bacterial, viral or other infection (including tuberculosis) despite appropriate antibiotics or other treatment at the time of leukapheresis or engineered cell administration; subject has presence of acute or chronic graft-versus-host disease (GVHD); subject with active autoimmune disease requiring immunosuppressive therapy; subject has cardiac disorders (CTCAE version 4.03 Grade 3 or 4) within the 6 months prior to leukapheresis or engineered cell administration; subject with a concomitant genetic syndrome, with the exception of Down's syndrome; subject with an active central nervous system (CNS) disease and significant neurological deterioration (subjects with CNS-2 or CNS-3 involvement can be eligible or can receive administration if they are asymptomatic and do not have significant neurological deterioration); subject with a history or presence of clinically relevant CNS pathology; subject that is pregnant or nursing.

In some disclosures, subjects who have received one or more of the following treatments or therapies may be ineligible for or are excluded from treatment according to the disclosed methods or uses: therapeutic doses of corticosteroids (defined as > 20 mg/day prednisone or equivalent) within 7 days prior to leukapheresis or 72 hours prior to engineered cell administration (with the exception of physiologic replacement, topical, and inhaled steroids); low-dose chemotherapy (e.g., vincristine, rituximab, cyclophosphamide ≤ 300 mg/m²) given after leukapheresis to maintain disease control must be discontinued ≥ 7 days prior to lymphodepletion; cytotoxic chemotherapeutic agents that are not considered lymphotoxic within 1 week prior to leukapheresis (with the exception of oral anticancer agents, including lenalidomide and ibrutinib, if at least 3 half-lives have elapsed prior to leukapheresis); lymphotoxic chemotherapeutic agents (e.g., cyclophosphamide, ifosfamide, bendamustine) within 2 weeks prior to leukapheresis; experimental agents within 4 weeks prior to leukapheresis unless no response or PD is documented on the experimental therapy and at least 3 half-lives have elapsed prior to leukapheresis; immunosuppressive therapies within 4 weeks prior to leukapheresis and engineered cell administration (e.g., calcineurin inhibitors, methotrexate or other chemotherapeutics, mycophenolate, rapamycin, thalidomide, immunosuppressive antibodies such as antitumor necrosis factor (TNF), anti-IL-6, or anti-IL-6R); donor lymphocyte infusions (DLI) within 6 weeks prior to engineered cell administration; radiation within 6 weeks prior to leukapheresis (subjects must have progressing disease (PD) in irradiated lesions or have additional non-irradiated lesions to be eligible to receive engineered cell administration, and radiation to a single lesion, if additional non-irradiated, measurable lesions are present, can be allowed up to 2 weeks prior to leukapheresis); or an allogeneic HSCT within 90 days prior to leukapheresis.

In some disclosures, the Eastern Cooperative Oncology Group (ECOG) performance status indicator can be used to assess or select subjects for treatment, e.g., subjects who have had poor performance from prior therapies (see, e.g., Oken et al. (1982) Am J Clin Oncol. 5:649-655). The ECOG Scale of Performance Status describes a patient's level of functioning in terms of their ability to care for themselves, daily activity, and physical ability (e.g., walking, working, etc.). In some disclosures, an ECOG performance status of 0 indicates that a subject can perform normal activity. In some disclosures, subjects with an ECOG performance status of 1 exhibit some restriction in physical activity but the subject is fully ambulatory. In some disclosures, patients with an ECOG performance status of 2 is more than 50% ambulatory. In some cases, the subject with an ECOG performance status of 2 may also be capable of self-care; see e.g., Sørensen et al., (1993) Br J Cancer 67(4) 773-775. The criteria reflective of the ECOG performance status are described in Table 1 below:

| **Table** 1. ECOG Performance Status Criteria | |
|---|---|
| Grade | ECOG performance status |
| 0 | Fully active, able to carry on all pre-disease performance without restriction |
| 1 | Restricted in physically strenuous activity but ambulatory and able to carry out work of a light or sedentary nature, e.g., light house work, office work |
| 2 | Ambulatory and capable of all self-care but unable to carry out any work activities; up and about more than 50% of waking hours |
| 3 | Capable of only limited self-care; confined to bed or chair more than 50% of waking hours |
| 4 | Completely disabled; cannot carry on any self-care; totally confined to bed or chair |
| 5 | Dead |

In some disclosures, the methods involve treating a subject that has an Eastern Cooperative Oncology Group Performance Status (ECOG) of 0-1 or 0-2. In some disclosures, the methods treat a poor-prognosis population or of DLBCL patients or subject thereof that generally responds poorly to therapies or particular reference therapies, such as one having one or more, such as two or three, chromosomal translocations (such as so-called "double-hit" or "triple-hit" lymphoma; having translocations MYC/8q24 loci, usually in combination with the t(14; 18) (q32; q21) bcl-2 gene or/and BCL6/3q27 chromosomal translocation; see, e.g., Xu et al. (2013) Int J Clin Exp Pathol. 6(4): 788-794), and/or one having relapsed, optionally relapsed within 12 months, following administration of an autologous stem cell transplant (ASCT), and/or one having been deemed chemorefractory.

In some disclosures, the engineered cells or compositions containing such cells used in the methods and uses disclosed herein contain recombinant receptors that target an antigen associated with a B cell malignancy or a hematological malignancy. In some disclosures, antigens targeted by the receptors in some disclosures include antigens associated with a B cell malignancy, such as any of a number of known B cell marker. In some disclosures, the antigen is or includes CD19, CD20, CD22, ROR1, CD45, CD21, CD5, CD33, Igkappa, Iglambda, CD79a, CD79b or CD30.

In some disclosures, the cell therapy, e.g., adoptive T cell therapy, is carried out by autologous transfer, in which the cells are isolated and/or otherwise prepared from the subject who is to receive the cell therapy, or from a sample derived from such a subject. Thus, in some disclosures, the cells are derived from a subject, e.g., patient, in need of a treatment and the cells, following isolation and processing are administered to the same subject.

In some disclosures, the cell therapy, e.g., adoptive T cell therapy, is carried out by allogeneic transfer, in which the cells are isolated and/or otherwise prepared from a subject other than a subject who is to receive or who ultimately receives the cell therapy, e.g., a first subject. In such disclosures, the cells then are administered to a different subject, e.g., a second subject, of the same species. In some disclosures, the first and second subjects are genetically identical. In some disclosures, the first and second subjects are genetically similar. In some disclosures, the second subject expresses the same HLA class or supertype as the first subject.

The cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some disclosures, they are administered by parenteral, intrapulmonary, and intranasal, and, if desired for local treatment, intralesional administration. Parenteral infusions include intramuscular, intravenous, intraarterial, intraperitoneal, or subcutaneous administration. In some disclosures, a given dose is administered by a single bolus administration of the cells. In some disclosures, it is administered by multiple bolus administrations of the cells, for example, over a period of no more than 3 days, or by continuous infusion administration of the cells. In some disclosures, administration of the cell dose or any additional therapies, e.g., the lymphodepleting therapy, intervention therapy and/or combination therapy, is carried out via outpatient delivery.

For the prevention or treatment of disease, the appropriate dosage may depend on the type of disease to be treated, the type of cells or recombinant receptors, the severity and course of the disease, whether the cells are administered for preventive or therapeutic purposes, previous therapy, the subject's clinical history and response to the cells, and the discretion of the attending physician. The compositions and cells are in some disclosures suitably administered to the subject at one time or over a series of treatments.

**In** some disclosures, the cells are administered as part of a combination treatment, such as simultaneously with or sequentially with, in any order, another or additional therapeutic intervention, such as an antibody or engineered cell or receptor or agent, such as a cytotoxic or therapeutic agent. The cells in some disclosures are co-administered with one or more additional therapeutic agents or in connection with another therapeutic intervention, either simultaneously or sequentially in any order. In some disclosures, the additional therapeutic agent is any interventions or agents described herein, such as any combination therapy agents described in Section IV, or such as any interventions or agents that can ameliorate symptoms of toxicity described herein, for example, in Section I.D. In some contexts, the cells are co-administered with another therapy sufficiently close in time such that the cell populations enhance the effect of one or more additional therapeutic agents, or vice versa. In some disclosures, the cells are administered prior to the one or more additional therapeutic agents. In some disclosures, the cells are administered after the one or more additional therapeutic agents. In some disclosures, the one or more additional agents include a cytokine, such as IL-2, for example, to enhance persistence. In some disclosures, the methods comprise administration of a chemotherapeutic agent.

**In** some disclosures, the methods comprise administration of a chemotherapeutic agent, e.g., a conditioning chemotherapeutic agent, for example, to reduce tumor burden prior to the administration.

Preconditioning subjects with immunodepleting (e.g., lymphodepleting) therapies in some disclosures can improve the effects of adoptive cell therapy (ACT).

Thus, in some disclosures, the methods include administering a preconditioning agent, such as a lymphodepleting or chemotherapeutic agent, such as cyclophosphamide, fludarabine, or combinations thereof, to a subject prior to the initiation of the cell therapy. For example, the subject may be administered a preconditioning agent at least 2 days prior, such as at least 3, 4, 5, 6, or 7 days prior, to the initiation of the cell therapy. In some disclosures, the subject is administered a preconditioning agent no more than 7 days prior, such as no more than 6, 5, 4, 3, or 2 days prior, to the initiation of the cell therapy.

**In** some disclosures, the subject is preconditioned with cyclophosphamide at a dose between or between about 20 mg/kg and 100 mg/kg body weight of the subject, such as between or between about 40 mg/kg and 80 mg/kg. In some disclosures, the subject is administered about 60 mg/kg of cyclophosphamide. In some disclosures, the cyclophosphamide is administered once daily for one or two days. In some disclosures, where the lymphodepleting agent comprises cyclophosphamide, the subject is administered cyclophosphamide at a dose between or between about 100 mg/m² and 500 mg/m² body surface area of the subject, such as between or between about 200 mg/m² and 400 mg/m², or 250 mg/m² and 350 mg/m², inclusive. In some instances disclosed herein, the subject is administered about 100 mg/m² of cyclophosphamide. In some instances disclosed herein, the subject is administered about 150 mg/m² of cyclophosphamide. In some instances disclosed herein, the subject is administered about 200 mg/m² of cyclophosphamide. In some instances disclosed herein, the subject is administered about 250 mg/m² of cyclophosphamide. In some instances disclosed herein, the subject is administered about 300 mg/m² of cyclophosphamide. In some disclosures, the cyclophosphamide can be administered in a single dose or can be administered in a plurality of doses, such as given daily, every other day or every three days. In some disclosures, cyclophosphamide is administered daily, such as for 1-5 days, for example, for 3 to 5 days. In some instances disclosed herein, the subject is administered about 300 mg/m² body surface area of the subject, of cyclophosphamide, daily for 3 days, prior to initiation of the cell therapy. In some disclosures, the subject is administered a total of at or about 300 mg/m², 400 mg/m², 500 mg/m², 600 mg/m², 700 mg/m², 800 mg/m², 900 mg/m², 1000 mg/m², 1200 mg/m², 1500 mg/m², 1800 mg/m², 2000 mg/m², 2500 mg/m², 2700 mg/m², 3000 mg/m², 3300 mg/m², 3600 mg/m², 4000 mg/m² or 5000 mg/m² cyclophosphamide, or a range defined by any of the foregoing, prior to initiation of the cell therapy.

**In** some disclosures, where the lymphodepleting agent comprises fludarabine, the subject is administered fludarabine at a dose between or between about 1 mg/m² and 100 mg/m² body surface area of the subject, such as between or between about 10 mg/m² and 75 mg/m², 15 mg/m² and 50 mg/m², 20 mg/m² and 40 mg/m², or 24 mg/m² and 35 mg/m², inclusive. In some instances disclosed herein, the subject is administered about 10 mg/m² of fludarabine. In some instances disclosed herein, the subject is administered about 15 mg/m² of fludarabine. In some instances disclosed herein, the subject is administered about 20 mg/m² of fludarabine. In some instances disclosed herein, the subject is administered about 25 mg/m² of fludarabine. In some instances disclosed herein, the subject is administered about 30 mg/m² of fludarabine. In some disclosures, the fludarabine can be administered in a single dose or can be administered in a plurality of doses, such as given daily, every other day or every three days. In some disclosures, fludarabine is administered daily, such as for 1-5 days, for example, for 2 to 4 days. In some instances disclosed herein, the subject is administered about 30 mg/m² body surface area of the subject, of fludarabine, daily for 3 days, prior to initiation of the cell therapy. In some disclosures, the subject is administered a total of at or about 10 mg/m², 20 mg/m², 25 mg/m², 30 mg/m², 40 mg/m², 50 mg/m², 60 mg/m², 70 mg/m², 80 mg/m², 90 mg/m², 100 mg/m², 120 mg/m², 150 mg/m², 180 mg/m², 200 mg/m², 250 mg/m², 270 mg/m², 300 mg/m², 330 mg/m², 360 mg/m², 400 mg/m² or 500 mg/m² cyclophosphamide, or a range defined by any of the foregoing, prior to initiation of the cell therapy.

**In** some disclosures, the lymphodepleting agent comprises a single agent, such as cyclophosphamide or fludarabine. In some disclosures, the subject is administered cyclophosphamide only, without fludarabine or other lymphodepleting agents. In some disclosures, prior to the administration, the subject has received a lymphodepleting therapy comprising the administration of cyclophosphamide at or about 200-400 mg/m² body surface area of the subject, optionally at or about 300 mg/m², daily, for 2-4 days. In some disclosures, the subject is administered fludarabine only, for example, without cyclophosphamide or other lymphodepleting agents. In some disclosures, prior to the administration, the subject has received a lymphodepleting therapy comprising the administration of fludarabine at or about 20-40 mg/m² body surface area of the subject, optionally at or about 30 mg/m², daily, for 2-4 days.

**In** some disclosures, the lymphodepleting agent comprises a combination of agents, such as a combination of cyclophosphamide and fludarabine. Thus, the combination of agents may include cyclophosphamide at any dose or administration schedule, such as those described above, and fludarabine at any dose or administration schedule, such as those described above. For example, in some disclosures, the subject is administered 60 mg/kg (~2 g/m²) of cyclophosphamide and 3 to 5 doses of 25 mg/m² fludarabine prior to the first or subsequent dose. In some the subject is administered fludarabine (30 mg/m²/day for 3 days) and cyclophosphamide (300 mg/m²/day for 3 days) (flu/cy) concurrently, intravenously, prior to administration of the cells.

Following administration of the cells, the biological activity of the engineered cell populations in some disclosures is measured, e.g., by any of a number of known methods. Parameters to assess include specific binding of an engineered or natural T cell or other immune cell to antigen, in vivo, e.g., by imaging, or ex vivo, e.g., by ELISA or flow cytometry. In certain disclosures, the ability of the engineered cells to destroy target cells can be measured using any suitable known methods, such as cytotoxicity assays described in, for example, Kochenderfer et al., J. Immunotherapy, 32(7): 689-702 (2009), and Herman et al. J. Immunological Methods, 285(1): 25-40 (2004). In certain disclosures, the biological activity of the cells is measured by assaying expression and/or secretion of one or more cytokines, such as CD107a, IFNγ, IL-2, and TNF. In some disclosures the biological activity is measured by assessing clinical outcome, such as reduction in tumor burden or load.

**In** certain disclosures, the engineered cells are further modified in any number of ways, such that their therapeutic or prophylactic efficacy is increased. For example, the engineered recombinant receptor expressed by the population can be conjugated either directly or indirectly through a linker to a targeting moiety. The practice of conjugating compounds, e.g., the CAR, to targeting moieties is known. See, for instance, Wadwa et al., J. Drug Targeting 3: 1 1 1 (1995), and U.S. Patent 5,087,616. In some disclosures, the cells are administered as part of a combination treatment, such as simultaneously with or sequentially with, in any order, another therapeutic intervention, such as an antibody or engineered cell or receptor or agent, such as a cytotoxic or therapeutic agent. The cells in some disclosures are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some disclosures, the cells are administered prior to the one or more additional therapeutic agents. In some disclosures, the cells are administered after the one or more additional therapeutic agents. In some disclosures, the one or more additional agent includes a cytokine, such as IL-2, for example, to enhance persistence.

### B. Dosing

**In** some disclosures, the methods and uses involve administering all or a portion of a composition containing cells, such as engineered T cells expressing a recombinant receptor, such as a chimeric antigen receptor (CAR). In some disclosures, a particular amount or number of cells, or a particular amount of the composition containing the particular amount or number of cells, is administered to a subject. In some disclosures, one or more doses of cells, containing a particular amount or number of cells or a particular amount of the composition containing the particular amount or number of cells, is administered to a subject. In some disclosures, a dose of cells is administered to subjects in accord with the disclosed methods, and/or with the disclosed articles of manufacture or compositions. In some disclosures, the size, amount or timing of the doses is determined as a function the age of the subject. In some disclosures, the size, amount or timing of the doses is determined as a function the body weight of the subject. In some disclosures, the size, amount or timing of the doses is determined as a function of the particular type of B cell malignancy or hematological malignancy in the subject.

**In** some disclosures, the amount or number of cells in the dose is determined as a function of the age of the subject. In some disclosures, the methods and uses involve administering CAR-expressing cells to a subject at or younger than 25 years of age. In some disclosures, the methods and uses involve administering CAR-expressing cells to a subject that is younger than 18 years of age. In some disclosures, the methods and uses involve administering CAR-expressing cells to a pediatric subject. In some disclosures, the methods and uses involve administering CAR-expressing cells to a young adult subject.

**In** some disclosures, the amount or number of cells that are administered in the methods and uses described herein is with reference to the amount or number of recombinant receptor-expressing cells, chimeric antigen receptor (CAR)-expressing cells, total T cells, or total peripheral blood mononuclear cells (PBMCs). **In** some disclosures, the amount or number of cells that are administered in the methods and uses described herein is with reference to the amount or number of CD3+ T cells, CD4+ T cells, CD8+ T cells, and in some cases also recombinant receptor-expressing or CAR-expressing T cells.

In some disclosures, the amount or number of cells in the dose is determined as a function of the body weight of the subject. In some disclosures, the methods and uses involve administering CAR-expressing cells to a subject that is less than 100 kilograms (kg) in body weight. In some disclosures, the methods and uses involve administering CAR-expressing cells to a subject that is at or greater than 100 kg in body weight. In some disclosures, based on the body weight of the subject, the subject is administered a dose that is calculated depending on the body weight (e.g., cells/kg body weight of the subject) or a flat or fixed dose.

In some disclosures, the methods involve administering, to a subject at or younger than 25 years of age, a T cell composition comprising T cells expressing an anti-CD19 chimeric antigen receptor (CAR), wherein the composition is administered in an amount selected from: (i) if the subject is less than 100 kilograms (kg) in body weight and is younger than 18 years of age, from at or about 0.05 x 10⁶ CAR+ T cells/kg body weight of the subject to at or about 1.5 x 10⁶ CAR+ T cells/kg body weight of the subject; and (ii) if the subject is at or greater than 100 kilograms (kg) in body weight or is between 18 and 25 years of age, inclusive, from at or about 0.05 x 10⁸ CAR+ T cells to at or about 1.5 x 10⁸ CAR+ T cells. In some disclosures, if the subject is between 18 and 25 years of age, inclusive, the T cell composition is administered in an amount from at or about 0.05 x 10⁸ CAR+ T cells to at or about 1.5 x 10⁸ CAR+ T cells.

In some disclosures, if the subject is less than 100 kilograms (kg) in body weight the T cell composition is administered in an amount from at or about 0.05 x 10⁶ CAR+ T cells/kg body weight of the subject to at or about 1 x 10⁶ CAR+ T cells/kg body weight of the subject.

In some disclosures, the methods and uses involve administering, to a subject at or younger than 25 years of age, a T cell composition comprising T cells expressing an anti-CD19 chimeric antigen receptor (CAR), wherein the composition is administered in an amount selected from: (i) if the subject is less than 100 kilograms (kg) in body weight, from at or about 0.05 x 10⁶ CAR+ T cells/kg body weight of the subject to at or about 1.5 x 10⁶ CAR+ T cells/kg body weight of the subject; and (ii) if the subject is at or greater than 100 kilograms (kg) in body weight, from at or about 0.05 x 10⁸ CAR+ T cells to at or about 1.5 x 10⁸ CAR+ T cells.

In some disclosures, the methods and uses involve administering, to a subject that is younger than 18 years of age, a T cell composition comprising T cells expressing an anti-CD19 chimeric antigen receptor (CAR), wherein the T cell composition is administered in an amount selected from: (i) if the subject is less than 100 kilograms (kg) in body weight, from at or about 0.05 x 10⁶ CAR+ T cells/kg body weight of the subject to at or about 1.5 x 10⁶ CAR+ T cells/kg body weight of the subject; and (ii) if the subject is at or greater than 100 kilograms (kg) in body weight, from at or about 0.05 x 10⁸ CAR+ T cells to at or about 1.5 x 10⁸ CAR+ T cells.

In some disclosures, if the subject is less than 100 kilograms (kg) in body weight the T cell composition is administered in an amount from at or about 0.05 x 10⁶ CAR+ T cells/kg body weight of the subject to at or about 1 x 10⁶ CAR+ T cells/kg body weight of the subject. In some disclosures, if the subject is at or greater than 100 kilograms (kg) in body weight, the T cell composition is administered in an amount from at or about 0.05 x 10⁸ CAR+ T cells to at or about 1 x 10⁸ CAR+ T cells.

Some instances, disclosed herein involve methods comprising administering, to a subject at or younger than 25 years of age, a T cell composition comprising T cells expressing an anti-CD19 chimeric antigen receptor (CAR), wherein the composition is administered in an amount selected from: (i) if the subject is younger than 18 years of age from at or about 0.05 x 10⁶ CAR+ T cells/kg body weight of the subject to at or about 1.5 x 10⁶ CAR+ T cells/kg body weight of the subject, but that does not exceed at or about 1.5 x 10⁸ total CAR+ T cells; or (ii) if the subject is between 18 and 25 years of age, inclusive, from at or about 0.05 x 10⁸ CAR+ T cells to at or about 1.5 x 10⁸ CAR+ T cells.

In some disclosures, if the subject is younger than 18 years of age the T cell composition is administered in an amount from at or about 0.05 x 10⁶ CAR+ T cells/kg body weight of the subject to at or about 1.0 x 10⁶ CAR+ T cells/kg body weight of the subject, but that does not exceed at or about 1.0 x 10⁸ total CAR+ T cells.

In some disclosures, subjects younger than 18 years of age, are administered a dose that is determined based on the body weight of the subject, but up to a maximum amount or maximum number of cells, or a cap amount or a cap number of cells. In some disclosures, the maximum or cap number or amount of cells in a dose can be a fixed number or amount. In some disclosures, the subject is younger than 18 years of age, and is administered a dose of cells, such as CAR-expressing cells, per kilogram body weight of the subject (cells/kg), up to a maximum number of cells. In some disclosures, the subject is younger than 18 years of age, and is administered from at or about 0.5 x 10⁶ CAR+ T cells/kg body weight of the subject to at or about 1.5 x 10⁶ CAR+ T cells/kg body weight of the subject, but an amount that does not exceed at or about 1.5 x 10⁸ total CAR+ T cells. In some disclosures, the subject is younger than 18 years of age, and is administered from at or about 0.5 x 10⁶ CAR+ T cells/kg body weight of the subject to at or about 1.0 x 10⁶ CAR+ T cells/kg body weight of the subject, but an amount that does not exceed at or about 1.0 x 10⁸ total CAR+ T cells. In some disclosures, the subject is younger than 18 years of age, and is administered at least at or about 0.5 x 10⁶ CAR+ T cells/kg body weight of the subject but an amount that does not exceed at or about 0.5 x 10⁸ total CAR+ T cells. In some disclosures, the subject is younger than 18 years of age, and is administered at least at or about that is at least at or about 1.0 x 10⁶ CAR+ T cells/kg body weight of the subject but an amount that does not exceed at or about 1.0 x 10⁸ total CAR+ T cells.

In some disclosures, the subject is younger than 18 years of age and is less than 100 kilograms (kg) in body weight, the subject is administered from at or about 0.5 x 10⁶ CAR+ T cells/kg body weight of the subject to at or about 1.5 x 10⁶ CAR+ T cells/kg body weight of the subject. In some disclosures, the subject is younger than 18 years of age and is less than 100 kilograms (kg) in body weight, the subject is administered at or about 0.5 x 10⁶ CAR+ T cells/kg body weight of the subject. In some disclosures, the subject is younger than 18 years of age and is less than 100 kilograms (kg) in body weight, the subject is administered at or about 1.0 x 10⁶ CAR+ T cells/kg body weight of the subject.

In some disclosures, for subjects that are administered a body weight-based dose of cells, the dose of cells, such as CAR-expressing cells, comprises between at or about 2 x 10⁵ cells per kg body weight of the subject (cells/kg) and at or about 2 x 10⁶ cells/kg body weight of the subject, such as between at or about 4 x 10⁵ cells/kg body weight of the subject and at or about 1 x 10⁶ cells/kg body weight of the subject or between at or about 6 x 10⁵ cells/kg body weight of the subject and at or about 8 x 10⁵ cells/kg body weight of the subject. In some disclosures, the dose of cells comprises no more than 2 x 10⁵ cells per kilogram body weight of the subject (cells/kg), such as no more than at or about 3 x 10⁵ cells/kg, no more than at or about 4 x 10⁵ cells/kg, no more than at or about 5 x 10⁵ cells/kg, no more than at or about 6 x 10⁵ cells/kg, no more than at or about 7 x 10⁵ cells/kg, no more than at or about 8 x 10⁵ cells/kg, no more than at or about 9 x 10⁵ cells/kg, no more than at or about 1 x 10⁶ cells/kg, or no more than at or about 2 x 10⁶ cells/kg. In some disclosures, the dose of cells comprises at least or at least about or at or about 2 x 10⁵ cells per kilogram body weight of the subject (cells/kg), such as at least or at least about or at or about 3 x 10⁵ cells/kg, at least or at least about or at or about 4 x 10⁵ cells/kg, at least or at least about or at or about 5 x 10⁵ cells/kg, at least or at least about or at or about 6 x 10⁵ cells/kg, at least or at least about or at or about 7 x 10⁵ cells/kg, at least or at least about or at or about 8 x 10⁵ cells/kg, at least or at least about or at or about 9 x 10⁵ cells/kg, at least or at least about or at or about 1 x 10⁶ cells/kg, or at least or at least about or at or about 2 x 10⁶ cells/kg.

In some disclosures, the subject is younger than 18 years of age and is at or greater than 100 kilograms (kg) in body weight, the subject is administered from at or about 0.5 x 10⁸ CAR+ T cells to at or about 1.5 x 10⁸ CAR+ T cells. In some disclosures, the subject is younger than 18 years of age and is at or greater than 100 kilograms (kg) in body weight, the subject is administered from at or about 0.5 x 10⁸ CAR+ T cells to at or about 1.0 x 10⁸ CAR+ T cells. In some disclosures, the subject is younger than 18 years of age and is at or greater than 100 kilograms (kg) in body weight, the subject is administered at or about 0.5 x 10⁸ CAR+ T cells. In some disclosures, the subject is younger than 18 years of age and is at or greater than 100 kilograms (kg) in body weight, the subject is administered at or about 1.0 x 10⁸ CAR+ T cells.

In some of any disclosures, the methods and uses involve administering, to a subject that is younger than 18 years of age, a T cell composition comprising T cells expressing an anti-CD19 chimeric antigen receptor (CAR), wherein the T cell composition is administered in an amount selected from: (i) if the subject is less than 100 kilograms (kg) in body weight, at or about 0.05 x 10⁶ CAR+ T cells/kg body weight of the subject; and (ii) if the subject is at or greater than 100 kilograms (kg) in body weight, at or about 0.05 x 10⁸ CAR+ T cells.

In some of any disclosures, the methods and uses involve administering, to a subject that is younger than 18 years of age, a T cell composition comprising T cells expressing an anti-CD19 chimeric antigen receptor (CAR), wherein the T cell composition is administered in an amount selected from: (i) if the subject is less than 100 kilograms (kg) in body weight, at or about 0.15 x 10⁶ CAR+ T cells/kg body weight of the subject; and (ii) if the subject is at or greater than 100 kilograms (kg) in body weight, at or about 0.15 x 10⁸ CAR+ T cells.

In some of any disclosures, the methods and uses involve administering, to a subject that is younger than 18 years of age, a T cell composition comprising T cells expressing an anti-CD19 chimeric antigen receptor (CAR), wherein the T cell composition is administered in an amount selected from: (i) if the subject is less than 100 kilograms (kg) in body weight, at or about 0.3 x 10⁶ CAR+ T cells/kg body weight of the subject; and (ii) if the subject is at or greater than 100 kilograms (kg) in body weight, at or about 0.3 x 10⁸ CAR+ T cells.

In some of any disclosures, the methods and uses involve administering, to a subject that is younger than 18 years of age, a T cell composition comprising T cells expressing an anti-CD19 chimeric antigen receptor (CAR), wherein the T cell composition is administered in an amount selected from: (i) if the subject is less than 100 kilograms (kg) in body weight, at or about 0.5 x 10⁶ CAR+ T cells/kg body weight of the subject; and (ii) if the subject is at or greater than 100 kilograms (kg) in body weight, at or about 0.5 x 10⁸ CAR+ T cells.

In some of any disclosures, the methods and uses involve administering, to a subject that is younger than 18 years of age, a T cell composition comprising T cells expressing an anti-CD19 chimeric antigen receptor (CAR), wherein the T cell composition is administered in an amount selected from: (i) if the subject is less than 100 kilograms (kg) in body weight, at or about 0.75 x 10⁶ CAR+ T cells/kg body weight of the subject; and (ii) if the subject is at or greater than 100 kilograms (kg) in body weight, at or about 0.75 x 10⁸ CAR+ T cells.

In some of any disclosures, the methods and uses involve administering, to a subject that is younger than 18 years of age, a T cell composition comprising T cells expressing an anti-CD19 chimeric antigen receptor (CAR), wherein the T cell composition is administered in an amount selected from: (i) if the subject is less than 100 kilograms (kg) in body weight, at or about 1.0 x 10⁶ CAR+ T cells/kg body weight of the subject; and (ii) if the subject is at or greater than 100 kilograms (kg) in body weight, at or about 1.0 x 10⁸ CAR+ T cells.

In some disclosures, if the subject is younger than 18 years of age, the T cell composition is administered in an amount that is at least at or about 0.05 x 10⁶ CAR+ T cells/kg body weight of the subject but that does not exceed at or about 0.05 x 10⁸ total CAR+ T cells. In some of such disclosures, after administration of at or about 0.05 x 10⁶ CAR+ T cells/kg body weight of the subject but that does not exceed at or about 0.05 x 10⁸ total CAR+ T cells, the subject does not exhibit a response or an adverse event, e.g., toxicity such as CRS or NT, at or around day 28 after the initial administration, the subject receives another lymphodepleting therapy and the dose is escalated to at or about 0.1 x 10⁶ CAR+ T cells/kg body weight of the subject but that does not exceed at or about 0.1 x 10⁸ total CAR+ T cells. In some disclosures, if the subject is younger than 18 years of age, the T cell composition is administered in an amount that is at least at or about 0.1 x 10⁶ CAR+ T cells/kg body weight of the subject but that does not exceed at or about 0.1 x 10⁸ total CAR+ T cells. In some disclosures, if the subject is younger than 18 years of age, the T cell composition is administered in an amount that is at least at or about 0.15 x 10⁶ CAR+ T cells/kg body weight of the subject but that does not exceed at or about 0.15 x 10⁸ total CAR+ T cells. In some disclosures, if the subject is younger than 18 years of age, the T cell composition is administered in an amount that is at least at or about 0.3 x 10⁶ CAR+ T cells/kg body weight of the subject but that does not exceed at or about 0.3 x 10⁸ total CAR+ T cells. In some disclosures, if the subject is younger than 18 years of age, the T cell composition is administered in an amount that is at least at or about 0.5 x 10⁶ CAR+ T cells/kg body weight of the subject but that does not exceed at or about 0.5 x 10⁸ total CAR+ T cells. In some disclosures, if the subject is younger than 18 years of age, the T cell composition is administered in an amount that is at least at or about 0.75 x 10⁶ CAR+ T cells/kg body weight of the subject but that does not exceed at or about 0.75 x 10⁸ total CAR+ T cells. In some disclosures, if the subject is younger than 18 years of age, the T cell composition is administered in an amount that is at least at or about 1.0 x 10⁶ CAR+ T cells/kg body weight of the subject but that does not exceed at or about 1.0 x 10⁸ total CAR+ T cells.

**In** some disclosures, if the subject is between 18 and 25 years of age, inclusive, the T cell composition is administered in an amount from at or about 0.05 x 10⁸ CAR+ T cells to at or about 1.0 x 10⁸ CAR+ T cells. In some disclosures, if the subject is between 18 and 25 years of age, inclusive, the T cell composition is administered in an amount that is at or about 0.05 x 10⁸ CAR+ T cells. In some disclosures, if the subject is between 18 and 25 years of age, inclusive, the T cell composition is administered in an amount that is at or about 0.15 x 10⁸ CAR+ T cells. In some disclosures, if the subject is between 18 and 25 years of age, inclusive, the T cell composition is administered in an amount that is at or about 0.3 x 10⁸ CAR+ T cells. In some disclosures, if the subject is between 18 and 25 years of age, inclusive, the T cell composition is administered in an amount that is at or about 0.5 x 10⁸ CAR+ T cells. In some disclosures, if the subject is between 18 and 25 years of age, inclusive, the T cell composition is administered in an amount that is at or about 0.75 x 10⁸ CAR+ T cells. In some disclosures, if the subject is between 18 and 25 years of age, inclusive, the T cell composition is administered in an amount that is at or about 1.0 x 10⁸ CAR+ T cells.

**In** some instances, disclosed herein are methods and uses that involve administering, to a subject at or younger than 25 years of age and weighing 12 kg or more, a T cell composition comprising T cells expressing an anti-CD19 chimeric antigen receptor (CAR), wherein the T cell composition is administered in an amount selected from: (i) if the subject is younger than 18 years of age, at or about 0.05 x 10⁶ CAR+ T cells/kg body weight of the subject, but not exceeding at or about 0.05 x 10⁸ total CAR+ T cells; and (ii) if the subject is between 18 and 25 years of age, inclusive, at or about 0.05 x 10⁸ CAR+ T cells.

**In** some disclosures, the methods and uses involve administering, to a subject at or younger than 25 years of age and weighing 12 kg or more, a T cell composition comprising T cells expressing an anti-CD19 chimeric antigen receptor (CAR), wherein the T cell composition is administered in an amount selected from: (i) if the subject is younger than 18 years of age, at or about 0.15 x 10⁶ CAR+ T cells/kg body weight of the subject, but not exceeding at or about 0.15 x 10⁸ total CAR+ T cells; and (ii) if the subject is between 18 and 25 years of age, inclusive, at or about 0.15 x 10⁸ CAR+ T cells.

**In** some disclosures, the methods and uses involve administering, to a subject at or younger than 25 years of age and weighing 12 kg or more, a T cell composition comprising T cells expressing an anti-CD19 chimeric antigen receptor (CAR), wherein the T cell composition is administered in an amount selected from: (i) if the subject is younger than 18 years of age, at or about 0.3 x 10⁶ CAR+ T cells/kg body weight of the subject, but not exceeding at or about 0.3 x 10⁸ total CAR+ T cells; and (ii) if the subject is between 18 and 25 years of age, inclusive, at or about 0.3 x 10⁸ CAR+ T cells.

**In** some disclosures, the methods and uses involve administering, to a subject at or younger than 25 years of age and weighing 12 kg or more, a T cell composition comprising T cells expressing an anti-CD19 chimeric antigen receptor (CAR), wherein the T cell composition is administered in an amount selected from: (i) if the subject is younger than 18 years of age, at or about 0.5 x 10⁶ CAR+ T cells/kg body weight of the subject, but not exceeding at or about 0.5 x 10⁸ total CAR+ T cells; and (ii) if the subject is between 18 and 25 years of age, inclusive, at or about 0.5 x 10⁸ CAR+ T cells.

**In** some disclosures, the methods and uses involve administering, to a subject at or younger than 25 years of age and weighing 12 kg or more, a T cell composition comprising T cells expressing an anti-CD19 chimeric antigen receptor (CAR), wherein the T cell composition is administered in an amount selected from: (i) if the subject is younger than 18 years of age, at or about 0.75 x 10⁶ CAR+ T cells/kg body weight of the subject, but not exceeding at or about 0.75 x 10⁸ total CAR+ T cells; and (ii) if the subject is between 18 and 25 years of age, inclusive, at or about 0.75 x 10⁸ CAR+ T cells.

**In** some disclosures, the methods and uses involve administering, to a subject at or younger than 25 years of age and weighing 12 kg or more, a T cell composition comprising T cells expressing an anti-CD19 chimeric antigen receptor (CAR), wherein the T cell composition is administered in an amount selected from: (i) if the subject is younger than 18 years of age, at or about 1 x 10⁶ CAR+ T cells/kg body weight of the subject, but not exceeding at or about 1 x 10⁸ total CAR+ T cells; and (ii) if the subject is between 18 and 25 years of age, inclusive, at or about 1 x 10⁸ CAR+ T cells.

In some disclosures, the methods and uses involve administering, to a subject at or younger than 25 years of age and weighing 6 kg or more, a T cell composition comprising T cells expressing an anti-CD19 chimeric antigen receptor (CAR), wherein the T cell composition is administered in an amount selected from: (i) if the subject is younger than 18 years of age at or about 1 x 10⁶ CAR+ T cells/kg body weight of the subject, but not exceeding at or about 1 x 10⁸ total CAR+ T cells; and (ii) if the subject is between 18 and 25 years of age, inclusive, at or about 1.0 x 10⁸ CAR+ T cells.

In some disclosures, the methods and uses involve administering, to a subject younger than 18 years of age and weighing 12 kg or more, a composition comprising T cells expressing an anti-CD19 chimeric antigen receptor (CAR), wherein the composition is administered in an amount that is at or about 0.05 x 10⁶ CAR+ T cells/kg body weight of the subject to at or about 1.5 x 10⁶ CAR+ T cells/kg body weight of the subject, but that does not exceed at or about 1.5 x 10⁸ total CAR+ T cells.

In some disclosures, subjects younger than 18 years of age, are administered a dose that is determined based on particular volume of the composition, but up to a maximum or cap amount or number of cells. In some disclosures, the subject is younger than 18 years of age, and is administered a dose of cells, such as CAR-expressing cells, that contains a minimum volume of a composition containing the cells at a particular concentration, up to a maximum number of cells. In some disclosures, the minimum volume of cells to be administered is at least at or about 0.05 mL. In some disclosures, the minimum volume of cells to be administered is at least at or about 0.1 mL. In some disclosures, the minimum volume of cells to be administered is at least at or about 0.5 mL. In some disclosures, the minimum volume of cells to be administered is at least at or about 0.75 mL. In some disclosures, the minimum volume of cells to be administered is at least at or about 1.0 mL.

In some disclosures, the composition is administered at or about 0.05 mL in volume. In some disclosures, the composition is administered at or about 0.1 mL in volume. In some disclosures, the composition is administered at or about 0.5 mL in volume. In some disclosures, the composition is administered at or about 0.75 mL in volume. In some disclosures, the composition is administered at or about 1.0 mL in volume.

In some disclosures, two separate compositions of cells are administered, such as a composition comprising CD4+ CAR+ T cells and a composition comprising CD8+ CAR+ T cells, and each composition is administered with a minimum volume of at least at or about 0.5 mL, or at least at or about 1.0 mL.

In some disclosures, a total volume of at least 0.05 mL at a concentration of at or greater than 2.5 x 10⁶ cells/mL of the T cell composition is administered. In some disclosures, a total volume of at least 0.5 mL at a concentration of at or greater than 2.5 x 10⁶ cells/mL of the T cell composition is administered.

In some disclosures, the methods involve administering, to a subject at or younger than 25 years of age, a T cell composition comprising T cells expressing an anti-CD19 chimeric antigen receptor (CAR) at a concentration of at or greater than 0.25 x 10⁶ cells/mL, wherein the T cell composition is administered in an amount selected from: (i) if the subject is younger than 18 years of age, an amount from at or about 0.05 x 10⁸ CAR+ T cells to at or about 1.5 x 10⁸ CAR+ T cell in a volume of at least 0.5 mL; and (ii) if the subject is between 18 and 25 years of age, inclusive, from at or about 0.05 x 10⁸ CAR+ T cells to at or about 1.5 x 10⁸ CAR+ T cell.

In some disclosures, the methods and uses involve administering, to a subject at or younger than 25 years of age, a T cell composition comprising T cells expressing an anti-CD19 chimeric antigen receptor (CAR) at a concentration of at or greater than 2.5 x 10⁶ cells/mL, wherein the T cell composition is administered in an amount selected from: (i) if the subject is younger than 18 years of age, an amount not exceeding at or about 0.5 x 10⁸ total CAR+ T cells in a volume of at least 0.5 mL; and (ii) if the subject is between 18 and 25 years of age, inclusive, at or about 0.5 x 10⁸ CAR+ T cells.

In some disclosures, the methods and uses involve administering, to a subject at or younger than 25 years of age, a T cell composition comprising T cells expressing an anti-CD19 chimeric antigen receptor (CAR) at a concentration of at or greater than 2.5 x 10⁵ cells/mL, wherein the T cell composition is administered in an amount selected from: (i) if the subject is younger than 18 years of age, an amount not exceeding at or about 0.05 x 10⁸ total CAR+ T cells in a volume of at least 0.5 mL; and (ii) if the subject is between 18 and 25 years of age, inclusive, at or about 0.05 x 10⁸ CAR+ T cells.

In some disclosures, the methods and uses involve administering, to a subject at or younger than 25 years of age, a T cell composition comprising T cells expressing an anti-CD19 chimeric antigen receptor (CAR) at a concentration of at or greater than 2.5 x 10⁶ cells/mL, wherein the T cell composition is administered in an amount selected from: (i) if the subject is younger than 18 years of age, an amount not exceeding at or about 1 x 10⁸ total CAR+ T cells in a volume of at least 0.5 mL; and (ii) if the subject is between 18 and 25 years of age, inclusive, at or about 1.0 x 10⁸ CAR+ T cells.

In some of any disclosures, the total volume of the T cell composition administered is at least 0.05 mL. In some of any disclosures, the total volume of the T cell composition administered is at least 0.1 mL. In some of any disclosures, the total volume of the T cell composition administered is at least 0.15 mL. In some of any disclosures, the total volume of the T cell composition administered is at least 0.3 mL. In some of any disclosures, the total volume of the T cell composition administered is at least 0.5 mL. In some of any disclosures, the total volume of the T cell composition administered is at least 0.75 mL. In some of any disclosures, the total volume of the T cell composition administered is at least 1.0 mL.

In some of any disclosures, the concentration of the T cell composition is greater than at or about 0.5 x 10⁶ cells/mL or is or is about 0.5 x 10⁶ cells/mL. In some of any disclosures, the concentration of the T cell composition is greater than at or about 1 x 10⁶ cells/mL or is or is about 1 x 10⁶ cells/mL. In some of any disclosures, the concentration of the T cell composition is greater than at or about 1.5 x 10⁶ cells/mL or is or is about 1.5 x 10⁶ cells/mL. In some of any disclosures, the concentration of the T cell composition is greater than at or about 3 x 10⁶ cells/mL or is or is about 3 x 10⁶ cells/mL. In some of any disclosures, the concentration of the T cell composition is greater than at or about 5 x 10⁶ cells/mL or is or is about 5 x 10⁶ cells/mL. In some of any disclosures, the concentration of the T cell composition is greater than at or about 7.5 x 10⁶ cells/mL or is or is about 7.5 x 10⁶ cells/mL. In some of any disclosures, the concentration of the T cell composition is greater than at or about 10 x 10⁶ cells/mL or is or is about 10 x 10⁶ cells/mL. In some of any disclosures, the concentration of the T cell composition is greater than or greater than about 15 x 10⁶ cells/mL or is or is about 15 x 10⁶ cells/mL.

In some disclosures, the compositions are formulated at particular concentration of cells. In some disclosures, the concentration of cells in the composition is between about 10 x 10⁶ cells/mL and about 70 x 10⁶ cells/mL, between about 10 x 10⁶ cells/mL and about 50 x 10⁶ cells/mL, between about 10 x 10⁶ cells/mL and about 25 x 10⁶ cells/mL, between about 10 x 10⁶ cells/mL and about 15 x 10⁶ cells/mL, 15 x 10⁶ cells/mL and about 70 x 10⁶ cells/mL, between about 15 x 10⁶ cells/mL and about 50 x 10⁶ cells/mL, between about 15 x 10⁶ cells/mL and about 25 x 10⁶ cells/mL, between about 25 x 10⁶ cells/mL and about 70 x 10⁶ cells/mL, between about 25 x 10⁶ cells/mL and about 50 x 10⁶ cells/mL, and between about 50 x 10⁶ cells/mL and about 70 x 10⁶ cells/mL. In some disclosures, the concentration of the T cell composition is greater than at or about 5 x 10⁶ cells/mL or is or is about 5 x 10⁶ cells/mL. In some disclosures, the concentration of the T cell composition is greater than at or about 10 x 10⁶ cells/mL or is or is about 10 x 10⁶ cells/mL. In some disclosures, the concentration of the T cell composition is greater than or greater than about 15 x 10⁶ cells/mL or is or is about 15 x 10⁶ cells/mL. In some disclosures, a minimum volume of at least at or about 0.5 mL or at least at or about 1.0 mL of any of such compositions can be administered.

In some disclosures, subjects between 18 and 25 years of age, inclusive, are administered a dose that is a flat dose of cells or fixed dose of cells such that the dose of cells is not tied to or based on the body surface area or weight of a subject. In some disclosures, the subject is between 18 and 25 years of age, inclusive, and is administered from at or about 0.5 x 10⁸ CAR+ T cells to at or about 1.5 x 10⁸ CAR+ T cells. In some disclosures, the subject is between 18 and 25 years of age, inclusive, and is administered from at or about 0.5 x 10⁸ CAR+ T cells to at or about 1.0 x 10⁸ CAR+ T cells. In some disclosures, the subject is between 18 and 25 years of age, inclusive, and is administered at or about 0.5 x 10⁸ CAR+ T cells. In some disclosures, the subject is between 18 and 25 years of age, inclusive, and is administered at or about at or about 1.0 x 10⁸ CAR+ T cells.

In some disclosures, the maximum (or cap) amount or number of cells to be administered is an amount or number that is equivalent to a body weight-based dose for a subject with a particular body weight, such as between at or about 50 kg to at or about 150 kg. In some disclosures, the maximum (or cap) amount or number of cells to be administered is an amount or number that is equivalent to a body weight-based dose for a subject that has a body weight of 100 kg. In some disclosures, the maximum (or cap) amount or number of cells to be administered is an amount or number that is equivalent to a body weight-based dose for a subject that has a body weight of 150 kg. In some disclosures, the body weight-based dose is from at or about 0.05 x 10⁶ CAR+ T cells/kg body weight of the subject to at or about 1.5 x 10⁶ CAR+ T cells/kg body weight of the subject; or is from at or about 0.05 x 10⁶ CAR+ T cells/kg body weight of the subject to at or about 1.0 x 10⁶ CAR+ T cells/kg body weight of the subject; or is at or about 0.05 x 10⁶ CAR+ T cells/kg body weight of the subject; or is at or about 1.5 x 10⁶ CAR+ T cells/kg body weight of the subject. In some disclosures, the body weight-based dose is from at or about 0.5 x 10⁶ CAR+ T cells/kg body weight of the subject to at or about 1.5 x 10⁶ CAR+ T cells/kg body weight of the subject; or is from at or about 0.5 x 10⁶ CAR+ T cells/kg body weight of the subject to at or about 1.0 x 10⁶ CAR+ T cells/kg body weight of the subject; or is at or about 0.5 x 10⁶ CAR+ T cells/kg body weight of the subject; or is at or about 1.5 x 10⁶ CAR+ T cells/kg body weight of the subject.

**In** some disclosures, the flat or fixed amount or number of cells to be administered to a subject, for example, a subject that is between 18 and 25 years of age, inclusive, is an amount or number that is equivalent to a body weight-based dose for a subject with a particular body weight, such as between at or about 50 kg to at or about 150 kg. In some disclosures, the flat or fixed amount or number of cells to be administered to a subject, for example, a subject that is between 18 and 25 years of age, inclusive, is an amount or number that is equivalent to a body weight-based dose for a subject that has a body weight of 100 kg. In some disclosures, the flat or fixed amount or number of cells to be administered to a subject, for example, a subject that is between 18 and 25 years of age, inclusive, is an amount or number that is equivalent to a body weight-based dose for a subject that has a body weight of 150 kg. In some disclosures, the body weight-based dose is from at or about 0.05 x 10⁶ CAR+ T cells/kg body weight of the subject to at or about 1.5 x 10⁶ CAR+ T cells/kg body weight of the subject; or is from at or about 0.05 x 10⁶ CAR+ T cells/kg body weight of the subject to at or about 1.0 x 10⁶ CAR+ T cells/kg body weight of the subject; or is at or about 0.05 x 10⁶ CAR+ T cells/kg body weight of the subject; or is at or about 1.5 x 10⁶ CAR+ T cells/kg body weight of the subject. In some disclosures, the body weight-based dose is from at or about 0.5 x 10⁶ CAR+ T cells/kg body weight of the subject to at or about 1.5 x 10⁶ CAR+ T cells/kg body weight of the subject; or is from at or about 0.5 x 10⁶ CAR+ T cells/kg body weight of the subject to at or about 1.0 x 10⁶ CAR+ T cells/kg body weight of the subject; or is at or about 0.5 x 10⁶ CAR+ T cells/kg body weight of the subject; or is at or about 1.5 x 10⁶ CAR+ T cells/kg body weight of the subject.

**In** some disclosures, for subjects that are administered a flat or fixed number amount of cells, the dose of cells, such that the dose of cells is not tied to or based on the body surface area or weight of a subject. In some disclosures, for the subjects that are administered a flat or fixed dose of cells, the dose comprises a range of about one million to about 100 billion cells and/or that amount of cells per kilogram of body weight, such as, e.g., 1 million to about 50 billion cells (e.g., about 5 million cells, about 25 million cells, about 500 million cells, about 1 billion cells, about 5 billion cells, about 20 billion cells, about 30 billion cells, about 40 billion cells, or a range defined by any two of the foregoing values), such as about 10 million to about 100 billion cells (e.g., about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or a range defined by any two of the foregoing values), and in some cases about 100 million cells to about 50 billion cells (e.g., about 120 million cells, about 250 million cells, about 350 million cells, about 450 million cells, about 650 million cells, about 800 million cells, about 900 million cells, about 3 billion cells, about 30 billion cells, about 45 billion cells) or any value in between these ranges. In some disclosures, the amount or number of cells described herein is with reference to the amount or number of recombinant receptor-expressing cells, chimeric antigen receptor (CAR)-expressing cells, total T cells, or total peripheral blood mononuclear cells (PBMCs), or the amount or number of CD3+ T cells, CD4+ T cells, CD8+ T cells, and in some cases also recombinant receptor-expressing or CAR-expressing T cells.

In some disclosures, the flat or fixed dose of cells comprises from or from about 1 x 10⁵ to 5 x 10⁸ total CAR+ T cells, 1 x 10⁵ to 2.5 x 10⁸ total CAR+ T cells, 1 x 10⁵ to 1 x 10⁸ total CAR+ T cells, 1 x 10⁵ to 5 x 10⁷ total CAR+ T cells, 1 x 10⁵ to 2.5 x 10⁷ total CAR+ T cells, 1 x 10⁵ to 1 x 10⁷ total CAR+ T cells, 1 x 10⁵ to 5 x 10⁶ total CAR+ T cells, 1 x 10⁵ to 2.5 x 10⁶ total CAR+ T cells, 1 x 10⁵ to 1 x 10⁶ total CAR+ T cells, 1 x 10⁶ to 5 x 10⁸ total CAR+ T cells, 1 x 10⁶ to 2.5 x 10⁸ total CAR+ T cells, 1 x 10⁶ to 1 x 10⁸ total CAR+ T cells, 1 x 10⁶ to 5 x 10⁷ total CAR+ T cells, 1 x 10⁶ to 2.5 x 10⁷ total CAR+ T cells, 1 x 10⁶ to 1 x 10⁷ total CAR+ T cells, 1 x 10⁶ to 5 x 10⁶ total CAR+ T cells, 1 x 10⁶ to 2.5 x 10⁶ total CAR+ T cells, 2.5 x 10⁶ to 5 x 10⁸ total CAR+ T cells, 2.5 x 10⁶ to 2.5 x 10⁸ total CAR+ T cells, 2.5 x 10⁶ to 1 x 10⁸ total CAR+ T cells, 2.5 x 10⁶ to 5 x 10⁷ total CAR+ T cells, 2.5 x 10⁶ to 2.5 x 10⁷ total CAR+ T cells, 2.5 x 10⁶ to 1 x 10⁷ total CAR+ T cells, 2.5 x 10⁶ to 5 x 10⁶ total CAR+ T cells, 5 x 10⁶ to 5 x 10⁸ total CAR+ T cells, 5 x 10⁶ to 2.5 x 10⁸ total CAR+ T cells, 5 x 10⁶ to 1 x 10⁸ total CAR+ T cells, 5 x 10⁶ to 5 x 10⁷ total CAR+ T cells, 5 x 10⁶ to 2.5 x 10⁷ total CAR+ T cells, 5 x 10⁶ to 1 x 10⁷ total CAR+ T cells, 1 x 10⁷ to 5 x 10⁸ total CAR+ T cells, 1 x 10⁷ to 2.5 x 10⁸ total CAR+ T cells, 1 x 10⁷ to 1 x 10⁸ total CAR+ T cells, 1 x 10⁷ to 5 x 10⁷ total CAR+ T cells, 1 x 10⁷ to 2.5 x 10⁷ total CAR+ T cells, 2.5 x 10⁷ to 5 x 10⁸ total CAR+ T cells, 2.5 x 10⁷ to 2.5 x 10⁸ total CAR+ T cells, 2.5 x 10⁷ to 1 x 10⁸ total CAR+ T cells, 2.5 x 10⁷ to 5 x 10⁷ total CAR+ T cells, 5 x 10⁷ to 5 x 10⁸ total CAR+ T cells, 5 x 10⁷ to 2.5 x 10⁸ total CAR+ T cells, 5 x 10⁷ to 1 x 10⁸ total CAR+ T cells, 1 x 10⁸ to 5 x 10⁸ total CAR+ T cells, 1.5 x 10⁸ to 2.5 x 10⁸ total CAR+ T cells, or 2.5 x 10⁸ to 5 x 10⁸ total CAR+ T cells.

In some disclosures, the dose of genetically engineered cells comprises at least or at least about 1 x 10⁵ CAR+ T cells, at least or at least about 2.5 x 10⁵ CAR+ T cells, at least or at least about 5 x 10⁵ CAR+ T cells, at least or at least about 1 x 10⁶ CAR+ T cells, at least or at least about 2.5 x 10⁶ CAR+ T cells, at least or at least about 5 x 10⁶ CAR+ T cells, at least or at least about 1 x 10⁷ CAR+ T cells, at least or at least about 2.5 x 10⁷ CAR+ T cells, at least or at least about 5 x 10⁷ CAR+ T cells, at least or at least about 1 x 10⁸ CAR+ T cells, at least or at least about 2.5 x 10⁸ CAR+ T cells, or at least or at least about 5 x 10⁸ CAR+ T cells.

In some disclosures, the T cells of the dose include CD4⁺ T cells, CD8⁺ T cells or CD4⁺ and CD8⁺ T cells.

In some disclosures, for example, the CD8⁺ T cells of the dose, including in a dose including CD4⁺ and CD8⁺ T cells, includes between about 1 x 10⁶ and 5 x 10⁸ total CAR+ CD8⁺cells, e.g., in the range of about 5 x 10⁶ to 1 x 10⁸ such cells, such cells 5 x 10⁶, 1 x 10⁷, 1.5 x 10⁷, 3 x 10⁷, 2.5 x 10⁷, 5 x 10⁷, 7.5 x 10⁷, 1 x 10³, 1.5 x 10³, or 5 x 10⁸ total such cells, or the range between any two of the foregoing values. In some disclosures, the subject is administered multiple doses, and each of the doses or the total dose can be within any of the foregoing values. In some disclosures, the dose of cells comprises the administration of from or from about 1 x 10⁷ to 0.75 x 10⁸ total CAR+ CD8⁺ T cells, 1 x 10⁷ to 2.5 x 10⁷ total CAR+ CD8⁺ T cells, from or from about 1 x 10⁷ to 0.75 x 10⁸ total CAR+ CD8⁺ T cells, each inclusive. In some disclosures, the dose of cells comprises the administration of or about 5 x 10⁶, 1 x 10⁷, 1.5 x 10⁷, 3 x 10⁷, 2.5 x 10⁷ 5 x 10⁷, 7.5 x 10⁷, 1 x 10³, 1.5 x 10³, or 5 x 10⁸ total CAR+ CD8⁺ T cells. In some disclosures, the dose of T cells comprises: at or about 5 x 10⁷ CAR+ T cells or at or about 2.5 x 10⁷ CAR+ CD8⁺ T cells. In some disclosures, the dose of T cells comprises: at or about 1 x 10⁸ CAR+ T cells or at or about 5 x 10⁷ CAR+ CD8⁺ T cells. In some disclosures, the dose of T cells comprises: at or about 1.5 x 10⁸ CAR+ T cells or at or about 0.75 x 10⁸ CAR+ CD8⁺ T cells.

In some disclosures, the dose of cells, e.g., recombinant receptor-expressing T cells, is administered to the subject as a single dose or is administered only one time within a period of two weeks, one month, three months, six months, 1 year or more.

In the context of adoptive cell therapy, administration of a given "dose" encompasses administration of the given amount or number of cells as a single composition and/or single uninterrupted administration, e.g., as a single injection or continuous infusion, and also encompasses administration of the given amount or number of cells as a split dose or as a plurality of compositions, provided in multiple individual compositions or infusions, over a specified period of time, such as over no more than 3 days. Thus, in some contexts, the dose is a single or continuous administration of the specified number of cells, given or initiated at a single point in time. In some contexts, however, the dose is administered in multiple injections or infusions over a period of no more than three days, such as once a day for three days or for two days or by multiple infusions over a single day period.

Thus, in some disclosures, the cells of the dose are administered in a single pharmaceutical composition. In some disclosures, the cells of the dose are administered in a plurality of compositions, collectively containing the cells of the dose.

In some disclosures, the term "split dose" refers to a dose that is split so that it is administered over more than one day. This type of dosing is encompassed by the present methods and is considered to be a single dose.

Thus, the dose of cells may be administered as a split dose, e.g., a split dose administered over time. For example, in some disclosures, the dose may be administered to the subject over 2 days or over 3 days. Exemplary methods for split dosing include administering 25% of the dose on the first day and administering the remaining 75% of the dose on the second day. In other disclosures, 33% of the dose may be administered on the first day and the remaining 67% administered on the second day. In some disclosures, 10% of the dose is administered on the first day, 30% of the dose is administered on the second day, and 60% of the dose is administered on the third day. In some disclosures, the split dose is not spread over more than 3 days.

In some disclosures, cells of the dose may be administered by administration of a plurality of compositions or solutions, such as a first and a second, optionally more, each containing some cells of the dose. In some disclosures, the plurality of compositions, each containing a different population and/or sub-types of cells, are administered separately or independently, optionally within a certain period of time. For example, the populations or sub-types of cells can include CD8⁺ and CD4⁺ T cells, respectively, and/or CD8⁺- and CD4⁺-enriched populations, respectively, e.g., CD4⁺ and/or CD8⁺ T cells each individually including cells genetically engineered to express the recombinant receptor. In some disclosures, the administration of the dose comprises administration of a first composition comprising a dose of CD8⁺ T cells or a dose of CD4⁺ T cells and administration of a second composition comprising the other of the dose of CD4⁺ T cells and the CD8⁺ T cells.

In some disclosures, the administration of the composition or dose, e.g., administration of the plurality of cell compositions, involves administration of the cell compositions separately. In some disclosures, the separate administrations are carried out simultaneously, or sequentially, in any order. In some disclosures, the dose comprises a first composition and a second composition, and the first composition and second composition are administered 0 to 12 hours apart, 0 to 6 hours apart or 0 to 2 hours apart. In some disclosures, the initiation of administration of the first composition and the initiation of administration of the second composition are carried out no more than 2 hours, no more than 1 hour, or no more than 30 minutes apart, no more than 15 minutes, no more than 10 minutes or no more than 5 minutes apart. In some disclosures, the initiation and/or completion of administration of the first composition and the completion and/or initiation of administration of the second composition are carried out no more than 2 hours, no more than 1 hour, or no more than 30 minutes apart, no more than 15 minutes, no more than 10 minutes or no more than 5 minutes apart.

In some composition, the first composition, e.g., first composition of the dose, comprises CD4⁺ T cells. In some composition, the first composition, e.g., first composition of the dose, comprises CD8⁺ T cells. In some disclosures, the first composition is administered prior to the second composition.

In some disclosures, the dose or composition of cells includes a defined or target ratio of CD4⁺ cells expressing a recombinant receptor to CD8⁺ cells expressing a recombinant receptor and/or of CD4⁺ cells to CD8⁺ cells, which ratio optionally is approximately 1:1 or is between approximately 1:3 and approximately 3:1, such as approximately 1:1. In some disclosures, the administration of a composition or dose with the target or desired ratio of different cell populations (such as CD4⁺:CD8⁺ ratio or CAR⁺CD4⁺:CAR⁺CD8⁺ ratio, e.g., 1:1) involves the administration of a cell composition containing one of the populations and then administration of a separate cell composition comprising the other of the populations, where the administration is at or approximately at the target or desired ratio. In some disclosures, administration of a dose or composition of cells at a defined ratio leads to improved expansion, persistence and/or antitumor activity of the T cell therapy.

In some disclosures, the subject receives multiple doses, e.g., two or more doses or multiple consecutive doses, of the cells. In some disclosures, two doses are administered to a subject. In some disclosures, the subject receives the consecutive dose, e.g., second dose, is administered approximately 4, 5, 6, 7, 8, 9, 10, 11, 12, 13, 14, 15, 16, 17, 18, 19, 20 or 21 days after the first dose. In some disclosures, multiple consecutive doses are administered following the first dose, such that an additional dose or doses are administered following administration of the consecutive dose. In some disclosures, the number of cells administered to the subject in the additional dose is the same as or similar to the first dose and/or consecutive dose. In some disclosures, the additional dose or doses are larger than prior doses.

In some disclosures, the size of the first and/or consecutive dose is determined based on one or more criteria such as response of the subject to prior treatment, e.g. chemotherapy, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

In some disclosures, the time between the administration of the first dose and the administration of the consecutive dose is about 9 to about 35 days, about 14 to about 28 days, or 15 to 27 days. In some disclosures, the administration of the consecutive dose is at a time point more than about 14 days after and less than about 28 days after the administration of the first dose. In some disclosures, the time between the first and consecutive dose is about 21 days. In some disclosures, an additional dose or doses, e.g. consecutive doses, are administered following administration of the consecutive dose. In some disclosures, the additional consecutive dose or doses are administered at least about 14 and less than about 28 days following administration of a prior dose. In some disclosures, the additional dose is administered less than about 14 days following the prior dose, for example, 4, 5, 6, 7, 8, 9, 10, 11, 12, or 13 days after the prior dose. In some disclosures, no dose is administered less than about 14 days following the prior dose and/or no dose is administered more than about 28 days after the prior dose.

In some disclosures, the dose of cells, e.g., recombinant receptor-expressing cells, comprises two doses (e.g., a double dose), comprising a first dose of the T cells and a consecutive dose of the T cells, wherein one or both of the first dose and the second dose comprises administration of the split dose of T cells.

In some disclosures, the dose of cells is generally large enough to be effective in reducing disease burden.

In some disclosures, the cells are administered at a desired dosage, which in some disclosures includes a desired dose or number of cells or cell type(s) and/or a desired ratio of cell types. Thus, the dosage of cells in some disclosures is based on a total number of cells (or number per kg body weight) and a desired ratio of the individual populations or sub-types, such as the CD4⁺ to CD8⁺ ratio. In some disclosures, the dosage of cells is based on a desired total number (or number per kg of body weight) of cells in the individual populations or of individual cell types. In some disclosures, the dosage is based on a combination of such features, such as a desired number of total cells, desired ratio, and desired total number of cells in the individual populations.

In some disclosures, the populations or sub-types of cells, such as CD8⁺ and CD4⁺ T cells, are administered at or within a tolerated difference of a desired dose of total cells, such as a desired dose of T cells. In some disclosures, the desired dose is a desired number of cells or a desired number of cells per unit of body weight of the subject to whom the cells are administered, e.g., cells/kg. In some disclosures, the desired dose is at or above a minimum number of cells or minimum number of cells per unit of body weight. In some disclosures, among the total cells, administered at the desired dose, the individual populations or sub-types are present at or near a desired output ratio (such as CD4⁺ to CD8⁺ ratio), e.g., within a certain tolerated difference or error of such a ratio.

In some disclosures, the cells are administered at or within a tolerated difference of a desired dose of one or more of the individual populations or sub-types of cells, such as a desired dose of CD4⁺ cells and/or a desired dose of CD8⁺ cells. In some disclosures, the desired dose is a desired number of cells of the sub-type or population, or a desired number of such cells per unit of body weight of the subject to whom the cells are administered, e.g., cells/kg. In some disclosures, the desired dose is at or above a minimum number of cells of the population or sub-type, or minimum number of cells of the population or sub-type per unit of body weight.

Thus, in some disclosures, the dosage is based on a desired fixed dose of total cells and a desired ratio, and/or based on a desired fixed dose of one or more, e.g., each, of the individual sub-types or sub-populations. Thus, in some disclosures, the dosage is based on a desired fixed or minimum dose of T cells and a desired ratio of CD4⁺ to CD8⁺ cells, and/or is based on a desired fixed or minimum dose of CD4⁺ and/or CD8⁺ cells.

In some disclosures, the cells are administered at or within a tolerated range of a desired output ratio of multiple cell populations or sub-types, such as CD4⁺ and CD8⁺ cells or sub-types. In some disclosures, the desired ratio can be a specific ratio or can be a range of ratios. for example, in some disclosures, the desired ratio (e.g., ratio of CD4⁺ to CD8⁺ cells) is between at or about 5:1 and at or about 5:1 (or greater than about 1:5 and less than about 5:1), or between at or about 1:3 and at or about 3:1 (or greater than about 1:3 and less than about 3:1), such as between at or about 2:1 and at or about 1:5 (or greater than about 1:5 and less than about 2:1, such as at or about 5:1, 4.5:1, 4:1, 3.5:1, 3:1, 2.5:1, 2:1, 1.9:1, 1.8:1, 1.7:1, 1.6:1, 1.5:1, 1.4:1, 1.3:1, 1.2:1, 1.1:1, 1:1, 1:1.1, 1:1.2, 1:1.3, 1:1.4, 1:1.5, 1:1.6, 1:1.7, 1:1.8, 1:1.9: 1:2, 1:2.5, 1:3, 1:3.5, 1:4, 1:4.5, or 1:5. In some disclosures, the tolerated difference is within about 1%, about 2%, about 3%, about 4% about 5%, about 10%, about 15%, about 20%, about 25%, about 30%, about 35%, about 40%, about 45%, about 50% of the desired ratio, including any value in between these ranges.

In particular disclosures, the numbers and/or concentrations of cells refer to the number of recombinant receptor (e.g., CAR)-expressing cells. In other disclosures, the numbers and/or concentrations of cells refer to the number or concentration of all cells, T cells, or peripheral blood mononuclear cells (PBMCs) administered.

In some disclosures, the size of the dose is determined based on one or more criteria such as response of the subject to prior treatment, e.g. chemotherapy, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

In some disclosures, the methods and uses also include administering one or more additional doses of cells expressing a chimeric antigen receptor (CAR) and/or lymphodepleting therapy and/or a combination therapy, and/or one or more steps of the methods are repeated. In some disclosures, the one or more additional dose is the same as the initial dose. In some disclosures, the one or more additional dose is different from the initial dose, e.g., higher, such as 2-fold, 3-fold, 4-fold, 5-fold, 6-fold, 7-fold, 8-fold, 9-fold or 10-fold or more higher than the initial dose, or lower, such as e.g., higher, such as 2-fold, 3-fold, 4-fold, 5-fold, 6-fold, 7-fold, 8-fold, 9-fold or 10-fold or more lower than the initial dose. In some disclosures, administration of one or more additional doses is determined based on response of the subject to the initial treatment or any prior treatment, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

### C. Response, Efficacy and Survival

In some disclosures, the administration according to the methods and uses can result in treatment of the disease or condition in the subject. In some disclosures, the administration according to the methods and uses can result in the subject exhibiting a response (e.g., a clinical response) to treatment. In some disclosures, the administration according to the methods and uses can treat the subject despite the subject having become resistant to another therapy.

In some disclosures, at least 30%, at least 35%, at least 40% or at least 50% of subjects treated according to the method achieve complete remission (CR); and/or at least about 40%, at least about 50%, at least about 60% or at least about 70% of the subjects treated according to the method achieve an objective response (OR). In some disclosures, at least or about at least 50% of subjects, at least or about at least 60% of the subjects, at least or about at least 70% of the subjects, at least or about at least 80% of the subjects or at least or about at least 90% of the subjects treated according to the method and uses achieve CR and/or achieve an objective response (OR).

In some disclosures, criteria assessed for response to treatment includes overall response rate (ORR; also known in some cases as objective response rate), complete response (CR; also known in some cases as complete response), duration of response (DOR), progression-free survival (PFS), overall survival (OS), minimal residual disease (MRD) negative rate, relapse-free survival (RFS), event-free survival (EFS), rate of hematopoietic stem cell transplant (HSCT) after administration of the engineered cells, and/or pharmacokinetic parameters such as C_{max,} T_{max,} and area under the curve (AUC).

In some disclosures, the measure or criterion assessed is the overall response rate (ORR). In some disclosures, ORR for certain subjects can be described as the total number of subjects achieving a complete response (CR) or CR with incomplete blood count recovery (CRi), after a period of time after administration of the engineered cells, e.g., on day 28 and/or day 56. In some disclosures, the ORR of the subjects treated according to the method and uses is at least at or about 50%, at least at or about 60%, at or about 70%, at least at or about 80%, at least at or about 85%, at least at or about 90% or at least at or about 95%.

In some disclosures, the measure or criterion assessed is the minimal residual disease (MRD) negative rate. In some disclosures, MRD negative response is described as the total number of subjects achieving a MRD negative response, after a period of time after administration of the engineered cells, e.g., on day 28 and/or day 56. In some disclosures, the MRD negative response rate of the subjects treated according to the method and uses is at least at or about 50%, at least at or about 60%, at or about 70%, at least at or about 80%, at least at or about 85%, at least at or about 90% or at least at or about 95%. In some disclosures, MRD can be measured using any criteria described herein. In some disclosures, a MRD relapse after treatment can be described as an MRD detection by validated assay at a frequency of 1 x 10⁻⁴ or greater in BM cells following an initial MRD negative (less than 1 x 10⁻⁴) complete response (CR) or complete response with incomplete blood count recovery (CRi).

In some disclosures, MRD negative rate can be described as the proportion of subjects achieving a CR or CRi with an MRD negative BM on day 28, confirmed on day 56, divided by the number of total subjects included in the analysis. In some disclosures, the first assessment is performed at 28 days after administration of the CAR-expressing cells. In some disclosures, if the subject cannot be assessed for CR/CRi due to hypoplastic marrow, a repeat marrow examination is performed when there is evidence of hematopoietic recovery such that MRD negative remission can be assessed. In some cases, for the best overall disease response to be considered MRD negative CR or CRi, there is no clinical evidence of relapse as assessed by peripheral blood, bone marrow, cerebrospinal fluid and extramedullary disease assessment (where applicable) with no MRD detected in the bone marrow (below the level of detection, <0.01% by a validated assay). In some disclosures, the MRD result is confirmed at a minimum of 4 weeks (28 days) after the initial achievement of MRD negative CR or CRi. In some disclosures, additional assessments also are included in the same evaluation. In some disclosures, the measure or criterion assessed is the overall response rate (ORR). In some disclosures, ORR for certain subjects can be described as the total number of subjects achieving a complete response (CR) or a partial response (PR), after a period of time after administration of the engineered cells, e.g., on day 28 and/or day 56. In some disclosures, the ORR of the subjects treated according to the method and uses is at least at or about 50%, at least at or about 60%, at or about 70%, at least at or about 80%, at least at or about 85%, at least at or about 90% or at least at or about 95%.

In some disclosures, the measure or criterion assessed is duration of response (DOR). In some disclosures, DOR can be described as the time from first response until progressive disease (PD), disease relapse, or death from any cause, whichever occurs first. In some disclosures, the DOR is measured until up to approximately 2, 3, or 4 years after administration of the engineered cells. In some disclosures, the response is durable for at least 3, 4, 5, 6, 7, 8, 9, 10, 11, 12, 18, 24, 36 or 48 months after initiation of administration of the engineered cells, in at least at or about 20%, at least at or about 30%, at or about 40%, at least at or about 50%, at least at or about 60%, at or about 70%, at least at or about 80%, at least at or about 85%, at least at or about 90% or at least at or about 95%, of the subjects that were treated according to the method and uses.

In some disclosures, the measure or criterion assessed is relapse-free survival (RFS). In some disclosures, RFS can be described as the time from first response to documentation of progressive disease (PD), disease relapse, or death due to any cause, whichever occurs first. In some disclosures, the RFS is measured until up to approximately 2, 3, or 4 years after administration of the engineered cells. In some disclosures, the RFS is at least 3, 4, 5, 6, 7, 8, 9, 10, 11, 12, 18, 24, 36 or 48 months after initiation of administration of the engineered cells, in at least at or about 20%, at least at or about 30%, at or about 40%, at least at or about 50%, at least at or about 60%, at or about 70%, at least at or about 80%, at least at or about 85%, at least at or about 90% or at least at or about 95%, of the subjects that were treated according to the method and uses.

In some disclosures, the measure or criterion assessed is event-free survival (EFS). In some disclosures, EFS can be described as the time from initiation of administration of infusion to progressive disease (PD), disease relapse, start of a new anticancer therapy, or death from any cause, whichever occurs first. In some disclosures, the EFS is measured until up to approximately 2, 3, or 4 years after administration of the engineered cells. In some disclosures, the EFS is at least 3, 4, 5, 6, 7, 8, 9, 10, 11, 12, 18, 24, 36 or 48 months after initiation of administration of the engineered cells, in at least at or about 20%, at least at or about 30%, at or about 40%, at least at or about 50%, at least at or about 60%, at or about 70%, at least at or about 80%, at or about 85%, at least at or about 90% or at least at or about 95%, of the subjects that were treated according to the method and uses.

In some disclosures, the measure or criterion assessed is overall survival (OS). In some disclosures, OS can be described as the time from initiation of administration of the engineered cells to time of death due to any cause. In some disclosures, the OS is measured until up to approximately 2, 3, or 4 years after administration of the engineered cells. In some disclosures, the OS is at least 3, 4, 5, 6, 7, 8, 9, 10, 11, 12, 18, 24, 36 or 48 months after initiation of administration of the engineered cells, in at least at or about 20%, at least at or about 30%, at or about 40%, at least at or about 50%, at least at or about 60%, at or about 70%, at least at or about 80%, at least at or about 85%, at least at or about 90% or at least at or about 95%, of the subjects that were treated according to the method and uses.

In some disclosures, the measure or criterion assessed is the minimal residual disease (MRD) negative rate. In some disclosures, MRD negative response in some subjects can be described as the percentage of B-ALL subjects achieving a CR or CRi and a negative MRD bone marrow, after a period of time after administration of the engineered cells. In some disclosures, the MRD negative response is measured until up to approximately 2, 3, or 4 years after administration of the engineered cells. In some disclosures, the MRD negative response rate of the subjects treated according to the method and uses is at least at or about 20%, at least at or about 30%, at or about 40%, at least at or about 50%, at least at or about 60%, at or about 70%, at least at or about 80%, at least at or about 85%, at least at or about 90% or at least at or about 95%.

In some disclosures, the measure or criterion assessed is the rate of hematopoietic stem cell transplant (HSCT) after response to administration of engineered cells. In some disclosures, rate of HSCT can be described as the percentage of subjects who achieve a response after the administration of engineered cells, and then proceed to receive HSCT. In some disclosures, the rate of HSCT is measured until up to approximately 2, 3, or 4 years after administration of the engineered cells. In some disclosures, the rate of HSCT after response of the subjects treated according to the method and uses is less than at or about 80%, less than at or about 70%, at or about 60%, less than at or about 50%, less than at or about 40%, at or about 30%, less than at or about 20% or less than at or about 10%.

In some disclosures, at least 40% or at least 50% of subjects treated according to the methods disclosed herein achieve complete remission (CR; also known in some cases as complete response), exhibit progression-free survival (PFS) and/or overall survival (OS) of greater than at or about 3 months, 6 months or 12 months or greater than 13 months or approximately 14 months; on average, subjects treated according to the method exhibit a median PFS or OS of greater than at or about 6 months, 12 months, or 18 months; and/or the subject exhibits PFS or OS following therapy for at least at or about 6, 12, 18 or more months or longer.

In some disclosures, at least 35%, at least 40%, at least 50%, at least 55%, at least 60%, at least 65%, at least 70%, or at least 75% or more of the subjects treated according to the disclosed methods, and/or with the disclosed articles of manufacture or compositions, achieve a complete response (CR). In some disclosures, at least 50%, at least 60%, at least 70%, at least 80%, or at least 90% of the subjects treated according to the disclosed methods, and/or with the disclosed articles of manufacture or compositions, achieve an objective response (OR). In some disclosures, at least 60%, at least 70%, at least 80%, at least 90%, at least 95% or more of the subjects treated according to the disclosed methods, and/or with the disclosed articles of manufacture or compositions, achieve a CR or OR by one month, by two months or by three months.

In some disclosures, by three months, four months, five months, six months or more after initiation of administration of the cell therapy, at least 60%, at least 70%, at least 80%, at least 90%, at least 95% or more of the subjects treated according to the disclosed methods, and/or with the disclosed articles of manufacture or compositions, remain in response, such as remain in CR or OR. In some disclosures, such response, such as CR or OR, is durable for at least three months, four months, five months, six months, seven months, eight months or nine months, such as in at least or about at least 60%, at least 70%, at least 80%, at least 90%, at least 95% or more of the subjects treated according to the disclosed methods or in such subjects who achieve a CR by one month or by three months. In some disclosures, at least 60%, at least 70%, at least 80%, at least 90%, at least 95% or more of the subjects treated according to the disclosed methods, and/or with the disclosed articles of manufacture or compositions, or such subjects who achieve a CR by one month or by three months, survive or survive without progression for greater than or greater than about three months, four months, five months, six months, seven months, eight months or nine months.

In some disclosures, the resulting response observed in such subjects by the treatment in accord with the disclosed methods, and/or with the disclosed articles of manufacture or compositions, is associated with or results in a low risk of any toxicity or a low risk of severe toxicity in a majority of the subjects treated. In some disclosures, greater than or greater than about 30%, 35%, 40%, 50%, 55%, 60% or more of the subjects treated according to the disclosed methods and/or with the disclosed articles of manufacture or compositions do not exhibit any grade of CRS or any grade of neurotoxicity (NT). In some disclosures, greater than or greater than about 50%, 60%, 70%, 80% or more of the subjects treated according to the disclosed methods and/or with the disclosed articles of manufacture or compositions do not exhibit severe CRS or grade 3 or higher CRS. In some disclosures, greater than or greater than about 50%, 60%, 70%, 80% or more of the subjects treated according to the disclosed methods, and/or with the disclosed articles of manufacture or compositions, do not exhibit severe neurotoxicity or grade 3 or higher neurotoxicity, such as grade 4 or 5 neurotoxicity.

In some disclosures, at least at or about 45%, 50%, 60%, 65%, 70%, 75%, 80%, 85%, 90%, or 95% of subjects treated according to the method and/or with the disclosed articles of manufacture or compositions do not exhibit early onset CRS or neurotoxicity and/or do not exhibit onset of CRS earlier than 1 day, 2 days, 3 days or 4 days following initiation of the administration. In some disclosures, at least at or about 45%, 50%, 60%, 65%, 70%, 75%, 80%, 85%, 90%, or 95% of subjects treated according to the methods, and/or with the disclosed articles of manufacture or compositions, do not exhibit onset of neurotoxicity earlier than 3 days, 4 days, 5 days, six days or 7 days following initiation of the administration. In some disclosures, the median onset of neurotoxicity among subjects treated according to the methods, and/or with the disclosed articles of manufacture or compositions, is at or after the median peak of, or median time to resolution of, CRS in subjects treated according to the method. In some cases, the median onset of neurotoxicity among subjects treated according to the method is greater than at or about 8, 9, 10, or 11 days.

In some disclosures, the dose is within a range in which a correlation is observed (optionally a linear relationship) between the number of such cells (e.g., of total CAR⁺ T cells or of CD8⁺ and/or CD4⁺ CAR⁺ T cells) and one or more outcomes indicative of therapeutic response, or duration thereof (e.g., likelihood of achieving a remission, a complete remission, and/or a particular duration of remission) and/or duration of any of the foregoing. In some disclosures, it is found that the higher dose of cells administered can result in greater response without or without substantially impacting or affecting the incidence or risk of toxicity (e.g. CRS or neurotoxicity), or degree of incidence or risk of toxicity, in the subject e.g. severe CRS or severe neurotoxicity.

In some disclosures, the methods can achieve a high or a particular rate of response (such as a rate of response among a population as assessed after a certain period post-administration, such as three months or six months), e.g., ORR (such as a 6-month or 3-month ORR) of 40% or more, 45% or more, 50% or more, 55% or more, 60% or more, 65% or more, 70% or more, 75% or 80% or 81%, 82%, 83%, 84% or 85% or more and CR rate (such as a 6-month or 3-month CR rate) of 30% or more, 35% or more, 40% or more, 45% or more, 50% or more, 55% or more, 60% or more, 65% or more, 70% or more, 71%, 72%, 73% or more or approximately 75% or more, which also is durable such as for a particular period of time or at least a particular period of time, e.g., is sustained for more than 1, 3 or 6 months or more or 9 months or more after initiation of therapy. In some disclosures, such rates of response and durability are received following only a single administration or dose of such therapy. Treatment of such subjects by the disclosed methods, and/or with the disclosed articles of manufacture or compositions, in some disclosures, also result in the subjects achieving the high rate of response, yet not exhibiting higher incidence of developing toxicities, such as neurotoxicity or CRS, even at a higher cell dosage. In some disclosures, about or greater than 50%, 55% or 60% of subjects achieving such responses do not develop any grade of toxicity, such as any grade of CRS and/or neurotoxicity.

In some disclosures, response rates in subjects, such as subjects with ALL or NHL, are based on the Lugano criteria. (Cheson et al., (2014) JCO 32(27):3059-3067; Johnson et al., (2015) Radiology 2:323-338; Cheson, B.D. (2015) Chin Clin Oncol 4(1):5). In some disclosures, response assessment utilizes any of clinical, hematologic, and/or molecular methods. In some disclosures, response assessed using the Lugano criteria involves the use of positron emission tomography (PET)-computed tomography (CT) and/or CT as appropriate. PET-CT evaluations may further comprise the use of fluorodeoxyglucose (FDG) for FDG-avid lymphomas. In some disclosures, response assessed using the Lugano criteria involves the use of positron emission tomography (PET)-computed tomography (CT) and/or CT as appropriate for imaging evaluation. FDG-avid lymphomas include Hodgkin lymphoma (HL) and certain non-Hodgkin lymphomas (NHL), including diffuse large B cellular lymphoma (DLBCL), marginal zone NHL with an aggressive transformation, and FDG-avid nodal lymphomas (essentially all histologic types except: chronic lymphocytic leukemia (CLL), small lymphocytic lymphoma, lymphoplasmacytic lymphoma / Waldenström macroglobulinaemia, and mycosis fungoides). In some cases, for non-FDG-avid histologies, CT is the preferred imaging method. In some disclosures, the post-treatment scans are taken as long as possible after administration of treatment. In some disclosures the post-treatment scans are taken a minimum of 3 weeks after therapy, such as 3 weeks, 4 weeks, 5 weeks, 6 weeks, 7 weeks, 8 weeks, 9 weeks, 10 weeks, 11 weeks, 12, weeks or more after administration of treatment. In some disclosures, where PET-CT will be used to assess response in FDG-avid histologies, a 5-point scale may be used. In some respects, the 5-point scale comprises the following criteria: 1, no uptake above background; 2, uptake ≤ mediastinum; 3, uptake > mediastinum but ≤ liver; 4, uptake moderately > liver; 5, uptake markedly higher than liver and/or new lesions; X, new areas of uptake unlikely to be related to lymphoma.

In some disclosures, where PET-CT will be used to assess response in FDG-avid histologies, a 5-point scale, such as the Deauville five-point scale (Deauville 5ps), may be used for evaluation or staging. The Deauville score is based on visual interpretation of fluorodeoxyglucose (FDG) uptake, visualized by PET/CT scans, of each lesion, compared to two reference organs, the mediastinum (*i.e.,* blood pool) and the liver. One assessment (initial staging) is made prior to treatment and a second round of FDG PET/CT scans is used to evaluate residual masses (in comparison to the FDG update in the reference organs) during and/or after treatment. The scale ranges from 1 to 5, where 1 is best and 5 is the worst. Each FDG-avid (or previously FDG-avid) lesion is rated independently. In some respects, the 5-point scale comprises the following criteria: 1, no uptake above background; 2, uptake ≤ mediastinum; 3, uptake > mediastinum but ≤ liver; 4, uptake moderately > liver; 5, uptake markedly higher than liver (e.g., maximum standard uptake value (SUV_{MAX} >2x liver; 5a) and/or new lesion (on response evaluation) that is possibly related to lymphoma (5b); X, new areas of uptake unlikely to be related to lymphoma.

A Deauville score of 1 or 2 is considered to represent complete metabolic response (CMR) at interim and end of treatment. A Deauville score of 3 also represents CMR, but interpretation of score 3 depends on the timing of the assessment, the clinical context and the treatment. A Deauville score of 4 or 5 at interim is considered to represent partial metabolic response. However, a Deauville score of 4 or 5 at the end of treatment represents residual metabolic disease, if the uptake has reduced from baseline; no metabolic response (NMR) if there is no change in uptake from baseline; and progressive metabolic disease (PMD) if there in an increase in uptake from baseline and/or there are new lesions. At interim and end of treatment, NMR or PMD indicates treatment failure.

In some disclosures, a complete response as described using the Lugano criteria involves a complete metabolic response and a complete radiologic response at various measureable sites. In some disclosures, these sites include lymph nodes and extralymphatic sites, wherein a CR is described as a score of 1, 2, or 3 with or without a residual mass on the 5-point scale, when PET-CT is used. In some disclosures, in Waldeyer's ring or extranodal sites with high physiologic uptake or with activation within spleen or marrow (e.g., with chemotherapy or myeloid colony-stimulating factors), uptake may be greater than normal mediastinum and/or liver. In this circumstance, complete metabolic response may be inferred if uptake at sites of initial involvement is no greater than surrounding normal tissue even if the tissue has high physiologic uptake. In some disclosures, response is assessed in the lymph nodes using CT, wherein a CR is described as no extralymphatic sites of disease and target nodes/nodal masses must regress to ≤ 1.5 cm in longest transverse diameter of a lesion (LDi). Further sites of assessment include the bone marrow wherein PET-CT-based assessment should indicate a lack of evidence of FDG-avid disease in marrow and a CT-based assessment should indicate a normal morphology, which if indeterminate should be IHC negative. Further sites may include assessment of organ enlargement, which should regress to normal. In some disclosures, non-measured lesions and new lesions are assessed, which in the case of CR should be absent (Cheson et al., (2014) JCO 32(27):3059-3067; Johnson et al., (2015) Radiology 2:323-338; Cheson, B.D. (2015) Chin Clin Oncol 4(1):5).

In some disclosures, a partial response (PR; also known in some cases as partial remission) as described using the Lugano criteria involves a partial metabolic and/or radiological response at various measureable sites. In some disclosures, these sites include lymph nodes and extralymphatic sites, wherein a PR is described as a score of 4 or 5 with reduced uptake compared with baseline and residual mass(es) of any size, when PET-CT is used. At interim, such findings can indicate responding disease. At the end of treatment, such findings can indicate residual disease. In some disclosures, response is assessed in the lymph nodes using CT, wherein a PR is described as ≥50% decrease in SPD of up to 6 target measureable nodes and extranodal sites. if a lesion is too small to measure on CT, 5 mm × 5 mm is assigned as the default value; if the lesion is no longer visible, the value is 0 mm × 0 mm; for a node >5 mm × 5 mm, but smaller than normal, actual measurements are used for calculation. Further sites of assessment include the bone marrow wherein PET-CT-based assessment should indicate residual uptake higher than uptake in normal marrow but reduced compared with baseline (diffuse uptake compatible with reactive changes from chemotherapy allowed). In some disclosures, if there are persistent focal changes in the marrow in the context of a nodal response, consideration should be given to further evaluation with MRI or biopsy, or an interval scan. In some disclosures, further sites may include assessment of organ enlargement, where the spleen must have regressed by >50% in length beyond normal. In some disclosures, non-measured lesions and new lesions are assessed, which in the case of PR should be absent/normal, regressed, but no increase. No response/stable disease (SD) or progressive disease (PD) can also be measured using PET-CT and/or CT based assessments (Cheson et al., (2014) JCO 32(27):3059-3067; Johnson et al., (2015) Radiology 2:323-338; Cheson, B.D. (2015) Chin Clin Oncol 4(1):5).

In some respects, progression-free survival (PFS) is described as the length of time during and after the treatment of a disease, such as cancer, that a subject lives with the disease but it does not get worse. In some disclosures, objective response (OR) is described as a measurable response. In some disclosures, objective response rate (ORR; also known in some cases as overall response rate) is described as the proportion of patients who achieved CR or PR. In some disclosures, overall survival (OS) is described as the length of time from either the date of diagnosis or the start of treatment for a disease, such as cancer, that subjects diagnosed with the disease are still alive. In some disclosures, event-free survival (EFS) is described as the length of time after treatment for a cancer ends that the subject remains free of certain complications or events that the treatment was intended to prevent or delay. These events may include the return of the cancer or the onset of certain symptoms, such as bone pain from cancer that has spread to the bone, or death.

In some disclosures, the measure of duration of response (DOR) includes the time from documentation of tumor response to disease progression. In some disclosures, the parameter for assessing response can include durable response, e.g., response that persists after a period of time from initiation of therapy. In some disclosures, durable response is indicated by the response rate at approximately 1, 2, 3, 4, 5, 6, 7, 8, 9, 10, 11, 12, 18 or 24 months after initiation of therapy. In some disclosures, the response is durable for greater than 3 months or greater than 6 months.

In some disclosures, the RECIST criteria is used to determine objective tumor response; in some disclosures, in solid tumors (Eisenhauer et al., European Journal of Cancer 45 (2009) 228-247.) In some disclosures, the RECIST criteria is used to determine objective tumor response for target lesions. In some respects, a complete response as determined using RECIST criteria is described as the disappearance of all target lesions and any pathological lymph nodes (whether target or non-target) must have reduction in short axis to <10 mm. In other disclosures, a partial response as determined using RECIST criteria is described as at least a 30% decrease in the sum of diameters of target lesions, taking as reference the baseline sum diameters. In other disclosures, progressive disease (PD) is described as at least a 20% increase in the sum of diameters of target lesions, taking as reference the smallest sum on study (this includes the baseline sum if that is the smallest on study). In addition to the relative increase of 20%, the sum must also demonstrate an absolute increase of at least 5 mm (in some disclosures the appearance of one or more new lesions is also considered progression). In other disclosures, stable disease (SD) is described as neither sufficient shrinkage to qualify for PR nor sufficient increase to qualify for PD, taking as reference the smallest sum diameters while on study.

In some disclosures, the administration in accord with the disclosed methods, and/or with the disclosed articles of manufacture or compositions, generally reduces or prevents the expansion or burden of the disease or condition in the subject. For example, where the disease or condition is a tumor, the methods generally reduce tumor size, bulk, metastasis, percentage of blasts in the bone marrow or molecularly detectable cancer and/or improve prognosis or survival or other symptom associated with tumor burden.

Disease burden can encompass a total number of cells of the disease in the subject or in an organ, tissue, or bodily fluid of the subject, such as the organ or tissue of the tumor or another location, e.g., which would indicate metastasis. For example, tumor cells may be detected and/or quantified in the blood or bone marrow in the context of certain hematological malignancies. Disease burden can include, in some disclosures, the mass of a tumor, the number or extent of metastases and/or the percentage of blast cells present in the bone marrow.

In some disclosures, a subject has leukemia. The extent of disease burden can be determined by assessment of residual leukemia in blood or bone marrow.

In some disclosures, response rates in subjects, such as subjects with CLL, are based on the International Workshop on Chronic Lymphocytic Leukemia (IWCLL) response criteria (Hallek, et al., Blood 2008, Jun 15; 111(12): 5446-5456). In some disclosures, these criteria are described as follows: complete remission (CR; also known in some cases as complete response), which in some disclosures requires the absence of peripheral blood clonal lymphocytes by immunophenotyping, absence of lymphadenopathy, absence of hepatomegaly or splenomegaly, absence of constitutional symptoms and satisfactory blood counts; complete remission with incomplete marrow recovery (CRi), which in some disclosures is described as CR above, but without normal blood counts; partial remission (PR; also known in some cases as partial response), which in some disclosures is described as ≥ 50% fall in lymphocyte count, ≥ 50% reduction in lymphadenopathy or ≥ 50% reduction in liver or spleen, together with improvement in peripheral blood counts; progressive disease (PD), which in some disclosures is described as ≥ 50% rise in lymphocyte count to > 5 x10⁹/L, ≥ 50% increase in lymphadenopathy, ≥ 50% increase in liver or spleen size, Richter's transformation, or new cytopenias due to CLL; and stable disease, which in some disclosures is described as not meeting criteria for CR, CRi, PR or PD.

In some disclosures, the subjects exhibits a CR or OR if, within 1 month of the administration of the dose of cells, lymph nodes in the subject are less than at or about 20 mm in size, less than at or about 10 mm in size or less than at or about 10 mm in size.

In some disclosures, an index clone of the CLL is not detected in the bone marrow of the subject (or in the bone marrow of greater than 50%, 60%, 70%, 80%, 90% or more of the subjects treated according to the methods. In some disclosures, an index clone of the CLL is assessed by IgH deep sequencing. In some disclosures, the index clone is not detected at a time that is at or about or at least at or about 1, 2, 3, 4, 5, 6, 12, 18 or 24 months following the administration of the cells.

In some disclosures, a subject exhibits morphologic disease if there are greater than or equal to 5% blasts in the bone marrow, for example, as detected by light microscopy, such as greater than or equal to 10% blasts in the bone marrow, greater than or equal to 20% blasts in the bone marrow, greater than or equal to 30% blasts in the bone marrow, greater than or equal to 40% blasts in the bone marrow or greater than or equal to 50% blasts in the bone marrow. In some disclosures, a subject exhibits complete or clinical remission if there are less than 5% blasts in the bone marrow.

In some disclosures, a subject has leukemia. The extent of disease burden can be determined by assessment of residual leukemia in blood or bone marrow.

In some disclosures, a subject exhibits morphologic disease if there are greater than or equal to 5% blasts in the bone marrow, for example, as detected by light microscopy, such as greater than or equal to 10% blasts in the bone marrow, greater than or equal to 20% blasts in the bone marrow, greater than or equal to 30% blasts in the bone marrow, greater than or equal to 40% blasts in the bone marrow or greater than or equal to 50% blasts in the bone marrow. In some disclosures, a subject exhibits complete or clinical remission if there are less than 5% blasts in the bone marrow.

In some disclosures, a subject may exhibit complete remission, but a small proportion of morphologically undetectable (by light microscopy techniques) residual leukemic cells are present. A subject is said to exhibit minimum residual disease (MRD) if the subject exhibits less than 5% blasts in the bone marrow and exhibits molecularly detectable cancer. In some disclosures, molecularly detectable cancer can be assessed using any of a variety of molecular techniques that permit sensitive detection of a small number of cells. In some disclosures, MRD is detected by a validated assay at a frequency of 1 x 10⁻⁴ or greater in BM cells. In some disclosures, such techniques include PCR assays, which can determine unique Ig/T-cell receptor gene rearrangements or fusion transcripts produced by chromosome translocations. In some disclosures, flow cytometry can be used to identify cancer cell based on leukemia-specific immunophenotypes. In some disclosures, molecular detection of cancer can detect as few as 1 leukemia cell in 100,000 normal cells. In some disclosures, a subject exhibits MRD that is molecularly detectable if at least or greater than 1 leukemia cell in 100,000 cells is detected, such as by PCR or flow cytometry. In some disclosures, the disease burden of a subject is molecularly undetectable or MRD⁻, such that, in some cases, no leukemia cells are able to be detected in the subject using PCR or flow cytometry techniques.

In some disclosures, an index clone of the leukemia, is not detected in the bone marrow of the subject (or in the bone marrow of greater than 50%, 60%, 70%, 80%, 90% or more of the subjects treated according to the methods. In some disclosures, an index clone of the leukemia, is assessed by IGH deep sequencing. In some disclosures, the index clone is not detected at a time that is at or about or at least at or about 1, 2, 3, 4, 5, 6, 12, 18 or 24 months following the administration of the cells.

In some disclosures, MRD is detected by flow cytometry. Flow cytometry can be used to monitor bone marrow and peripheral blood samples for cancer cells. In particular disclosures, flow cytometry is used to detect or monitor the presence of cancer cells in bone marrow. In some disclosures, multiparameter immunological detection by flow cytometry is used to detect cancer cells (see for example, Coustan-Smith et al., (1998) Lancet 351:550-554). In some disclosures, multiparameter immunological detection by mass cytometry is used to detect cancer cells. In some disclosures, 1, 2, 3, 4, 5, 6, 7, 8, 9, 10, 11, 12, 13, 14, 15, 20, 25, 30, 35, 40, 45 or 50 parameters can be used to detect cancer cells. The antigens used for detection are selected based on the cancer being detected (Foon and Todd (1986) Blood 68:1-31).

In some disclosures, bone marrow is harvested by bone marrow aspirates or bone marrow biopsies, and lymphocytes are isolated for analysis. Monoclonal and/or polyclonal antibodies conjugated to a fluorochrome (e.g., fluorescein isothiocyanate (FITC), phycoerythrin, peridinin chlorophyll protein, or biotin) can be used to detect epitopes, such as terminal deoxynucleotidyl transferase (TdT), CD3, CD10, CD11c, CD13, CD14, CD33, CD19, CD20, CD21, CD22, CD23, CD34, CD45, CD56, CD79b, IgM, and/or KORSA3544, on isolated lymphocytes. Labeled cells can then be detected using flow cytometry, such as multiparameter flow cytometry, or mass cytometry, to detect multiple epitopes.

In some disclosures, the presence of MRD in ALL can be determined based on having less than 5% lymphoblasts by morphology, and/or MRD detected by a validated assay at a frequency of 1 x10⁻⁴ or greater in bone marrow cells after two lines of therapy. In some disclosures, the MRD response rate can be determined from the proportion of subjects achieving a CR or CRi with no MRD detected in bone marrow (e.g., <0.01 % by a validated assay), up to 24 months after administration of the engineered cell composition, over the number of subjects available for the analysis.

Lymphoid cells can be identified and gated based on a light-scatter dot plot and then secondarily gated to identify cell populations expressing the immunophenotypic features of interest. Exemplary epitopes are set forth in **Table 2** below. Other immunologic classification of leukemias and lymphomas include those described in, for example, Foon and Todd Blood (1986) 68(1): 1-31, and Mrozek et al., Hematol Oncol Clin North Am. 2009 October; 23(5): 991-v. In some disclosures, flow cytometric assessment of MRD can be achieved by quantifying live lymphocytes bearing one or more ALL or NHL phenotypes. In some disclosures, flow cytometric assessment of MRD can be achieved by quantifying live lymphocytes bearing one or more CLL immunophenotypes (e.g., low forward/side scatter; CD3^{neg}; CD5⁺; CD14^{neg}; CD19⁺; CD23⁺; CD45⁺; CD56^{neg}).

| **Table 2. Exemplary Immnunophenotype and Cytogentics Characteristics** | | |
|---|---|---|
| **Disease** | **Immunophenotype** | **Cytogenetics** |
| Acute Lymphoblastic Leukemia (ALL) | B cell lineage: | cryptic t(12;21) |
| | CD19+; CD22+; CD79a+; TdT+; CD10+/-; cytoplasmic Ig +/-; surface Ig +/- | t(1;19)(q23;p13) |
| | | Philadelphia chromosome (t(9;22)(q34;q11)) |
| | | t(4;11)(q21;q23) |
| | | t(8;14)(q24;q32) |
| | | t(11;14)(p13;q11) |
| | T cell lineage: CD2+; CD3+; CD4+; CD5+; CD7+; CD8+; TdT+ | t(v;11q23.3) |
| | | hyperdiploidy |
| | | hypodiploidy |
| | | recurrent genetic abnormalities |
| | | Philadelphia chromosome-like |
| B-Cell Acute Lymphoblastic Leukemia/Lymphoma | CD19+; CD22+; CD79a+; TdT+; CD10+/-; cytoplasmic Ig +/-; surface Ig +/- | 1(9:22)(q34.1;,q11.2) |
| | | t(v;11q23.3) |
| | | t(12;21)(p13.2;q22.1) |
| | | t(5;14)(q31.1;q32.3) |
| | | t(1;19)(q23;p13.3) |
| Chronic Lymphocytic Leukemia (CLL) | Pan-B+; CD5+; CD23+: CD79b/CD22 weak; FMC7-; sIg weak | Trisomy12 |
| | | del(13)(q14.3) |
| | | del 11q22-q23 |
| | | del 17p13 (p53) |
| | | t(11;14)(q13;q32) BCL1/IgH rearrangement |
| | | t(14;19)(q32;q13) |
| | | IgH deletion (14q32) |
| | | del(6q) |
| | | +8q24 |
| | | +3 |
| | | +18 |
| | | del 6q21 |
| Small lymphocytic lymphoma (SLL) | Pan-B+; CD5+; CD23+: CD10-; sIgM+ faint | del(6)(q21-23) |
| Lymphoplasmacytic lymphoma | Pan-B+; CD5-; CD10-; cyIgM+ | t(9;14)(p13;q32) PAX5/IgH |
| Follicle center cell lymphoma | Pan-B+; CD10+/-; CD5-; sIg+ | t(14;18)(q32;q21) / BCL2 Rearr |
| Diffuse large cell lymphoma | CD19+; CD22+; CD10-/+; SIg+ | t(14;18) and p53 mutations |
| | | t(3;V)(q27;V)/ BCL6 Rearr |
| | | variants c-MYC Rearr |
| Burkitt's lymphoma (BL) | Pan-B+; TdT-; CD10+; CD5-; sIgM+ | t(8;14)(q24;q32) or variants / c-MYC Rearr |
| Burkitt-like lymphoma | Pan-B+; TdT-; CD10-/+ CD5-; sIg+ | t(8;14) or variants |
| | | t(8;14)+ t(14;18) |
| Mantle cell lymphoma | Pan-B +; CD5+; CD23-: CD10-/+; sIgM+ bright | t(11;14)(q13;q32) / BCL1 Rearr |
| Marginal zone B-cell lymphoma (MZBCL) | pan-B+; CD5-/+; CD10-; CD23-; CD11c+/-; cyIg + (40% of the | t(11;18)(q21;q21) / PI2 / MLT fusion: Extra-nodal low-grade MALT lymphoma; indolent disease |
| | cells), sIgM+ bright; sIgD- | t(1;14)(p21;q32): Extra-nodal MALT lymphoma |
| | | del(7)(q22-31): Splenic MZBCL /+3q: Nodal, extra-nodal and splenic MZBCL |
| +: positive in >90% of the cases | | |
| +/-: positive in more than 50% of the cases | | |
| -/+: positive in less than 50% of cases | | |
| -: positive in <10% of the cases | | |
| Pan-B markers: e.g., CD19, CD20, CD79a | | |
| sIG: surface immunoglobulins | | |
| cyIg: cytoplasmic immunoglobulins | | |

In some disclosures, deep sequencing of the immunoglobulin heavy chain (IGH) locus of harvested B cells can be used to detect minimal residual disease (MRD). Clonal presence of a particular IgG rearrangement can provide a marker to detect the presence of B cell malignancies, such as ALL or NHL and/or residual presence of malignant cells thereof. In some disclosures cells such as a population containing or suspected of containing B cells are harvested and isolated from blood. In some disclosures, cells are harvested and isolated from bone marrow, e.g., from bone marrow aspirates or bone marrow biopsies and/or from other biological samples. In some disclosures, polymerase chain reaction (PCR) amplification of the complementarity determining region 3 (CDR3) is achieved using primers to highly conserved sequences within the V and J regions of the gene locus, which may be used to identify clonal populations of cells for purposes of assessing minimal residual disease. Other methods for detecting clonal populations, such as single cell sequencing approaches, including those providing information regarding number of cells of a particular lineage and/or expressing a particular variable chain such as variable heavy chain or binding site thereof, such as a clonal population, may be used. In some disclosures, the IGH DNA is amplified using a degenerate primers or primers recognizing regions of variable chains shared among different cell clones, such as those recognizing consensus V and degenerate consensus J region of the IGH sequence. An exemplary sequence of the V region is ACACGGCCTCGTGTATTACTGT (SEQ ID NO: 57). An exemplary degenerate consensus sequence of the J region is ACCTGAGGAGACGGTGACC (SEQ ID NO: 58).

The PCR product or sequencing result in some disclosures is specific to the rearranged allele and serves as a clonal marker for MRD detection. Following PCR amplification of the CDR3 region, PCR products can be sequenced to yield patient-specific oligonucleotides constructed as probes for allele-specific PCR for sensitive detection of MRD following treatment of B-cell malignancies with CAR-T cell therapy, e.g. CD19 CAR- T cell therapy. In disclosures where a PCR product is not generated using the consensus primers, V region family-specific primers for the framework region 1 can be used instead.

In some disclosures, persistence of PCR-detectable tumor cells such as cells of the B cell malignancy such as ALL or NHL, such as detectable IGH sequences corresponding to the malignant or clonal IGH sequences, after treatment is associated with increased risk of relapse. In some disclosures, patients who are negative for malignant IGH sequences following treatment (in some disclosures, even in the context of other criteria indicating progressive disease or only a partial response, such as persistence of enlarged lymph nodes or other criteria that may in some contexts be associated with disease or lack of complete response) may be deemed to have increased likelihood of PFS or to enter into CR or durable CR or prolonged survival, compared to patients with persistent malignant IGH sequences. In some disclosures, such prognostic and staging determinations are particularly relevant for treatments in which clearance of malignant cells is observed within a short period of time following administration of the therapy, e.g., in comparison to resolution of other clinical symptoms such as lymph node size or other staging criteria. For example, in some such disclosures, absence of detectable IGH or minimal residual disease in a sample such as the bone marrow may be a preferred readout for response or likelihood of response or durability thereof, as compared to other available staging or prognostic approaches. In some disclosures, results from MRD, e.g., IGH deep sequencing information, may inform further intervention or lack thereof. For example, the methods and other disclosures in some contexts provide that a subject deemed negative for malignant IGH may in some disclosures be not further treated or not be further administered a dose of the therapy provided, or that the subject be administered a lower or reduced dose. Conversely, it may be provided or specified that a subject exhibiting MRD via IGH deep sequencing be further treated, e.g., with the therapy initially administered at a similar or higher dose or with a further treatment. In some disclosures, the disease or condition persists following administration of the first dose and/or administration of the first dose is not sufficient to eradicate the disease or condition in the subject.

In some disclosures, the method reduces the burden of the disease or condition, e.g., number of tumor cells, size of tumor, duration of patient survival or event-free survival, to a greater degree and/or for a greater period of time as compared to the reduction that would be observed with a comparable method using an alternative dosing regimen, such as one in which the subject receives one or more alternative therapeutic agents and/or one in which the subject does not receive a dose of cells and/or a lymphodepleting agent in accord with the disclosed methods, and/or with the disclosed articles of manufacture or compositions. In some disclosures, the burden of a disease or condition in the subject is detected, assessed, or measured. Disease burden may be detected in some disclosures by detecting the total number of disease or disease-associated cells, e.g., tumor cells, in the subject, or in an organ, tissue, or bodily fluid of the subject, such as blood or serum. In some disclosures, survival of the subject, survival within a certain time period, extent of survival, presence or duration of event-free or symptom-free survival, or relapse-free survival, is assessed. In some disclosures, any symptom of the disease or condition is assessed. In some disclosures, the measure of disease or condition burden is specified.

In some disclosures, the event-free survival rate or overall survival rate of the subject is improved by the methods, as compared with other methods, for example, methods in which the subject receives one or more alternative therapeutic agents and/or one in which the subject does not receive a dose of cells and/or a lymphodepleting agent in accord with the disclosed methods, and/or with the disclosed articles of manufacture or compositions. For example, in some disclosures, event-free survival rate or probability for subjects treated by the methods at 6 months following the dose is greater than about 40%, greater than about 50%, greater than about 60%, greater than about 70%, greater than about 80%, greater than about 90%, or greater than about 95%. In some disclosures, overall survival rate is greater than about 40%, greater than about 50%, greater than about 60%, greater than about 70%, greater than about 80%, greater than about 90%, or greater than about 95%. In some disclosures, the subject treated with the methods exhibits event-free survival, relapse-free survival, or survival to at least 6 months, or at least 1, 2, 3, 4, 5, 6, 7, 8, 9, or 10 years. In some disclosures, the time to progression is improved, such as a time to progression of greater than at or about 6 months, or at least 1, 2, 3, 4, 5, 6, 7, 8, 9, or 10 years.

In some disclosures, following treatment by the methods or uses, the probability of relapse is reduced as compared to other methods, for example, methods in which the subject receives one or more alternative therapeutic agents and/or one in which the subject does not receive a dose of cells and/or a lymphodepleting agent in accord with the disclosed methods, and/or with the disclosed articles of manufacture or compositions. For example, in some disclosures, the probability of relapse at 6 months following the first dose is less than about 80%, less than about 70%, less than about 60%, less than about 50%, less than about 40%, less than about 30%, less than about 20%, or less than about 10%.

In some disclosures, the measure or criterion assessed is a pharmacokinetic parameter or a parameter that is associated with exposure. In some disclosures, the pharmacokinetic parameter is the maximum concentration (Cₘₐₓ) of engineered cells present in the subject, after a period of time after administration of the engineered cells. In some disclosures, the Cₘₐₓ is measured until up to approximately 1, 2, 3, 4, 5, 6, 7, 8, 9, 10, 11, 12, 18 or 24 months after administration of the engineered cells. In some disclosures, the pharmacokinetic parameter is the time to maximum concentration (Tₘₐₓ) of engineered cells present in the subject. In some disclosures, the Tₘₐₓ is measured until up to approximately 1, 2, 3, 4, 5, 6, 7, 8, 9, 10, 11, 12, 18 or 24 months after administration of the engineered cells. In some disclosures, the pharmacokinetic parameter is the area under the curve (AUC) of engineered cells present in the subject. In some disclosures, the AUC is measured until up to approximately 1, 2, 3, 4, 5, 6, 7, 8, 9, 10, 11, 12, 18 or 24 months after administration of the engineered cells.

In some cases, pharmacokinetics can be assessed by measuring such parameters as the maximum (peak) plasma concentration (Cₘₐₓ), the peak time (*i.e*. when maximum plasma concentration (Cₘₐₓ) occurs; Tₘₐₓ), the minimum plasma concentration (*i.e.* the minimum plasma concentration between doses of a therapeutic agent, e.g., CAR+ T cells; Cₘᵢₙ), the elimination half-life (T_{1/2}) and area under the curve (*i.e.* the area under the curve generated by plotting time versus plasma concentration of the therapeutic agent CAR+ T cells; AUC), following administration. The concentration of a particular therapeutic agent, e.g., CAR+ T cells, in the plasma following administration can be measured using any method known in the art suitable for assessing concentrations of the therapeutic agents, e.g., CAR+ T cells, in samples of blood, or any methods described herein. For example, nucleic acid-based methods, such as quantitative PCR (qPCR) or flow cytometry-based methods, or other assays, such as an immunoassay, ELISA, or chromatography/mass spectrometry-based assays can be used.

In some disclosures, the pharmacokinetics (PK) of administered cells, e.g., CAR⁺ T cell composition, are determined to assess the availability, e.g., bioavailability, of the administered cells. In some disclosures, the determined pharmacokinetic parameters of the administered cells include maximum (peak) plasma concentrations (Cₘₐₓ), such as Cₘₐₓ of CD3⁺ CAR⁺ cells, CD4⁺ CAR⁺ cells and or CD8⁺ CAR⁺ T cells; the time point at which Cₘₐₓ is achieved (Tₘₐₓ), such as the Tₘₐₓ of CD3⁺ CAR⁺ cells, CD4⁺ CAR⁺ cells and or CD8⁺ CAR⁺ T cells, and or area under the curve (AUC), such as the AUC₀₋₂₈, of CD3⁺ CAR⁺ cells, CD4⁺ CAR⁺ cells and or CD8⁺ CAR⁺ T cells. In some disclosures, the pharmacokinetic parameter is peak CD3⁺ CAR⁺ T cell concentration (Cₘₐₓ CD3⁺ CAR⁺ T cells), or CD8⁺ CAR⁺ T cell concentration (Cₘₐₓ CD8⁺ CAR⁺ T cells). In some disclosures, the pharmacokinetic parameter is AUC₀₋₂₈, of CD3⁺ CAR⁺ T cells, (AUC0-28 CD3⁺ CAR⁺ T cells), or AUC₀₋₂₈, of CD8⁺ CAR⁺ T cells, (AUC₀₋₂₈ CD8⁺ CAR⁺ T cells),

In some disclosures, "exposure" can refer to the body exposure of a therapeutic agent, e.g., CAR+ T cells in the plasma (blood or serum) after administration of the therapeutic agent over a certain period of time. In some disclosures exposure can be set forth as the area under the therapeutic agent concentration-time curve (AUC) as determined by pharmacokinetic analysis after administration of a dose of the therapeutic agent, e.g., CAR+ T cells. In some cases, the AUC is expressed in cells*days/µL, for cells administered in cell therapy, or in corresponding units thereof. In some disclosures, the AUC is measured as an average AUC in a patient population, such as a sample patient population, e.g., the average AUC from one or more patient(s). In some disclosures, systemic exposure refers to the area under the curve (AUC) within a certain period of time, e.g., from day 0 to day 1, 2, 3, 4, 5, 6, 7, 8, 9, 10, 11, 12, 13, 14, 21, 28 days or more, or week 1, 2, 3, 4, 5, 6, 7, 8, 9, 10, 11, 12, 13, 14, 15 or more, or month 1, 2, 3, 4, 5, 6, 7, 8, 9, 10, 11, 12, 18, 24, 48 or more. In some disclosures, the AUC is measured as an AUC from day 0 to day 28 (AUC₀₋₂₈) after administration of the therapeutic agent, e.g., CAR+ T cells, including all measured data and data extrapolated from measured pharmacokinetic (PK) parameters, such as an average AUC from a patient population, such as a sample patient population. In some disclosures, to determine exposure over time, e.g., AUC for a certain period of time, such as AUC₀₋₂₈, a therapeutic agent concentration-time curve is generated, using multiple measurements or assessment of parameters, e.g., cell concentrations, over time, e.g., measurements taken every 1, 2, 3, 4, 5, 6, 7, 8, 9, 10, 11, 12, 13, 14, 21 or 28 days or more.

In some disclosures, the presence and/or amount of cells expressing the recombinant receptor (*e.g*., CAR-expressing cells administered for T cell based therapy) in the subject following the administration of the T cells and before, during and/or after the administration of the therapy is detected. In some disclosures, nucleic acid-based methods, such as quantitative PCR (qPCR), is used to assess the quantity of cells expressing the recombinant receptor (*e.g*., CAR-expressing cells administered for T cell based therapy) in the blood or serum or organ or tissue sample (*e.g.,* disease site, *e.g.,* tumor sample) of the subject. In some disclosures, persistence is quantified as copies of DNA or plasmid encoding the receptor, *e.g.,* CAR, per microgram of DNA, or as the number of receptor-expressing, *e.g.,* CAR-expressing, cells per microliter of the sample, *e.g.,* of blood or serum, or per total number of peripheral blood mononuclear cells (PBMCs) or white blood cells or T cells per microliter of the sample. In some disclosures, the primers or probe used for qPCR or other nucleic acid-based methods are specific for binding, recognizing and/or amplifying nucleic acids encoding the recombinant receptor, and/or other components or elements of the plasmid and/or vector, including regulatory elements, e.g., promoters, transcriptional and/or post-transcriptional regulatory elements or response elements, or markers, e.g., surrogate markers. In some disclosures, the primers can be specific for regulatory elements, such as the woodchuck hepatitis virus post-transcriptional regulatory element (WPRE).

In some disclosures, the cells are detected in the subject at or at least at 4, 14, 15, 27, or 28 days following the administration of the T cells, e.g., CAR-expressing T cells. In some disclosures, the cells are detected at or at least at 2, 4, or 6 weeks following, or 3, 6, or 12, 18, or 24, or 30 or 36 months, or 1, 2, 3, 4, 5, or more years, following the administration of the T cells, e.g., CAR-expressing T cells.

In some disclosures, the peak levels and/or AUC are assessed and/or the sample is obtained from the subject at a time that is at least 8 days, 9 days, 10 days, 11 days, 12 days, 13 days, 14 days, 15 days, 16 days, 17 days, 18 days, 19 days, 20 days or 21 days after initiation of administration of the genetically engineered cells. In some disclosures the peak levels and/or AUC are assessed and/or the sample is obtained from the subject at a time that is between or between about 11 to 22 days, 12 to 18 days or 14 to 16 days, each inclusive, after initiation of administration of the genetically engineered cells.

The exposure, *e.g.,* number or concentration of cells, *e.g.* T cells administered for T cell therapy, indicative of expansion and/or persistence, may be stated in terms of maximum numbers or concentration of the cells to which the subject is exposed, duration of detectable cells or cells above a certain number or percentage, area under the curve (AUC) for number or concentration of cells over time, and/or combinations thereof and indicators thereof. Such outcomes may be assessed using known methods, such as qPCR to detect copy number of nucleic acid encoding the recombinant receptor compared to total amount of nucleic acid or DNA in the particular sample, *e.g.,* blood, serum, plasma or tissue, such as a tumor sample, and/or flow cytometric assays detecting cells expressing the receptor generally using antibodies specific for the receptors. Cell-based assays may also be used to detect the number or percentage or concentration of functional cells, such as cells capable of binding to and/or neutralizing and/or inducing responses, *e.g*., cytotoxic responses, against cells of the disease or condition or expressing the antigen recognized by the receptor.

In some cases, the pharmacokinetics of administered cells, e.g., adoptively transferred cells are determined to assess the availability, e.g., bioavailability of the administered cells. Methods for determining the pharmacokinetics of adoptively transferred cells may include drawing peripheral blood from subjects that have been administered engineered cells, and determining the number or ratio of the engineered cells in the peripheral blood. Approaches for selecting and/or isolating cells may include use of chimeric antigen receptor (CAR)-specific antibodies (e.g., Brentjens et al., Sci. Transl. Med. 2013 Mar; 5(177): 177ra38) Protein L (Zheng et al., J. Transl. Med. 2012 Feb; 10:29), epitope tags, such as Strep-Tag sequences, introduced directly into specific sites in the CAR, whereby binding reagents for Strep-Tag are used to directly assess the CAR (Liu et al. (2016) Nature Biotechnology, 34:430; international patent application Pub. No. WO2015095895) and monoclonal antibodies that specifically bind to a CAR polypeptide (see international patent application Pub. No. WO2014190273). Extrinsic marker genes may in some cases be utilized in connection with engineered cell therapies to permit detection or selection of cells and, in some cases, also to promote cell suicide. A truncated epidermal growth factor receptor (EGFRt) in some cases can be co-expressed with a transgene of interest (e.g., encoding a CAR) in transduced cells (see e.g. U.S. Patent No. 8,802,374). EGFRt may contain an epitope recognized by the antibody cetuximab (Erbitux^{®}) or other therapeutic anti-EGFR antibody or binding molecule, which can be used to identify or select cells that have been engineered with the EGFRt construct and another recombinant receptor, such as a chimeric antigen receptor (CAR), and/or to eliminate or separate cells expressing the receptor. See U.S. Patent No. 8,802,374 and Liu et al., Nature Biotech. 2016 April; 34(4): 430-434).

In some disclosures, the number of CAR⁺ T cells in a biological sample obtained from the patient, e.g., blood, can be determined at a period of time after administration of the cell therapy, e.g., to determine the pharmacokinetics of the cells. In some disclosures, number of CAR⁺ T cells, optionally CAR⁺ CD8⁺ T cells and/or CAR⁺ CD4⁺ T cells, detectable in the blood of the subject, or in a majority of subjects so treated by the method, is greater than 1 cells per µL, greater than 5 cells per µL or greater than per 10 cells per µL.

### D. Toxicity

In some disclosures, the methods and uses can include features that result in a lower rate and/or lower degree of toxicity, toxic outcome or symptom, toxicity-promoting profile, factor, or property, such as a symptom or outcome associated with or indicative of cytokine release syndrome (CRS) or neurotoxicity, for example, compared to administration of an alternative cell therapy, such as an alternative CAR⁺ T cell composition and/or an alternative dosing of cells, e.g. a dosing of cells that is not administered at a defined ratio.

In some disclosures, the methods and uses does not result in a high rate or likelihood of toxicity or toxic outcomes, or reduces the rate or likelihood of toxicity or toxic outcomes, such as neurotoxicity (NT), cytokine release syndrome (CRS), such as compared to certain other cell therapies. In some disclosures, the methods do not result in, or do not increase the risk of, severe NT (sNT), severe CRS (sCRS), macrophage activation syndrome, tumor lysis syndrome, fever of at least at or about 38 degrees Celsius for three or more days and a plasma level of CRP of at least at or about 20 mg/dL. In some disclosures, greater than or greater than about 30%, 35%, 40%, 50%, 55%, 60% or more of the subjects treated according to the disclosed methods do not exhibit any grade of CRS or any grade of neurotoxicity. In some disclosures, no more than 50% of subjects treated (e.g. at least 60%, at least 70%, at least 80%, at least 90% or more of the subjects treated) exhibit a cytokine release syndrome (CRS) higher than grade 2 and/or a neurotoxicity higher than grade 2. In some disclosures, at least 50% of subjects treated according to the method (e.g. at least 60%, at least 70%, at least 80%, at least 90% or more of the subjects treated) do not exhibit a severe toxic outcome (e.g. severe CRS or severe neurotoxicity), such as do not exhibit grade 3 or higher neurotoxicity and/or does not exhibit severe CRS, or does not do so within a certain period of time following the treatment, such as within a week, two weeks, or one month of the administration of the cells. In some disclosures, parameters assessed to determine certain toxicities include adverse events (AEs), dose-limiting toxicities (DLTs), CRS and NT.

Administration of adoptive T cell therapy, such as treatment with T cells expressing chimeric antigen receptors, can induce toxic effects or outcomes such as cytokine release syndrome and neurotoxicity. In some disclosures, such effects or outcomes parallel high levels of circulating cytokines, which may underlie the observed toxicity.

In some disclosures, the presence or development of adverse events (AEs), is measured or determined after administration of the engineered cells. In some disclosures, the type, frequency and severity of adverse events (AEs), serious adverse events (SAE), and laboratory abnormalities (overall and in clinical, histological and molecular subgroups), are measured. In some disclosures, greater than or greater than about 20%, 25%, 30%, 35%, 40%, 50%, 55%, 60% or more of the subjects treated according to the disclosed methods do not exhibit any AEs or SAEs.

In some disclosures, the toxic outcome is or is associated with or indicative of cytokine release syndrome (CRS) or severe CRS (sCRS). CRS, e.g., sCRS, can occur in some cases following adoptive T cell therapy and administration to subjects of other biological products. See Davila et al., Sci Transl Med 6, 224ra25 (2014); Brentjens et al., Sci. Transl. Med. 5, 177ra38 (2013); Grupp et al., N. Engl. J. Med. 368, 1509-1518 (2013); and Kochenderfer et al., Blood 119, 2709-2720 (2012); Xu et al., Cancer Letters 343 (2014) 172-78.

Typically, CRS is caused by an exaggerated systemic immune response mediated by, for example, T cells, B cells, NK cells, monocytes, and/or macrophages. Such cells may release a large amount of inflammatory mediators such as cytokines and chemokines. Cytokines may trigger an acute inflammatory response and/or induce endothelial organ damage, which may result in microvascular leakage, heart failure, or death. Severe, life-threatening CRS can lead to pulmonary infiltration and lung injury, renal failure, or disseminated intravascular coagulation. Other severe, life-threatening toxicities can include cardiac toxicity, respiratory distress, neurologic toxicity and/or hepatic failure.

CRS may be treated using anti-inflammatory therapy such as an anti-IL-6 therapy, e.g., anti-IL-6 antibody, e.g., tocilizumab, or antibiotics or other agents as described. Outcomes, signs and symptoms of CRS are known and include those described herein. In some disclosures, where a particular dosage regimen or administration effects or does not effect a given CRS-associated outcome, sign, or symptom, particular outcomes, signs, and symptoms and/or quantities or degrees thereof may be specified.

In the context of administering CAR-expressing cells, CRS typically occurs 6-20 days after infusion of cells that express a CAR. See Xu et al., Cancer Letters 343 (2014) 172-78. In some cases, CRS occurs less than 6 days or more than 20 days after CAR T cell infusion. The incidence and timing of CRS may be related to baseline cytokine levels or tumor burden at the time of infusion. Commonly, CRS involves elevated serum levels of interferon (IFN)-y, tumor necrosis factor (TNF)-α, and/or interleukin (IL)-2. Other cytokines that may be rapidly induced in CRS are IL-1β, IL-6, IL-8, and IL-10.

Exemplary outcomes associated with CRS include fever, rigors, chills, hypotension, dyspnea, acute respiratory distress syndrome (ARDS), encephalopathy, ALT/AST elevation, renal failure, cardiac disorders, hypoxia, neurologic disturbances, and death. Neurological complications include delirium, seizure-like activity, confusion, word-finding difficulty, aphasia, and/or becoming obtunded. Other CRS-related outcomes include fatigue, nausea, headache, seizure, tachycardia, myalgias, rash, acute vascular leak syndrome, liver function impairment, and renal failure. In some disclosures, CRS is associated with an increase in one or more factors such as serum-ferritin, d-dimer, aminotransferases, lactate dehydrogenase and triglycerides, or with hypofibrinogenemia or hepatosplenomegaly. Other exemplary signs or symptoms associated with CRS include hemodynamic instability, febrile neutropenia, increase in serum C-reactive protein (CRP), changes in coagulation parameters (for example, international normalized ratio (INR), prothrombin time (PTI) and/or fibrinogen), changes in cardiac and other organ function, and/or absolute neutrophil count (ANC).

In some disclosures, outcomes associated with CRS include one or more of: persistent fever, e.g., fever of a specified temperature, e.g., greater than at or about 38 degrees Celsius, for two or more, e.g., three or more, e.g., four or more days or for at least three consecutive days; fever greater than at or about 38 degrees Celsius; elevation of cytokines, such as a max fold change, e.g., of at least at or about 75, compared to pre-treatment levels of at least two cytokines (e.g., at least two of the group consisting of interferon gamma (IFNy), GM-CSF, IL-6, IL-10, Flt-3L, fracktalkine, and IL-5, and/or tumor necrosis factor alpha (TNFα)), or a max fold change, e.g., of at least at or about 250 of at least one of such cytokines; and/or at least one clinical sign of toxicity, such as hypotension (e.g., as measured by at least one intravenous vasoactive pressor); hypoxia (e.g., plasma oxygen (PO₂) levels of less than at or about 90%); and/or one or more neurologic disorders (including mental status changes, obtundation, and seizures).

Exemplary CRS-related outcomes include increased or high serum levels of one or more factors, including cytokines and chemokines and other factors associated with CRS. Exemplary outcomes further include increases in synthesis or secretion of one or more of such factors. Such synthesis or secretion can be by the T cell or a cell that interacts with the T cell, such as an innate immune cell or B cell.

In some disclosures, the CRS-associated serum factors or CRS-related outcomes include inflammatory cytokines and/or chemokines, including interferon gamma (IFN-γ), TNF-a, IL-1β, IL-2, IL-6, IL-7, IL-8, IL-10, IL-12, sIL-2Ra, granulocyte macrophage colony stimulating factor (GM-CSF), macrophage inflammatory protein (MIP)-1, tumor necrosis factor alpha (TNFα), IL-6, and IL-10, IL-1β, IL-8, IL-2, MIP-1, Flt-3L, fracktalkine, and/or IL-5. In some disclosures, the factor or outcome includes C reactive protein (CRP). In addition to being an early and easily measurable risk factor for CRS, CRP also is a marker for cell expansion. In some disclosures, subjects that are measured to have high levels of CRP, such as ≥ 15 mg/dL, have CRS. In some disclosures, subjects that are measured to have high levels of CRP do not have CRS. In some disclosures, a measure of CRS includes a measure of CRP and another factor indicative of CRS.

In some disclosures, one or more inflammatory cytokines or chemokines are monitored before, during, or after CAR treatment. In some disclosures, the one or more cytokines or chemokines include IFN-γ, TNF-α, IL-2, IL-1β, IL-6, IL-7, IL-8, IL-10, IL-12, sIL-2Rα, granulocyte macrophage colony stimulating factor (GM-CSF), or macrophage inflammatory protein (MIP). In some disclosures, IFN-γ, TNF-α, and IL-6 are monitored.

CRS criteria that appear to correlate with the onset of CRS to predict which patients are more likely to be at risk for developing sCRS have been developed (see Davilla et al. Science translational medicine. 2014;6(224):224ra25). Factors include fevers, hypoxia, hypotension, neurologic changes, elevated serum levels of inflammatory cytokines, such as a set of seven cytokines (IFNγ, IL-5, IL-6, IL-10, Flt-3L, fractalkine, and GM-CSF) whose treatment-induced elevation can correlate well with both pretreatment tumor burden and sCRS symptoms. Other guidelines on the diagnosis and management of CRS are known (see e.g., Lee et al, Blood. 2014;124(2): 188-95). In some disclosures, the criteria reflective of CRS grade are those detailed in Table 3 below.

| **Table 3: Exemplary Grading Criteria for CRS** | |
|---|---|
| **Grade** | **Description of Symptoms** |
| 1 | Not life-threatening, require only symptomatic treatment such as antipyretics and anti-emetics (e.g., fever, nausea, fatigue, headache, myalgias, malaise) |
| Mild | |
| 2 | Require and respond to moderate intervention: |
| Moderate | • Oxygen requirement < 40%, or |
| | • Hypotension responsive to fluids or low dose of a single vasopressor, or |
| | • Grade 2 organ toxicity (by CTCAE v4.0) |
| 3 | Require and respond to aggressive intervention: |
| Severe | • Oxygen requirement ≥ 40%, or |
| | • Hypotension requiring high dose of a single vasopressor (e.g., norepinephrine ≥ 20 µg/kg/min, dopamine ≥ 10 µg/kg/min, phenylephrine ≥ 200 µg/kg/min, or epinephrine ≥ 10 µg/kg/min), or |
| | • Hypotension requiring multiple vasopressors (e.g., vasopressin + one of the above agents, or combination vasopressors equivalent to ≥ 20 µg/kg/min norepinephrine), or |
| | • Grade 3 organ toxicity or Grade 4 transaminitis (by CTCAE v4.0) |
| 4 | Life-threatening: |
| Life-threatening | • Requirement for ventilator support, or |
| | • Grade 4 organ toxicity (excluding transaminitis) |
| 5 | Death |
| Fatal | |

In some disclosures, a subject is deemed to develop "severe CRS" ("sCRS") in response to or secondary to administration of a cell therapy or dose of cells thereof, if, following administration, the subject displays: (1) fever of at least 38 degrees Celsius for at least three days; (2) cytokine elevation that includes either (a) a max fold change of at least 75 for at least two of the following group of seven cytokines compared to the level immediately following the administration: interferon gamma (IFNy), GM-CSF, IL-6, IL-10, Flt-3L, fracktalkine, and IL-5 and/or (b) a max fold change of at least 250 for at least one of the following group of seven cytokines compared to the level immediately following the administration: interferon gamma (IFNy), GM-CSF, IL-6, IL-10, Flt-3L, fracktalkine, and IL-5; and (c) at least one clinical sign of toxicity such as hypotension (requiring at least one intravenous vasoactive pressor) or hypoxia (PO₂ < 90%) or one or more neurologic disorder(s) (including mental status changes, obtundation, and/or seizures). In some disclosures, severe CRS includes CRS with a grade of 3 or greater, such as set forth in **Table 3.**

In some disclosures, outcomes associated with severe CRS or grade 3 CRS or greater, such as grade 4 or greater, include one or more of: persistent fever, e.g., fever of a specified temperature, e.g., greater than at or about 38 degrees Celsius, for two or more, e.g., three or more, e.g., four or more days or for at least three consecutive days; fever greater than at or about 38 degrees Celsius; elevation of cytokines, such as a max fold change, e.g., of at least at or about 75, compared to pre-treatment levels of at least two cytokines (e.g., at least two of the group consisting of interferon gamma (IFNy), GM-CSF, IL-6, IL-10, Flt-3L, fracktalkine, and IL-5, and/or tumor necrosis factor alpha (TNFα)), or a max fold change, e.g., of at least at or about 250 of at least one of such cytokines; and/or at least one clinical sign of toxicity, such as hypotension (e.g., as measured by at least one intravenous vasoactive pressor); hypoxia (e.g., plasma oxygen (PO₂) levels of less than at or about 90%); and/or one or more neurologic disorders (including mental status changes, obtundation, and seizures). In some disclosures, severe CRS includes CRS that requires management or care in the intensive care unit (ICU).

In some disclosures, the CRS, such as severe CRS, encompasses a combination of (1) persistent fever (fever of at least 38 degrees Celsius for at least three days) and (2) a serum level of CRP of at least at or about 20 mg/dL. In some disclosures, the CRS encompasses hypotension requiring the use of two or more vasopressors or respiratory failure requiring mechanical ventilation. In some disclosures, the dosage of vasopressors is increased in a second or subsequent administration.

In some disclosures, severe CRS or grade 3 CRS encompasses an increase in alanine aminotransferase, an increase in aspartate aminotransferase, chills, febrile neutropenia, headache, left ventricular dysfunction, encephalopathy, hydrocephalus, and/or tremor.

The method of measuring or detecting the various outcomes may be specified.

In some disclosures, the toxic outcome is or is associated with neurotoxicity. In some disclosures, symptoms associated with a clinical risk of neurotoxicity include confusion, delirium, aphasia, expressive aphasia, obtundation, myoclonus, lethargy, altered mental status, convulsions, seizure-like activity, seizures (optionally as confirmed by electroencephalogram [EEG]), elevated levels of beta amyloid (Aβ), elevated levels of glutamate, and elevated levels of oxygen radicals. In some disclosures, neurotoxicity is graded based on severity (e.g., using a Grade 1-5 scale (see, e.g., Guido Cavaletti & Paola Marmiroli Nature Reviews Neurology 6, 657-666 (December 2010); National Cancer Institute-Common Toxicity Criteria version 4.03 (NCI-CTCAE v4.03).

In some instances disclosed herein, neurologic symptoms may be the earliest symptoms of sCRS. In some disclosures, neurologic symptoms are seen to begin 5 to 7 days after cell therapy infusion. In some disclosures, duration of neurologic changes may range from 3 to 19 days. In some cases, recovery of neurologic changes occurs after other symptoms of sCRS have resolved. In some disclosures, time or degree of resolution of neurologic changes is not hastened by treatment with anti-IL-6 and/or steroid(s).

In some disclosures, a subject is deemed to develop "severe neurotoxicity" in response to or secondary to administration of a cell therapy or dose of cells thereof, if, following administration, the subject displays symptoms that limit self-care (e.g. bathing, dressing and undressing, feeding, using the toilet, taking medications) from among: 1) symptoms of peripheral motor neuropathy, including inflammation or degeneration of the peripheral motor nerves; 2) symptoms of peripheral sensory neuropathy, including inflammation or degeneration of the peripheral sensory nerves, dysesthesia, such as distortion of sensory perception, resulting in an abnormal and unpleasant sensation, neuralgia, such as intense painful sensation along a nerve or a group of nerves, and/or paresthesia, such as functional disturbances of sensory neurons resulting in abnormal cutaneous sensations of tingling, numbness, pressure, cold and warmth in the absence of stimulus. In some disclosures, severe neurotoxicity includes neurotoxicity with a grade of 3 or greater, such as set forth in **Table 4.**

| **Table 4: Exemplary Grading Criteria for neurotoxicity** | |
|---|---|
| **Grade** | **Description of Symptoms** |
| 1 | Mild or asymptomatic symptoms |
| Asymptomatic or Mild | |
| 2 | Presence of symptoms that limit instrumental activities of daily living (ADL), such as preparing meals, shopping for groceries or clothes, using the telephone, managing money |
| Moderate | |
| 3 | Presence of symptoms that limit self-care ADL, such as bathing, dressing and undressing, feeding self, using the toilet, taking medications |
| Severe | |
| 4 | Symptoms that are life-threatening, requiring urgent intervention |
| Life-threatening | |
| 5 | Death |
| Fatal | |

In some disclosures, the methods reduce symptoms associated with CRS or neurotoxicity compared to other methods. In some disclosures, the methods reduce symptoms, outcomes or factors associated with CRS, including symptoms, outcomes or factors associated with severe CRS or grade 3 or higher CRS, compared to other methods. For example, subjects treated according to the present methods may lack detectable and/or have reduced symptoms, outcomes or factors of CRS, e.g. severe CRS or grade 3 or higher CRS, such as any described, e.g. set forth in **Table 3.** In some disclosures, subjects treated according to the present methods may have reduced symptoms of neurotoxicity, such as limb weakness or numbness, loss of memory, vision, and/or intellect, uncontrollable obsessive and/or compulsive behaviors, delusions, headache, cognitive and behavioral problems including loss of motor control, cognitive deterioration, and autonomic nervous system dysfunction, and sexual dysfunction, compared to subjects treated by other methods. In some disclosures, subjects treated according to the present methods may have reduced symptoms associated with peripheral motor neuropathy, peripheral sensory neuropathy, dysethesia, neuralgia or paresthesia.

In some disclosures, the methods reduce outcomes associated with neurotoxicity including damages to the nervous system and/or brain, such as the death of neurons. In some disclosures, the methods reduce the level of factors associated with neurotoxicity such as beta amyloid (Aβ), glutamate, and oxygen radicals.

In some disclosures, the toxicity outcome is a dose-limiting toxicity (DLT). In some disclosures, the toxic outcome is a dose-limiting toxicity. In some disclosures, the toxic outcome is the absence of a dose-limiting toxicity. In some disclosures, a dose-limiting toxicity (DLT) is defined as any grade 3 or higher toxicity as assessed by any known or published guidelines for assessing the particular toxicity, such as any described above and including the National Cancer Institute (NCI) Common Terminology Criteria for Adverse Events (CTCAE) version 4.0.

In some disclosures, the low rate, risk or likelihood of developing a toxicity, e.g. CRS or neurotoxicity or severe CRS or neurotoxicity, e.g. grade 3 or higher CRS or neurotoxicity, observed with administering a dose of T cells in accord with the disclosed methods, and/or with the disclosed articles of manufacture or compositions, permits administration of the cell therapy on an outpatient basis. In some disclosures, the administration of the cell therapy, e.g. dose of T cells (e.g. CAR⁺ T cells) in accord with the disclosed methods, and/or with the disclosed articles of manufacture or compositions, is performed on an outpatient basis or does not require admission to the subject to the hospital, such as admission to the hospital requiring an overnight stay.

In some disclosures, subjects administered the cell therapy, e.g. dose of T cells (e.g. CAR⁺ T cells) in accord with the disclosed methods, and/or with the disclosed articles of manufacture or compositions, including subjects treated on an outpatient basis, are not administered an intervention for treating any toxicity prior to or with administration of the cell dose, unless or until the subject exhibits a sign or symptom of a toxicity, such as of a neurotoxicity or CRS. Exemplary agents for treating, delaying, attenuating or ameliorating a toxicity are described herein.

In some disclosures, if a subject administered the cell therapy, e.g. dose of T cells (e.g. CAR⁺ T cells), including subjects treated on an outpatient basis, exhibits a fever the subject is given or is instructed to receive or administer a treatment to reduce the fever. In some disclosures, the fever in the subject is characterized as a body temperature of the subject that is (or is measured at) at or above a certain threshold temperature or level. In some disclosures, the threshold temperature is that associated with at least a low-grade fever, with at least a moderate fever, and/or with at least a high-grade fever. In some disclosures, the threshold temperature is a particular temperature or range. For example, the threshold temperature may be at or about or at least at or about 38, 39, 40, 41, or 42 degrees Celsius, and/or may be a range of at or about 38 degrees Celsius to at or about 39 degrees Celsius, a range of at or about 39 degrees Celsius to at or about 40 degrees Celsius, a range of at or about 40 degrees Celsius to at or about 41 degrees, or a range of at or about 41 degrees Celsius to at or about 42 degrees Celsius.

In some disclosures, the treatment designed to reduce fever includes treatment with an antipyretic. An antipyretic may include any agent, e.g., compound, composition, or ingredient, that reduces fever, such as one of any number of agents known to have antipyretic effects, such as NSAIDs (such as ibuprofen, naproxen, ketoprofen, and nimesulide), salicylates, such as aspirin, choline salicylate, magnesium salicylate, and sodium salicylate, paracetamol, acetaminophen, Metamizole, Nabumetone, Phenaxone, antipyrine, febrifuges. In some disclosures, the antipyretic is acetaminophen. In some disclosures, acetaminophen can be administered at a dose of 12.5 mg/kg orally or intravenously up to every four hours. In some disclosures, it is or comprises ibuprofen or aspirin.

In some disclosures, if the fever is a sustained fever, the subject is administered an alternative treatment for treating the toxicity, such as any described herein. For subjects treated on an outpatient basis, the subject is instructed to return to the hospital if the subject has and/or is determined to or to have a sustained fever. In some disclosures, the subject has, and/or is determined to or considered to have, a sustained fever if he or she exhibits a fever at or above the relevant threshold temperature, and where the fever or body temperature of the subject is not reduced, or is not reduced by or by more than a specified amount (e.g., by more than 1 °C, and generally does not fluctuate by about, or by more than about, 0.5 °C, 0.4 °C, 0.3 °C, or 0.2 °C), following a specified treatment, such as a treatment designed to reduce fever such as treatment with an antipyreticm, e.g. NSAID or salicylates, e.g. ibuprofen, acetaminophen or aspirin. For example, a subject is considered to have a sustained fever if he or she exhibits or is determined to exhibit a fever of at least at or about 38 or 39 degrees Celsius, which is not reduced by or is not reduced by more than at or about 0.5 °C, 0.4 °C, 0.3 °C, or 0.2 °C, or by at or about 1%, 2%, 3%, 4%, or 5%, over a period of 6 hours, over a period of 8 hours, or over a period of 12 hours, or over a period of 24 hours, even following treatment with the antipyretic such as acetaminophen. In some disclosures, the dosage of the antipyretic is a dosage ordinarily effective in such as subject to reduce fever or fever of a particular type such as fever associated with a bacterial or viral infection, e.g., a localized or systemic infection.

In some disclosures, the subject has, and/or is determined to or considered to have, a sustained fever if he or she exhibits a fever at or above the relevant threshold temperature, and where the fever or body temperature of the subject does not fluctuate by about, or by more than about, 1 °C, and generally does not fluctuate by about, or by more than about, 0.5 °C, 0.4 °C, 0.3 °C, or 0.2 °C. Such absence of fluctuation above or at a certain amount generally is measured over a given period of time (such as over a 24-hour, 12-hour, 8-hour, 6-hour, 3-hour, or 1-hour period of time, which may be measured from the first sign of fever or the first temperature above the indicated threshold). For example, in some disclosures, a subject is considered to or is determined to exhibit sustained fever if he or she exhibits a fever of at least at or about or at least at or about 38 or 39 degrees Celsius, which does not fluctuate in temperature by more than at or about 0.5°C, 0.4 °C, 0.3 °C, or 0.2 °C, over a period of 6 hours, over a period of 8 hours, or over a period of 12 hours, or over a period of 24 hours.

In some disclosures, the fever is a sustained fever; in some disclosures, the subject is treated at a time at which a subject has been determined to have a sustained fever, such as within one, two, three, four, five six, or fewer hours of such determination or of the first such determination following the initial therapy having the potential to induce the toxicity, such as the cell therapy, such as dose of T cells, e.g. CAR⁺ T cells.

In some disclosures, one or more interventions or agents for treating the toxicity, such as a toxicity-targeting therapies, is administered at a time at which or immediately after which the subject is determined to or confirmed to (such as is first determined or confirmed to) exhibit sustained fever, for example, as measured according to any of the aforementioned disclosures. In some disclosures, the one or more toxicity-targeting therapies is administered within a certain period of time of such confirmation or determination, such as within 30 minutes, 1 hour, 2 hours, 3 hours, 4 hours, 6 hours, or 8 hours thereof.

### II. RECOMBINANT RECEPTORS

In some disclosures, the cells for use in or administered in connection with the disclosed methods contain or are engineered to contain an engineered receptor, e.g., an engineered antigen receptor, such as a chimeric antigen receptor (CAR). Also disclosed are populations of such cells, compositions containing such cells and/or enriched for such cells, such as in which cells of a certain type such as T cells or CD8⁺ or CD4⁺ cells are enriched or selected. Among the compositions are pharmaceutical compositions and formulations for administration, such as for adoptive cell therapy. Also disclosed are therapeutic methods for administering the cells and compositions to subjects, e.g., patients, in accord with the disclosed methods, and/or with the disclosed articles of manufacture or compositions.

In some disclosures, the cells contain one or more nucleic acids introduced via genetic engineering, and thereby express recombinant or genetically engineered products of such nucleic acids. In some disclosures, gene transfer is accomplished by first stimulating the cells, such as by combining it with a stimulus that induces a response such as proliferation, survival, and/or activation, e.g., as measured by expression of a cytokine or activation marker, followed by introduction of the nucleic acids, e.g., by transduction, into the stimulated cells, and optionally incubation or expansion in culture to numbers sufficient for clinical applications.

The cells generally express recombinant receptors, such as antigen receptors including chimeric antigen receptors (CARs), and other antigen-binding receptors such as transgenic T cell receptors (TCRs). Also among the receptors are other chimeric receptors.

### A. Chimeric Antigen Receptors (CARs)

In some disclosures of the methods and uses, chimeric receptors, such as a chimeric antigen receptors, contain one or more domains that combine an antigen- or ligand-binding domain (e.g. antibody or antibody fragment) that provides specificity for a desired antigen (e.g., tumor antigen) with intracellular signaling domains. In some disclosures, the intracellular signaling domain is a stimulating or an activating intracellular domain portion, such as a T cell stimulating or activating domain, providing a primary activation signal or a primary signal. In some disclosures, the intracellular signaling domain contains or additionally contains a costimulatory signaling domain to facilitate effector functions. In some disclosures, chimeric receptors when genetically engineered into immune cells can modulate T cell activity, and, in some cases, can modulate T cell differentiation or homeostasis, thereby resulting in genetically engineered cells with improved longevity, survival and/or persistence *in vivo,* such as for use in adoptive cell therapy methods.

Exemplary antigen receptors, including CARs, and methods for engineering and introducing such receptors into cells, include those described, for example, in WO200014257, WO2013126726, WO2012/129514, WO2014031687, WO2013/166321, WO2013/071154, WO2013/123061, U.S. patent app. Pub. Nos. US2002131960, US2013287748, US20130149337, U.S. Patent Nos. 6,451,995, 7,446,190, 8,252,592, 8,339,645, 8,398,282, 7,446,179, 6,410,319, 7,070,995, 7,265,209, 7,354,762, 7,446,191, 8,324,353, and 8,479,118, and European patent app. No. EP2537416, and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some disclosures, the antigen receptors include a CAR as described in U.S. Patent No.: 7,446,190, and those described in WO/2014055668. Examples of the CARs include CARs as disclosed in any of the aforementioned publications, such as WO2014031687, US 8,339,645, US 7,446,179, US 2013/0149337, US 7,446,190, US 8,389,282, Kochenderfer et al., (2013) Nature Reviews Clinical Oncology, 10, 267-276; Wang et al. (2012) J. Immunother. 35(9): 689-701; and Brentjens et al., Sci Transl Med. 2013 5(177). See also WO2014031687, US 8,339,645, US 7,446,179, US 2013/0149337, US 7,446,190, and US 8,389,282.

The recombinant receptors, such as CARs, generally include an extracellular antigen binding domain, such as a portion of an antibody molecule, generally a variable heavy (V_{H}) chain region and/or variable light (V_{L}) chain region of the antibody, e.g., an scFv antibody fragment.

In some disclosures, the antigen targeted by the receptor is a polypeptide. In some disclosures, it is a carbohydrate or other molecule. In some disclosures, the antigen is selectively expressed or overexpressed on cells of the disease or condition, e.g., the tumor or pathogenic cells, as compared to normal or non-targeted cells or tissues. In other disclosures, the antigen is expressed on normal cells and/or is expressed on the engineered cells.

In some disclosures, the antigen targeted by the receptor includes antigens associated with a B cell malignancy, such as any of a number of known B cell marker. In some disclosures, the antigen targeted by the receptor is CD20, CD19, CD22, ROR1, CD45, CD21, CD5, CD33, Igkappa, Iglambda, CD79a, CD79b or CD30.

In some disclosures, the chimeric antigen receptor includes an extracellular portion containing an antibody or antibody fragment. In some disclosures, the chimeric antigen receptor includes an extracellular portion containing the antibody or fragment and an intracellular signaling domain. In some disclosures, the antibody or fragment includes an scFv.

In some disclosures, the antigen targeted by the antigen-binding domain is CD19. In some disclosures, the antigen-binding domain of the recombinant receptor, e.g., CAR, and the antigen-binding domain binds, such as specifically binds or specifically recognizes, a CD19, such as a human CD19. In some disclosures, the scFv contains a V_{H} and a V_{L} derived from an antibody or an antibody fragment specific to CD19. In some disclosures, the antibody or antibody fragment that binds CD19 is a mouse derived antibody such as FMC63 and SJ25C1. In some disclosures, the antibody or antibody fragment is a human antibody, e.g., as described in U.S. Patent Publication No. US 2016/0152723.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, heavy chain variable (V_{H}) regions capable of specifically binding the antigen, single chain antibody fragments, including single chain variable fragments (scFv), and single domain antibodies (*e.g*., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, *e.g*., bispecific or trispecific, antibodies, diabodies, triabodies, and tetrabodies, tandem di-scFv, tandem tri-scFv. Unless otherwise stated, the term "antibody" should be understood to encompass functional antibody fragments thereof also referred to herein as "antigen-binding fragments." The term also encompasses intact or full-length antibodies, including antibodies of any class or sub-class, including IgG and sub-classes thereof, IgM, IgE, IgA, and IgD.

The terms "complementarity determining region," and "CDR," synonymous with "hypervariable region" or "HVR," are known to refer to non-contiguous sequences of amino acids within antibody variable regions, which confer antigen specificity and/or binding affinity. In general, there are three CDRs in each heavy chain variable region (CDR-H1, CDR-H2, CDR-H3) and three CDRs in each light chain variable region (CDR-L1, CDR-L2, CDR-L3). "Framework regions" and "FR" are known to refer to the non-CDR portions of the variable regions of the heavy and light chains. In general, there are four FRs in each full-length heavy chain variable region (FR-H1, FR-H2, FR-H3, and FR-H4), and four FRs in each full-length light chain variable region (FR-L1, FR-L2, FR-L3, and FR-L4).

The precise amino acid sequence boundaries of a given CDR or FR can be readily determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273,927-948 ("Chothia" numbering scheme); MacCallum et al., J. Mol. Biol. 262:732-745 (1996), "Antibody-antigen interactions: Contact analysis and binding site topography," J. Mol. Biol. 262, 732-745." ("Contact" numbering scheme); Lefranc MP et al., "IMGT unique numbering for immunoglobulin and T cell receptor variable domains and Ig superfamily V-like domains," Dev Comp Immunol, 2003 Jan;27(1):55-77 ("IMGT" numbering scheme); Honegger A and Plückthun A, "Yet another numbering scheme for immunoglobulin variable domains: an automatic modeling and analysis tool," J Mol Biol, 2001 Jun 8;309(3):657-70, ("Aho" numbering scheme); and Martin et al., "Modeling antibody hypervariable loops: a combined algorithm," PNAS, 1989, 86(23):9268-9272, ("AbM" numbering scheme).

The boundaries of a given CDR or FR may vary depending on the scheme used for identification. For example, the Kabat scheme is based on structural alignments, while the Chothia scheme is based on structural information. Numbering for both the Kabat and Chothia schemes is based upon the most common antibody region sequence lengths, with insertions accommodated by insertion letters, for example, "30a," and deletions appearing in some antibodies. The two schemes place certain insertions and deletions ("indels") at different positions, resulting in differential numbering. The Contact scheme is based on analysis of complex crystal structures and is similar in many respects to the Chothia numbering scheme. The AbM scheme is a compromise between Kabat and Chothia definitions based on that used by Oxford Molecular's AbM antibody modeling software.

**Table 5,** below, lists exemplary position boundaries of CDR-L1, CDR-L2, CDR-L3 and CDR-H1, CDR-H2, CDR-H3 as identified by Kabat, Chothia, AbM, and Contact schemes, respectively. For CDR-H1, residue numbering is listed using both the Kabat and Chothia numbering schemes. FRs are located between CDRs, for example, with FR-L1 located before CDR-L1, FR-L2 located between CDR-L1 and CDR-L2, FR-L3 located between CDR-L2 and CDR-L3 and so forth. It is noted that because the shown Kabat numbering scheme places insertions at H35A and H35B, the end of the Chothia CDR-H1 loop when numbered using the shown Kabat numbering convention varies between H32 and H34, depending on the length of the loop.

**Table 5. Boundaries of CDRs according to various numbering schemes.**

| **CDR** | **Kabat** | **Chothia** | **AbM** | **Contact** |
|---|---|---|---|---|
| CDR-L1 | L24--L34 | L24--L34 | L24--L34 | L30--L36 |
| CDR-L2 | L50--L56 | L50--L56 | L50--L56 | L46--L55 |
| CDR-L3 | L89--L97 | L89--L97 | L89--L97 | L89--L96 |
| CDR-H1 (Kabat Numbering¹) | H31--H35B | H26--H32.34 | H26--H35B | H30--H35B |
| CDR-H1 (Chothia Numbering²) | H31--H35 | H26--H32 | H26--H35 | H30--H35 |
| CDR-H2 | H50--H65 | H52--H56 | H50--H58 | H47--H58 |
| CDR-H3 | H95--H102 | H95--H102 | H95--H102 | H93--H101 |

| | | | | |
|---|---|---|---|---|
| 1 - Kabat et al. (1991), "Sequences of Proteins of Immunological Interest," 5th Ed. Public Health Service, National Institutes of Health, Bethesda, MD 2 - Al-Lazikani et al., (1997) JMB 273,927-948 | | | | |

Thus, unless otherwise specified, a "CDR" or "complementary determining region," or individual specified CDRs (*e.g*., CDR-H1, CDR-H2, CDR-H3), of a given antibody or region thereof, such as a variable region thereof, should be understood to encompass a (or the specific) complementary determining region as defined by any of the aforementioned schemes, or other known schemes. For example, where it is stated that a particular CDR (*e.g.,* a CDR-H3) contains the amino acid sequence of a corresponding CDR in a given V_{H} or V_{L} region amino acid sequence, it is understood that such a CDR has a sequence of the corresponding CDR (*e.g.,* CDR-H3) within the variable region, as defined by any of the aforementioned schemes, or other known schemes. In some disclosures, specific CDR sequences are specified. Exemplary CDR sequences of disclosed antibodies are described using various numbering schemes, although it is understood that a disclosed antibody can include CDRs as described according to any of the other aforementioned numbering schemes or other numbering schemes known to a skilled artisan.

Likewise, unless otherwise specified, a FR or individual specified FR(s) (*e.g.,* FR-H1, FR-H2, FR-H3, FR-H4), of a given antibody or region thereof, such as a variable region thereof, should be understood to encompass a (or the specific) framework region as defined by any of the known schemes. In some instances disclosed herein, the scheme for identification of a particular CDR, FR, or FRs or CDRs is specified, such as the CDR as defined by the Kabat, Chothia, AbM or Contact method, or other known schemes. In other cases, the particular amino acid sequence of a CDR or FR is given.

The term "variable region" or "variable domain" refers to the domain of an antibody heavy or light chain that is involved in binding the antibody to antigen. The variable regions of the heavy chain and light chain (V_{H} and V_{L}, respectively) of a native antibody generally have similar structures, with each domain comprising four conserved framework regions (FRs) and three CDRs. (See, *e.g.,* Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, *e.g.,* Portolano et al., J. Immunol. 150:880-887 (1993); Clarkson et al., Nature 352:624-628 (1991).

Among the antibodies included in the disclosed CARs are antibody fragments. An "antibody fragment" or "antigen-binding fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; heavy chain variable (V_{H}) regions, single-chain antibody molecules such as scFvs and single-domain antibodies comprising only the V_{H} region; and multispecific antibodies formed from antibody fragments. In some disclosures, the antigen-binding domain in the disclosed CARs is or comprises an antibody fragment comprising a variable heavy chain (V_{H}) and a variable light chain (V_{L}) region. In particular disclosures, the antibodies are single-chain antibody fragments comprising a heavy chain variable (V_{H}) region and/or a light chain variable (V_{L}) region, such as scFvs.

In some disclosures, the antigen is CD19. In some disclosures, the scFv contains a V_{H} and a V_{L} derived from an antibody or an antibody fragment specific to CD19. In some disclosures, the antibody or antibody fragment that binds CD19 is a mouse derived antibody such as FMC63 and SJ25C1. In some disclosures, the antibody or antibody fragment is a human antibody, e.g., as described in U.S. Patent Publication No. US 2016/0152723.

In some disclosures, the scFv is derived from FMC63. FMC63 generally refers to a mouse monoclonal IgG1 antibody raised against Nalm-1 and -16 cells expressing CD19 of human origin (Ling, N. R., et al. (1987). Leucocyte typing III. 302). In some disclosures, the FMC63 antibody comprises a CDR-H1 and a CDR-H2 set forth in SEQ ID NOS: 38 and 39, respectively, and a CDR-H3 set forth in SEQ ID NO: 40 or 54; and a CDR-L1 set forth in SEQ ID NO: 35 and a CDR-L2 set forth in SEQ ID NO: 36 or 55 and a CDR-L3 set forth in SEQ ID NO: 37 or 34. In some disclosures, the FMC63 antibody comprises a heavy chain variable region (V_{H}) comprising the amino acid sequence of SEQ ID NO: 41 and a light chain variable region (V_{L}) comprising the amino acid sequence of SEQ ID NO: 42.

In some disclosures, the scFv comprises a variable light chain containing a CDR-L1 sequence of SEQ ID NO:35, a CDR-L2 sequence of SEQ ID NO:36, and a CDR-L3 sequence of SEQ ID NO:37 and/or a variable heavy chain containing a CDR-H1 sequence of SEQ ID NO:38, a CDR-H2 sequence of SEQ ID NO:39, and a CDR-H3 sequence of SEQ ID NO:40. In some disclosures, the scFv comprises a variable heavy chain region set forth in SEQ ID NO:41 and a variable light chain region set forth in SEQ ID NO:42. In some disclosures, the variable heavy and variable light chains are connected by a linker. In some disclosures, the linker is set forth in SEQ ID NO:56. In some disclosures, the scFv comprises, in order, a V_{H}, a linker, and a V_{L}. In some disclosures, the scFv comprises, in order, a V_{L}, a linker, and a V_{H}. In some disclosures, the scFv is encoded by a sequence of nucleotides set forth in SEQ ID NO:57 or a sequence that exhibits at least 85%, 86%, 87%, 88%, 89%, 90%, 91%, 92%, 93%, 94%, 95%, 96%, 97%, 98%, or 99% sequence identity to SEQ ID NO:57. In some disclosures, the scFv comprises the sequence of amino acids set forth in SEQ ID NO:43 or a sequence that exhibits at least 85%, 86%, 87%, 88%, 89%, 90%, 91%, 92%, 93%, 94%, 95%, 96%, 97%, 98%, or 99% sequence identity to SEQ ID NO:43.

In some disclosures the scFv is derived from SJ25C1. SJ25C1 is a mouse monoclonal IgG1 antibody raised against Nalm-1 and -16 cells expressing CD19 of human origin (Ling, N. R., et al. (1987). Leucocyte typing III. 302). In some disclosures, the SJ25C1 antibody comprises a CDR-H1, a CDR-H2 and a CDR-H3 sequence set forth in SEQ ID NOS: 47-49, respectively, and a CDR-L1, a CDR-L2 and a CDR-L3 sequence set forth in SEQ ID NOS: 44-46, respectively. In some disclosures, the SJ25C1 antibody comprises a heavy chain variable region (V_{H}) comprising the amino acid sequence of SEQ ID NO: 50 and a light chain variable region (V_{L}) comprising the amino acid sequence of SEQ ID NO: 51.

In some disclosures, the scFv comprises a variable light chain containing a CDR-L1 sequence of SEQ ID NO:44, a CDR-L2 sequence of SEQ ID NO: 45, and a CDR-L3 sequence of SEQ ID NO:46 and/or a variable heavy chain containing a CDR-H1 sequence of SEQ ID NO:47, a CDR-H2 sequence of SEQ ID NO:48, and a CDR-H3 sequence of SEQ ID NO:49. In some disclosures, the scFv comprises a variable heavy chain region set forth in SEQ ID NO:50 and a variable light chain region set forth in SEQ ID NO:51. In some disclosures, the variable heavy and variable light chain are connected by a linker. In some disclosures, the linker is set forth in SEQ ID NO:52. In some disclosures, the scFv comprises, in order, a V_{H}, a linker, and a V_{L}. In some disclosures, the scFv comprises, in order, a V_{L}, a linker, and a V_{H}. In some disclosures, the scFv comprises the sequence of amino acids set forth in SEQ ID NO:53 or a sequence that exhibits at least 85%, 86%, 87%, 88%, 89%, 90%, 91%, 92%, 93%, 94%, 95%, 96%, 97%, 98%, or 99% sequence identity to SEQ ID NO:53.

In some disclosures, the antibody portion of the recombinant receptor, e.g., CAR, further includes at least a portion of an immunoglobulin constant region, such as a hinge region, e.g., an IgG4 hinge region, and/or a C_{H}1/C_{L} and/or Fc region. In some disclosures, the constant region or portion is of a human IgG, such as IgG4 or IgG1. In some disclosures, the portion of the constant region serves as a spacer region between the antigen-recognition component, e.g., scFv, and transmembrane domain. The spacer can be of a length that provides for increased responsiveness of the cell following antigen binding, as compared to in the absence of the spacer. Exemplary spacers include, but are not limited to, those described in Hudecek et al. (2013) Clin. Cancer Res., 19:3153, WO2014031687, U.S. Patent No. 8,822,647 or published app. No. US 2014/0271635.

In some disclosures, the constant region or portion is of a human IgG, such as IgG4 or IgG1. In some disclosures, the spacer has the sequence ESKYGPPCPPCP (set forth in SEQ ID NO: 1), and is encoded by the sequence set forth in SEQ ID NO: 2. In some disclosures, the spacer has the sequence set forth in SEQ ID NO: 3. In some disclosures, the spacer has the sequence set forth in SEQ ID NO: 4. In some disclosures, the constant region or portion is of IgD. In some disclosures, the spacer has the sequence set forth in SEQ ID NO: 5. In some disclosures, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 1, 3, 4 or 5. In some disclosures, the spacer has the sequence set forth in SEQ ID NOS: 26-34. In some disclosures, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 26-34.

In some disclosures, the antigen receptor comprises an intracellular domain linked directly or indirectly to the extracellular domain. In some disclosures, the chimeric antigen receptor includes a transmembrane domain linking the extracellular domain and the intracellular signaling domain. In some disclosures, the intracellular signaling domain comprises an ITAM. For example, in some disclosures, the antigen recognition domain (e.g. extracellular domain) generally is linked to one or more intracellular signaling components, such as signaling components that mimic activation through an antigen receptor complex, such as a TCR complex, in the case of a CAR, and/or signal via another cell surface receptor. In some disclosures, the chimeric receptor comprises a transmembrane domain linked or fused between the extracellular domain (e.g. scFv) and intracellular signaling domain. Thus, in some disclosures, the antigen-binding component (e.g., antibody) is linked to one or more transmembrane and intracellular signaling domains.

In one disclosure, a transmembrane domain that naturally is associated with one of the domains in the receptor, e.g., CAR, is used. In some instances disclosed herein, the transmembrane domain is selected or modified by amino acid substitution to avoid binding of such domains to the transmembrane domains of the same or different surface membrane proteins to minimize interactions with other members of the receptor complex.

The transmembrane domain in some disclosures is derived either from a natural or from a synthetic source. Where the source is natural, the domain in some disclosures is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD 154. Alternatively the transmembrane domain in some disclosures is synthetic. In some disclosures, the synthetic transmembrane domain comprises predominantly hydrophobic residues such as leucine and valine. In some disclosures, a triplet of phenylalanine, tryptophan and valine will be found at each end of a synthetic transmembrane domain. In some disclosures, the linkage is by linkers, spacers, and/or transmembrane domain(s). In some disclosures, the transmembrane domain contains a transmembrane portion of CD28.

In some disclosures, the extracellular domain and transmembrane domain can be linked directly or indirectly. In some disclosures, the extracellular domain and transmembrane are linked by a spacer, such as any described herein. In some disclosures, the receptor contains extracellular portion of the molecule from which the transmembrane domain is derived, such as a CD28 extracellular portion.

Among the intracellular signaling domains are those that mimic or approximate a signal through a natural antigen receptor, a signal through such a receptor in combination with a costimulatory receptor, and/or a signal through a costimulatory receptor alone. In some disclosures, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

T cell activation is in some disclosures described as being mediated by two classes of cytoplasmic signaling sequences: those that initiate antigen-dependent primary activation through the TCR (primary cytoplasmic signaling sequences), and those that act in an antigen-independent manner to provide a secondary or co-stimulatory signal (secondary cytoplasmic signaling sequences). In some disclosures, the CAR includes one or both of such signaling components.

The receptor, e.g., the CAR, generally includes at least one intracellular signaling component or components. In some disclosures, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from CD3 zeta chain, FcR gamma, CD3 gamma, CD3 delta and CD3 epsilon. In some disclosures, cytoplasmic signaling molecule(s) in the CAR contain(s) a cytoplasmic signaling domain, portion thereof, or sequence derived from CD3 zeta.

In some disclosures, the receptor includes an intracellular component of a TCR complex, such as a TCR CD3 chain that mediates T-cell activation and cytotoxicity, e.g., CD3 zeta chain. Thus, in some disclosures, the antigen-binding portion is linked to one or more cell signaling modules. In some disclosures, cell signaling modules include CD3 transmembrane domain, CD3 intracellular signaling domains, and/or other CD transmembrane domains. In some disclosures, the receptor, e.g., CAR, further includes a portion of one or more additional molecules such as Fc receptor γ, CD8, CD4, CD25, or CD16. For example, in some disclosures, the CAR or other chimeric receptor includes a chimeric molecule between CD3-zeta (CD3-ζ) or Fc receptor γ and CD8, CD4, CD25 or CD16.

In some disclosures, upon ligation of the CAR or other chimeric receptor, the cytoplasmic domain or intracellular signaling domain of the receptor activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some disclosures, a truncated portion of an intracellular signaling domain of an antigen receptor component or costimulatory molecule is used in place of an intact immunostimulatory chain, for example, if it transduces the effector function signal. In some disclosures, the intracellular signaling domain or domains include the cytoplasmic sequences of the T cell receptor (TCR), and in some disclosures also those of co-receptors that in the natural context act in concert with such receptors to initiate signal transduction following antigen receptor engagement.

In the context of a natural TCR, full activation generally requires not only signaling through the TCR, but also a costimulatory signal. Thus, in some disclosures, to promote full activation, a component for generating secondary or co-stimulatory signal is also included in the CAR. In other disclosures, the CAR does not include a component for generating a costimulatory signal. In some disclosures, an additional CAR is expressed in the same cell and provides the component for generating the secondary or costimulatory signal.

In some disclosures, the chimeric antigen receptor contains an intracellular domain of a T cell costimulatory molecule. In some disclosures, the CAR includes a signaling domain and/or transmembrane portion of a costimulatory receptor, such as CD28, 4-1BB, OX40, DAP10, and ICOS. In some disclosures, the same CAR includes both the activating and costimulatory components. In some disclosures, the chimeric antigen receptor contains an intracellular domain derived from a T cell costimulatory molecule or a functional variant thereof, such as between the transmembrane domain and intracellular signaling domain. In some disclosures, the T cell costimulatory molecule is CD28 or 41BB.

In some disclosures, the activating domain is included within one CAR, whereas the costimulatory component is provided by another CAR recognizing another antigen. In some disclosures, the CARs include activating or stimulatory CARs, costimulatory CARs, both expressed on the same cell (see WO2014/055668). In some disclosures, the cells include one or more stimulatory or activating CAR and/or a costimulatory CAR. In some disclosures, the cells further include inhibitory CARs (iCARs, see Fedorov et al., Sci. Transl. Medicine, 5(215) (December, 2013), such as a CAR recognizing an antigen other than the one associated with and/or specific for the disease or condition whereby an activating signal delivered through the disease-targeting CAR is diminished or inhibited by binding of the inhibitory CAR to its ligand, e.g., to reduce off-target effects.

In certain disclosures, the intracellular signaling domain comprises a CD28 transmembrane and signaling domain linked to a CD3 (e.g., CD3-zeta) intracellular domain. In some disclosures, the intracellular signaling domain comprises a chimeric CD28 and CD137 (4-1BB, TNFRSF9) co-stimulatory domains, linked to a CD3 zeta intracellular domain.

In some disclosures, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some disclosures, the antigen receptor further includes a marker and/or cells expressing the CAR or other antigen receptor further includes a surrogate marker, such as a cell surface marker, which may be used to confirm transduction or engineering of the cell to express the receptor. In some disclosures, the marker includes all or part (e.g., truncated form) of CD34, a NGFR, or epidermal growth factor receptor, such as truncated version of such a cell surface receptor (e.g., tEGFR). In some disclosures, the nucleic acid encoding the marker is operably linked to a polynucleotide encoding for a linker sequence, such as a cleavable linker sequence, e.g., T2A. For example, a marker, and optionally a linker sequence, can be any as disclosed in published patent application No. WO2014031687. For example, the marker can be a truncated EGFR (tEGFR) that is, optionally, linked to a linker sequence, such as a T2A cleavable linker sequence.

An exemplary polypeptide for a truncated EGFR (e.g. tEGFR) comprises the sequence of amino acids set forth in SEQ ID NO: 7 or 16 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16. An exemplary T2A linker sequence comprises the sequence of amino acids set forth in SEQ ID NO: 6 or 17 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 6 or 17.

In some disclosures, the marker is a molecule, e.g., cell surface protein, not naturally found on T cells or not naturally found on the surface of T cells, or a portion thereof. In some disclosures, the molecule is a non-self molecule, e.g., non-self protein, i.e., one that is not recognized as "self" by the immune system of the host into which the cells will be adoptively transferred.

In some disclosures, the marker serves no therapeutic function and/or produces no effect other than to be used as a marker for genetic engineering, e.g., for selecting cells successfully engineered. In other disclosures, the marker may be a therapeutic molecule or molecule otherwise exerting some desired effect, such as a ligand for a cell to be encountered in vivo, such as a costimulatory or immune checkpoint molecule to enhance and/or dampen responses of the cells upon adoptive transfer and encounter with ligand.

In some cases, CARs are referred to as first, second, and/or third generation CARs. In some disclosures, a first generation CAR is one that solely provides a CD3-chain induced signal upon antigen binding; in some disclosures, a second-generation CARs is one that provides such a signal and costimulatory signal, such as one including an intracellular signaling domain from a costimulatory receptor such as CD28 or CD137; in some disclosures, a third generation CAR is one that includes multiple costimulatory domains of different costimulatory receptors.

For example, in some disclosures, the CAR contains an antibody, e.g., an antibody fragment, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some disclosures, the CAR contains an antibody, e.g., antibody fragment, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such disclosures, the receptor further includes a spacer containing a portion of an Ig molecule, such as a human Ig molecule, such as an Ig hinge, e.g. an IgG4 hinge, such as a hinge-only spacer.

In some disclosures, the transmembrane domain of the recombinant receptor, e.g., the CAR, is or includes a transmembrane domain of human CD28 (e.g. Accession No. P01747.1) or variant thereof, such as a transmembrane domain that comprises the sequence of amino acids set forth in SEQ ID NO: 8 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 8; in some disclosures, the transmembrane-domain containing portion of the recombinant receptor comprises the sequence of amino acids set forth in SEQ ID NO: 9 or a sequence of amino acids having at least at or about 85%, 86%, 87%, 88%, 89%, 90%, 91%, 92%, 93%, 94%, 95%, 96%, 97%, 98%, 99% or more sequence identity thereto.

In some disclosures, the intracellular signaling component(s) of the recombinant receptor, *e.g.* the CAR, contains an intracellular costimulatory signaling domain of human CD28 or a functional variant or portion thereof, such as a domain with an LL to GG substitution at positions 186-187 of a native CD28 protein. For example, the intracellular signaling domain can comprise the sequence of amino acids set forth in SEQ ID NO: 10 or 11 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 10 or 11. In some disclosures, the intracellular domain comprises an intracellular costimulatory signaling domain of 4-1BB (e.g. (Accession No. Q07011.1) or functional variant or portion thereof, such as the sequence of amino acids set forth in SEQ ID NO: 12 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 12.

In some disclosures, the intracellular signaling domain of the recombinant receptor, e.g. the CAR, comprises a human CD3 zeta stimulatory signaling domain or functional variant thereof, such as an 112 AA cytoplasmic domain of isoform 3 of human CD3ζ (Accession No.: P20963.2) or a CD3 zeta signaling domain as described in U.S. Patent No.: 7,446,190 or U.S. Patent No. 8,911,993. For example, in some disclosures, the intracellular signaling domain comprises the sequence of amino acids as set forth in SEQ ID NO: 13, 14 or 15 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 13, 14 or 15.

In some disclosures, the spacer contains only a hinge region of an IgG, such as only a hinge of IgG4 or IgG1, such as the hinge only spacer set forth in SEQ ID NO: 1. In other disclosures, the spacer is or contains an Ig hinge, e.g., an IgG4-derived hinge, optionally linked to a C_{H}2 and/or C_{H}3 domains. In some disclosures, the spacer is an Ig hinge, e.g., an IgG4 hinge, linked to C_{H}2 and C_{H}3 domains, such as set forth in SEQ ID NO: 4. In some disclosures, the spacer is an Ig hinge, e.g., an IgG4 hinge, linked to a C_{H}3 domain only, such as set forth in SEQ ID NO: 3. In some disclosures, the spacer is or comprises a glycine-serine rich sequence or other flexible linker such as known flexible linkers.

For example, in some disclosures, the CAR includes an antibody such as an antibody fragment, including scFvs, a spacer, such as a spacer containing a portion of an immunoglobulin molecule, such as a hinge region and/or one or more constant regions of a heavy chain molecule, such as an Ig-hinge containing spacer, a transmembrane domain containing all or a portion of a CD28-derived transmembrane domain, a CD28-derived intracellular signaling domain, and a CD3 zeta signaling domain. In some disclosures, the CAR includes an antibody or fragment, such as scFv, a spacer such as any of the Ig-hinge containing spacers, a CD28-derived transmembrane domain, a 4-1BB-derived intracellular signaling domain, and a CD3 zeta-derived signaling domain.

In some disclosures, nucleic acid molecules encoding such CAR constructs further includes a sequence encoding a T2A ribosomal skip element and/or a tEGFR sequence, e.g., downstream of the sequence encoding the CAR. In some disclosures, the sequence encodes a T2A ribosomal skip element set forth in SEQ ID NO: 6 or 17, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 6 or 17. In some disclosures, T cells expressing an antigen receptor (e.g. CAR) can also be generated to express a truncated EGFR (EGFRt) as a non-immunogenic selection epitope (e.g. by introduction of a construct encoding the CAR and EGFRt separated by a T2A ribosome switch to express two proteins from the same construct), which then can be used as a marker to detect such cells (see e.g. U.S. Patent No. 8,802,374). In some disclosures, the sequence encodes an tEGFR sequence set forth in SEQ ID NO: 7 or 16, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16. In some cases, the peptide, such as T2A, can cause the ribosome to skip (ribosome skipping) synthesis of a peptide bond at the C-terminus of a 2A element, leading to separation between the end of the 2A sequence and the next peptide downstream (*see,* for example, de Felipe. Genetic Vaccines and Ther. 2:13 (2004) and deFelipe et al. Traffic 5:616-626 (2004)). Many 2A elements are known. Examples of 2A sequences that can be used in the methods and nucleic acids disclosed herein, without limitation, 2A sequences from the foot-and-mouth disease virus (F2A, *e.g.,* SEQ ID NO: 21), equine rhinitis A virus (E2A, *e.g.,* SEQ ID NO: 20), Thosea asigna virus (T2A, *e.g.,* SEQ ID NO: 6 or 17), and porcine teschovirus-1 (P2A, *e.g.,* SEQ ID NO: 18 or 19) as described in U.S. Patent Publication No. 20070116690.

The recombinant receptors, such as CARs, expressed by the cells administered to the subject generally recognize or specifically bind to a molecule that is expressed in, associated with, and/or specific for the disease or condition or cells thereof being treated. Upon specific binding to the molecule, e.g., antigen, the receptor generally delivers an immunostimulatory signal, such as an ITAM-transduced signal, into the cell, thereby promoting an immune response targeted to the disease or condition. For example, in some disclosures, the cells express a CAR that specifically binds to an antigen expressed by a cell or tissue of the disease or condition or associated with the disease or condition.

### C. Multi-targeting

In some disclosures, the cells used in connection with the disclosed methods, uses, articles of manufacture and compositions include cells employing multi-targeting strategies, such as expression of two or more genetically engineered receptors on the cell, each recognizing the same of a different antigen and typically each including a different intracellular signaling component. Such multi-targeting strategies are described, for example, in WO 2014055668 (describing combinations of activating and costimulatory CARs, e.g., targeting two different antigens present individually on off-target, e.g., normal cells, but present together only on cells of the disease or condition to be treated) and Fedorov et al., Sci. Transl. Medicine, 5(215) (2013) (describing cells expressing an activating and an inhibitory CAR, such as those in which the activating CAR binds to one antigen expressed on both normal or non-diseased cells and cells of the disease or condition to be treated, and the inhibitory CAR binds to another antigen expressed only on the normal cells or cells which it is not desired to treat).

For example, in some disclosures, the cells include a receptor expressing a first genetically engineered antigen receptor (e.g., CAR) which is capable of inducing an activating or stimulatory signal to the cell, generally upon specific binding to the antigen recognized by the first receptor, e.g., the first antigen. In some disclosures, the cell further includes a second genetically engineered antigen receptor (e.g., CAR), e.g., a chimeric costimulatory receptor, which is capable of inducing a costimulatory signal to the immune cell, generally upon specific binding to a second antigen recognized by the second receptor. In some disclosures, the first antigen and second antigen are the same. In some disclosures, the first antigen and second antigen are different.

In some disclosures, the first and/or second genetically engineered antigen receptor (e.g. CAR) is capable of inducing an activating signal to the cell. In some disclosures, the receptor includes an intracellular signaling component containing ITAM or ITAM-like motifs. In some disclosures, the activation induced by the first receptor involves a signal transduction or change in protein expression in the cell resulting in initiation of an immune response, such as ITAM phosphorylation and/or initiation of ITAM-mediated signal transduction cascade, formation of an immunological synapse and/or clustering of molecules near the bound receptor (e.g. CD4 or CD8, etc.), activation of one or more transcription factors, such as NF-κB and/or AP-1, and/or induction of gene expression of factors such as cytokines, proliferation, and/or survival.

In some disclosures, the first and/or second receptor includes intracellular signaling domains or regions of costimulatory receptors such as CD28, CD137 (4-1BB), OX40, and/or ICOS. In some disclosures, the first and second receptor include an intracellular signaling domain of a costimulatory receptor that are different. In one disclosure, the first receptor contains a CD28 costimulatory signaling region and the second receptor contain a 4-1BB co-stimulatory signaling region or vice versa.

In some disclosures, the first and/or second receptor includes both an intracellular signaling domain containing ITAM or ITAM-like motifs and an intracellular signaling domain of a costimulatory receptor.

In some disclosures, the first receptor contains an intracellular signaling domain containing ITAM or ITAM-like motifs and the second receptor contains an intracellular signaling domain of a costimulatory receptor. The costimulatory signal in combination with the activating signal induced in the same cell is one that results in an immune response, such as a robust and sustained immune response, such as increased gene expression, secretion of cytokines and other factors, and T cell mediated effector functions such as cell killing.

In some disclosures, neither ligation of the first receptor alone nor ligation of the second receptor alone induces a robust immune response. In some disclosures, if only one receptor is ligated, the cell becomes tolerized or unresponsive to antigen, or inhibited, and/or is not induced to proliferate or secrete factors or carry out effector functions. In some such disclosures, however, when the plurality of receptors are ligated, such as upon encounter of a cell expressing the first and second antigens, a desired response is achieved, such as full immune activation or stimulation, e.g., as indicated by secretion of one or more cytokine, proliferation, persistence, and/or carrying out an immune effector function such as cytotoxic killing of a target cell.

In some disclosures, the two receptors induce, respectively, an activating and an inhibitory signal to the cell, such that binding by one of the receptor to its antigen activates the cell or induces a response, but binding by the second inhibitory receptor to its antigen induces a signal that suppresses or dampens that response. Examples are combinations of activating CARs and inhibitory CARs or iCARs. Such a strategy may be used, for example, in which the activating CAR binds an antigen expressed in a disease or condition but which is also expressed on normal cells, and the inhibitory receptor binds to a separate antigen which is expressed on the normal cells but not cells of the disease or condition.

In some disclosures, the multi-targeting strategy is employed in a case where an antigen associated with a particular disease or condition is expressed on a non-diseased cell and/or is expressed on the engineered cell itself, either transiently (e.g., upon stimulation in association with genetic engineering) or permanently. In such cases, by requiring ligation of two separate and individually specific antigen receptors, specificity, selectivity, and/or efficacy may be improved.

In some disclosures, the plurality of antigens, e.g., the first and second antigens, are expressed on the cell, tissue, or disease or condition being targeted, such as on the cancer cell. In some disclosures, the cell, tissue, disease or condition is multiple myeloma or a multiple myeloma cell. In some disclosures, one or more of the plurality of antigens generally also is expressed on a cell which it is not desired to target with the cell therapy, such as a normal or non-diseased cell or tissue, and/or the engineered cells themselves. In such disclosures, by requiring ligation of multiple receptors to achieve a response of the cell, specificity and/or efficacy is achieved.

### E. Methods of Engineering Cells

In some disclosures, the engineered cells, for example engineered cells used in connection with the methods and uses disclosed herein, are produced by a process that generates an output composition of enriched T cells from one or more input compositions and/or from a single biological sample. In certain disclosures, the output composition contains cells that express a recombinant receptor, e.g., a CAR, such as an anti-CD19 CAR. In particular disclosures, the cells of the output compositions are suitable for administration to a subject as a therapy, such as in accordance with any of the methods and uses disclosed herein, including in an autologous cell therapy. In some disclosures, the output composition is a composition of enriched CD4+ or CD8+ T cells.

In some disclosures, the process for generating or producing engineered cells is by a process that includes some or all of the steps of: collecting or obtaining a biological sample; isolating, selecting, or enriching input cells from the biological sample; cryopreserving and storing the input cells; thawing and/or incubating the input cells under stimulating conditions; engineering the stimulated cells to express or contain a recombinant polynucleotide, e.g., a polynucleotide encoding a recombinant receptor such as a CAR; cultivating the engineered cells, e.g. to a threshold amount, density, or expansion; formulating the cultivated cells in an output composition; and/or cryopreserving and storing the formulated output cells until the cells are released for infusion and/or are suitable to be administered to a subject. In certain disclosures, the process is performed with two or more input compositions of enriched T cells, such as a separate CD4+ composition and a separate CD8+ composition, that are separately processed and engineered from the same starting or initial biological sample and re-infused back into the subject at a defined ratio, e.g. 1:1 ratio of CD4+ to CD8+ T cells. In some disclosures, the enriched T cells are or include engineered T cells, e.g., T cells transduced to express a recombinant receptor.

In particular disclosures, an output composition of engineered cells expressing a recombinant receptor (e.g. anti-CD 19 CAR) is produced from an initial and/or input composition of cells. In some disclosures, the input composition is a composition of enriched T cells, enriched CD4+ T cells, and/or enriched CD8+ T cells (herein after also referred to as compositions of enriched T cells, compositions of enriched CD4+ T cells, and compositions of enriched CD8+ T cells, respectively). In some disclosures, a composition enriched in CD4+ T cells contains at least 60%, 65%, 70%, 75%, 80%, 85%, 90%, 95%, 98%, 99%, or 99.9% CD4+ T cells. In particular disclosures, the composition of enriched CD4+ T cells contains 100% CD4+ T cells contains about 100% CD4+ T cells. In certain disclosures, the composition of enriched T cells includes or contains less than 20%, less than 10%, less than 5%, less than 1%, less than 0.1%, or less than 0.01% CD8+ T cells, and/or contains no CD8+ T cells, and/or is free or substantially free of CD8+ T cells. In some disclosures, the populations of cells consist essentially of CD4+ T cells. In some disclosures, a composition enriched in CD8+ T cells contains at least 75%, 80%, 85%, 90%, 95%, 98%, 99%, or 99.9% CD8+ T cells, or contains or contains about 100% CD8+ T cells. In certain disclosures, the composition of enriched CD8+ T cells includes or contains less than 20%, less than 10%, less than 5%, less than 1%, less than 0.1%, or less than 0.01% CD4+ T cells, and/or contains no CD4+ T cells, and/or is free or substantially free of CD4+ T cells. In some disclosures, the populations of cells consist essentially of CD8+ T cells.

In certain disclosures, the process for producing engineered cells further can include one or more of: activating and/or stimulating a cells, e.g., cells of an input composition; genetically engineering the activated and/or stimulated cells, e.g., to introduce a polynucleotide encoding a recombinant protein by transduction or transfection; and/or cultivating the engineered cells, e.g., under conditions that promote proliferation and/or expansion. In particular disclosures, the methods may be used in connection with harvesting, collecting, and/or formulating output compositions produced after the cells have been incubated, activated, stimulated, engineered, transduced, transfected, and/or cultivated.

In some disclosures, engineered cells, such as those that express an anti-CD19 CAR as described, used in accord with the disclosed methods and uses are produced or generated by a process for selecting, isolating, activating, stimulating, expanding, cultivating, and/or formulating cells. In some disclosures, such methods include any as described.

In some disclosures, engineered cells, such as those that express an anti-CD19 CAR as described, used in accord with the disclosed methods and uses are produced or generated by exemplary processes as described in, for example, WO 2019/089855 and WO 2015/164675.

In some of any disclosures, exemplary processes for generating, producing or manufacturing the engineered cells, such as those that express an anti-CD19 CAR as described, or a composition comprising such cells, such as a composition comprising engineered CD4+ T cells and engineered CD8+ T cells each expressing the same anti-CD19 chimeric antigen receptor (CAR), involve subjecting enriched CD4+ and enriched CD8+ cell populations, separately, to process steps. In some disclosures of the exemplary process for generating or manufacturing engineered cells, CD4+ and CD8+ cells are separately selected from human peripheral blood mononuclear cells (PBMCs), for example, that are obtained by leukapheresis, generating separate enriched CD4+ and enriched CD8+ cell compositions. In some disclosures, such cells can be cryopreserved. In some disclosures, the CD4+ and CD8+ compositions can be subsequently thawed and separately subject to steps for stimulation, transduction, and expansion.

In some disclosures of the exemplary process for generating or manufacturing engineered cells, thawed CD4+ and CD8+ cells are separately stimulated, for example, in the presence of paramagnetic polystyrene-coated beads coupled to anti-CD3 and anti-CD28 antibodies (such as at a 1:1 bead to cell ratio). In some disclosures, the stimulation is carried out in media containing human recombinant IL-2, human recombinant IL-15, and N-Acetyl Cysteine (NAC). In some disclosures, the cell culture media for CD4+ cells also can include human recombinant IL-7.

In some disclosures of the exemplary process for generating or manufacturing engineered cells, following the introduction of the beads, CD4+ and CD8+ cells are separately transduced with a lentiviral vector encoding the same CAR, such as the same anti-CD19 CAR. In some disclosures, the CAR can contain an anti-CD19 scFv derived from a murine antibody, an immunoglobulin spacer, a transmembrane domain derived from CD28, a costimulatory region derived from 4-1BB, and a CD3-zeta intracellular signaling domain. In some disclosures, the vector can encode a truncated receptor that serves as a surrogate marker for CAR expression that is connected to the CAR construct by a T2A sequence. In some disclosures of the exemplary process, the cells are transduced in the presence of 10 µg/ml protamine sulfate.

In some disclosures of the exemplary process for generating or manufacturing engineered cells, following transduction, the beads are removed from the cell compositions by exposure to a magnetic field. In some disclosures, the CD4+ and CD8+ cell compositions are separately cultivated for expansion with continual mixing and oxygen transfer by a bioreactor (for example, a Xuri W25 Bioreactor). In some cases, poloxamer is added to the media. In some disclosures, both the CD4+ and the CD8+ cell compositions are cultivated in the presence of IL-2 and IL-15. In some disclosures, the CD4+ cell media also included IL-7. In some cases, the CD4+ and CD8+ cells are each cultivated, prior to harvest, to 4-fold expansion. In some disclosures, one day after reaching the threshold, cells from each composition can be separately harvested, formulated, and cryopreserved. In some disclosures, the exemplary processes for generating, producing or manufacturing the engineered cells, such as those that express an anti-CD19 CAR as described, or a composition comprising such cells, such as a composition comprising engineered CD4+ T cells and engineered CD8+ T cells each expressing the same anti-CD19 chimeric antigen receptor (CAR), include those described in **Table 6** below.

**Table 6: Exemplary process for generating CD4+ and CD8+ CAR-T cells**

| **Stage** | **CD4+ cells** | | **CD8+ cells** | |
|---|---|---|---|---|
| **Stimulation** (day 1-2) | | • anti-CD3/CD28 antibody conjugated beads | | • anti-CD3/CD28 antibody conjugated beads |
| | | • 1:1 bead to cell ratio | | • 1:1 bead to cell ratio |
| | | • media: IL-2, IL-7, IL-15, and NAC | | • media: IL-2, IL-15, and NAC |
| **Transduction** (day 2-5) | | • transduction adjuvant (e.g. 10 µg/ml protamine sulfate) | | • transduction adjuvant (e.g. 10 µg/ml protamine sulfate) |
| **Bead removal** (day 5*) | | • magnetic bead removal | | • magnetic bead removal |
| **Expansion (day 5* - Harvest)** | | • rocking motion bioreactor and/or continuous mixing | | • rocking motion bioreactor and/or continuous mixing |
| | | • media: IL-2, IL-7, IL15, and poloxamer | | • media: IL-2, IL15, and poloxamer |

| | | | | |
|---|---|---|---|---|
| * Approximate | | | | |

In other disclosures, a different exemplary process for generating, producing or manufacturing the engineered cells or a composition comprising such cells include a process that differs from the exemplary process above in that: NAC is not added to the media during stimulation; CD4+ cell media does not contain IL-2; cells are stimulated at a bead to cell ratio of 3:1; cells are transduced with a higher concentration of protamine sulfate; bead removal occurs at about day 7; and expansion is performed at a static setting, i.e., without continual mixing or perfusion (e.g., semi-continuous and/or stepwise perfusion), and without poloxamer.

In some disclosures, at least one separate composition of enriched CD4+ T cells and at least one separate composition of enriched CD8+ T cells are isolated, selected, enriched, or obtained from a single biological sample, e.g., a sample of PBMCs or other white blood cells from the same donor such as a patient or healthy individual. In some disclosures, a separate composition of enriched CD4+ T cells and a separate composition of enriched CD8+ T cells originated, e.g., were initially isolated, selected, and/or enriched, from the same biological sample, such as a single biological sample obtained, collected, and/or taken from a single subject. In some disclosures, a biological sample is first subjected to selection of CD4+ T cells, where both the negative and positive fractions are retained, and the negative fraction is further subjected to selection of CD8+ T cells. In other disclosures, a biological sample is first subjected to selection of CD8⁺ T cells, where both the negative and positive fractions are retained, and the negative fraction is further subjected to selection of CD4+ T cells. In some disclosures, methods of selection are carried out as described in International PCT publication No. WO2015/164675. In some disclosures, methods of selection are carried out as described in International PCT publication No. WO 2019/089855. In some disclosures, a biological sample is first positively selected for CD8+ T cells to generate at least one composition of enriched CD8+ T cells, and the negative fraction is then positively selected for CD4+ T cells to generate at least one composition of enriched CD4+ T cells, such that the at least one composition of enriched CD8+ T cells and the at least one composition of enriched CD4+ T cells are separate compositions from the same biological sample, e.g., from the same donor patient or healthy individual. In some disclosures, two or more separate compositions of enriched T cells, e.g., at least one being a composition of enriched CD4+ T cells and at least one being a separate composition of enriched CD8+ T cells from the same donor, are separately frozen, e.g., cryoprotected or cryopreserved in a cryopreservation media.

In some disclosures, two or more separate compositions of enriched T cells, e.g., at least one being a composition of enriched CD4+ T cells and at least one being a separate composition of enriched CD8+ T cells from the same biological sample, are activated and/or stimulated by contacting with a stimulatory reagent (e.g., by incubation with CD3/CD28 conjugated magnetic beads for T cell activation). In some disclosures, each of the activated/stimulated cell composition is engineered, transduced, and/or transfected, e.g., using a viral vector encoding a recombinant protein (e.g. CAR), to express the same recombinant protein in the CD4+ T cells and CD8+ T cells of each cell composition. In some disclosures, the method comprises removing the stimulatory reagent, e.g., magnetic beads, from the cell composition. In some disclosures, a cell composition containing engineered CD4+ T cells and a cell composition containing engineered CD8+ T cells are separately cultivated, e.g., for separate expansion of the CD4+ T cell and CD8+ T cell populations therein. In certain disclosures, a cell composition from the cultivation is harvested and/or collected and/or formulated, e.g., by washing the cell composition in a formulation buffer. In certain disclosures, a formulated cell composition comprising CD4+ T cells and a formulated cell composition comprising CD8+ T cells is frozen, e.g., cryoprotected or cryopreserved in a cryopreservation media. In some disclosures, engineered CD4+ T cells and CD8+ T cells in each formulation originate from the same donor or biological sample and express the same recombination protein (e.g., CAR, such as anti-CD19 CAR). In some disclosures, a separate engineered CD4+ formulation and a separate engineered CD8+ formulation are administered at a defined ratio, e.g. 1:1, to a subject in need thereof such as the same donor.

### 1. Cells and Preparation of Cells for Genetic Engineering

In some disclosures, cells, such as T cells, used in connection with the disclosed methods, uses, articles of manufacture or compositions are cells have been genetically engineered to express a recombinant receptor, e.g., a CAR or a TCR, such as those described herein. In some disclosures, the engineered cells are used in the context of cell therapy, e.g., adoptive cell therapy. In some disclosures, the engineered cells are immune cells. In some disclosures, the engineered cells are T cells, such as CD4+ or CD8+ T cells.

In some disclosures, the nucleic acids, such as nucleic acids encoding a recombinant receptor, are heterologous, i.e., normally not present in a cell or sample obtained from the cell, such as one obtained from another organism or cell, which for example, is not ordinarily found in the cell being engineered and/or an organism from which such cell is derived. In some disclosures, the nucleic acids are not naturally occurring, such as a nucleic acid not found in nature, including one comprising chimeric combinations of nucleic acids encoding various domains from multiple different cell types.

The cells generally are eukaryotic cells, such as mammalian cells, and typically are human cells. In some disclosures, the cells are derived from the blood, bone marrow, lymph, or lymphoid organs, are cells of the immune system, such as cells of the innate or adaptive immunity, *e.g*., myeloid or lymphoid cells, including lymphocytes, typically T cells and/or NK cells. Other exemplary cells include stem cells, such as multipotent and pluripotent stem cells, including induced pluripotent stem cells (iPSCs). The cells typically are primary cells, such as those isolated directly from a subject and/or isolated from a subject and frozen. In some disclosures, the cells include one or more subsets of T cells or other cell types, such as whole T cell populations, CD4⁺ cells, CD8⁺ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. Among the methods include off-the-shelf methods. In some disclosures, such as for off-the-shelf technologies, the cells are pluripotent and/or multipotent, such as stem cells, such as induced pluripotent stem cells (iPSCs). In some disclosures, the methods include isolating cells from the subject, preparing, processing, culturing, and/or engineering them, and re-introducing them into the same subject, before or after cryopreservation.

Among the sub-types and subpopulations of T cells and/or of CD4⁺ and/or of CD8⁺ T cells are naive T (T_{N}) cells, effector T cells (T_{EFF}), memory T cells and sub-types thereof, such as stem cell memory T (T_{SCM}), central memory T (T_{CM}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (T_{REG}) cells, helper T cells, such as TH1 cells, TH2 cells, TH3 cells, TH17 cells, TH9 cells, TH22 cells, follicular helper T cells, alpha/beta T cells, and delta/gamma T cells.

In some disclosures, the cells are natural killer (NK) cells. In some disclosures, the cells are monocytes or granulocytes, e.g., myeloid cells, macrophages, neutrophils, dendritic cells, mast cells, eosinophils, and/or basophils.

In some disclosures, the cells include one or more nucleic acids introduced via genetic engineering, and thereby express recombinant or genetically engineered products of such nucleic acids. In some disclosures, the nucleic acids are heterologous, i.e., normally not present in a cell or sample obtained from the cell, such as one obtained from another organism or cell, which for example, is not ordinarily found in the cell being engineered and/or an organism from which such cell is derived. In some disclosures, the nucleic acids are not naturally occurring, such as a nucleic acid not found in nature, including one comprising chimeric combinations of nucleic acids encoding various domains from multiple different cell types.

In some disclosures, preparation of the engineered cells includes one or more culture and/or preparation steps. The cells for introduction of the nucleic acid encoding the transgenic receptor such as the CAR, may be isolated from a sample, such as a biological sample, e.g., one obtained from or derived from a subject. In some disclosures, the subject from which the cell is isolated is one having the disease or condition or in need of a cell therapy or to which cell therapy will be administered. The subject in some disclosures is a human in need of a particular therapeutic intervention, such as the adoptive cell therapy for which cells are being isolated, processed, and/or engineered.

Accordingly, the cells in some disclosures are primary cells, e.g., primary human cells. The samples include tissue, fluid, and other samples taken directly from the subject, as well as samples resulting from one or more processing steps, such as separation, centrifugation, genetic engineering (e.g. transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some disclosures, the sample from which the cells are derived or isolated is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some disclosures, the cells are derived from cell lines, e.g., T cell lines. The cells in some disclosures are obtained from a xenogeneic source, for example, from mouse, rat, non-human primate, and pig.

In some disclosures, isolation of the cells includes one or more preparation and/or non-affinity based cell separation steps. In some disclosures, cells are washed, centrifuged, and/or incubated in the presence of one or more reagents, for example, to remove unwanted components, enrich for desired components, lyse or remove cells sensitive to particular reagents. In some disclosures, cells are separated based on one or more property, such as density, adherent properties, size, sensitivity and/or resistance to particular components.

In some disclosures, cells from the circulating blood of a subject are obtained, e.g., by apheresis or leukapheresis. The samples, in some disclosures, contain lymphocytes, including T cells, monocytes, granulocytes, B cells, other nucleated white blood cells, red blood cells, and/or platelets, and in some disclosures contains cells other than red blood cells and platelets.

In some disclosures, the blood cells collected from the subject are washed, e.g., to remove the plasma fraction and to place the cells in an appropriate buffer or media for subsequent processing steps. In some disclosures, the cells are washed with phosphate buffered saline (PBS). In some disclosures, the wash solution lacks calcium and/or magnesium and/or many or all divalent cations. In some disclosures, a washing step is accomplished a semi-automated "flow-through" centrifuge (for example, the Cobe 2991 cell processor, Baxter) according to the manufacturer's instructions. In some disclosures, a washing step is accomplished by tangential flow filtration (TFF) according to the manufacturer's instructions. In some disclosures, the cells are resuspended in a variety of biocompatible buffers after washing, such as, for example, Ca⁺⁺/Mg⁺⁺ free PBS. In certain disclosures, components of a blood cell sample are removed and the cells directly resuspended in culture media.

In some disclosures, the methods include density-based cell separation methods, such as the preparation of white blood cells from peripheral blood by lysing the red blood cells and centrifugation through a Percoll or Ficoll gradient.

In some disclosures, at least a portion of the selection step includes incubation of cells with a selection reagent. The incubation with a selection reagent or reagents, e.g., as part of selection methods which may be performed using one or more selection reagents for selection of one or more different cell types based on the expression or presence in or on the cell of one or more specific molecules, such as surface markers, e.g., surface proteins, intracellular markers, or nucleic acid. In some disclosures, any known method using a selection reagent or reagents for separation based on such markers may be used. In some disclosures, the selection reagent or reagents result in a separation that is affinity- or immunoaffinity-based separation. For example, the selection in some disclosures includes incubation with a reagent or reagents for separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

In some disclosures of such processes, a volume of cells is mixed with an amount of a desired affinity-based selection reagent. The immunoaffinity-based selection can be carried out using any system or method that results in a favorable energetic interaction between the cells being separated and the molecule specifically binding to the marker on the cell, e.g., the antibody or other binding partner on the solid surface, e.g., particle. In some disclosures, methods are carried out using particles such as beads, e.g. magnetic beads, that are coated with a selection agent (e.g. antibody) specific to the marker of the cells. The particles (e.g. beads) can be incubated or mixed with cells in a container, such as a tube or bag, while shaking or mixing, with a constant cell density-to-particle (e.g., bead) ratio to aid in promoting energetically favored interactions. In other cases, the methods include selection of cells in which all or a portion of the selection is carried out in the internal cavity of a centrifugal chamber, for example, under centrifugal rotation. In some disclosures, incubation of cells with selection reagents, such as immunoaffinity-based selection reagents, is performed in a centrifugal chamber. In certain disclosures, the isolation or separation is carried out using a system, device, or apparatus described in International Patent Application, Publication Number WO2009/072003, or US 20110003380 A1. In one disclosure, the system is a system as described in International Publication Number WO2016/073602.

In some disclosures, by conducting such selection steps or portions thereof (e.g., incubation with antibody-coated particles, e.g., magnetic beads) in the cavity of a centrifugal chamber, the user is able to control certain parameters, such as volume of various solutions, addition of solution during processing and timing thereof, which can provide advantages compared to other available methods. For example, the ability to decrease the liquid volume in the cavity during the incubation can increase the concentration of the particles (e.g. bead reagent) used in the selection, and thus the chemical potential of the solution, without affecting the total number of cells in the cavity. This in turn can enhance the pairwise interactions between the cells being processed and the particles used for selection. In some disclosures, carrying out the incubation step in the chamber, e.g., when associated with the systems, circuitry, and control as described herein, permits the user to effect agitation of the solution at desired time(s) during the incubation, which also can improve the interaction.

In some disclosures, at least a portion of the selection step is performed in a centrifugal chamber, which includes incubation of cells with a selection reagent. In some disclosures of such processes, a volume of cells is mixed with an amount of a desired affinity-based selection reagent that is far less than is normally employed when performing similar selections in a tube or container for selection of the same number of cells and/or volume of cells according to manufacturer's instructions. In some disclosures, an amount of selection reagent or reagents that is/are no more than 5%, no more than 10%, no more than 15%, no more than 20%, no more than 25%, no more than 50%, no more than 60%, no more than 70% or no more than 80% of the amount of the same selection reagent(s) employed for selection of cells in a tube or container-based incubation for the same number of cells and/or the same volume of cells according to manufacturer's instructions is employed.

In some disclosures, for selection, e.g., immunoaffinity-based selection of the cells, the cells are incubated in the cavity of the chamber in a composition that also contains the selection buffer with a selection reagent, such as a molecule that specifically binds to a surface marker on a cell that it desired to enrich and/or deplete, but not on other cells in the composition, such as an antibody, which optionally is coupled to a scaffold such as a polymer or surface, e.g., bead, e.g., magnetic bead, such as magnetic beads coupled to monoclonal antibodies specific for CD4 and CD8. In some disclosures, as described, the selection reagent is added to cells in the cavity of the chamber in an amount that is substantially less than (e.g. is no more than 5%, 10%, 20%, 30%, 40%, 50%, 60%, 70% or 80% of the amount) as compared to the amount of the selection reagent that is typically used or would be necessary to achieve about the same or similar efficiency of selection of the same number of cells or the same volume of cells when selection is performed in a tube with shaking or rotation. In some disclosures, the incubation is performed with the addition of a selection buffer to the cells and selection reagent to achieve a target volume with incubation of the reagent of, for example, 10 mL to 200 mL, such as at least or at least about 10 mL, 20 mL, 30 mL, 40 mL, 50 mL, 60 mL, 70 mL, 80 mL, 90 mL, 100 mL, 150 mL or 200 mL. In some disclosures, the selection buffer and selection reagent are pre-mixed before addition to the cells. In some disclosures, the selection buffer and selection reagent are separately added to the cells. In some disclosures, the selection incubation is carried out with periodic gentle mixing condition, which can aid in promoting energetically favored interactions and thereby permit the use of less overall selection reagent while achieving a high selection efficiency.

In some disclosures, the total duration of the incubation with the selection reagent is from or from about 5 minutes to 6 hours, such as 30 minutes to 3 hours, for example, at least or at least about 30 minutes, 60 minutes, 120 minutes or 180 minutes.

In some disclosures, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some disclosures, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In some disclosures, such process is carried out within the entirely closed system to which the chamber is integral. In some disclosures, this process (and in some disclosures also one or more additional step, such as a previous wash step washing a sample containing the cells, such as an apheresis sample) is carried out in an automated fashion, such that the cells, reagent, and other components are drawn into and pushed out of the chamber at appropriate times and centrifugation effected, so as to complete the wash and binding step in a single closed system using an automated program.

In some disclosures, after the incubation and/or mixing of the cells and selection reagent and/or reagents, the incubated cells are subjected to a separation to select for cells based on the presence or absence of the particular reagent or reagents. In some disclosures, the separation is performed in the same closed system in which the incubation of cells with the selection reagent was performed. In some disclosures, after incubation with the selection reagents, incubated cells, including cells in which the selection reagent has bound are transferred into a system for immunoaffinity-based separation of the cells. In some disclosures, the system for immunoaffinity-based separation is or contains a magnetic separation column.

In some disclosures, the isolation methods include the separation of different cell types based on the expression or presence in the cell of one or more specific molecules, such as surface markers, e.g., surface proteins, intracellular markers, or nucleic acid. In some disclosures, any known method for separation based on such markers may be used. In some disclosures, the separation is affinity- or immunoaffinity-based separation. For example, the isolation in some disclosures includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some disclosures, both fractions are retained for further use. In some disclosures, negative selection can be particularly useful where no antibody is available that specifically identifies a cell type in a heterogeneous population, such that separation is best carried out based on markers expressed by cells other than the desired population.

The separation need not result in 100% enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some disclosures, multiple rounds of separation steps are carried out, where the positively or negatively selected fraction from one step is subjected to another separation step, such as a subsequent positive or negative selection. In some disclosures, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some disclosures, specific subpopulations of T cells, such as cells positive or expressing high levels of one or more surface markers, e.g., CD28⁺, CD62L⁺, CCR7⁺, CD27⁺, CD127⁺, CD4⁺, CD8⁺, CD45RA⁺, and/or CD45RO⁺ T cells, are isolated by positive or negative selection techniques.

For example, CD3⁺, CD28⁺ T cells can be positively selected using anti-CD3/anti-CD28 conjugated magnetic beads (e.g., DYNABEADS^{®} M-450 CD3/CD28 T Cell Expander).

In some disclosures, isolation is carried out by enrichment for a particular cell population by positive selection, or depletion of a particular cell population, by negative selection. In some disclosures, positive or negative selection is accomplished by incubating cells with one or more antibodies or other binding agent that specifically bind to one or more surface markers expressed or expressed (marker⁺) at a relatively higher level (marker^{high} on the positively or negatively selected cells, respectively.

In particular disclosures, a biological sample, e.g., a sample of PBMCs or other white blood cells, are subjected to selection of CD4+ T cells, where both the negative and positive fractions are retained. In certain disclosures, CD8+ T cells are selected from the negative fraction. In some disclosures, a biological sample is subjected to selection of CD8+ T cells, where both the negative and positive fractions are retained. In certain disclosures, CD4+ T cells are selected from the negative fraction.

In some disclosures, T cells are separated from a PBMC sample by negative selection of markers expressed on non-T cells, such as B cells, monocytes, or other white blood cells, such as CD14. In some disclosures, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

In some disclosures, CD8⁺ cells are further enriched for or depleted of naive, central memory, effector memory, and/or central memory stem cells, such as by positive or negative selection based on surface antigens associated with the respective subpopulation. In some disclosures, enrichment for central memory T (T_{CM}) cells is carried out to increase efficacy, such as to improve long-term survival, expansion, and/or engraftment following administration, which in some disclosures is particularly robust in such sub-populations. *See* Terakura et al. (2012) Blood. 1:72-82; Wang et al. (2012) J Immunother. 35(9):689-701. In some disclosures, combining T_{CM}-enriched CD8⁺ T cells and CD4⁺ T cells further enhances efficacy.

In disclosures, memory T cells are present in both CD62L⁺ and CD62L⁻ subsets of CD8⁺ peripheral blood lymphocytes. PBMC can be enriched for or depleted of CD62L⁻CD8⁺ and/or CD62L⁺CD8⁺ fractions, such as using anti-CD8 and anti-CD62L antibodies.

In some disclosures, the enrichment for central memory T (T_{CM}) cells is based on positive or high surface expression of CD45RO, CD62L, CCR7, CD28, CD3, and/or CD127; in some disclosures, it is based on negative selection for cells expressing or highly expressing CD45RA and/or granzyme B. In some disclosures, isolation of a CD8⁺ population enriched for T_{CM} cells is carried out by depletion of cells expressing CD4, CD14, CD45RA, and positive selection or enrichment for cells expressing CD62L. In one disclosure, enrichment for central memory T (T_{CM}) cells is carried out starting with a negative fraction of cells selected based on CD4 expression, which is subjected to a negative selection based on expression of CD14 and CD45RA, and a positive selection based on CD62L. Such selections in some disclosures are carried out simultaneously and in other disclosures are carried out sequentially, in either order. In some disclosures, the same CD4 expression-based selection step used in preparing the CD8⁺ cell population or subpopulation, also is used to generate the CD4⁺ cell population or sub-population, such that both the positive and negative fractions from the CD4-based separation are retained and used in subsequent steps of the methods, optionally following one or more further positive or negative selection steps.

In a particular disclosure, a sample of PBMCs or other white blood cell sample is subjected to selection of CD4⁺ cells, where both the negative and positive fractions are retained. The negative fraction then is subjected to negative selection based on expression of CD14 and CD45RA or CD19, and positive selection based on a marker characteristic of central memory T cells, such as CD62L or CCR7, where the positive and negative selections are carried out in either order.

CD4⁺ T helper cells are sorted into naïve, central memory, and effector cells by identifying cell populations that have cell surface antigens. CD4⁺ lymphocytes can be obtained by standard methods. In some disclosures, naive CD4⁺ T lymphocytes are CD45RO⁻, CD45RA⁺, CD62L⁺, CD4⁺ T cells. In some disclosures, central memory CD4⁺ cells are CD62L⁺ and CD45RO⁺. In some disclosures, effector CD4⁺ cells are CD62L⁻ and CD45RO⁻.

In one disclosure, to enrich for CD4⁺ cells by negative selection, a monoclonal antibody cocktail typically includes antibodies to CD14, CD20, CD11b, CD16, HLA-DR, and CD8. In some disclosures, the antibody or binding partner is bound to a solid support or matrix, such as a magnetic bead or paramagnetic bead, to allow for separation of cells for positive and/or negative selection. For example, in some disclosures, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In Vitro and In Vivo, p 17-25 Edited by: S. A. Brooks and U. Schumacher © Humana Press Inc., Totowa, NJ).

In some disclosures, the sample or composition of cells to be separated is incubated with small, magnetizable or magnetically responsive material, such as magnetically responsive particles or microparticles, such as paramagnetic beads (e.g., such as Dynalbeads or MACS beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some disclosures, the magnetic particle or bead comprises a magnetically responsive material bound to a specific binding member, such as an antibody or other binding partner. There are many well-known magnetically responsive materials used in magnetic separation methods. Suitable magnetic particles include those described in Molday, U.S. Pat. No. 4,452,773, and in European Patent Specification EP 452342 B. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some disclosures, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some disclosures, a combination of positive and negative selection is performed during the same selection step, where the positive and negative fractions are retained and further processed or subject to further separation steps.

In certain disclosures, the magnetically responsive particles are coated in primary antibodies or other binding partners, secondary antibodies, lectins, enzymes, or streptavidin. In certain disclosures, the magnetic particles are attached to cells via a coating of primary antibodies specific for one or more markers. In certain disclosures, the cells, rather than the beads, are labeled with a primary antibody or binding partner, and then cell-type specific secondary antibody- or other binding partner (e.g., streptavidin)-coated magnetic particles, are added. In certain disclosures, streptavidin-coated magnetic particles are used in conjunction with biotinylated primary or secondary antibodies.

In some disclosures, the magnetically responsive particles are left attached to the cells that are to be subsequently incubated, cultured and/or engineered; in some disclosures, the particles are left attached to the cells for administration to a patient. In some disclosures, the magnetizable or magnetically responsive particles are removed from the cells. Methods for removing magnetizable particles from cells are known and include, e.g., the use of competing non-labeled antibodies, and magnetizable particles or antibodies conjugated to cleavable linkers. In some disclosures, the magnetizable particles are biodegradable.

In some disclosures, the affinity-based selection is via magnetic-activated cell sorting (MACS) (Miltenyi Biotec, Auburn, CA). Magnetic Activated Cell Sorting (MACS) systems are capable of high-purity selection of cells having magnetized particles attached thereto. In certain disclosures, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain disclosures, the non-target cells are labelled and depleted from the heterogeneous population of cells.

In certain disclosures, the isolation or separation is carried out using a system, device, or apparatus that carries out one or more of the isolation, cell preparation, separation, processing, incubation, culture, and/or formulation steps of the methods. In some disclosures, the system is used to carry out each of these steps in a closed or sterile environment, for example, to minimize error, user handling and/or contamination. In one disclosure, the system is a system as described in WO2009/072003, or US 20110003380.

In some disclosures, the system or apparatus carries out one or more, e.g., all, of the isolation, processing, engineering, and formulation steps in an integrated or self-contained system, and/or in an automated or programmable fashion. In some disclosures, the system or apparatus includes a computer and/or computer program in communication with the system or apparatus, which allows a user to program, control, assess the outcome of, and/or adjust various aspects of the processing, isolation, engineering, and formulation steps.

In some disclosures, the separation and/or other steps is carried out using CliniMACS system (Miltenyi Biotec), for example, for automated separation of cells on a clinical-scale level in a closed and sterile system. Components can include an integrated microcomputer, magnetic separation unit, peristaltic pump, and various pinch valves. The integrated computer in some disclosures controls all components of the instrument and directs the system to perform repeated procedures in a standardized sequence. The magnetic separation unit in some disclosures includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some disclosures uses antibody-coupled magnetizable particles that are supplied in a sterile, non-pyrogenic solution. In some disclosures, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some disclosures, the cell populations for use with the methods described herein are unlabeled and are not retained in the column. In some disclosures, the cell populations for use with the methods described herein are labeled and are retained in the column. In some disclosures, the cell populations for use with the methods described herein are eluted from the column after removal of the magnetic field, and are collected within the cell collection bag.

In certain disclosures, separation and/or other steps are carried out using the CliniMACS Prodigy system (Miltenyi Biotec). The CliniMACS Prodigy system in some disclosures is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood is automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. (2012) J Immunother. 35(9): 651-660, Terakura et al. (2012) Blood.1:72-82, and Wang et al. (2012) J Immunother. 35(9):689-701.

In some disclosures, a cell population described herein is collected and enriched (or depleted) via flow cytometry, in which cells stained for multiple cell surface markers are carried in a fluidic stream. In some disclosures, a cell population described herein is collected and enriched (or depleted) via preparative scale (FACS)-sorting. In certain disclosures, a cell population described herein is collected and enriched (or depleted) by use of microelectromechanical systems (MEMS) chips in combination with a FACS-based detection system (see, e.g., WO 2010/033140, Cho et al. (2010) Lab Chip 10, 1567-1573; and Godin et al. (2008) J Biophoton. 1(5):355-376. In both cases, cells can be labeled with multiple markers, allowing for the isolation of well-defined T cell subsets at high purity.

In some disclosures, the antibodies or binding partners are labeled with one or more detectable marker, to facilitate separation for positive and/or negative selection. For example, separation may be based on binding to fluorescently labeled antibodies. In some disclosures, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flow-cytometric detection system. Such methods allow for positive and negative selection based on multiple markers simultaneously.

In some disclosures, the preparation methods include steps for freezing, e.g., cryopreserving, the cells, either before or after isolation, incubation, and/or engineering. In some disclosures, the freeze and subsequent thaw step removes granulocytes and, to some extent, monocytes in the cell population. In some disclosures, the cells are suspended in a freezing solution, e.g., following a washing step to remove plasma and platelets. Any of a variety of known freezing solutions and parameters in some disclosures may be used. One disclosure involves using PBS containing 20% DMSO and 8% human serum albumin (HSA), or other suitable cell freezing media. This is then diluted 1:1 with media so that the final concentration of DMSO and HSA are 10% and 4%, respectively. The cells are generally then frozen to -80° C. at a rate of 1° per minute and stored in the vapor phase of a liquid nitrogen storage tank.

In some disclosures, the isolation and/or selection results in one or more input compositions of enriched T cells, e.g., CD3+ T cells, CD4+ T cells, and/or CD8+ T cells. In some disclosures, two or more separate input composition are isolated, selected, enriched, or obtained from a single biological sample. In some disclosures, separate input compositions are isolated, selected, enriched, and/or obtained from separate biological samples collected, taken, and/or obtained from the same subject.

In certain disclosures, the one or more input compositions is or includes a composition of enriched T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD3+ T cells. In particular disclosure, the input composition of enriched T cells consists essentially of CD3+ T cells.

In certain disclosures, the one or more input compositions is or includes a composition of enriched CD4+ T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD4+ T cells. In certain disclosures, the input composition of CD4+ T cells includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells. In some disclosures, the composition of enriched T cells consists essentially of CD4+ T cells.

In certain disclosures, the one or more compositions is or includes a composition of CD8+ T cells that is or includes at least 60%, at least 65%, at least 70%, at least 75%, at least 80%, at least 85%, at least 90%, at least 95%, at least 98%, at least 99%, at least 99.5%, at least 99.9%, or at or at about 100% CD8+ T cells. In certain disclosures, the composition of CD8+ T cells contains less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free of or substantially free of CD4+ T cells. In some disclosures, the composition of enriched T cells consists essentially of CD8+ T cells.

### 2. Activation and Stimulation

In some disclosures, the cells are incubated and/or cultured prior to or in connection with genetic engineering. The incubation steps can include culture, cultivation, stimulation, activation, and/or propagation. The incubation and/or engineering may be carried out in a culture vessel, such as a unit, chamber, well, column, tube, tubing set, valve, vial, culture dish, bag, or other container for culture or cultivating cells. In some disclosures, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some disclosures, the stimulating conditions or agents include one or more agent, e.g., ligand, which is capable of stimulating or activating an intracellular signaling domain of a TCR complex. In some disclosures, the agent turns on or initiates TCR/CD3 intracellular signaling cascade in a T cell. Such agents can include antibodies, such as those specific for a TCR, e.g. anti-CD3. In some disclosures, the stimulating conditions include one or more agent, e.g. ligand, which is capable of stimulating a costimulatory receptor, e.g., anti-CD28. In some disclosures, such agents and/or ligands may be, bound to solid support such as a bead, and/or one or more cytokines. Optionally, the expansion method may further comprise the step of adding anti-CD3 and/or anti-CD28 antibody to the culture medium (e.g., at a concentration of at least about 0.5 ng/ml). In some disclosures, the stimulating agents include IL-2, IL-15 and/or IL-7. In some disclosures, the IL-2 concentration is at least about 10 units/mL.

In some disclosures, incubation is carried out in accordance with techniques such as those described in US Patent No. 6,040,177 to Riddell et al., Klebanoff et al.(2012) J Immunother. 35(9): 651-660, Terakura et al. (2012) Blood.1:72-82, and/or Wang et al. (2012) J Immunother. 35(9):689-701.

In some disclosures, the T cells are expanded by adding to a culture-initiating composition feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g. for a time sufficient to expand the numbers of T cells). In some disclosures, the non-dividing feeder cells can comprise gamma-irradiated PBMC feeder cells. In some disclosures, the PBMC are irradiated with gamma rays in the range of about 3000 to 3600 rads to prevent cell division. In some disclosures, the feeder cells are added to culture medium prior to the addition of the populations of T cells.

In some disclosures, the stimulating conditions include temperature suitable for the growth of human T lymphocytes, for example, at least about 25 degrees Celsius, generally at least about 30 degrees, and generally at or about 37 degrees Celsius. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some disclosures is provided in any suitable amount, such as a ratio of LCL feeder cells to initial T lymphocytes of at least about 10:1.

In disclosures, antigen-specific T cells, such as antigen-specific CD4⁺ and/or CD8⁺ T cells, are obtained by stimulating naive or antigen specific T lymphocytes with antigen. For example, antigen-specific T cell lines or clones can be generated to cytomegalovirus antigens by isolating T cells from infected subjects and stimulating the cells in vitro with the same antigen.

In some disclosures, at least a portion of the incubation in the presence of one or more stimulating conditions or a stimulatory agents is carried out in the internal cavity of a centrifugal chamber, for example, under centrifugal rotation, such as described in International Publication Number WO2016/073602. In some disclosures, at least a portion of the incubation performed in a centrifugal chamber includes mixing with a reagent or reagents to induce stimulation and/or activation. In some disclosures, cells, such as selected cells, are mixed with a stimulating condition or stimulatory agent in the centrifugal chamber. In some disclosures of such processes, a volume of cells is mixed with an amount of one or more stimulating conditions or agents that is far less than is normally employed when performing similar stimulations in a cell culture plate or other system.

In some disclosures, the stimulating agent is added to cells in the cavity of the chamber in an amount that is substantially less than (e.g. is no more than 5%, 10%, 20%, 30%, 40%, 50%, 60%, 70% or 80% of the amount) as compared to the amount of the stimulating agent that is typically used or would be necessary to achieve about the same or similar efficiency of selection of the same number of cells or the same volume of cells when selection is performed without mixing in a centrifugal chamber, e.g. in a tube or bag with periodic shaking or rotation. In some disclosures, the incubation is performed with the addition of an incubation buffer to the cells and stimulating agent to achieve a target volume with incubation of the reagent of, for example, 10 mL to 200 mL, such as at least or at least about or about or 10 mL, 20 mL, 30 mL, 40 mL, 50 mL, 60 mL, 70 mL, 80 mL, 90 mL, 100 mL, 150 mL or 200 mL. In some disclosures, the incubation buffer and stimulating agent are pre-mixed before addition to the cells. In some disclosures, the incubation buffer and stimulating agent are separately added to the cells. In some disclosures, the stimulating incubation is carried out with periodic gentle mixing condition, which can aid in promoting energetically favored interactions and thereby permit the use of less overall stimulating agent while achieving stimulating and activation of cells.

In some disclosures, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some disclosures, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In some disclosures, the total duration of the incubation, e.g. with the stimulating agent, is between or between about 1 hour and 96 hours, 1 hour and 72 hours, 1 hour and 48 hours, 4 hours and 36 hours, 8 hours and 30 hours or 12 hours and 24 hours, such as at least or at least about 6 hours, 12 hours, 18 hours, 24 hours, 36 hours or 72 hours. In some disclosures, the further incubation is for a time between or about between 1 hour and 48 hours, 4 hours and 36 hours, 8 hours and 30 hours or 12 hours and 24 hours, inclusive.

In particular disclosures, the stimulating conditions include incubating, culturing, and/or cultivating a composition of enriched T cells with and/or in the presence of one or more cytokines. In particular disclosures, the one or more cytokines are recombinant cytokines. In some disclosures, the one or more cytokines are human recombinant cytokines. In certain disclosures, the one or more cytokines bind to and/or are capable of binding to receptors that are expressed by and/or are endogenous to T cells. In particular disclosures, the one or more cytokines is or includes a member of the 4-alpha-helix bundle family of cytokines. In some disclosures, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF).

In some disclosures, the stimulation results in activation and/or proliferation of the cells, for example, prior to transduction.

### 3. Vectors and Methods for Genetic Engineering

In some disclosures, engineered cells, such as T cells, used in connection with the disclosed methods, uses, articles of manufacture or compositions are cells have been genetically engineered to express a recombinant receptor, e.g., a CAR, such as those described herein. In some disclosures, the cells are engineered by introduction, delivery or transfer of nucleic acid sequences that encode the recombinant receptor and/or other molecules.

In some disclosures, methods for producing engineered cells includes the introduction of a polynucleotide encoding a recombinant receptor (e.g. anti-CD19 CAR) into a cell, e.g., such as a stimulated or activated cell. In particular disclosures, the recombinant proteins are recombinant receptors, such as any described. Introduction of the nucleic acid molecules encoding the recombinant protein, such as recombinant receptor, in the cell may be carried out using any of a number of known vectors. Such vectors include viral and non-viral systems, including lentiviral and gammaretroviral systems, as well as transposon-based systems such as PiggyBac or Sleeping Beauty-based gene transfer systems. Exemplary methods include those for transfer of nucleic acids encoding the receptors, including via viral, e.g., retroviral or lentiviral, transduction, transposons, and electroporation. In some disclosures, the engineering produces one or more engineered compositions of enriched T cells.

In certain disclosures, the one or more compositions of stimulated T cells are or include two separate stimulated compositions of enriched T cells. In particular disclosures, two separate compositions of enriched T cells, e.g., two separate compositions of enriched T cells that have been selected, isolated, and/or enriched from the same biological sample, are separately engineered. In certain disclosures, the two separate compositions include a composition of enriched CD4+ T cells. In particular disclosures, the two separate compositions include a composition of enriched CD8+ T cells. In some disclosures, two separate compositions of enriched CD4+ T cells and enriched CD8+ T cells are genetically engineered separately.

In some disclosures, gene transfer is accomplished by first stimulating the cell, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications. In certain disclosures, the gene transfer is accomplished by first incubating the cells under stimulating conditions, such as by any of the methods described.

In some disclosures, methods for genetic engineering are carried out by contacting one or more cells of a composition with a nucleic acid molecule encoding the recombinant protein, e.g. recombinant receptor. In some disclosures, the contacting can be effected with centrifugation, such as spinoculation (e.g. centrifugal inoculation). Such methods include any of those as described in International Publication Number WO2016/073602. Exemplary centrifugal chambers include those produced and sold by Biosafe SA, including those for use with the Sepax^{®} and Sepax^{®} 2 system, including an A-200/F and A-200 centrifugal chambers and various kits for use with such systems. Exemplary chambers, systems, and processing instrumentation and cabinets are described, for example, in US Patent No. 6,123,655, US Patent No. 6,733,433 and Published U.S. Patent Application, Publication No.: US 2008/0171951, and published international patent application, publication no. WO 00/38762. Exemplary kits for use with such systems include, but are not limited to, single-use kits sold by BioSafe SA under product names CS-430.1, CS-490.1, CS-600.1 or CS-900.2.

In some disclosures, the contacting can be effected with centrifugation, such as spinoculation (e.g., centrifugal inoculation). In some disclosures, the composition containing cells, the vector, e.g., viral particles and reagent can be rotated, generally at relatively low force or speed, such as speed lower than that used to pellet the cells, such as from or from about 600 rpm to 1700 rpm (e.g., at or about or at least 600 rpm, 1000 rpm, or 1500 rpm or 1700 rpm). In some disclosures, the rotation is carried at a force, e.g., a relative centrifugal force, of from or from about 100 g to 3200 g (e.g., at or about or at least at or about 100 g, 200 g, 300 g, 400 g, 500 g, 1000 g, 1500 g, 2000 g, 2500 g, 3000 g or 3200 g), as measured for example at an internal or external wall of the chamber or cavity. The term "relative centrifugal force" or RCF is generally understood to be the effective force imparted on an object or substance (such as a cell, sample, or pellet and/or a point in the chamber or other container being rotated), relative to the earth's gravitational force, at a particular point in space as compared to the axis of rotation. The value may be determined using well-known formulas, taking into account the gravitational force, rotation speed and the radius of rotation (distance from the axis of rotation and the object, substance, or particle at which RCF is being measured).

In some disclosures, the system is included with and/or placed into association with other instrumentation, including instrumentation to operate, automate, control and/or monitor aspects of the transduction step and one or more various other processing steps performed in the system, e.g. one or more processing steps that can be carried out with or in connection with the centrifugal chamber system as described herein or in International Publication Number WO2016/073602. This instrumentation in some disclosures is contained within a cabinet. In some disclosures, the instrumentation includes a cabinet, which includes a housing containing control circuitry, a centrifuge, a cover, motors, pumps, sensors, displays, and a user interface. An exemplary device is described in US Patent No. 6,123,655, US Patent No. 6,733,433 and US 2008/0171951.

In some disclosures, the system comprises a series of containers, e.g., bags, tubing, stopcocks, clamps, connectors, and a centrifuge chamber. In some disclosures, the containers, such as bags, include one or more containers, such as bags, containing the cells to be transduced and the viral vector particles, in the same container or separate containers, such as the same bag or separate bags. In some disclosures, the system further includes one or more containers, such as bags, containing medium, such as diluent and/or wash solution, which is pulled into the chamber and/or other components to dilute, resuspend, and/or wash components and/or compositions during the methods. The containers can be connected at one or more positions in the system, such as at a position corresponding to an input line, diluent line, wash line, waste line and/or output line.

In some disclosures, the chamber is associated with a centrifuge, which is capable of effecting rotation of the chamber, such as around its axis of rotation. Rotation may occur before, during, and/or after the incubation in connection with transduction of the cells and/or in one or more of the other processing steps. Thus, in some disclosures, one or more of the various processing steps is carried out under rotation, e.g., at a particular force. The chamber is typically capable of vertical or generally vertical rotation, such that the chamber sits vertically during centrifugation and the side wall and axis are vertical or generally vertical, with the end wall(s) horizontal or generally horizontal.

In some disclosures, during at least a part of the genetic engineering, e.g. transduction, and/or subsequent to the genetic engineering the cells are transferred to a bioreactor bag assembly for culture of the genetically engineered cells, such as for cultivation or expansion of the cells.

In some disclosures, recombinant nucleic acids are transferred into cells using recombinant infectious virus particles, such as, e.g., vectors derived from simian virus 40 (SV40), adenoviruses, adeno-associated virus (AAV). In some disclosures, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some disclosures, the retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV) or spleen focus forming virus (SFFV). Most retroviral vectors are derived from murine retroviruses. In some disclosures, the retroviruses include those derived from any avian or mammalian cell source. The retroviruses typically are amphotropic, meaning that they are capable of infecting host cells of several species, including humans. In one disclosure, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

In some disclosures, the viral vector particles contain a genome derived from a retroviral genome based vector, such as derived from a lentiviral genome based vector. In some instances of the disclosed viral vectors, the heterologous nucleic acid encoding a recombinant receptor, such as an antigen receptor, such as a CAR, is contained and/or located between the 5' LTR and 3' LTR sequences of the vector genome.

In some disclosures, the viral vector genome is a lentivirus genome, such as an HIV-1 genome or an SIV genome. For example, lentiviral vectors have been generated by multiply attenuating virulence genes, for example, the genes env, vif, vpu and nef can be deleted, making the vector safer for therapeutic purposes. Lentiviral vectors are known. See Naldini et al., (1996 and 1998); Zufferey et al.,

(1997); Dull et al., 1998, U.S. Pat. Nos. 6,013,516; and 5,994,136). In some disclosures, these viral vectors are plasmid-based or virus-based, and are configured to carry the essential sequences for incorporating foreign nucleic acid, for selection, and for transfer of the nucleic acid into a host cell. Known lentiviruses can be readily obtained from depositories or collections such as the American Type Culture Collection ("ATCC"; 10801 University Blvd., Manassas, Va. 20110-2209), or isolated from known sources using commonly available techniques.

Non-limiting examples of lentiviral vectors include those derived from a lentivirus, such as Human Immunodeficiency Virus 1 (HIV-1), HIV-2, an Simian Immunodeficiency Virus (SIV), Human T-lymphotropic virus 1 (HTLV-1), HTLV-2 or equine infection anemia virus (E1AV). For example, lentiviral vectors have been generated by multiply attenuating the HIV virulence genes, for example, the genes env, vif, vpr, vpu and nef are deleted, making the vector safer for therapeutic purposes. Lentiviral vectors are known in the art, see Naldini et al., (1996 and 1998); Zufferey et al., (1997); Dull et al., 1998, U.S. Pat. Nos. 6,013,516; and 5,994,136). In some disclosures, these viral vectors are plasmid-based or virus-based, and are configured to carry the essential sequences for incorporating foreign nucleic acid, for selection, and for transfer of the nucleic acid into a host cell. Known lentiviruses can be readily obtained from depositories or collections such as the American Type Culture Collection ("ATCC"; 10801 University Blvd., Manassas, Va. 20110-2209), or isolated from known sources using commonly available techniques.

In some disclosures, the viral genome vector can contain sequences of the 5' and 3' LTRs of a retrovirus, such as a lentivirus. In some disclosures, the viral genome construct may contain sequences from the 5' and 3' LTRs of a lentivirus, and in particular can contain the R and U5 sequences from the 5' LTR of a lentivirus and an inactivated or self-inactivating 3' LTR from a lentivirus. The LTR sequences can be LTR sequences from any lentivirus from any species. For example, they may be LTR sequences from HIV, SIV, FIV or BIV. Typically, the LTR sequences are HIV LTR sequences.

In some disclosures, the nucleic acid of a viral vector, such as an HIV viral vector, lacks additional transcriptional units. The vector genome can contain an inactivated or self-inactivating 3' LTR (Zufferey et al. J Virol 72: 9873, 1998; Miyoshi et al., J Virol 72:8150, 1998). For example, deletion in the U3 region of the 3' LTR of the nucleic acid used to produce the viral vector RNA can be used to generate self-inactivating (SIN) vectors. This deletion can then be transferred to the 5' LTR of the proviral DNA during reverse transcription. A self-inactivating vector generally has a deletion of the enhancer and promoter sequences from the 3' long terminal repeat (LTR), which is copied over into the 5' LTR during vector integration. In some disclosures enough sequence can be eliminated, including the removal of a TATA box, to abolish the transcriptional activity of the LTR. This can prevent production of full-length vector RNA in transduced cells. In some disclosures, the U3 element of the 3' LTR contains a deletion of its enhancer sequence, the TATA box, Sp1, and NF-kappa B sites. As a result of the self-inactivating 3' LTR, the provirus that is generated following entry and reverse transcription contains an inactivated 5' LTR. This can improve safety by reducing the risk of mobilization of the vector genome and the influence of the LTR on nearby cellular promoters. The self-inactivating 3' LTR can be constructed by any method known in the art. In some disclosures, this does not affect vector titers or the in vitro or in vivo properties of the vector.

Optionally, the U3 sequence from the lentiviral 5' LTR can be replaced with a promoter sequence in the viral construct, such as a heterologous promoter sequence. This can increase the titer of virus recovered from the packaging cell line. An enhancer sequence can also be included. Any enhancer/promoter combination that increases expression of the viral RNA genome in the packaging cell line may be used. In one disclosure, the CMV enhancer/promoter sequence is used (U.S. Pat. No. 5,385,839 and U.S. Pat. No. 5,168,062).

In certain disclosures, the risk of insertional mutagenesis can be minimized by constructing the retroviral vector genome, such as lentiviral vector genome, to be integration defective. A variety of approaches can be pursued to produce a non-integrating vector genome. In some disclosures, a mutation(s) can be engineered into the integrase enzyme component of the pol gene, such that it encodes a protein with an inactive integrase. In some disclosures, the vector genome itself can be modified to prevent integration by, for example, mutating or deleting one or both attachment sites, or making the 3' LTR-proximal polypurine tract (PPT) non-functional through deletion or modification. In some disclosures, non-genetic approaches are available; these include pharmacological agents that inhibit one or more functions of integrase. The approaches are not mutually exclusive; that is, more than one of them can be used at a time. For example, both the integrase and attachment sites can be non-functional, or the integrase and PPT site can be non-functional, or the attachment sites and PPT site can be non-functional, or all of them can be non-functional. Such methods and viral vector genomes are known and available (see Philpott and Thrasher, Human Gene Therapy 18:483, 2007; Engelman et al. J Virol 69:2729, 1995; Brown et al J Virol 73:9011 (1999); WO 2009/076524; McWilliams et al., J Virol 77:11150, 2003; Powell and Levin J Virol 70:5288, 1996).

In some disclosures, the vector contains sequences for propagation in a host cell, such as a prokaryotic host cell. In some disclosures, the nucleic acid of the viral vector contains one or more origins of replication for propagation in a prokaryotic cell, such as a bacterial cell. In some disclosures, vectors that include a prokaryotic origin of replication also may contain a gene whose expression confers a detectable or selectable marker such as drug resistance.

The viral vector genome is typically constructed in a plasmid form that can be transfected into a packaging or producer cell line. Any of a variety of known methods can be used to produce retroviral particles whose genome contains an RNA copy of the viral vector genome. In some disclosures, at least two components are involved in making a virus-based gene delivery system: first, packaging plasmids, encompassing the structural proteins as well as the enzymes necessary to generate a viral vector particle, and second, the viral vector itself, i.e., the genetic material to be transferred. Biosafety safeguards can be introduced in the design of one or both of these components.

In some disclosures, the packaging plasmid can contain all retroviral, such as HIV-1, proteins other than envelope proteins (Naldini et al., 1998). In other disclosures, viral vectors can lack additional viral genes, such as those that are associated with virulence, e.g., vpr, vif, vpu and nef, and/or Tat, a primary transactivator of HIV. In some disclosures, lentiviral vectors, such as HIV-based lentiviral vectors, comprise only three genes of the parental virus: gag, pol and rev, which reduces or eliminates the possibility of reconstitution of a wild-type virus through recombination.

In some disclosures, the viral vector genome is introduced into a packaging cell line that contains all the components necessary to package viral genomic RNA, transcribed from the viral vector genome, into viral particles. Alternatively, the viral vector genome may comprise one or more genes encoding viral components in addition to the one or more sequences, e.g., recombinant nucleic acids, of interest. In some disclosures, in order to prevent replication of the genome in the target cell, however, endogenous viral genes required for replication are removed and provided separately in the packaging cell line.

In some disclosures, a packaging cell line is transfected with one or more plasmid vectors containing the components necessary to generate the particles. In some disclosures, a packaging cell line is transfected with a plasmid containing the viral vector genome, including the LTRs, the cis-acting packaging sequence and the sequence of interest, i.e. a nucleic acid encoding an antigen receptor, such as a CAR; and one or more helper plasmids encoding the virus enzymatic and/or structural components, such as Gag, pol and/or rev. In some disclosures, multiple vectors are utilized to separate the various genetic components that generate the retroviral vector particles. In some such disclosures, providing separate vectors to the packaging cell reduces the chance of recombination events that might otherwise generate replication competent viruses. In some disclosures, a single plasmid vector having all of the retroviral components can be used.

In some disclosures, the retroviral vector particle, such as lentiviral vector particle, is pseudotyped to increase the transduction efficiency of host cells. For example, a retroviral vector particle, such as a lentiviral vector particle, in some disclosures is pseudotyped with a VSV-G glycoprotein, which provides a broad cell host range extending the cell types that can be transduced. In some disclosures, a packaging cell line is transfected with a plasmid or polynucleotide encoding a non-native envelope glycoprotein, such as to include xenotropic, polytropic or amphotropic envelopes, such as Sindbis virus envelope, GALV or VSV-G.

In some disclosures, the packaging cell line provides the components, including viral regulatory and structural proteins, that are required in trans for the packaging of the viral genomic RNA into lentiviral vector particles. In some disclosures, the packaging cell line may be any cell line that is capable of expressing lentiviral proteins and producing functional lentiviral vector particles. In some disclosures, suitable packaging cell lines include 293 (ATCC CCL X), 293T, HeLA (ATCC CCL 2), D17 (ATCC CCL 183), MDCK (ATCC CCL 34), BHK (ATCC CCL-10) and Cf2Th (ATCC CRL 1430) cells.

In some disclosures, the packaging cell line stably expresses the viral protein(s). For example, in some disclosures, a packaging cell line containing the gag, pol, rev and/or other structural genes but without the LTR and packaging components can be constructed. In some disclosures, a packaging cell line can be transiently transfected with nucleic acid molecules encoding one or more viral proteins along with the viral vector genome containing a nucleic acid molecule encoding a heterologous protein, and/or a nucleic acid encoding an envelope glycoprotein.

In some disclosures, the viral vectors and the packaging and/or helper plasmids are introduced via transfection or infection into the packaging cell line. The packaging cell line produces viral vector particles that contain the viral vector genome. Methods for transfection or infection are well known. Non-limiting examples include calcium phosphate, DEAE-dextran and lipofection methods, electroporation and microinjection.

When a recombinant plasmid and the retroviral LTR and packaging sequences are introduced into a special cell line (e.g., by calcium phosphate precipitation for example), the packaging sequences may permit the RNA transcript of the recombinant plasmid to be packaged into viral particles, which then may be secreted into the culture media. The media containing the recombinant retroviruses in some disclosures is then collected, optionally concentrated, and used for gene transfer. For example, in some disclosures, after cotransfection of the packaging plasmids and the transfer vector to the packaging cell line, the viral vector particles are recovered from the culture media and titered by standard methods used by those of skill in the art.

In some disclosures, a retroviral vector, such as a lentiviral vector, can be produced in a packaging cell line, such as an exemplary HEK 293T cell line, by introduction of plasmids to allow generation of lentiviral particles. In some disclosures, a packaging cell is transfected and/or contains a polynucleotide encoding gag and pol, and a polynucleotide encoding a recombinant receptor, such as an antigen receptor, for example, a CAR. In some disclosures, the packaging cell line is optionally and/or additionally transfected with and/or contains a polynucleotide encoding a rev protein. In some disclosures, the packaging cell line is optionally and/or additionally transfected with and/or contains a polynucleotide encoding a non-native envelope glycoprotein, such as VSV-G. In some such disclosures, approximately two days after transfection of cells, *e.g.,* HEK 293T cells, the cell supernatant contains recombinant lentiviral vectors, which can be recovered and titered.

Recovered and/or produced retroviral vector particles can be used to transduce target cells using the methods as described. Once in the target cells, the viral RNA is reverse-transcribed, imported into the nucleus and stably integrated into the host genome. One or two days after the integration of the viral RNA, the expression of the recombinant protein, e.g., antigen receptor, such as CAR, can be detected.

In some disclosures, the methods involve methods of transducing cells by contacting, e.g., incubating, a cell composition comprising a plurality of cells with a viral particle. In some disclosures, the cells to be transfected or transduced are or comprise primary cells obtained from a subject, such as cells enriched and/or selected from a subject.

In some disclosures, the concentration of cells to be transduced of the composition is from or from about 1.0 x 10⁵ cells/mL to 1.0 x 10⁸ cells/mL, such as at least or at least about or about 1.0 x 10⁵ cells/mL, 5 x 10⁵ cells/mL, 1 x 10⁶ cells/mL, 5 x 10⁶ cells/mL, 1 x 10⁷ cells/mL, 5 x 10⁷ cells/mL or 1 x 10⁸ cells/mL.

In some disclosures, the viral particles are provided at a certain ratio of copies of the viral vector particles or infectious units (IU) thereof, per total number of cells to be transduced (IU/cell). For example, in some disclosures, the viral particles are present during the contacting at or about or at least at or about 0.5, 1, 2, 3, 4, 5, 10, 15, 20, 30, 40, 50, or 60 IU of the viral vector particles per one of the cells.

In some disclosures, the titer of viral vector particles is between or between about 1 x 10⁶ IU/mL and 1 x 10⁸ IU/mL, such as between or between about 5 x 10⁶ IU/mL and 5 x 10⁷ IU/mL, such as at least 6 x 10⁶ IU/mL, 7 x 10⁶ IU/mL, 8 x 10⁶ IU/mL, 9 x 10⁶ IU/mL, 1 x 10⁷ IU/mL, 2 x 10⁷ IU/mL, 3 x 10⁷ IU/mL, 4 x 10⁷ IU/mL, or 5 x10⁷ IU/mL.

In some disclosures, transduction can be achieved at a multiplicity of infection (MOI) of less than 100, such as generally less than 60, 50, 40, 30, 20, 10, 5 or less.

In some disclosures, the method involves contacting or incubating, the cells with the viral particles. In some disclosures, the contacting is for 30 minutes to 72 hours, such as 30 minute to 48 hours, 30 minutes to 24 hours or 1 hour to 24 hours, such as at least or at least about 30 minutes, 1 hour, 2 hours, 6 hours, 12 hours, 24 hours, 36 hours or more.

In some disclosures, contacting is performed in solution. In some disclosures, the cells and viral particles are contacted in a volume of from or from about 0.5 mL to 500 mL, such as from or from about 0.5 mL to 200 mL, 0.5 mL to 100 mL, 0.5 mL to 50 mL, 0.5 mL to 10 mL, 0.5 mL to 5 mL, 5 mL to 500 mL, 5 mL to 200 mL, 5 mL to 100 mL, 5 mL to 50 mL, 5 mL to 10 mL, 10 mL to 500 mL, 10 mL to 200 mL, 10 mL to 100 mL, 10 mL to 50 mL, 50 mL to 500 mL, 50 mL to 200 mL, 50 mL to 100 mL, 100 mL to 500 mL, 100 mL to 200 mL or 200 mL to 500 mL.

In certain disclosures, the input cells are treated, incubated, or contacted with particles that comprise binding molecules that bind to or recognize the recombinant receptor that is encoded by the viral DNA.

In some disclosures, the incubation of the cells with the viral vector particles results in or produces an output composition comprising cells transduced with the viral vector particles.

In some disclosures, recombinant nucleic acids are transferred into T cells via electroporation (see, e.g., Chicaybam et al, (2013) PLoS ONE 8(3): e60298 and Van Tedeloo et al. (2000) Gene Therapy 7(16): 1431-1437). In some disclosures, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA co-precipitation (Brash et al., Mol. Cell Biol., 7: 2031-2034 (1987)).

Other approaches and vectors for transfer of the nucleic acids encoding the recombinant products are those described, e.g., in international patent application, Publication No.: WO2014055668, and U.S. Patent No. 7,446,190.

In some disclosures, the cells, e.g., T cells, may be transfected or transduced either during or after expansion e.g., with nucleic acids encoding a chimeric antigen receptor (CAR). This transduction or transfection for the introduction of the gene of the desired receptor can be carried out with any suitable retroviral vector, for example. The genetically modified cell population can then be liberated from the initial stimulus (the anti-CD3/anti-CD28 stimulus, for example) and subsequently be stimulated with a second type of stimulus e.g. via a de novo introduced receptor). This second type of stimulus may include an antigenic stimulus in form of a peptide/MHC molecule, the cognate (cross-linking) ligand of the genetically introduced receptor (e.g. natural ligand of a CAR) or any ligand (such as an antibody) that directly binds within the framework of the new receptor (e.g. by recognizing constant regions within the receptor). See, for example, Cheadle et al, "Chimeric antigen receptors for T-cell based therapy" Methods Mol Biol. 2012; 907:645-66 or Barrett et al., Chimeric Antigen Receptor Therapy for Cancer Annual Review of Medicine Vol. 65: 333-347 (2014).

In some cases, a vector may be used that does not require that the cells, e.g., T cells, are activated. In some such disclosures, the cells may be selected and/or transduced prior to activation. Thus, the cells may be engineered prior to, or subsequent to culturing of the cells, and in some cases at the same time as or during at least a portion of the culturing.

Among additional nucleic acids, e.g., genes for introduction are those to improve the efficacy of therapy, such as by promoting viability and/or function of transferred cells; genes to provide a genetic marker for selection and/or evaluation of the cells, such as to assess *in vivo* survival or localization; genes to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also the publications of PCT/US91/08442 and PCT/US94/05601 by Lupton et al. describing the use of bifunctional selectable fusion genes derived from fusing a dominant positive selectable marker with a negative selectable marker. *See,* e.g., Riddell et al., US Patent No. 6,040,177, at columns 14-17.

### 4. Cultivation, Expansion and Formulation of Engineered Cells

In some disclosures, the methods for generating the engineered cells, e.g., for cell therapy in accord with any of disclosed methods, uses, articles of manufacture or compositions, include one or more steps for cultivating cells, e.g., cultivating cells under conditions that promote proliferation and/or expansion. In some disclosures, cells are cultivated under conditions that promote proliferation and/or expansion subsequent to a step of genetically engineering, e.g., introducing a recombinant polypeptide to the cells by transduction or transfection. In particular disclosures, the cells are cultivated after the cells have been incubated under stimulating conditions and transduced or transfected with a recombinant polynucleotide, e.g., a polynucleotide encoding a recombinant receptor. Thus, in some disclosures, a composition of CAR-positive T cells that has been engineered by transduction or transfection with a recombinant polynucleotide encoding the CAR, is cultivated under conditions that promote proliferation and/or expansion.

In certain disclosures, the one or more compositions of engineered T cells are or include two separate compositions of enriched T cells, such as two separate compositions of enriched T cells that have been engineered with a polynucleotide encoding a recombinant receptor, e.g. a CAR. In particular disclosures, two separate compositions of enriched T cells, e.g., two separate compositions of enriched T cells selected, isolated, and/or enriched from the same biological sample, are separately cultivated under stimulating conditions, such as subsequent to a step of genetically engineering, e.g., introducing a recombinant polypeptide to the cells by transduction or transfection. In certain disclosures, the two separate compositions include a composition of enriched CD4+ T cells, such as a composition of enriched CD4+ T cells that have been engineered with a polynucleotide encoding a recombinant receptor, e.g. a CAR. In particular disclosures, the two separate compositions include a composition of enriched CD8+ T cells, such as a composition of enriched CD4+ T cells that have been engineered with a polynucleotide encoding a recombinant receptor, e.g. a CAR. In some disclosures, two separate compositions of enriched CD4+ T cells and enriched CD8+ T cells, such as a composition of enriched CD4+ T cells and a composition of enriched CD8+ T cells that have each been separately engineered with a polynucleotide encoding a recombinant receptor, e.g. a CAR, are separately cultivated, e.g., under conditions that promote proliferation and/or expansion.

In some disclosures, cultivation is carried out under conditions that promote proliferation and/or expansion. In some disclosures, such conditions may be designed to induce proliferation, expansion, activation, and/or survival of cells in the population. In particular disclosures, the stimulating conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to promote growth, division, and/or expansion of the cells.

In particular disclosures, the cells are cultivated in the presence of one or more cytokines. In particular disclosures, the one or more cytokines are recombinant cytokines. In some disclosures, the one or more cytokines are human recombinant cytokines. In certain disclosures, the one or more cytokines bind to and/or are capable of binding to receptors that are expressed by and/or are endogenous to T cells. In particular disclosures, the one or more cytokines, e.g. a recombinant cytokine, is or includes a member of the 4-alpha-helix bundle family of cytokines. In some disclosures, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF). In some disclosures, the one or more recombinant cytokine includes IL-2, IL-7 and/or IL-15. In some disclosures, the cells, e.g., engineered cells, are cultivated in the presence of a cytokine, e.g., a recombinant human cytokine, at a concentration of between 1 IU/mL and 2,000 IU/mL, between 10 IU/mL and 100 IU/mL, between 50 IU/mL and 200 IU/mL, between 100 IU/mL and 500 IU/mL, between 100 IU/mL and 1,000 IU/mL, between 500 IU/mL and 2,000 IU/mL, or between 100 IU/mL and 1,500 IU/mL.

In some disclosures, a composition of enriched of T cells, such as separate compositions of engineered CD4+ T cells and CD8+ T cells, is cultivated with recombinant IL-2, e.g., human recombinant IL-2, at a concentration between 2 IU/ml and 500 IU/ml, between 10 IU/ml and 250 IU/ml, between 100 IU/ml and 500 IU/ml, or between 100 IU/ml and 400 IU/ml. In particular disclosures, the composition of enriched T cells is cultivated with IL-2 at a concentration at or at about 50 IU/ml, 75 IU/ml, 100 IU/ml, 125 IU/ml, 150 IU/ml, 175 IU/ml, 200 IU/ml, 225 IU/ml, 250 IU/ml, 300 IU/ml, or 400 IU/ml. In some disclosures, the composition of enriched T cells is cultivated with recombinant IL-2 at a concentration of 200 IU/ml. In some disclosures, the composition of enriched T cells is a composition of enriched CD4+ T cells, such as a composition of engineered CD4+ T cells. In particular disclosures, the composition of enriched T cells is a composition of enriched CD8+ T cells, such as a composition of engineered CD8+ T cells.

In some disclosures, a composition of enriched T cells, such as separate compositions of engineered CD4+ T cells and CD8+ T cells, is cultivated with IL-7, e.g., human recombinant IL-7, at a concentration between 10 IU/ml and 5,000 IU/ml, between 500 IU/ml and 2,000 IU/ml, between 600 IU/ml and 1,500 IU/ml, between 500 IU/ml and 2,500 IU/ml, between 750 IU/ml and 1,500 IU/ml, or between 1,000 IU/ml and 2,000 IU/ml. In particular disclosures, the composition of enriched T cells is cultivated with IL-7 at a concentration at or at about 100 IU/ml, 200 IU/ml, 300 IU/ml, 400 IU/ml, 500 IU/ml, 600 IU/ml, 700 IU/ml, 800 IU/ml, 900 IU/ml, 1,000 IU/ml, 1,200 IU/ml, 1,400 IU/ml, or 1,600 IU/ml. In some disclosures, the cells are cultivated in the presence of recombinant IL-7 at a concertation of or of about 1,200 IU/ml. In some disclosures, the composition of enriched T cells is a composition of enriched CD4+ T cells, such as engineered CD4+ T cells.

In some disclosures, a composition of enriched T cells, such as separate compositions of engineered CD4+ T cells and CD8+ T cells, is cultivated with IL-15, e.g., human recombinant IL-15, at a concentration between 0.1 IU/ml and 200 IU/ml, between 1 IU/ml and 50 IU/ml, between 5 IU/ml and 25 IU/ml, between 25 IU/ml and 50IU/ml, between 5 IU/ml and 15 IU/ml, or between 10 IU/ml and 00 IU/ml. In particular disclosures, the composition of enriched T cells is cultivated with IL-15 at a concentration at or at about 1 IU/ml, 2 IU/ml, 3 IU/ml, 4 IU/ml, 5 IU/ml, 6 IU/ml, 7 IU/ml, 8 IU/ml, 9 IU/ml, 10 IU/ml, 11 IU/ml, 12 IU/ml, 13 IU/ml, 14 IU/ml, 15 IU/ml, 20 IU/ml, 25 IU/ml, 30 IU/ml, 40 IU/ml, 50 IU/ml, 100 IU/ml, or 200 IU/ml. In particular disclosures, a composition of enriched T cells is cultivated with recombinant IL-15 at a concentration of 20 IU/ml. In some disclosures, the composition of enriched T cells is a composition of enriched CD4+ T cells, such as engineered CD4+ T cells. In particular disclosures, the composition of enriched T cells is a composition of enriched CD8+ T cells, such as engineered CD8+ T cells.

In particular disclosures, a composition of enriched CD8+ T cells, such as engineered CD8+ T cells, is cultivated in the presence of IL-2 and/or IL-15, such as in amounts as described. In certain disclosures, a composition of enriched CD4+ T cells, such as engineered CD4+ T cells, is cultivated in the presence of IL-2, IL-7, and/or IL-15, such as in amounts as described. In some disclosures, the IL-2, IL-7, and/or IL-15 are recombinant. In certain disclosures, the IL-2, IL-7, and/or IL-15 are human. In particular disclosures, the one or more cytokines are or include human recombinant IL-2, IL-7, and/or IL-15.

In some disclosures, the cultivation is performed under conditions that generally include a temperature suitable for the growth of primary immune cells, such as human T lymphocytes, for example, at least about 25 degrees Celsius, generally at least about 30 degrees, and generally at or about 37 degrees Celsius. In some disclosures, the composition of enriched T cells is incubated at a temperature of 25 to 38°C, such as 30 to 37°C, for example at or about 37 °C ± 2 °C. In some disclosures, the incubation is carried out for a time period until the culture, e.g. cultivation or expansion, results in a desired or threshold density, number or dose of cells. In some disclosures, the incubation is greater than or greater than about or is for about or 24 hours, 48 hours, 72 hours, 96 hours, 5 days, 6 days, 7 days, 8 days, 9 days or more.

In particular disclosures, the cultivation is performed in a closed system. In certain disclosures, the cultivation is performed in a closed system under sterile conditions. In particular disclosures, the cultivation is performed in the same closed system as one or more steps of the disclosed systems. In some disclosures the composition of enriched T cells is removed from a closed system and placed in and/or connected to a bioreactor for the cultivation. Examples of suitable bioreactors for the cultivation include, but are not limited to, GE Xuri W25, GE Xuri W5, Sartorius BioSTAT RM 20 | 50, Finesse SmartRocker Bioreactor Systems, and Pall XRS Bioreactor Systems. In some disclosures, the bioreactor is used to perfuse and/or mix the cells during at least a portion of the cultivation step.

In some disclosures, the mixing is or includes rocking and/or motioning. In some cases, the bioreactor can be subject to motioning or rocking, which, in some disclosures, can increase oxygen transfer. Motioning the bioreactor may include, but is not limited to rotating along a horizontal axis, rotating along a vertical axis, a rocking motion along a tilted or inclined horizontal axis of the bioreactor or any combination thereof. In some disclosures, at least a portion of the incubation is carried out with rocking. The rocking speed and rocking angle may be adjusted to achieve a desired agitation. In some disclosures the rock angle is 20°, 19°, 18°, 17°, 16°, 15°, 14°, 13°, 12°, 11°, 10°, 9°, 8°, 7°, 6°, 5°, 4°, 3°, 2° or 1°. In certain disclosures, the rock angle is between 6-16°. In other disclosures, the rock angle is between 7-16°. In other disclosures, the rock angle is between 8-12°. In some disclosures, the rock rate is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 rpm. In some disclosures, the rock rate is between 4 and 12 rpm, such as between 4 and 6 rpm, inclusive.

In some disclosures, the bioreactor maintains the temperature at or near 37°C and CO2 levels at or near 5% with a steady air flow at, at about, or at least 0.01 L/min, 0.05 L/min, 0.1 L/min, 0.2 L/min, 0.3 L/min, 0.4 L/min, 0.5 L/min, 1.0 L/min, 1.5 L/min, or 2.0 L/min or greater than 2.0 L/min. In certain disclosures, at least a portion of the cultivation is performed with perfusion, such as with a rate of 290 ml/day, 580 ml/day, and/or 1160 ml/day, e.g., depending on the timing in relation to the start of the cultivation and/or density of the cultivated cells. In some disclosures, at least a portion of the cell culture expansion is performed with a rocking motion, such as at an angle of between 5° and 10°, such as 6°, at a constant rocking speed, such as a speed of between 5 and 15 RPM, such as 6 RMP or 10 RPM.

In some disclosures, the methods for manufacturing, generating or producing a cell therapy and/or engineered cells, in accord with the disclosed methods, uses or articles of manufacture, may include formulation of cells, such as formulation of genetically engineered cells resulting from the processing steps prior to or after the incubating, engineering, and cultivating, and/or one or more other processing steps as described. In some disclosures, one or more of the processing steps, including formulation of cells, can be carried out in a closed system. In some cases, the cells are processed in one or more steps (e.g. carried out in the centrifugal chamber and/or closed system) for manufacturing, generating or producing a cell therapy and/or engineered cells may include formulation of cells, such as formulation of genetically engineered cells resulting from the transduction processing steps prior to or after the culturing, e.g. cultivation and expansion, and/or one or more other processing steps as described. In some disclosures, the genetically engineered cells are formulated as unit dose form compositions including the number of cells for administration in a given dose or fraction thereof.

In some disclosures, the dose of cells comprising cells engineered with a recombinant antigen receptor, e.g. CAR or TCR, is provided as a composition or formulation, such as a pharmaceutical composition or formulation. Such compositions can be used in accord with the disclosed methods, such as in the treatment of diseases, conditions, and disorders, or in detection, diagnostic, and prognostic methods, and uses and articles of manufacture. In some cases, the cells can be formulated in an amount for dosage administration, such as for a single unit dosage administration or multiple dosage administration.

In some disclosures, the cells can be formulated into a container, such as a bag or vial. In some disclosures, the vial may be an infusion vial. In some disclosures, the vial is formulated with a single unit dose of the engineered cells, such as including the number of cells for administration in a given dose or fraction thereof.

In some disclosures, the cells are formulated in a pharmaceutically acceptable buffer, which may, in some disclosures, include a pharmaceutically acceptable carrier or excipient. In some disclosures, the processing includes exchange of a medium into a medium or formulation buffer that is pharmaceutically acceptable or desired for administration to a subject. In some disclosures, the processing steps can involve washing the transduced and/or expanded cells to replace the cells in a pharmaceutically acceptable buffer that can include one or more optional pharmaceutically acceptable carriers or excipients. Exemplary of such pharmaceutical forms, including pharmaceutically acceptable carriers or excipients, can be any described below in conjunction with forms acceptable for administering the cells and compositions to a subject. The pharmaceutical composition in some disclosures contains the cells in amounts effective to treat or prevent the disease or condition, such as a therapeutically effective or prophylactically effective amount.

In some disclosures, the formulation buffer contains a cryopreservative. In some disclosures, the cell are formulated with a cryopreservative solution that contains 1.0% to 30% DMSO solution, such as a 5% to 20% DMSO solution or a 5% to 10% DMSO solution. In some disclosures, the cryopreservation solution is or contains, for example, PBS containing 20% DMSO and 8% human serum albumin (HSA), or other suitable cell freezing media. In some disclosures, the cryopreservative solution is or contains, for example, at least or about 7.5% DMSO. In some disclosures, the processing steps can involve washing the transduced and/or expanded cells to replace the cells in a cryopreservative solution. In some disclosures, the cells are frozen, e.g., cryoprotected or cryopreserved, in media and/or solution with a final concentration of or of about 12.5%, 12.0%, 11.5%, 11.0%, 10.5%, 10.0%, 9.5%, 9.0%, 8.5%, 8.0%, 7.5%, 7.0%, 6.5%, 6.0%, 5.5%, or 5.0% DMSO, or between 1% and 15%, between 6% and 12%, between 5% and 10%, or between 6% and 8% DMSO. In particular disclosures, the cells are frozen, e.g., cryoprotected or cryopreserved, in media and/or solution with a final concentration of or of about 5.0%, 4.5%, 4.0%, 3.5%, 3.0%, 2.5%, 2.0%, 1.5%, 1.25%, 1.0%, 0.75%, 0.5%, or 0.25% HSA, or between 0.1% and 5%, between 0.25% and 4%, between 0.5% and 2%, or between 1% and 2% HSA.

In some disclosures, the formulation is carried out using one or more processing step including washing, diluting or concentrating the cells, such as the cultured or expanded cells. In some disclosures, the processing can include dilution or concentration of the cells to a desired concentration or number, such as unit dose form compositions including the number of cells for administration in a given dose or fraction thereof. In some disclosures, the processing steps can include a volume-reduction to thereby increase the concentration of cells as desired. In some disclosures, the processing steps can include a volume-addition to thereby decrease the concentration of cells as desired. In some disclosures, the processing includes adding a volume of a formulation buffer to transduced and/or expanded cells. In some disclosures, the volume of formulation buffer is from or from about 10 mL to 1000 mL, such as at least or at least about or about or 50 mL, 100 mL, 200 mL, 300 mL, 400 mL, 500 mL, 600 mL, 700 mL, 800 mL, 900 mL or 1000 mL.

In some disclosures, such processing steps for formulating a cell composition is carried out in a closed system. Exemplary of such processing steps can be performed using a centrifugal chamber in conjunction with one or more systems or kits associated with a cell processing system, such as a centrifugal chamber produced and sold by Biosafe SA, including those for use with the Sepax^{®} or Sepax 2^{®} cell processing systems. An exemplary system and process is described in International Publication Number WO2016/073602. In some disclosures, the method includes effecting expression from the internal cavity of the centrifugal chamber a formulated composition, which is the resulting composition of cells formulated in a formulation buffer, such as pharmaceutically acceptable buffer, in any of the above disclosures as described. In some disclosures, the expression of the formulated composition is to a container, such as the vials of the biomedical material vessels described herein, that is operably linked as part of a closed system with the centrifugal chamber. In some disclosures, the biomedical material vessels are configured for integration and or operable connection and/or is integrated or operably connected, to a closed system or device that carries out one or more processing steps. In some disclosures, the biomedical material vessel is connected to a system at an output line or output position. In some cases, the closed system is connected to the vial of the biomedical material vessel at the inlet tube. Exemplary close systems for use with the biomedical material vessels described herein include the Sepax^{®} and Sepax^{®} 2 system.

In some disclosures, the closed system, such as associated with a centrifugal chamber or cell processing system, includes a multi-port output kit containing a multi-way tubing manifold associated at each end of a tubing line with a port to which one or a plurality of containers can be connected for expression of the formulated composition. In some disclosures, a desired number or plurality of vials, can be sterilely connected to one or more, generally two or more, such as at least 3, 4, 5, 6, 7, 8 or more of the ports of the multi-port output. For example, in some disclosures, one or more containers, e.g., biomedical material vessels, can be attached to the ports, or to fewer than all of the ports. Thus, in some disclosures, the system can effect expression of the output composition into a plurality of vials of the biomedical material vessels.

In some disclosures, cells can be expressed to the one or more of the plurality of output containers, e.g., vials, in an amount for dosage administration, such as for a single unit dosage administration or multiple dosage administration. For example, in some disclosures, the vials, may each contain the number of cells for administration in a given dose or fraction thereof. Thus, each vial, in some disclosures, may contain a single unit dose for administration or may contain a fraction of a desired dose such that more than one of the plurality of vials, such as two of the vials, or 3 of the vials, together constitute a dose for administration. In some disclosures, 4 vials together constitute a dose for administration.

Thus, the containers, e.g. bags or vials, generally contain the cells to be administered, e.g., one or more unit doses thereof. The unit dose may be an amount or number of the cells to be administered to the subject or twice the number (or more) of the cells to be administered. It may be the lowest dose or lowest possible dose of the cells that would be administered to the subject. In some disclosures, the articles of manufacture includes one or more of the plurality of output containers.

In some disclosures, each of the containers, e.g. bags or vials, individually comprises a unit dose of the cells. Thus in some disclosures, each of the containers comprises the same or approximately or substantially the same number of cells. In some disclosures, each unit dose contains at or about or at least or at least about 1 x 10⁶, 2 x 10⁶, 5 x 10⁶, 1 x 10⁷, 5 x 10⁷, or 1 x 10⁸ engineered cells, total cells, T cells, or PBMCs. In some disclosures, each unit dose contains at or about or at least or at least about 1 x 10⁶, 2 x 10⁶, 5 x 10⁶, 1 x 10⁷, 5 x 10⁷, or 1 x 10⁸ CAR+ T cells that are CD3+, such as CD4+ or CD8+, or a viable subset thereof. In some disclosures, the volume of the formulated cell composition in each container, e.g. bag or vial, is between at or about 10 mL and at or about 100 mL, such as at or about or at least or at least about 20 mL, 30 mL, 40 mL, 50 mL, 60 mL, 70 mL, 80 mL, 90 mL or 100 mL. In some disclosures, the volume of the formulated cell composition in each container, e.g. bag or vial, is between at or about 1 mL and at or about 10 mL, such as between at or about 1 mL and at or about 5 mL. In some disclosures, the volume of the formulated cell composition in each container, e.g. bag or vial, is between at or about 4 mL and at or about 5 mL. In some disclosures, the volume of the formulated cell composition in each container, e.g. bag or vial, is or is about 4.4 mL. In some disclosures, the volume of the formulated cell composition in each container, e.g. bag or vial, is or is about 4.5 mL. In some disclosures, the volume of the formulated cell composition in each container, e.g. bag or vial, is or is about 4.6 mL. In some disclosures, the volume of the formulated cell composition in each container, e.g. bag or vial, is or is about 4.7 mL. In some disclosures, the volume of the formulated cell composition in each container, e.g. bag or vial, is or is about 4.8 mL. In some disclosures, the volume of the formulated cell composition in each container, e.g. bag or vial, is or is about 4.9 mL. In some disclosures, the volume of the formulated cell composition in each container, e.g. bag or vial, is or is about 5.0 mL.

In some disclosures, the formulated cell composition has a concentration of greater than at or about 0.5 x 10⁶ recombinant receptor-expressing (e.g. CAR⁺)/CD3+ cells or such viable cells per mL, greater than at or about 1.0 x 10⁶ recombinant receptor-expressing (e.g. CAR⁺)/CD3+ cells or such viable cells per mL, greater than at or about 1.5 x 10⁶ recombinant receptor-expressing (e.g. CAR⁺)/CD3+ cells or such viable cells per mL, greater than at or about 2.0 x 10⁶ recombinant receptor-expressing (e.g. CAR⁺)/CD3+ cells or such viable cells per mL. greater than at or about 2.5 x 10⁶ recombinant receptor-expressing (e.g. CAR⁺)/CD3+ cells or such viable cells per mL, greater than at or about 2.6 x 10⁶ recombinant receptor-expressing (e.g. CAR⁺)/CD3+ cells or such viable cells per mL, greater than at or about 2.7 x 10⁶ recombinant receptor-expressing (e.g. CAR⁺)/CD3+ cells or such viable cells per mL, greater than at or about 2.8 x 10⁶ recombinant receptor-expressing (e.g. CAR⁺)/CD3+ cells or such viable cells per mL, greater than at or about 2.9 x 10⁶ recombinant receptor-expressing (e.g. CAR⁺)/CD3+ cells or such viable cells per mL greater than at or about 3.0 x 10⁶ recombinant receptor-expressing (e.g. CAR⁺)/CD3+ cells or such viable cells per mL, greater than at or about 3.5 x 10⁶ recombinant receptor-expressing (e.g. CAR⁺)/CD3+ cells or such viable cells per mL, greater than at or about 4.0 x 10⁶ recombinant receptor-expressing (e.g. CAR⁺)/CD3+ cells or such viable cells per mL, greater than at or about 4.5 x 10⁶ recombinant receptor-expressing (e.g. CAR⁺)/CD3+ cells or such viable cells per mL or greater than at or about 5 x 10⁶ recombinant receptor-expressing (e.g. CAR⁺)/CD3+ cells or such viable cells per mL. In some disclosures, the CD3+ cells are CD4+ T cells. In some disclosures, the CD3+ cells are CD8+ T cells. In some disclosures, the CD3+ T cells are CD4+ and CD8+ T cells.

In some disclosures, the cells in the container, e.g. bag or vials, can be cryopreserved. In some disclosures, the container, e.g. vials, can be stored in liquid nitrogen until further use.

In some disclosures, such cells produced by the method, or a composition comprising such cells, are administered to a subject for treating a disease or condition, for example, in accord with the methods, uses and articles of manufacture described herein.

### III. COMPOSITIONS AND FORMULATIONS

In some disclosures, the cells engineered with a recombinant antigen receptor, e.g. CAR, for administration to a subject according to the methods and uses disclosed herein, is provided as a composition or formulation, such as a pharmaceutical composition or formulation. Such compositions can be used in accord with the disclosed methods or uses, and/or with the disclosed articles of manufacture or compositions, such as in the prevention or treatment of diseases, conditions, and disorders, such as a B cell malignancy or a hematological malignancy, or in detection, diagnostic, and prognostic methods.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some disclosures, the choice of carrier is determined in part by the particular cell or agent and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some disclosures, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some disclosures are included in the compositions. Suitable buffering agents include, for example, citric acid, sodium citrate, phosphoric acid, potassium phosphate, and various other acids and salts. In some disclosures, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulation or composition may also contain more than one active ingredient useful for the particular indication, disease, or condition being prevented or treated with the cells or cell compositions, where the respective activities do not adversely affect one another. Such active ingredients are suitably present in combination in amounts that are effective for the purpose intended. Thus, in some disclosures, the pharmaceutical composition further includes other pharmaceutically active agents or drugs, such as any of the combination therapy agents described herein, e.g., in Section IV, and chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. In some disclosures, the further agents are administered in the form of a salt, e.g., a pharmaceutically acceptable salt. Suitable pharmaceutically acceptable acid addition salts include those derived from mineral acids, such as hydrochloric, hydrobromic, phosphoric, metaphosphoric, nitric, and sulphuric acids, and organic acids, such as tartaric, acetic, citric, malic, lactic, fumaric, benzoic, glycolic, gluconic, succinic, and arylsulphonic acids, for example, p-toluenesulphonic acid.

The pharmaceutical composition in some disclosures contains agents or cells in amounts effective to treat or prevent the disease or condition, such as a therapeutically effective or prophylactically effective amount. Therapeutic or prophylactic efficacy in some disclosures is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

The cells and/or additional agents can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some disclosures, they are administered by parenteral, intrapulmonary, and intranasal, and, if desired for local treatment, intralesional administration. Parenteral infusions include intramuscular, intravenous, intraarterial, intraperitoneal, or subcutaneous administration. In some disclosures, a given dose is administered by a single bolus administration of the cells or agent. In some disclosures, it is administered by multiple bolus administrations of the cells or agent, for example, over a period of no more than 3 days, or by continuous infusion administration of the cells or agent.

For the prevention or treatment of disease, the appropriate dosage may depend on the type of disease to be treated, the type of agent or agents, the type of cells or recombinant receptors, the severity and course of the disease, whether the agent or cells are administered for preventive or therapeutic purposes, previous therapy, the subject's clinical history and response to the agent or the cells, and the discretion of the attending physician. The compositions are in some disclosures suitably administered to the subject at one time or over a series of treatments.

The cells and/or additional agents may be administered using standard administration techniques, formulations, and/or devices. Disclosed herein are formulations and devices, such as syringes and vials, for storage and administration of the compositions. In some disclosures, the cells and/or additional agents is administered using a small syringe. With respect to cells, administration can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell or an agent that treats or ameliorates symptoms of neurotoxicity), it will generally be formulated in a unit dosage injectable form (solution, suspension, emulsion).

Formulations include those for oral, intravenous, intraperitoneal, subcutaneous, pulmonary, transdermal, intramuscular, intranasal, buccal, sublingual, or suppository administration. In some disclosures, the agent or cell populations are administered parenterally. The term "parenteral," as used herein, includes intravenous, intramuscular, subcutaneous, rectal, vaginal, and intraperitoneal administration. In some disclosures, the agent or cell populations are administered to a subject using peripheral systemic delivery by intravenous, intraperitoneal, or subcutaneous injection.

Compositions in some disclosures are provided as sterile liquid preparations, e.g., isotonic aqueous solutions, suspensions, emulsions, dispersions, or viscous compositions, which may in some disclosures be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the agent or cells in a solvent, such as in admixture with a suitable carrier, diluent, or excipient such as sterile water, physiological saline, glucose, dextrose, or the like.

The formulations to be used for in vivo administration are generally sterile. Sterility may be readily accomplished, e.g., by filtration through sterile filtration membranes.

### IV. COMBINATION THERAPY

In some disclosures of the methods, articles of manufacture, uses or compositions, the cell therapy, e.g. dose of T cells (e.g. CAR⁺ T cells) is administered to subjects in combination with an additional therapeutic agent or therapy, generally other than the cell therapy or another cell therapy, such as other than a CAR⁺ T cell therapy. In some disclosures, the cell therapy, e.g. dose of genetically engineered T cells, such as CAR⁺ T cells, in the disclosed methods or uses, and/or with the articles of manufacture or compositions, is administered as part of a combination treatment or combination therapy, such as simultaneously with, sequentially with or intermittently with, in any order, one or more additional therapeutic intervention. In some disclosures, the one or more additional therapeutic intervention includes any agent or treatment for treating or preventing the disease or condition, such as the B cell malignancy, e.g. ALL or NHL, and/or any agent or treatment to increase the efficacy, persistence, and/or activity of the engineered cell therapy.

In some disclosures, an additional therapeutic agent or therapy is administered to subjects who are or are likely to be or who are predicted to be poor responders and/or who do not, are likely not to and/or who are predicted not to respond or do not respond within a certain time and/or to a certain extent to treatment with the cell therapy, e.g. dose of T cells (e.g. CAR⁺ T cells). In some disclosures, the additional therapeutic agent is administered to subjects who do not or are not likely to or are not predicted to exhibit a complete response or overall response, such as within 1 month, within two months or within three months after initiation of administration of the cell therapy. In some disclosures, the additional therapeutic agent is administered to subjects who exhibit or are likely to exhibit or who are predicted to exhibit progressive disease (PD), such as within 1 month, two months or three months, following administration of the cell therapy. In some disclosures, a subject is likely or predicted not to exhibit a response or a certain response based on a plurality of similarly situated subjects so treated or previously treated with the cell therapy.

In some disclosures, it is observed that a subject that may or that is more likely to exhibit a poor response to cell therapy, e.g. dose of T cells (e.g. CAR⁺ T cells) includes a subject with ALL or NHL that is or has been identified to have stable or progressive disease (SD/PD) following treatment with a prior therapy, optionally a prior therapy with a chemotherapeutic agent. In some disclosures, the additional agent or therapy can be administered prior to, concomitantly with or at the same time and/or subsequently to initiation of administration of the cell therapy, e.g. dose of T cells (e.g. CAR⁺ T cells).

In certain disclosures, it is found that the pharmacokinetics (PK) of the cell therapy in the blood of subjects following administration of the cell therapy is similar or not substantially different between subjects that respond (e.g. exhibit a CR or OR) versus do not respond (e.g. exhibit PD) to the cell therapy. In some disclosures, such observations indicate that the cell therapy has or is expanding in the subject but may not exhibit optimal efficacy.

In some contexts, optimal efficacy of a cell therapy can depend on the ability of the administered cells to recognize and bind to a target, e.g., target antigen, to traffic, localize to and successfully enter appropriate sites within the subject, tumors, and environments thereof. In some contexts, optimal efficacy can depend on the ability of the administered cells to become activated, expand, to exert various effector functions, including cytotoxic killing and secretion of various factors such as cytokines, to persist, including long-term, to differentiate, transition or engage in reprogramming into certain phenotypic states (such as long-lived memory, less-differentiated, and effector states), to avoid or reduce immunosuppressive conditions in the local microenvironment of a disease, to provide effective and robust recall responses following clearance and re-exposure to target ligand or antigen, and avoid or reduce exhaustion, anergy, peripheral tolerance, terminal differentiation, and/or differentiation into a suppressive state.

In some disclosures, the efficacy of the immunotherapy, e.g., T cell therapy, may be limited by the immunosuppressive activity or factors present in the local microenvironment of the disease or disorder, e.g., the TME. In some disclosures, the TME contains or produces factors or conditions that can suppress the activity, function, proliferation, survival and/or persistence of T cells administered for T cell therapy.

In some disclosures, administration of an additional agent or therapy, prior to, concomitantly with or at the same time and/or subsequently to initiation of administration of the cell therapy, e.g. dose of T cells (e.g. CAR⁺ T cells) can result in improved activity, efficacy and/or persistence of the cell therapy and/or improve responses of the treated subject. In some disclosures, the additional agent for combination treatment or combination therapy enhances, boosts and/or promotes the efficacy and/or safety of the therapeutic effect of the cell therapy, e.g. engineered T cell therapy, such as CAR⁺ T cells. In some disclosures, the additional agent enhances or improves the efficacy, survival or persistence of the administered cells, e.g., cells expressing the recombinant receptor, e.g. CAR.

In some disclosures, the additional agent of therapy is an antibody or a cytotoxic or therapeutic agent, e.g., a chemotherapeutic agent. In some disclosures, the one or more additional agents for treatment or therapy is an immunomodulatory agent, immune checkpoint inhibitor, adenosine pathway or adenosine receptor antagonist or agonist and kinase inhibitors. In some disclosures, the combination treatment or combination therapy includes an additional treatment, such as a surgical treatment, transplant, and/or radiation therapy.

In some disclosures, the additional agent is selected from among a protein phosphatase inhibitor, a kinase inhibitor, a cytokine, an immunomodulator, or an agent that decreases the level or activity of a regulatory T (Treg) cell. In some disclosures, the additional agent enhances safety, by virtue of reducing or ameliorating adverse effects of the administered cell therapy. In some disclosures, the additional agent can treat the same disease, condition or a comorbidity. In some disclosures, the additional agent can ameliorate, reduce or eliminate one or more toxicities, adverse effects or side effects that are associated with administration of the cells, e.g., CAR-expressing cells.

In some disclosures, the additional therapy, treatment or agent includes chemotherapy, radiation therapy, surgery, transplantation, adoptive cell therapy, antibodies, cytotoxic agents, chemotherapeutic agents, cytokines, growth inhibitory agents, anti-hormonal agents, kinase inhibitors, anti-angiogenic agents, cardioprotectants, immunostimulatory agents, immunosuppressive agents, immune checkpoint inhibitors, antibiotics, angiogenesis inhibitors, metabolic modulators or other therapeutic agents or any combination thereof. In some disclosures, the additional agent is a protein, a peptide, a nucleic acid, a small molecule agent, a cell, a toxin, a lipid, a carbohydrate or combinations thereof, or any other type of therapeutic agent, e.g. radiation. In some disclosures, the additional therapy, agent or treatment includes surgery, chemotherapy, radiation therapy, transplantation, administration of cells expressing a recombinant receptor, e.g., CAR. kinase inhibitor, immune checkpoint inhibitor, mTOR pathway inhibitor, immunosuppressive agents, immunomodulators, antibodies, immunoablative agents, antibodies and/or antigen binding fragments thereof, antibody conjugates, other antibody therapies, cytotoxins, steroids, cytokines, peptide vaccines, hormone therapy, antimetabolites, metabolic modulators, drugs that inhibit either the calcium dependent phosphatase calcineurin or the p70S6 kinase FK506) or inhibit the p70S6 kinase, alkylating agents, anthracyclines, vinca alkaloids, proteosome inhibitors, GITR agonists, protein tyrosine phosphatase inhibitors, protein kinase inhibitors, an oncolytic virus, and/or other types of immunotherapy. In some disclosures, the additional agent or treatment is bone marrow transplantation, T cell ablative therapy using chemotherapy agents such as, fludarabine, external-beam radiation therapy (XRT), cyclophosphamide, and/or antibody therapy.

In some disclosures, the additional agent is a kinase inhibitor, e.g., an inhibitor of Bruton's tyrosine kinase (Btk), e.g., ibrutinib. In some disclosures, the additional agent is an adenosine pathway or adenosine receptor antagonist or agonist. In some disclosures, the additional agent is an immunomodulator such as thalidomide or a thalidomide derivative (e.g., lenalidomide). In some disclosures, the additional therapy, agent or treatment is a cytotoxic or chemotherapy agent, a biologic therapy (e.g., antibody, e.g., monoclonal antibody, or cellular therapy), or an inhibitor (e.g., kinase inhibitor).

In some disclosures, the additional agent is a chemotherapeutic agent. Exemplary chemotherapeutic agents include an anthracycline (e.g., doxorubicin, such as liposomal doxorubicin); a vinca alkaloid (e.g., vinblastine, vincristine, vindesine, vinorelbine); an alkylating agent (e.g., cyclophosphamide, decarbazine, melphalan, ifosfamide, temozolomide); an immune cell antibody (e.g., alemtuzamab, gemtuzumab, rituximab, tositumomab); an antimetabolite (including, e.g., folic acid antagonists, pyrimidine analogs, purine analogs and adenosine deaminase inhibitors such as fludarabine); a TNFR glucocorticoid induced TNFR related protein (GITR) agonist; a proteasome inhibitor (e.g., aclacinomycin A, gliotoxin or bortezomib); an immunomodulatory such as thalidomide or a thalidomide derivative (e.g., lenalidomide).

In some disclosures, the additional agent is an immunomodulatory agent. In some disclosures, the combination therapy includes an immunomodulatory agent that can stimulate, amplify and/or otherwise enhance an anti-tumor immune response, e.g. anti-tumor immune response from the administered engineered cells, such as by inhibiting immunosuppressive signaling or enhancing immunostimulant signaling. In some disclosures, the immunomodulatory agent is a peptide, protein or is a small molecule. In some disclosures, the protein can be a fusion protein or a recombinant protein. In some disclosures, the immunomodulatory agent binds to an immunologic target, such as a cell surface receptor expressed on immune cells, such a T cells, B cells or antigen-presenting cells. For example, in some disclosures, the immunomodulatory agent is an antibody or antigen-binding antibody fragment, a fusion protein, a small molecule or a polypeptide. In some disclosures, the binding molecules, recombinant receptors, cells and/or compositions are administered in combination with an additional agent that is an antibody or an antigen-binding fragment thereof, such as a monoclonal antibody.

In some disclosures, the immunomodulatory agent blocks, inhibits or counteracts a component of the immune checkpoint pathway. The immune system has multiple inhibitory pathways that are involved in maintaining self-tolerance and for modulating immune responses. Tumors can use certain immune-checkpoint pathways as a major mechanism of immune resistance, particularly against T cells that are specific for tumor antigens (Pardoll (2012) Nature Reviews Cancer 12:252-264), e.g., engineered cells such as CAR-expressing cells. Because many such immune checkpoints are initiated by ligand-receptor interactions, they can be readily blocked by antibodies against the ligands and/or their receptors. In contrast to the majority of anti-cancer agents, checkpoint inhibitors do not necessarily target tumor cells directly, but rather target lymphocyte receptors or their ligands in order to enhance the endogenous antitumor activity of the immune system.

In some disclosures, the additional agent is an immunomodulatory agent that is an antagonist molecule or is an immune checkpoint inhibitor capable of inhibiting or blocking a function of a molecule, or signaling pathway, involving an immune checkpoint molecule. In some disclosures, the immune checkpoint molecule or pathway is PD-1, PD-L1, PD-L2, CTLA-4, LAG-3, TIM3, VISTA, adenosine 2A Receptor (A2AR), or adenosine or a pathway involving any of the foregoing. In certain disclosures, antagonistic molecules blocking an immune checkpoint pathway, such as small molecules, nucleic acid inhibitors (*e.g*., RNAi) or antibody molecules, are becoming promising avenues of immunotherapy for cancer and other diseases.

In some disclosures, the immune checkpoint inhibitor is a molecule that totally or partially reduces, inhibits, interferes with or modulate one or more checkpoint proteins. Checkpoint proteins regulate T-cell activation or function. These proteins are responsible for co-stimulatory or inhibitory interactions of T-cell responses. Immune checkpoint proteins regulate and maintain self-tolerance and the duration and amplitude of physiological immune responses.

Immune checkpoint inhibitors include any agent that blocks or inhibits in a statistically significant manner, the inhibitory pathways of the immune system. Such inhibitors may include small molecule inhibitors or may include antibodies, or antigen binding fragments thereof, that bind to and block or inhibit immune checkpoint receptors, ligands and/or receptor-ligand interaction. In some disclosures, modulation, enhancement and/or stimulation of particular receptors can overcome immune checkpoint pathway components. Illustrative immune checkpoint molecules that may be targeted for blocking, inhibition, modulation, enhancement and/or stimulation include, but are not limited to, PD-1 (CD279), PD-L1 (CD274, B7-H1), PDL2 (CD273, B7-DC), CTLA-4, LAG-3 (CD223), TIM-3, 4-1BB (CD137), 4-1BBL (CD137L), GITR (TNFRSF18, AITR), CD40, OX40 (CD134, TNFRSF4), CXCR2, tumor associated antigens (TAA), B7-H3, B7-H4, BTLA, HVEM, GAL9, B7H3, B7H4, VISTA, KIR, 2B4 (belongs to the CD2 family of molecules and is expressed on all NK, γδ, and memory CD8⁺ (αβ) T cells), CD160 (also referred to as BY55), CGEN-15049, CEACAM (e.g., CEACAM-1, CEACAM-3 and/or CEACAM-5), TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and a transforming growth factor receptor (TGFR; e.g., TGFR beta). Immune checkpoint inhibitors include antibodies, or antigen binding fragments thereof, or other binding proteins, that bind to and block or inhibit and/or enhance or stimulate the activity of one or more of any of the said molecules.

Exemplary immune checkpoint inhibitors include Tremelimumab (CTLA-4 blocking antibody, also known as ticilimumab, CP-675,206), anti-OX40, PD-L1 monoclonal antibody (Anti-B7-H1; MEDI4736), MK-3475 (PD-1 blocker), nivolumab (anti-PD-1 antibody), CT-011 (anti-PD-1 antibody), BY55 monoclonal antibody, AMP224 (anti-PD-L1 antibody), BMS-936559 (anti-PD-L1 antibody), MPLDL3280A (anti-PD-L1 antibody), MSB0010718C (anti-PD-L1 antibody) and ipilimumab (anti-CTLA-4 antibody, also known as Yervoy^{®}, MDX-010 and MDX-101). Exemplary of immunomodulatory antibodies include, but are not limited to, Daclizumab (Zenapax), Bevacizumab (Avastin ^{®}), Basiliximab, Ipilimumab, Nivolumab, pembrolizumab, MPDL3280A, Pidilizumab (CT-011), MK-3475, BMS-936559, MPDL3280A (Atezolizumab), tremelimumab, IMP321, BMS-986016, LAG525, urelumab, PF-05082566, TRX518, MK-4166, dacetuzumab (SGN-40), lucatumumab (HCD122), SEA-CD40, CP-870, CP-893, MEDI6469, MEDI6383, MOXR0916, AMP-224, MSB0010718C (Avelumab), MEDI4736, PDR001, rHIgM12B7, Ulocuplumab, BKT140, Varlilumab (CDX-1127), ARGX-110, MGA271, lirilumab (BMS-986015, IPH2101), IPH2201, ARGX-115, Emactuzumab, CC-90002 and MNRP1685A or an antibody-binding fragment thereof. Other exemplary immunomodulators include, e.g., afutuzumab (available from Roche^{®}); pegfilgrastim (Neulasta^{®}); lenalidomide (CC-5013, Revlimid^{®}); thalidomide (Thalomid^{®}), actimid (CC4047); and IRX-2 (mixture of human cytokines including interleukin 1, interleukin 2, and interferon.gamma., CAS 951209-71-5, available from IRX Therapeutics).

In some disclosures, the additional agent that is administered in accord with the disclosed methods, and/or with the disclosed articles of manufacture or compositions, is an agent that binds to and/or inhibits Programmed cell death 1 (PD-1). PD-1 is an immune checkpoint protein that is expressed in B cells, NK cells, and T cells (Shinohara et al., 1995, Genomics 23:704-6; Blank et al., 2007, Cancer Immunol Immunother 56:739-45; Finger et al., 1997, Gene 197:177-87; Pardoll (2012) Nature Reviews Cancer 12:252-264). The major role of PD-1 is to limit the activity of T cells in peripheral tissues during inflammation in response to infection, as well as to limit autoimmunity. PD-1 expression is induced in activated T cells and binding of PD-1 to one of its endogenous ligands acts to inhibit T-cell activation by inhibiting stimulatory kinases. PD-1 also acts to inhibit the TCR "stop signal." PD-1 is highly expressed on Treg cells and may increase their proliferation in the presence of ligand (Pardoll (2012) Nature Reviews Cancer 12:252-264). Anti-PD 1 antibodies have been used for treatment of melanoma, non-small-cell lung cancer, bladder cancer, prostate cancer, colorectal cancer, head and neck cancer, triple-negative breast cancer, leukemia, lymphoma and renal cell cancer (Topalian et al., 2012, N Engl J Med 366:2443-54; Lipson et al., 2013, Clin Cancer Res 19:462-8; Berger et al., 2008, Clin Cancer Res 14:3044-51; Gildener-Leapman et al., 2013, Oral Oncol 49:1089-96; Menzies & Long, 2013, Ther Adv Med Oncol 5:278-85). Exemplary anti-PD-1 antibodies include nivolumab (Opdivo by BMS), pembrolizumab (Keytruda by Merck), pidilizumab (CT-011 by Cure Tech), lambrolizumab (MK-3475 by Merck), and AMP-224 (Merck), nivolumab (also referred to as Opdivo, BMS-936558 or MDX1106; Bristol-Myers Squibb) is a fully human IgG4 monoclonal antibody which specifically blocks PD-1. Nivolumab (clone 5C4) and other human monoclonal antibodies that specifically bind to PD-1 are described in US 8,008,449 and WO2006/121168. Pidilizumab (CT-011; Cure Tech) is a humanized IgG1k monoclonal antibody that binds to PD-1. Pidilizumab and other humanized anti-PD-1 monoclonal antibodies are described in WO2009/101611. Pembrolizumab (formerly known as lambrolizumab, and also referred to as Keytruda, MK03475; Merck) is a humanized IgG4 monoclonal antibody that binds to PD-1. Pembrolizumab and other humanized anti-PD-1 antibodies are described in US 8,354,509 and WO2009/114335. Other anti-PD-1 antibodies include AMP 514 (Amplimmune), among others, e.g., anti-PD-1 antibodies described in US 8,609,089, US 2010028330, US 20120114649 and/or US 20150210769. AMP-224 (B7-DClg; Amplimmune; e.g., described in WO2010/027827 and WO2011/066342), is a PD-L2 Fc fusion soluble receptor that blocks the interaction between PD-1 and B7-H1.

In some disclosures, the additional agent that is administered in accord with the disclosed methods, and/or with the disclosed articles of manufacture or compositions, is an agent that binds to or inhibits PD-L1 (also known as CD274 and B7-H1) and/or PD-L2 (also known as CD273 and B7-DC). PD-L1 and PD-L2 are ligands for PD-1, found on activated T cells, B cells, myeloid cells, macrophages, and some types of tumor cells. Anti-tumor therapies have focused on anti-PD-L1 antibodies. The complex of PD-1 and PD-L1 inhibits proliferation of CD8⁺ T cells and reduces the immune response (Topalian et al., 2012, N Engl J Med 366:2443-54; Brahmer et al., 2012, N Eng J Med 366:2455-65). Anti-PD-L1 antibodies have been used for treatment of non-small cell lung cancer, melanoma, colorectal cancer, renal-cell cancer, pancreatic cancer, gastric cancer, ovarian cancer, breast cancer, and hematologic malignancies (Brahmer et al., 2012, N Eng J Med 366:2455-65; Ott et al., 2013, Clin Cancer Res 19:5300-9; Radvanyi et al., 2013, Clin Cancer Res 19:5541; Menzies & Long, 2013, Ther Adv Med Oncol 5:278-85; Berger et al., 2008, Clin Cancer Res 14:13044-51). Exemplary anti-PD-L1 antibodies include MDX-1105 (Medarex), MEDI4736 (Medimmune) MPDL3280A (Genentech), BMS-935559 (Bristol-Myers Squibb) and MSB0010718C. MEDI4736 (Medimmune) is a human monoclonal antibody that binds to PD-L1, and inhibits interaction of the ligand with PD-1. MDPL3280A (Genentech/Roche) is a human Fc optimized IgG1 monoclonal antibody that binds to PD-L1. MDPL3280A and other human monoclonal antibodies to PD-L1 are described in U.S. Patent No. 7,943,743 and U.S Publication No. 20120039906. Other anti-PD-L1 binding agents include YW243.55.S70 (see WO2010/077634) and MDX-1105 (also referred to as BMS-936559, and, e.g., anti-PD-L1 binding agents described in WO2007/005874).

In some disclosures, the additional agent that is administered in accord with the disclosed methods, and/or with the disclosed articles of manufacture or compositions, is an agent that is an inhibitor of Cytotoxic T-lymphocyte-associated antigen (CTLA-4), also known as CD152, or binds to CTLA-4. CTLA-4 is a co-inhibitory molecule that functions to regulate T-cell activation. CTLA-4 is a member of the immunoglobulin superfamily that is expressed exclusively on T-cells. CTLA-4 acts to inhibit T-cell activation and is reported to inhibit helper T-cell activity and enhance regulatory T-cell immunosuppressive activity. Although the precise mechanism of action of CTLA-4 remains under investigation, it has been suggested that it inhibits T cell activation by outcompeting CD28 in binding to CD80 and CD86, as well as actively delivering inhibitor signals to the T cell (Pardoll (2012) Nature Reviews Cancer 12:252-264). Anti-CTLA-4 antibodies have been used in clinical trials for the treatment of melanoma, prostate cancer, small cell lung cancer, non-small cell lung cancer (Robert & Ghiringhelli, 2009, Oncologist 14:848-61; Ott et al., 2013, Clin Cancer Res 19:5300; Weber, 2007, Oncologist 12:864-72; Wada et al., 2013, J Transl Med 11:89). A significant feature of anti-CTLA-4 is the kinetics of anti-tumor effect, with a lag period of up to 6 months after initial treatment required for physiologic response. In some cases, tumors may actually increase in size after treatment initiation, before a reduction is seen (Pardoll (2012) Nature Reviews Cancer 12:252-264). Exemplary anti-CTLA-4 antibodies include ipilimumab (Bristol-Myers Squibb) and tremelimumab (Pfizer). Ipilimumab has recently received FDA approval for treatment of metastatic melanoma (Wada et al., 2013, J Transl Med 11:89).

In some disclosures, the additional agent that is administered in accord with the disclosed methods, and/or with the disclosed articles of manufacture or compositions, is an agent that bind to and/or inhibits Lymphocyte activation gene-3 (LAG-3), also known as CD223. LAG-3 is another immune checkpoint protein. LAG-3 has been associated with the inhibition of lymphocyte activity and in some cases the induction of lymphocyte anergy. LAG-3 is expressed on various cells in the immune system including B cells, NK cells, and dendritic cells. LAG-3 is a natural ligand for the MHC class II receptor, which is substantially expressed on melanoma-infiltrating T cells including those endowed with potent immune-suppressive activity. Exemplary anti-LAG-3 antibodies include BMS-986016 (Bristol-Myers Squib), which is a monoclonal antibody that targets LAG-3. IMP701 (Immutep) is an antagonist LAG-3 antibody and IMP731 (Immutep and GlaxoSmithKline) is a depleting LAG-3 antibody. Other LAG-3 inhibitors include IMP321 (Immutep), which is a recombinant fusion protein of a soluble portion of LAG-3 and Ig that binds to MHC class II molecules and activates antigen presenting cells (APC). Other antibodies are described, e.g., in WO2010/019570 and US 2015/0259420.

In some disclosures, the additional agent that is administered in accord with the disclosed methods, and/or with the disclosed articles of manufacture or compositions, is an agent that bins to and/or inhibits T-cell immunoglobulin domain and mucin domain-3 (TIM-3). TIM-3 was initially identified on activated Th1 cells, has been shown to be a negative regulator of the immune response. Blockade of TIM-3 promotes T-cell mediated anti-tumor immunity and has anti-tumor activity in a range of mouse tumor models. Combinations of TIM-3 blockade with other immunotherapeutic agents such as TSR-042, anti-CD137 antibodies and others, can be additive or synergistic in increasing anti-tumor effects. TIM-3 expression has been associated with a number of different tumor types including melanoma, NSCLC and renal cancer, and additionally, expression of intratumoral TIM-3 has been shown to correlate with poor prognosis across a range of tumor types including NSCLC, cervical, and gastric cancers. Blockade of TIM-3 is also of interest in promoting increased immunity to a number of chronic viral diseases. TIM-3 has also been shown to interact with a number of ligands including galectin-9, phosphatidylserine and HMGB1, although which of these, if any, are relevant in regulation of anti-tumor responses is not clear at present. In some disclosures, antibodies, antibody fragments, small molecules, or peptide inhibitors that target TIM-3 can bind to the IgV domain of TIM-3 to inhibit interaction with its ligands. Exemplary antibodies and peptides that inhibit TIM-3 are described in US 2015/0218274, WO2013/006490 and US 2010/0247521. Other anti-TIM-3 antibodies include humanized versions of RMT3-23 (Ngiow et al., 2011, Cancer Res, 71:3540-3551), and clone 8B.2C12 (Monney et al., 2002, Nature, 415:536-541). Bi-specific antibodies that inhibit TIM-3 and PD-1 are described in US 2013/0156774.

In some disclosures, the additional agent that is administered in accord with the disclosed methods, and/or with the disclosed articles of manufacture or compositions, is an agent that is a CEACAM inhibitor (e.g., CEACAM-1, CEACAM-3, and/or CEACAM-5 inhibitor). In some disclosures, the inhibitor of CEACAM is an anti-CEACAM antibody molecule. Exemplary anti-CEACAM-1 antibodies are described in WO 2010/125571, WO 2013/082366 WO 2014/059251 and WO 2014/022332, e.g., a monoclonal antibody 34B1, 26H7, and 5F4; or a recombinant form thereof, as described in, e.g., US 2004/0047858, US 7,132,255 and WO 99/052552. In some disclosures, the anti-CEACAM antibody binds to CEACAM-5 as described in, e.g., Zheng et al. PloS One. (2011) 6(6): e21146), or crossreacts with CEACAM-1 and CEACAM-5 as described in, e.g., WO 2013/054331 and US 2014/0271618.

In some disclosures, the additional agent that is administered in accord with the disclosed methods, and/or with the disclosed articles of manufacture or compositions, is an agent that binds to and/or inhibits 4-1BB, also known as CD137. 4-1BB is a transmembrane glycoprotein belonging to the TNFR superfamily. 4-1BB receptors are present on activated T cells and B cells and monocytes. An exemplary anti-4-1BB antibody is urelumab (BMS-663513), which has potential immunostimulatory and antineoplastic activities.

In some disclosures, the additional agent that is administered in accord with the disclosed methods, and/or with the disclosed articles of manufacture or compositions, is an agent that binds to and/or inhibits Tumor necrosis factor receptor superfamily, member 4 (TNFRSF4), also known as OX40 and CD134. TNFRSF4 is another member of the TNFR superfamily. OX40 is not constitutively expressed on resting naive T cells and acts as a secondary co-stimulatory immune checkpoint molecule. Exemplary anti-OX40 antibodies are MEDI6469 and MOXR0916 (RG7888, Genentech).

In some disclosures, the additional agent that is administered in accord with the disclosed methods, and/or with the disclosed articles of manufacture or compositions, is an agent or a molecule that decreases the regulatory T cell (Treg) population. Methods that decrease the number of (e.g., deplete) Treg cells are known and include, e.g., CD25 depletion, cyclophosphamide administration, and modulating Glucocorticoid-induced TNFR family related gene (GITR) function. GITR is a member of the TNFR superfamily that is upregulated on activated T cells, which enhances the immune system. Reducing the number of Treg cells in a subject prior to apheresis or prior to administration of engineered cells, e.g., CAR-expressing cells, can reduce the number of unwanted immune cells (e.g., Tregs) in the tumor microenvironment and reduces the subject's risk of relapse. In some disclosures, the additional agent includes a molecule targeting GITR and/or modulating GITR functions, such as a GITR agonist and/or a GITR antibody that depletes regulatory T cells (Tregs). In some disclosures, the additional agent includes cyclophosphamide. In some disclosures, the GITR binding molecule and/or molecule modulating GITR function (e.g., GITR agonist and/or Treg depleting GITR antibodies) is administered prior to the engineered cells, e.g., CAR-expressing cells. For example, in some disclosures, the GITR agonist can be administered prior to apheresis of the cells. In some disclosures, cyclophosphamide is administered to the subject prior to administration (e.g., infusion or re-infusion) of the engineered cells, e.g., CAR-expressing cells or prior to apheresis of the cells. In some disclosures, cyclophosphamide and an anti-GITR antibody are administered to the subject prior to administration (e.g., infusion or re-infusion) of the engineered cells, e.g., CAR-expressing cells or prior to apheresis of the cells.

In some disclosures, the additional agent that is administered in accord with the disclosed methods, and/or with the disclosed articles of manufacture or compositions, is an agent that is a GITR agonist. Exemplary GITR agonists include, e.g., GITR fusion proteins and anti-GITR antibodies (e.g., bivalent anti-GITR antibodies) such as, e.g., a GITR fusion protein described in U.S. Patent No. 6,111,090, European Patent No. 090505B1, U.S Patent No. 8,586,023, PCT Publication Nos.: WO 2010/003118 and 2011/090754, or an anti-GITR antibody described, e.g., in U.S. Patent No. 7,025,962, European Patent No. 1947183B1, U.S. Patent No. 7,812,135, U.S. Patent No. 8,388,967, U.S. Patent No. 8,591,886, European Patent No. EP 1866339, PCT Publication No. WO 2011/028683, PCT Publication No. WO 2013/039954, PCT Publication No. WO2005/007190, PCT Publication No. WO 2007/133822, PCT Publication No. WO2005/055808, PCT Publication No. WO 99/40196, PCT Publication No. WO 2001/03720, PCT Publication No. WO99/20758, PCT Publication No. WO2006/083289, PCT Publication No. WO 2005/115451, U.S. Patent No. 7,618,632, and PCT Publication No. WO 2011/051726. An exemplary anti-GITR antibody is TRX518.

In some disclosures, the additional agent that is administered in accord with the disclosed methods, and/or with the disclosed articles of manufacture or compositions, enhances tumor infiltration or transmigration of the administered cells, e.g., CAR-expressing cells. For example, in some disclosures, the additional agent stimulates CD40, such as CD40L, e.g., recombinant human CD40L. Cluster of differentiation 40 (CD40) is also a member of the TNFR superfamily. CD40 is a costimulatory protein found on antigen-presenting cells and mediates a broad variety of immune and inflammatory responses. CD40 is also expressed on some malignancies, where it promotes proliferation. Exemplary anti-CD40 antibodies are dacetuzumab (SGN-40), lucatumumab (Novartis, antagonist), SEA-CD40 (Seattle Genetics), and CP-870,893. In some disclosures, the additional agent that enhances tumor infiltration includes tyrosine kinase inhibitor sunitnib, 144isclosure, and/or chemokine receptors such as CCR2, CCR4, and CCR7.

In some disclosures, the additional agent that is administered in accord with the disclosed methods, and/or with the disclosed articles of manufacture or compositions, is an immunomodulatory agent that is a structural or functional analog or derivative of thalidomide and/or an inhibitor of E3 ubiquitin ligase. In some disclosures, the immunomodulatory agent binds to cereblon (CRBN). In some disclosures, the immunomodulatory agent binds to the CRBN E3 ubiquitin-ligase complex. In some disclosures, the immunomodulatory agent binds to CRBN and the CRBN E3 ubiquitin-ligase complex. In some disclosures, the immunomodulatory agent up-regulates the protein or gene expression of CRBN. In some disclosures, CRBN is the substrate adaptor for the CRL4^{CRBN} E3 ubiquitin ligase, and modulates the specificity of the enzyme. In some disclosures, binding to CRB or the CRBN E3 ubiquitin ligase complex inhibits E3 ubiquitin ligase activity. In some disclosures, the immunomodulatory agent induces the ubiqutination of KZF1 (Ikaros) and IKZF3 (Aiolos) and/or induces degradation of IKZF1 (Ikaros) and IKZF3 (Aiolos). In some disclosures, the immunomodulatory agent induces the ubiquitination of casein kinase 1A1 (CK1α) by the CRL4^{CRBN} E3 ubiquitin ligase. In some disclosures, the ubiquitination of CK1α results in CK1α degradation.

In some disclosures, the immunomodulatory agent is an inhibitor of the Ikaros (IKZF1) transcription factor. In some disclosures, the immunomodulatory agent enhances ubiquitination of Ikaros. In some disclosures, the immunomodulatory agent enhances the degradation of Ikaros. In some disclosures, the immunomodulatory agent down-regulates the protein or gene expression of Ikaros. In some disclosures, administration of the immunomodulatory agent causes a decrease in Ikaros protein levels.

In some disclosures, the immunomodulatory agent is an inhibitor of the Aiolos (IKZF3) transcription factor. In some disclosures, the immunomodulatory agent enhances ubiquitination of Aiolos. In some disclosures, the immunomodulatory agent enhances the degradation of Aiolos. In some disclosures, the immunomodulatory agent down-regulates the protein or gene expression of Aiolos. In some disclosures, administration of the immunomodulatory agent causes a decrease in Aiolos protein levels.

In some disclosures, the immunomodulatory agent is an inhibitor of both the Ikaros (IKZF1) and Aiolos (IKZF3) transcription factors. In some disclosures, the immunomodulatory agent enhances ubiquitination of both Ikaros and Aiolos. In some disclosures, the immunomodulatory agent enhances the degradation of both Ikaros and Aiolos. In some disclosures, the immunomodulatory agent enhances ubiquitination and degradation of both Ikaros and Aiolos. In some disclosures, administration of the immunomodulatory agent causes both Aiolos protein levels and Ikaros protein levels to decrease.

In some disclosures, the immunomodulatory agent is a selective cytokine inhibitory drug (SelCID). In some disclosures, the immunomodulatory agent inhibits the activity of phosphodiesterase-4 (PDE4). In some disclosures, the immunomodulatory agent suppresses the enzymatic activity of the CDC25 phosphatases. In some disclosures, the immunomodulatory agent alters the intracellular trafficking of CDC25 phosphatases.

In some disclosures, the immunomodulatory agent is thalidomide (2-(2,6-dioxopiperidin-3-yl)-lH-isoindole- 1,3(2H)-dione) or an analog or derivative of thalidomide. In certain disclosures, a thalidomide derivative includes structural variants of thalidomide that have a similar biological activity. Exemplary thalidomide derivatives include, but are not limited to lenalidomide (REVLIMMUNOMODULATORY COMPOUND^{™}; Celgene Corporation), pomalidomide (also known as ACTIMMUNOMODULATORY COMPOUND^{™} or POMALYST^{™} (Celgene Corporation)), CC-1088, CDC-501, and CDC- 801, and the compounds disclosed in U.S. Pat. Nos. 5,712,291; 7,320,991; and 8,716,315; U.S. Appl. No. 2016/0313300; and PCT Pub. Nos. WO 2002/068414 and WO 2008/154252.

In some disclosures, the immunomodulatory agent is 1-oxo- and 1,3 dioxo-2-(2,6-dioxopiperldin-3-yl) isoindolines substituted with amino in the benzo ring as described in U.S. Pat. No. 5,635,517.

In some disclosures, the immunomodulatory agent is a compound of the following formula: 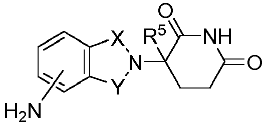 wherein one of X and Y is -C(O)- and the other of X and Y is -C(O)- or -CH₂-, and R⁵ is hydrogen or lower alkyl, or a pharmaceutically acceptable salt thereof. In some disclosures, X is -C(O)- and Y is - CH₂-. In some disclosures, both X and Y are -C(O)-. In some disclosures, R⁵ is hydrogen. In other disclosures, R⁵ is methyl.

In some disclosures, the immunomodulatory compound is a compound that belongs to a class of substituted 2-(2, 6-dioxopiperidin-3-yl)phthalimmunomodulatory compounds and substituted 2-(2,6-dioxopiperldin-3-yl)-1-oxoisoindoles, such as those described in U.S. Pat. Nos. 6,281,230; 6,316,471; 6,335,349; and 6,476,052, and International Patent Application No. PCT/US97/13375 (International Publication No. WO 98/03502).

In some disclosures, the immunomodulatory agent is a compound of the following formula: 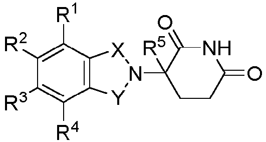 wherein
one of X and Y is -C(O)- and the other of X and Y is -C(O)- or -CH₂-;
   (1) each of R¹, R², R³, and R⁴ are independently halo, alkyl of 1 to 4 carbon atoms, or alkoxy or 1 to 4 carbon atoms, or
   (2) one of R¹, R³, R⁴, and R⁵ is -NHR^{a} and the remaining of R¹, R², R³, and R⁴ is are hydrogen,
wherein R^{a} is hydrogen or alkyl of 1 to 8 carbon atoms;
R⁵ is hydrogen or alkyl of 1 to 8 carbon atoms, benzyl, or halo; provided that R⁵ is other than hydrogen if X and Y are -C(O)- and (i) each of R¹, R², R³, and R⁴ is fluoro; or (ii) one of R¹, R², R³, and R⁴ is amino;
or a pharmaceutically acceptable salt thereof.

In some disclosures, the immunomodulatory agent is a compound that belongs to a class of isoindole-immunomodulatory compounds disclosed in U.S. Pat. No. 7,091,353, U.S. Patent Publication No. 2003/0045552, and International Application No. PCT/USOI/50401 (International Publication No. WO02/059106). For example, in some disclosures, the immunomodulatory agent is [2-(2,6-dioxo-piperidin-3-yl)-1,3-dioxo-2,3-dihydro-1H-isoindol-4-ylmethyl]-amide; (2-(2,6-dioxo-piperidin-3-yl)-1,3-dioxo-2,3-dihydro-1H-isoindol-4-ylmethyl)-carbamic acid tert-butyl ester; 4-(aminomethyl)-2-(2,6-dioxo(3-piperidyl))-isoindoline-1,3-dione; N-(2-(2,6-dioxo-piperidin-3-yl)-1,3-dioxo-2,3-dihydro-1H-isoindol-4-ylmethyl)-acetamide; N-{(2-(2,6-dioxo(3-piperidyl)-1,3-dioxoisoindolin-4-yl)methyl}cyclopropyl-carboxamide; 2-chloro-N-{(2-(2,6-dioxo(3-piperidyl))-1,3-dioxoisoindolin-4-yl)methyl}acetamide; N-(2-(2,6-dioxo(3-piperidyl))-1,3-dioxoisoindolin-4-yl)-3-pyridylcarboxamide; 3-{1-oxo-4-(benzylamino)isoindolin-2-yl}piperidine-2,6-dione; 2-(2,6-dioxo(3-piperidyl))-4-(benzylamino)isoindoline-1,3-dione; N-{(2-(2,6-dioxo(3-piperidyl))-1,3-dioxoisoindolin-4-yl)methyl}propanamide; N-{(2-(2,6-dioxo(3-piperidyl))-1,3-dioxoisoindolin-4-yl)methyl}-3-pyridylcarboxamide; N-{(2-(2,6-dioxo(3-piperidyl))-1,3-dioxoisoindolin-4-yl)methyl}heptanamide; N-{(2-(2,6-dioxo(3-piperidyl))-1,3-dioxoisoindolin-4-yl)methyl}-2-furylcarboxamide; {N-(2-(2,6-dioxo(3-piperidyl))-1,3-dioxoisoindolin-4-yl)carbamoyl}methyl acetate; N-(2-(2,6-dioxo(3-piperidyl))-1,3-dioxoisoindolin-4-yl)pentanamide; N-(2-(2,6-dioxo(3-piperidyl))-1,3-dioxoisoindolin-4-yl)-2-thienylcarboxamide; N-{[2-(2,6-dioxo(3-piperidyl))-1,3-dioxoisoindolin-4-yl|methyl}(butylamino)carboxamide; N-{[2-(2,6-dioxo(3-piperidyl))-1,3-dioxoisoindolin-4-yl]methyl}(octylamino)carboxamide; or N-{[2-(2,6-dioxo(3-piperidyl))-1,3-dioxoisoindolin-4-yl]methyl} (benzylamino)carboxamide.

In some disclosures, the immunomodulatory agent is a compound that belongs to a class of isoindole-immunomodulatory compounds disclosed in U.S. Patent Application Publication Nos. 2002/0045643, International Publication No. WO 98/54170, and U.S. Pat. No. 6,395,754. In some disclosures, the immunomodulatory agent is a tetra substituted 2-(2,6-dioxopiperdin-3-yl)-1-oxoisoindolines described in U.S. Pat. No. 5,798,368. In some disclosures, the immunomodulatory agent is 1-oxo and 1,3-dioxo-2-(2,6-dioxopiperidin-3-yl) isoindolines disclosed in U.S. Pat. No. 6,403,613. In some disclosures the immunomodulatory agent is a 1-oxo or 1,3-dioxoisoindoline substituted in the 4- or 5-position of the indoline ring as described in U.S. Pat. No. 6,380,239 and U.S. Pat. No. 7,244,759.

In some disclosures, the immunomodulatory agent is 2-(4-amino-1-oxo-1,3-dihydro-isoindol-2-yl)-4-carbamoyl-butyric acid or 4-(4-amino-1-oxo-1,3-dihydro-isoindol-2-yl)-4-carbamoyl-butyric acid. In some disclosures, the immunomodulatory compound is 4-carbamoyl-4-{4-[(furan-2-yl-methyl)-amino]-1,3-dioxo-1,3-dihydro-isoindol-2-yl}-butyric acid, 4-carbamoyl-2-{4-[(furan-2-yl-methyl)-amino]-1,3-dioxo-1,3-dihydro-isoindol-2-yl}-butyric acid, 2-{4-[(furan-2-yl-methyl)-amino]-1,3-dioxo-1,3-dihydro-isoindol-2-yl}-4-phenylcarbamoyl-butyric acid, or 2-{4-[(furan-2-yl-methyl)-amino]-1,3-dioxo-1,3-dihydro-isoindol-2-yl}-pentanedioic acid.

In some disclosures, the immunomodulatory agent is a isoindoline-1-one or isoindoline-1,3-dione substituted in the 2-position with 2,6-dioxo-3-hydroxypiperidin-5-yl as described in U.S. Pat. No. 6,458,810. In some disclosures, the immunomodulatory compound is 3-(5-amino-2-methyl-4-oxo-4H-quinazolin-3-yl)-piperidine-2,6-dione, or an enantiomer or a mixture of enantiomers thereof; or a pharmaceutically acceptable salt, solvate, hydrate, co-crystal, clathrate, or polymorph thereof. In some disclosures, the immunomodulatory compound is 3-[4-(4-morpholin-4-ylmethyl-benzyloxy)-1-oxo-1,3-dihydro-isoindol-2-yl]-piperidine-2,6-dione.

In some disclosures, the immunomodulatory agent is as described in Oshima, K. et al., Nihon Rinsho., 72(6):1130-5 (2014); Millrine, D. et al., Trends Mol Med., 23(4):348-364 (2017); and Collins, et al., Biochem J., 474(7):1127-1147 (2017).

In some disclosures, the immunomodulatory agent is lenalidomide, pomalidomide, avadomide, a stereoisomer of lenalidomide, pomalidomide, avadomide or a pharmaceutically acceptable salt, solvate, hydrate, co-crystal, clathrate, or polymorph thereof. In some disclosures, the immunomodulatory compound is lenalidomide, a stereoisomer of lenalidomide or a pharmaceutically acceptable salt, solvate, hydrate, co-crystal, clathrate, or polymorph thereof. In some disclosures, the immunomodulatory compound is lenalidomide, or ((RS)-3-(4-Amino-1-oxo-1,3-dihydro-2H-isoindol-2-yl)piperidine-2,6-dione).

In some disclosures, the additional agent includes thalidomide drugs or analogs thereof and/or derivatives thereof, such as lenalidomide, pomalidomide or apremilast. See, e.g., Bertilaccio et al., Blood (2013) 122:4171, Otahal et al., Oncoimmunology (2016) 5(4):e1115940; Fecteau et al., Blood (2014) 124(10):1637-1644 and Kuramitsu et al., Cancer Gene Therapy (2015) 22:487-495). Lenalidomide ((RS)-3-(4-Amino-1-oxo-1,3-dihydro-2H-isoindol-2-yl)piperidine-2,6-dione; also known as Revlimid) is a synthetic derivative of thalidomide, and has multiple immunomodulatory effects, including enforcement of immune synapse formation between T cell and antigen presenting cells (APCs). For example, in some cases, lenalidomide modulates T cell responses and results in increased interleukin (IL)-2 production in CD4⁺ and CD8⁺ T cells, induces the shift of T helper (Th) responses from Th2 to Th1, inhibits expansion of regulatory subset of T cells (Tregs), and improves functioning of immunological synapses in follicular lymphoma and chronic lymphocytic leukemia (CLL) (Otahal et al., Oncoimmunology (2016) 5(4):e1115940). Lenalidomide also has direct tumoricidal activity in patients with multiple myeloma (MM) and directly and indirectly modulates survival of CLL tumor cells by affecting supportive cells, such as nurse-like cells found in the microenvironment of lymphoid tissues. Lenalidomide also can enhance T-cell proliferation and interferon-γ production in response to activation of T cells via CD3 ligation or dendritic cell-mediated activation. Lenalidomide can also induce malignant B cells to express higher levels of immunostimulatory molecules such as CD80, CD86, HLA-DR, CD95, and CD40 (Fecteau et al., Blood (2014) 124(10):1637-1644). In some disclosures, lenalidomide is administered at a dosage of from about 1 mg to about 20 mg daily, e.g., from about 1 mg to about 10 mg, from about 2.5 mg to about 7.5 mg, from about 5 mg to about 15 mg, such as about 5 mg, 10 mg, 15 mg or 20 mg daily. In some disclosures, lenalidomide is administered at a dose of from about 10 µg/kg to 5 mg/kg, e.g., about 100 µg/kg to about 2 mg/kg, about 200 µg/kg to about 1 mg/kg, about 400 µg/kg to about 600 µg/kg, such as about 500 µg/kg.

In some disclosures, the additional agent that is administered in accord with the disclosed methods, and/or with the disclosed articles of manufacture or compositions, is a B-cell inhibitor. In some disclosures, the additional agent is one or more B-cell inhibitors selected from among inhibitors of CD10, CD19, CD20, CD22, CD34, CD123, CD79a, CD79b, CD179b, FLT-3, or ROR1, or a combination thereof. In some disclosures, the B-cell inhibitor is an antibody (e.g., a mono- or bispecific antibody) or an antigen binding fragment thereof. In some disclosures, the additional agent is an engineered cell expressing recombinant receptors that target B-cell targets, e.g., CD10, CD19, CD20, CD22, CD34, CD123, CD79a, CD79b, CD179b, FLT-3, or ROR1.

In some disclosures, the additional agent that is administered in accord with the disclosed methods, and/or with the disclosed articles of manufacture or compositions, is a CD20 inhibitor, e.g., an anti-CD20 antibody (e.g., an anti-CD20 mono- or bi-specific antibody) or a fragment thereof. Exemplary anti-CD20 antibodies include but are not limited to rituximab, ofatumumab, ocrelizumab (also known as GA101 or RO5072759), veltuzumab, obinutuzumab, TRU-015 (Trubion Pharmaceuticals), ocaratuzumab (also known as AME-133v or ocaratuzumab), and Pro131921 (Genentech). See, e.g., Lim et al. Haematologica. (2010) 95(1):135-43. In some disclosures, the anti-CD20 antibody comprises rituximab. Rituximab is a chimeric mouse/human monoclonal antibody IgG1 kappa that binds to CD20 and causes cytolysis of a CD20 expressing cell. In some disclosures, the additional agent includes rituximab. In some disclosures, the CD20 inhibitor is a small molecule.

In some disclosures, the additional agent that is administered in accord with the disclosed methods, and/or with the disclosed articles of manufacture or compositions, is a CD22 inhibitor, e.g., an anti-CD22 antibody (e.g., an anti-CD22 mono- or bi-specific antibody) or a fragment thereof. Exemplary anti-CD22 antibodies include epratuzumab and RFB4. In some disclosures, the CD22 inhibitor is a small molecule. In some disclosures, the antibody is a monospecific antibody, optionally conjugated to a second agent such as a chemotherapeutic agent. For instance, in some disclosures, the antibody is an anti-CD22 monoclonal antibody-MMAE conjugate (e.g., DCDT2980S). In some disclosures, the antibody is an scFv of an anti-CD22 antibody, e.g., an scFv of antibody RFB4. In some disclosures, the scFv is fused to all of or a fragment of Pseudomonas exotoxin-A (e.g., BL22). In some disclosures, the scFv is fused to all of or a fragment of (e.g., a 38 kDa fragment of) Pseudomonas exotoxin-A (e.g., moxetumomab pasudotox). In some disclosures, the anti-CD22 antibody is an anti-CD19/CD22 bispecific antibody, optionally conjugated to a toxin. For instance, in some disclosures, the anti-CD22 antibody comprises an anti-CD19/CD22 bispecific portion, (e.g., two scFv ligands, recognizing human CD19 and CD22) optionally linked to all of or a portion of diphtheria toxin (DT), e.g., first 389 amino acids of diphtheria toxin (DT), DT 390, e.g., a ligand-directed toxin such as DT2219ARL). In some disclosures, the bispecific portion (e.g., anti-CD 19/anti-CD22) is linked to a toxin such as deglycosylated ricin A chain (e.g., Combotox).

In some disclosures, the additional agent that is administered in accord with the disclosed methods, and/or with the disclosed articles of manufacture or compositions, is a cytokine or is an agent that induces increased expression of a cytokine in the tumor microenvironment. Cytokines have important functions related to T cell expansion, differentiation, survival, and homeostasis. Cytokines that can be administered to the subject receiving the combination therapy in the disclosed methods or uses, recombinant receptors, cells and/or compositions disclosed herein include one or more of IL-2, IL-4, IL-7, IL-9, IL-15, IL-18, and IL-21. In some disclosures, the cytokine administered is IL-7, IL-15, or IL-21, or a combination thereof. In some disclosures, administration of the cytokine to the subject that has sub-optimal response to the administration of the engineered cells, e.g., CAR-expressing cells improves efficacy and/or anti-tumor activity of the administered cells, e.g., CAR-expressing cells.

In some disclosures, the additional agent that is administered in accord with the disclosed methods, and/or with the disclosed articles of manufacture or compositions, is a cytokine, such as a protein that act on another cell as intercellular mediators. Examples of such cytokines are lymphokines, monokines, and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha and -beta; mullerian-inhibiting substance; mouse gonadotropin-associated peptide; inhibin; activin; vascular endothelial growth factor; integrin; thrombopoietin (TPO); nerve growth factors such as NGF-beta; platelet-growth factor; transforming growth factors (TGFs) such as TGF-alpha and TGF-beta; insulin-like growth factor-I and -II; erythropoietin (EPO); osteoinductive factors; interferons such as interferon-alpha, beta, and -gamma; colony stimulating factors (CSFs) such as macrophage-CSF (M-CSF); granulocyte-macrophage-CSF (GM-CSF); and granulocyte-CSF (G-CSF); interleukins (Ils) such as IL-1, IL-1alpha, IL-2, IL-3, IL-4, IL-5, IL-6, IL-7, IL-8, IL-9, IL-10, IL-11, IL-12; IL-15, a tumor necrosis factor such as TNF-alpha or TNF-beta; and other polypeptide factors including LIF and kit ligand (KL). As used herein, the term cytokine includes proteins from natural sources or from recombinant cell culture, and biologically active equivalents of the native sequence cytokines. For example, the immunomodulatory agent is a cytokine and the cytokine is IL-4, TNF-α, GM-CSF or IL-2.

In some disclosures, the additional agent that is administered in accord with the disclosed methods, and/or with the disclosed articles of manufacture or compositions, includes an interleukin-15 (IL-15) polypeptide, an interleukin-15 receptor alpha (IL-15Rα) polypeptide, or combination thereof, e.g., hetIL-15 (Admune Therapeutics, LLC). hetIL-15 is a heterodimeric non-covalent complex of IL-15 and IL-15Rα. hetIL-15 is described in, e.g., U.S. 8,124,084, U.S. 2012/0177598, U.S. 2009/0082299, U.S. 2012/0141413, and U.S. 2011/0081311. In some disclosures, the immunomodulatory agent can contain one or more cytokines. For example, the interleukin can include leukocyte interleukin injection (Multikine), which is a combination of natural cytokines.

In some disclosures, the additional agent that is administered in accord with the disclosed methods, and/or with the disclosed articles of manufacture or compositions, is a modulator of adenosine levels and/or an adenosine pathway component. Adenosine can function as an immunomodulatory agent in the body. For example, adenosine and some adenosine analogs that non-selectively activate adenosine receptor subtypes decrease neutrophil production of inflammatory oxidative products (Cronstein et al., Ann. N.Y. Acad. Sci. 451:291, 1985; Roberts et al., Biochem. J., 227:669, 1985; Schrier et al., J. Immunol. 137:3284, 1986; Cronstein et al., Clinical Immunol. Immunopath. 42:76, 1987). In some cases, concentration of extracellular adenosine or adenosine analogs can increase in specific environments, e.g., tumor microenvironment (TME). In some cases, adenosine or adenosine analog signaling depends on hypoxia or factors involved in hypoxia or its regulation, e.g., hypoxia inducible factor (HIF). In some disclosures, increase in adenosine signaling can increase in intracellular cAMP and cAMP-dependent protein kinase that results in inhibition of proinflammatory cytokine production, and can lead to the synthesis of immunosuppressive molecules and development of Tregs (Sitkovsky et al., Cancer Immunol Res (2014) 2(7):598-605). In some disclosures, the additional agent can reduce or reverse immunosuppressive effects of adenosine, adenosine analogs and/or adenosine signaling. In some disclosures, the additional agent can reduce or reverse hypoxia-driven A2-adenosinergic T cell immunosuppression. In some disclosures, the additional agent is selected from among antagonists of adenosine receptors, extracellular adenosine-degrading agents, inhibitors of adenosine generation by CD39/CD73 ectoenzymes, and inhibitors of hypoxia-HIF-1α signaling. In some disclosures, the additional agent is an adenosine receptor antagonist or agonist.

In some disclosures, the additional agent that is administered in accord with the disclosed methods, and/or with the disclosed articles of manufacture or compositions, is an agent that inhibits the activity and/or an amount of an adenosine receptor. Particular disclosures contemplate that inhibition or reduction of extracellular adenosine or the adenosine receptor by virtue of an inhibitor of extracellular adenosine (such as an agent that prevents the formation of, degrades, renders inactive, and/or decreases extracellular adenosine), and/or an adenosine receptor inhibitor (such as an adenosine receptor antagonist) can enhance immune response, such as a macrophage, neutrophil, granulocyte, dendritic cell, T- and/or B cell-mediated response. In addition, inhibitors of the Gs protein mediated cAMP dependent intracellular pathway and inhibitors of the adenosine receptor-triggered Gi protein mediated intracellular pathways, can also increase acute and chronic inflammation.

In some disclosures, the additional agent that is administered in accord with the disclosed methods, and/or with the disclosed articles of manufacture or compositions, is an adenosine receptor antagonist or agonist, e.g., an antagonist or agonist of one or more of the adenosine receptors A2a, A2b, A1, and A3. A1 and A3 inhibit, and A2a and A2b stimulate, respectively, adenylate cyclase activity. Certain adenosine receptors, such as A2a, A2b, and A3, can suppress or reduce the immune response during inflammation. Thus, antagonizing immunosuppressive adenosine receptors can augment, boost or enhance immune response, e.g., immune response from administered cells, e.g., CAR-expressing T cells. In some disclosures, the additional agent inhibits the production of extracellular adenosine and adenosine-triggered signaling through adenosine receptors. For example, enhancement of an immune response, local tissue inflammation, and targeted tissue destruction can be enhanced by inhibiting or reducing the adenosine-producing local tissue hypoxia; by degrading (or rendering inactive) accumulated extracellular adenosine; by preventing or decreasing expression of adenosine receptors on immune cells; and/or by inhibiting/antagonizing signaling by adenosine ligands through adenosine receptors.

In some disclosures, the additional agent that is administered in accord with the disclosed methods, and/or with the disclosed articles of manufacture or compositions, is an adenosine receptor antagonist. In some disclosures, the antagonist is small molecule or chemical compound of an adenosine receptor, such as the A2a, A2b, or A3 receptor. In some disclosures, the antagonist is a peptide, or a pepidomimetic, that binds the adenosine receptor but does not trigger a Gi protein dependent intracellular pathway. Examples of such antagonists are described in U.S. Pat. Nos. 5,565,566; 5,545, 627, 5,981,524; 5,861,405; 6,066,642; 6,326,390; 5,670,501; 6,117,998; 6,232,297; 5,786,360; 5,424,297; 6,313,131, 5,504,090; and 6,322,771.

In some disclosures, the additional agent is an A2 receptor (A2R) antagonist, such as an A2a antagonist. Exemplary A2R antagonists include, but are not limited to, KW6002 (istradefyline), SCH58261, caffeine, paraxanthine, 3,7-dimethyl-1-propargylxanthine (DMPX), 8-(m-chlorostyryl) caffeine (CSC), MSX-2, MSX-3, MSX-4, CGS-15943, ZM-241385, SCH-442416, preladenant, vipadenant (BII014), V2006, ST-1535, SYN-115, PSB-1115, ZM241365, FSPTP, and an inhibitory nucleic acid targeting A2R expression, e.g., siRNA or shRNA, or any antibodies or antigen-binding fragment thereof that targets an A2R. In some disclosures, the additional agent is an A2R antagonist described in, e.g., Ohta et al., Proc Natl Acad Sci U S A (2006) 103:13132-13137; Jin et al., Cancer Res. (2010) 70(6):2245-2255; Leone et al., Computational and Structural Biotechnology Journal (2015) 13:265-272; Beavis et al., Proc Natl Acad Sci U S A (2013) 110:14711-14716; and Pinna, A., Expert Opin Investig Drugs (2009) 18:1619-1631; Sitkovsky et al., Cancer Immunol Res (2014) 2(7):598-605; US 8,080,554; US 8,716,301; US 20140056922; WO2008/147482; US 8,883,500; US 20140377240; WO02/055083; US 7,141,575; US 7,405,219; US 8,883,500; US 8,450,329 and US 8,987,279).

In particular disclosures, an adenosine receptor antagonist that is an antisense molecule, inhibitory nucleic acid molecule (e.g., small inhibitory RNA (siRNA)) or catalytic nucleic acid molecule (e.g. a ribozyme) that specifically binds mRNA encoding an adenosine receptor. In some disclosures, the antisense molecule, inhibitory nucleic acid molecule or catalytic nucleic acid molecule binds nucleic acids encoding A2a, A2b, or A3. In some disclosures, an antisense molecule, inhibitory nucleic acid molecule or catalytic nucleic acid targets biochemical pathways downstream of the adenosine receptor. For example, the antisense molecule or catalytic nucleic acid can inhibit an enzyme involved in the Gs protein- or Gi protein-dependent intracellular pathway. In some disclosures, the additional agent includes dominant negative mutant form of an adenosine receptor, such as A2a, A2b, or A3.

In some disclosures, the additional agent that is administered in accord with the disclosed methods, and/or with the disclosed articles of manufacture or compositions, is an agent that inhibits extracellular adenosine. Agents that inhibit extracellular adenosine include agents that render extracellular adenosine non-functional (or decrease such function), such as a substance that modifies the structure of adenosine to inhibit the ability of adenosine to signal through adenosine receptors. In some disclosures, the additional agent is an extracellular adenosine-generating or adenosine-degrading enzyme, a modified form thereof or a modulator thereof. For example, in some disclosures, the additional agent is an enzyme (e.g. adenosine deaminase) or another catalytic molecule that selectively binds and destroys the adenosine, thereby abolishing or significantly decreasing the ability of endogenously formed adenosine to signal through adenosine receptors and terminate inflammation.

In some disclosures, the additional agent is an adenosine deaminase (ADA) or a modified form thereof, e.g., recombinant ADA and/or polyethylene glycol-modified ADA (ADA-PEG), which can inhibit local tissue accumulation of extracellular adenosine. ADA-PEG has been used in treatment of patients with ADA SCID (Hershfield (1995) Hum Mutat. 5:107). In some disclosures, an agent that inhibits extracellular adenosine includes agents that prevent or decrease formation of extracellular adenosine, and/or prevent or decrease the accumulation of extracellular adenosine, thereby abolishing, or substantially decreasing, the immunosuppressive effects of adenosine. In some disclosures, the additional agent specifically inhibits enzymes and proteins that are involved in regulation of synthesis and/or secretion of pro-inflammatory molecules, including modulators of nuclear transcription factors. Suppression of adenosine receptor expression or expression of the Gs protein- or Gi protein-dependent intracellular pathway, or the cAMP dependent intracellular pathway, can result in an increase/enhancement of immune response.

In some disclosures, the additional agent can target ectoenzymes that generate or produce extracellular adenosine. In some disclosures, the additional agent targets CD39 and CD73 ectoenzymes, which function in tandem to generate extracellular adenosine. CD39 (also called ectonucleoside triphosphate diphosphohydrolase) converts extracellular ATP (or ADP) to 5'AMP. Subsequently, CD73 (also called 5'nucleotidase) converts 5'AMP to adenosine. The activity of CD39 is reversible by the actions of NDP kinase and adenylate kinase, whereas the activity of CD73 is irreversible. CD39 and CD73 are expressed on tumor stromal cells, including endothelial cells and Tregs, and also on many cancer cells. For example, the expression of CD39 and CD73 on endothelial cells is increased under the hypoxic conditions of the tumor microenvironment. Tumor hypoxia can result from inadequate blood supply and disorganized tumor vasculature, impairing delivery of oxygen (Carroll and Ashcroft (2005), Expert. Rev. Mol. Med. 7(6):1-16). Hypoxia also inhibits adenylate kinase (AK), which converts adenosine to AMP, leading to very high extracellular adenosine concentration. Thus, adenosine is released at high concentrations in response to hypoxia, which is a condition that frequently occurs the tumor microenvironment (TME), in or around solid tumors. In some disclosures, the additional agent is one or more of anti-CD39 antibody or antigen binding fragment thereof, anti-CD73 antibody or antigen binding fragment thereof, e.g., MEDI9447 or TY/23, α-β-methylene-adenosine diphosphate (ADP), ARL 67156, POM-3, IPH52 (see, e.g., Allard et al. Clin Cancer Res (2013) 19(20):5626-5635; Hausler et al., Am J Transl Res (2014) 6(2):129-139; Zhang, B., Cancer Res. (2010) 70(16):6407-6411).

In some disclosures, the additional agent that is administered in accord with the disclosed methods, and/or with the disclosed articles of manufacture or compositions, is a chemotherapeutic agent (sometimes referred to as a cytotoxic agent). In particular disclosures, the chemotherapeutic agent is any agent known to be effective for the treatment, prevention or amelioration of hyperproliferative disorders such as cancer. Chemotherapeutic agents include, but are not limited to, small molecules, synthetic drugs, peptides, polypeptides, proteins, nucleic acids (e.g., DNA and RNA polynucleotides including, but not limited to, antisense nucleotide sequences, triple helices and nucleotide sequences encoding biologically active proteins, polypeptides or peptides), antibodies, synthetic or natural inorganic molecules, mimetic agents, and synthetic or natural organic molecules. In particular disclosures, chemotherapeutic drugs include alkylating agents, anthracyclines, cytoskeletal disruptors (taxanes), epothilones, histone deacetylase inhibitors, topoisomerase inhibitors, topoisomerase II inhibitors, kinase inhibitors, nucleotide analogs and precursor analogs, peptide antibiotics, platinum-based agents, and vinca alkaloids and derivatives.

Chemotherapeutic agents may include, but are not limited to, abarelix, aldesleukin, alemtuzumab, alitretinoin, allopurinol, altretamine, amifostine, anastrozole, arsenic trioxide, asparaginase, BCG live, bevaceizumab, bexarotene, bleomycin, bortezomib, busulfan, calusterone, camptothecin, capecitabine, carboplatin, carmustine, celecoxib, cetuximab, chlorambucil, cinacalcet, cisplatin, cladribine, cyclophosphamide, cytarabine, dacarbazine, dactinomycin, darbepoetin alfa, daunorubicin, denileukin diftitox, dexrazoxane, docetaxel, doxorubicin, dromostanolone, Elliott's B solution, epirubicin, epoetin alfa, estramustine, etoposide, exemestane, filgrastim, floxuridine, fludarabine, fluorouracil, fulvestrant, gemcitabine, gemtuzumab ozogamicin, gefitinib, goserelin, hydroxyurea, ibritumomab tiuxetan, idarubicin, ifosfamide, imatinib, interferon alfa-2a, interferon alfa-2b, irinotecan, letrozole, leucovorin, levamisole, lomustine, meclorethamine, megestrol, melphalan, mercaptopurine, mesna, methotrexate, methoxsalen, methylprednisolone, mitomycin C, mitotane, mitoxantrone, nandrolone, nofetumomab, oblimersen, oprelvekin, oxaliplatin, paclitaxel, pamidronate, pegademase, pegaspargase, pegfilgrastim, pemetrexed, pentostatin, pipobroman, plicamycin, polifeprosan, porfimer, procarbazine, quinacrine, rasburicase, rituximab, sargramostim, streptozocin, talc, tamoxifen, tarceva, temozolomide, teniposide, testolactone, thioguanine, thiotepa, topotecan, toremifene, tositumomab, trastuzumab, tretinoin, uracil mustard, valrubicin, vinblastine, vincristine, vinorelbine, and zoledronate.

In some disclosures, the additional agent is an inhibitor of hypoxia inducible factor 1 alpha (HIF-1α) signaling. Exemplary inhibitors of HIF-1α include digoxin, acriflavine, sirtuin-7 and ganetespib.

In some disclosures, the additional agent includes a protein tyrosine phosphatase inhibitor, e.g., a protein tyrosine phosphatase inhibitor described herein. In some disclosures, the protein tyrosine phosphatase inhibitor is an SHP-1 inhibitor, e.g., an SHP-1 inhibitor described herein, such as, e.g., sodium stibogluconate. In some disclosures, the protein tyrosine phosphatase inhibitor is an SHP-2 inhibitor, e.g., an SHP-2 inhibitor described herein.

In some disclosures, the additional agent is a kinase inhibitor. Kinase inhibitors, such as a CDK4 kinase inhibitor, a BTK kinase inhibitor, a MNK kinase inhibitor, or a DGK kinase inhibitor, can regulate the constitutively active survival pathways that exist in tumor cells and/or modulate the function of immune cells. In some disclosures, the kinase inhibitor is a Bruton's tyrosine kinase (BTK) inhibitor, e.g., ibrutinib. In some disclosures, the kinase inhibitor is a phosphatidylinositol-4,5-bisphosphate 3-kinase (PI3K) inhibitor. In some disclosures, the kinase inhibitor is a CDK4 inhibitor, e.g., a CDK4/6 inhibitor. In some disclosures, the kinase inhibitor is an mTOR inhibitor, such as, e.g., rapamycin, a rapamycin analog, OSI-027. The mTOR inhibitor can be, e.g., an mTORC1 inhibitor and/or an mTORC2 inhibitor, e.g., an mTORC1 inhibitor and/or mTORC2 inhibitor. In some disclosures, the kinase inhibitor is an MNK inhibitor, or a dual PI3K/mTOR inhibitor. In some disclosures, other exemplary kinase inhibitors include the AKT inhibitor perifosine, the mTOR inhibitor temsirolimus, the Src kinase inhibitors dasatinib and fostamatinib, the JAK2 inhibitors pacritinib and ruxolitinib, the PKCβ inhibitors enzastaurin and bryostatin, and the AAK inhibitor alisertib.

In some disclosures, the kinase inhibitor is a BTK inhibitor selected from ibrutinib (PCI-32765); GDC-0834; RN-486; CGI-560; CGI-1764; HM-71224; CC-292; ONO-4059; CNX-774; and LFM-A13. In some disclosures, the BTK inhibitor does not reduce or inhibit the kinase activity of interleukin-2-inducible kinase (ITK), and is selected from GDC-0834; RN-486; CGI-560; CGI-1764; HM-71224; CC-292; ONO-4059; CNX-774; and LFM-A13.

In some disclosures, the kinase inhibitor is a BTK inhibitor, e.g., ibrutinib (1-[(3R)-3-[4-Amino-3-(4-phenoxyphenyl)-1H-pyrazolo[3,4-d]pyrimidin-1-yl]piperidin-1-yl]prop-2-en-1-one; also known as PCI-32765). In some disclosures, the kinase inhibitor is a BTK inhibitor, e.g., ibrutinib (PCI-32765), and the ibrutinib is administered at a dose of about 250 mg, 300 mg, 350 mg, 400 mg, 420 mg, 440 mg, 460 mg, 480 mg, 500 mg, 520 mg, 540 mg, 560 mg, 580 mg, 600 mg (e.g., 250 mg, 420 mg or 560 mg) daily for a period of time, e.g., daily for 21 day cycle, or daily for 28 day cycle. In some disclosures, 1, 2, 3, 4, 5, 6, 7, 8, 9, 10, 11, 12 or more cycles of ibrutinib are administered. In some disclosures, the BTK inhibitor is a BTK inhibitor described in International Application WO 2015/079417.

In some disclosures, the kinase inhibitor is a PI3K inhibitor. PI3K is central to the PI3K/Akt/mTOR pathway involved in cell cycle regulation and lymphoma survival. Exemplary PI3K inhibitor includes idelalisib (PI3Kδ inhibitor). In some disclosures, the additional agent is idelalisib and rituximab.

In some disclosures, the additional agent is an inhibitor of mammalian target of rapamycin (mTOR). In some disclosures, the kinase inhibitor is an mTOR inhibitor selected from temsirolimus; ridaforolimus (also known as AP23573 and MK8669); everolimus (RAD001); rapamycin (AY22989); simapimod; AZD8055; PF04691502; SF1126; and XL765. In some disclosures, the additional agent is an inhibitor of mitogen-activated protein kinase (MAPK), such as vemurafenib, dabrafenib, and trametinib.

In some disclosures, the additional agent is an agent that regulates pro- or anti-apoptotic proteins. In some disclosures, the additional agent includes a B-cell lymphoma 2 (BCL-2) inhibitor (e.g., venetoclax, also called ABT-199 or GDC-0199; or ABT-737). Venetoclax is a small molecule (4-(4-{[2-(4-Chlorophenyl)-4,4-dimethyl-1-cyclohexen-1-yl]methyl}-1-piperazinyl)-N-({3-nitro-4-[(tetrahydro-2H-pyran-4-ylmethyl)amino]phenyl}sulfonyl)-2-(1H-pyrrolo[2,3-b]156isclosu-5-yloxy)benzamide) that inhibits the anti-apoptotic protein, BCL-2. Other agents that modulate pro- or anti-apoptotic protein include BCL-2 inhibitor ABT-737, navitoclax (ABT-263); Mcl-1 siRNA or Mcl-1 inhibitor retinoid N-(4-hydroxyphenyl) retinamide (4-HPR) for maximal efficacy. In some disclosures, the additional agent provides a pro-apoptotic stimuli, such as recombinant tumor necrosis factor-related apoptosis-inducing ligand (TRAIL), which can activate the apoptosis pathway by binding to TRAIL death receptors DR-4 and DR-5 on tumor cell surface, or TRAIL-R2 agonistic antibodies.

In some disclosures, the additional agent includes a cytotoxic agent, e.g., CPX-351 (Celator Pharmaceuticals), cytarabine, daunorubicin, vosaroxin (Sunesis Pharmaceuticals), sapacitabine (Cyclacel Pharmaceuticals), idarubicin, or mitoxantrone. In some disclosures, the additional agent includes a hypomethylating agent, e.g., a DNA methyltransferase inhibitor, e.g., azacitidine or decitabine.

In another disclosure, the additional therapy is a transplantation, e.g., allogeneic stem cell transplant.

In some disclosures, the additional therapy is a lymphodepleting therapy. In some disclosures, lymphodepletion is performed on a subject, e.g., prior to administering engineered cells, e.g., CAR-expressing cells. In some disclosures, the lymphodepletion comprises administering one or more of melphalan, Cytoxan, cyclophosphamide, and fludarabine. In some disclosures, a lymphodepleting chemotherapy is administered to the subject prior to, concurrently with, or after administration (e.g., infusion) of engineered cells, e.g., CAR-expressing cells. In a disclosure, the lymphodepleting chemotherapy is administered to the subject prior to administration of engineered cells, e.g., CAR-expressing cells.

**In** some disclosures, the additional agent is an oncolytic virus. In some disclosures, oncolytic viruses are capable of selectively replicating in and triggering the death of or slowing the growth of a cancer cell. In some cases, oncolytic viruses have no effect or a minimal effect on non-cancer cells. An oncolytic virus includes but is not limited to an oncolytic adenovirus, oncolytic Herpes Simplex Viruses, oncolytic retrovirus, oncolytic parvovirus, oncolytic vaccinia virus, oncolytic Sinbis virus, oncolytic influenza virus, or oncolytic RNA virus (e.g., oncolytic reovirus, oncolytic Newcastle Disease Virus (NDV), oncolytic measles virus, or oncolytic vesicular stomatitis virus (VSV)).

Other exemplary combination therapy, treatment and/or agents include anti-allergenic agents, anti-emetics, analgesics and adjunct therapies. In some disclosures, the additional agent includes cytoprotective agents, such as neuroprotectants, free-radical scavengers, cardioprotectors, anthracycline extravasation neutralizers and nutrients.

**In** some disclosures, an antibody used as an additional agent is conjugated or otherwise bound to a therapeutic agent, e.g., a chemotherapeutic agent (e.g., Cytoxan, fludarabine, histone deacetylase inhibitor, demethylating agent, peptide vaccine, anti-tumor antibiotic, tyrosine kinase inhibitor, alkylating agent, anti-microtubule or anti-mitotic agent), anti-allergic agent, anti-nausea agent (or anti-emetic), pain reliever, or cytoprotective agent described herein. In some disclosures, the additional agent is an antibody-drug conjugate.

Any of the additional agents described herein can be prepared and administered as a combination therapy described in the disclosed methods, uses, articles of manufacture or compositions, such as in pharmaceutical compositions comprising one or more agents of the combination therapy and a pharmaceutically acceptable carrier, such as any described herein. In some disclosures, the combination therapy in the disclosed methods, uses, articles of manufacture or compositions can be administered simultaneously, concurrently or sequentially, in any order with the additional agents, therapy or treatment, wherein such administration provides therapeutically effective levels each of the agents in the body of the subject. In some disclosures, the additional agent can be co-administered with the combination therapy in the disclosed methods, uses, articles of manufacture or compositions, for example, as part of the same pharmaceutical composition or using the same method of delivery. In some disclosures, the additional agent is administered simultaneously with the cell therapy, e.g. dose of engineered T cells (e.g. CAR⁺ T cells), but in separate compositions. In some disclosures, the additional agent is incubated with the engineered cell, e.g., CAR-expressing cells, prior to administration of the cells.

**In** some disclosures, the one or more additional agents are administered subsequent to or prior to the administration of the cell therapy, e.g. dose of engineered T cells (e.g. CAR⁺ T cells), separated by a selected time period. In some disclosures, the time period is 1 day, 2 days, 3 days, 4 days, 5 days, 6 days, 1 week, 2 weeks, 3 weeks, 1 month, 2 months, or 3 months. In some disclosures, the one or more additional agents are administered multiple times. In some disclosures, the additional agent is administered prior to the cell therapy, e.g. dose of engineered T cells (CAR⁺ T cells) in the disclosed methods, uses, articles of manufacture or compositions, e.g., two weeks, 12 days, 10 days, 8 days, one week, 6 days, 5 days, 4 days, 3 days, 2 days or 1 day before the administration. In some disclosures, the additional agent is administered after the cell therapy, e.g. dose of engineered T cells (e.g. CAR⁺ T cells) in the disclosed methods, uses, articles of manufacture or compositions, e.g., two weeks, 12 days, 10 days, 8 days, one week, 6 days, 5 days, 4 days, 3 days, 2 days or 1 day after the administration.

The dose of the additional agent can be any therapeutically effective amount, e.g., any dose amount described herein, and the appropriate dosage of the additional agent may depend on the type of B cell malignancy or hematological malignancy to be treated, the type, dose and/or frequency of the cell and/or composition administered, the age of the subject, the body weight of the subject, the severity and course of the disease, previous therapy, the patient's clinical history and response to cell therapy, e.g. dose of engineered T cells (CAR⁺ T cells), and the discretion of the attending physician.

### V. ARTICLES OF MANUFACTURE AND KITS

Also disclosed are articles of manufacture and kits containing engineered cells expressing a recombinant receptor (e.g., CAR) or compositions containing such cells, and optionally instructions for use, for example, instructions for administering, according to the provided methods and uses.

**In** some instances disclosed herein are articles of manufacture and/or kits that include a composition comprising a therapeutically effective amount of the engineered cells described herein, and instructions for administering, to a subject for treating a B cell malignancy or a hematological malignancy. In some disclosures, the instructions can specify some or all of the elements of the methods and uses provided herein. In some disclosures, the instructions specify particular instructions for administration of the cells for cell therapy, e.g., doses, timing, selection and/or identification of subjects for administration and conditions for administration. In some disclosures, the articles of manufacture and/or kits further include one or more additional agents for therapy, e.g., lymphodepleting therapy and/or combination therapy, such as any described herein and optionally further includes instructions for administering the additional agent for therapy. In some disclosures, the articles of manufacture and/or kits further comprise an agent for lymphodepleting therapy, and optionally further includes instructions for administering the lymphodepleting therapy. In some disclosures, the instructions can be included as a label or package insert accompanying the compositions for administration.

In some disclosures, the instructions specify the criteria for selection or identification of subjects for therapy. In some disclosures, the subjects include those any subjects described herein for administering the therapy, such as those described in Section I.A herein. In some disclosures, such criteria include subjects having a B cell malignancy or a hematologic malignancy. In some disclosures, such criteria include subjects having a B-cell acute lymphoblastic leukemia (B-ALL). In some disclosures, such criteria include subjects having a B-cell non-Hodgkin lymphoma (B-NHL) and/or any subtypes thereof, including diffuse large B-cell lymphoma (DLBCL), Burkitt lymphoma (BL) or primary mediastinal large B-cell lymphoma (PMBCL).

In some disclosures, the instructions specify the age of the subject for administration of the cells, e.g., CAR-expressing cells. In some disclosures, the instructions specify administration to subjects who are at or younger than 25 years of age. In some disclosures, the instructions specify administration to subjects that are younger than 18 years of age. In some disclosures, the instructions specify administration to pediatric subjects or young adult subjects.

In some disclosures, the instructions specify treating a subject having relapsed following remission after treatment with, or become refractory to, one or more prior therapies; or a subject that has relapsed or is refractory (R/R) to one or more prior therapies, e.g., one or more lines of standard therapy. In some disclosures, the instructions specify treating a specific group or subset of subjects, such as pediatric subjects identified as having a B cell malignancy that has R/R to standard therapy. In some disclosures, the subjects can have a high-risk disease, such as a B cell malignancy that is aggressive and/or has a poor prognosis or that has R/R to standard therapy. In some disclosures, the instructions specify treating subjects having a R/R B-ALL, a R/R B-NHL, or a B-ALL that exhibits minimum residual disease (MRD+ B-ALL). In some disclosures, the subject has a R/R DLBCL, a R/R BL or a R/R PMBCL. In some disclosures, the subject or population to be treated include those subjects having poor performance status. In some disclosures, the population to be treated includes, e.g., subjects having an Eastern Cooperative Oncology Group Performance Status (ECOG) that is 0-2. In other disclosures, the subjects to be treated include ECOG 0-1 or do not include ECOG2 subjects. In some disclosures, the subjects to be treated have failed one or more, or two or more prior therapies.

In some disclosures, the instructions specify the amount, number or dose of cells to be administered, or the amount or volume of the composition containing engineered cells to be administered. In some disclosures, the instructions specify doses according to the methods and uses provided herein, e.g., as described in Section I.B herein. For example, in some disclosures, the dose specified in the instructions include a dose that is based on body weight of the subject. In some disclosures, the dose specified in the instructions include a dose that is a fixed dose or a flat dose. In some disclosures, the instructions specify administration of multiple doses.

In some disclosures, the article of manufacture or kit comprises a container, optionally a vial comprising a plurality of CD4⁺ CAR+ T cells, and a container, optionally a vial comprising a plurality of CD8⁺ CAR+ T cells. In some disclosures, the article of manufacture or kit comprises a container, optionally a vial comprising a plurality of CD4⁺ CAR+ T cells, and further comprises, in the same container, a plurality of CD8⁺ CAR+ T cells. In some disclosures, a cryoprotectant is included with the cells. In some disclosures, the container is a bag.

**In** some disclosures, the container such as the vial comprises sufficient cells, such as sufficient CAR-expressing T cells (e.g., CD4+ CAR+ T cells or CD8+CAR+T cells or both), for a dose of administration according to the methods and uses provided herein, e.g., as described in Section I.B herein. In some disclosures, the container such as the vial comprises greater than or greater than about 10 x 10⁶ T cells or CAR+ T cells, greater than or greater than about 15 x 10⁶ T cells or CAR+ T cells, greater than or greater than about 25 x 10⁶ T cells or CAR+T cell.

In some disclosures, the vial comprises between about 10 x 10⁶ cells/mL and about 70 x 10⁶ cells/mL, between about 10 x 10⁶ cells/mL and about 50 x 10⁶ cells/mL, between about 10 x 10⁶ cells/mL and about 25 x 10⁶ cells/mL, between about 10 x 10⁶ cells/mL and about 15 x 10⁶ cells/mL, 15 x 10⁶ cells/mL and about 70 x 10⁶ cells/mL, between about 15 x 10⁶ cells/mL and about 50 x 10⁶ cells/mL, between about 15 x 10⁶ cells/mL and about 25 x 10⁶ cells/mL, between about 25 x 10⁶ cells/mL and about 70 x 10⁶ cells/mL, between about 25 x 10⁶ cells/mL and about 50 x 10⁶ cells/mL, and between about 50 x 10⁶ cells/mL and about 70 x 10⁶ cells/mL. In some disclosures, the concentration of the T cell composition in the container is greater than at or about 5 x 10⁶ cells/mL or is or is about 5 x 10⁶ cells/mL. In some disclosures, the concentration of the T cell composition in the container is greater than at or about 10 x 10⁶ cells/mL or is or is about 10 x 10⁶ cells/mL. In some disclosures, the concentration of the T cell composition in the container is greater than or greater than about 15 x 10⁶ cells/mL or is or is about 15 x 10⁶ cells/mL. In some disclosures, the concentration of cells in the container is of viable cells in the container.

In some disclosures, the plurality of vials or plurality of cells or unit dose of cells specified for administration, collectively, comprises a specified amount, number or dose of cells, such as any doses according to the methods and uses provided herein, e.g., as described in Section I.B herein. In some disclosures, the article comprises one or more unit dose of the CD4⁺ and/or CD8⁺ cells or of the CD4⁺receptor⁺ cells and/or CD8⁺receptor⁺ cells (e.g., CD4+ CAR+ cells and/or CD8+CAR+ cells). In some disclosures, the unit dose comprises between at or about 1 x 10⁷ and at or about 2 x 10⁸ CAR+ T cells, between at or about 5 x 10⁷ and at or about 1.5 x 10⁸ CAR+ T cells, at or about 5 x 10⁷ CAR+ T cells, at or about 1 x 10⁸ CAR+ T cells, or at or about 1.5 x 10⁸ CAR+ T cells. In some disclosures, the information in the article specifies administration of one or of a plurality of unit doses and/or a volume corresponding to such one or plurality of unit doses. In some disclosures, each vial or the plurality of vials or plurality of cells or unit dose of cells specified for administration, collectively, comprises an amount, number or dose of cells determined based on the body weight of the subject, up to a maximum (or cap) number or amount of cells. In some disclosures, each vial or the plurality of vials or plurality of cells or unit dose of cells specified for administration, collectively, comprises a flat number, amount or dose of cells or fixed number, amount or dose of cells such that the dose of cells is not tied to or based on the body surface area or weight of a subject. In some disclosures, a unit dose of a cell is or comprises the number or amount of cells, such as engineered T cells, that can be administered to a subject or a patient in a single dose.

In some disclosures, the instructions can specify dosage regimen and timing of the administration. For example, in some disclosures, the instructions can specify administering to the subject multiple doses, e.g., two or more doses, of the cells. In some disclosures, the instructions specify the timing of the multiple doses, e.g., the second dose being administered approximately 4, 5, 6, 7, 8, 9, 10, 11, 12, 13, 14, 15, 16, 17, 18, 19, 20 or 21 days after the first dose; and/or the dosage amount in each dose.

In some disclosures, the article of manufacture or kit comprises a plurality of CD4⁺ CAR+ T cells, and instructions for administering, to a subject having a disease or condition, all or a portion of the plurality of CD4⁺ T cells and further administering CD8⁺ CAR+ T cells. In some disclosures, the instructions specify administering the CD4⁺ T cells prior to administering the CD8⁺ cells. In some cases, the instructions specify administering the CD8⁺ T cells prior to administering the CD4⁺ cells. In some disclosures, the article of manufacture or kit comprises a plurality of CD8⁺ CAR+ T cells, and instructions for administering, to a subject having a disease or condition, all or a portion of the plurality of CD8⁺ T cells and CD4⁺ CAR+ T cells. In some disclosures, the instructions specify dosage regimen and timing of the administration of the cells.

In some disclosures, the instructions specify administering all or a portion of the CD4⁺ T cells and the all or a portion of the CD8⁺ T cells 0 to 12 hours apart, 0 to 6 hours apart or 0 to 2 hours apart. In some cases, the instructions specify administering the CD4⁺ T cells and the CD8⁺ T cells no more than 2 hours, no more than 1 hour, no more than 30 minutes, no more than 15 minutes, no more than 10 minutes or no more than 5 minutes apart.

In some disclosures, the instructions specify the dose or number of cells or cell type(s) and/or a ratio of cell types, e.g., individual populations or sub-types, such as the CD4⁺ to CD8⁺ ratio. In some disclosures, the populations or sub-types of cells, such as CD8⁺ and CD4⁺ T cells. For example, in some disclosures, the instructions specify that the cells are administered at or within a tolerated range of an output ratio of multiple cell populations or sub-types, such as CD4⁺ and CD8⁺ cells or sub-types, of between at or about 5:1 and at or about 5:1 (or greater than about 1:5 and less than about 5:1), or between at or about 1:3 and at or about 3:1 (or greater than about 1:3 and less than about 3:1), such as between at or about 2:1 and at or about 1:5 (or greater than about 1:5 and less than about 2:1, such as at or about 5:1, 4.5:1, 4:1, 3.5:1, 3:1, 2.5:1, 2:1, 1.9:1, 1.8:1, 1.7:1, 1.6:1, 1.5:1, 1.4:1, 1.3:1, 1.2:1, 1.1:1, 1:1, 1:1.1, 1:1.2, 1:1.3, 1:1.4, 1:1.5, 1:1.6, 1:1.7, 1:1.8, 1:1.9: 1:2, 1:2.5, 1:3, 1:3.5, 1:4, 1:4.5, or 1:5. In some disclosures, the tolerated difference is within about 1%, about 2%, about 3%, about 4% about 5%, about 10%, about 15%, about 20%, about 25%, about 30%, about 35%, about 40%, about 45%, about 50% of the desired ratio, including any value in between these ranges.

**In** some disclosures, the articles of manufacture and/or kits further include one or more additional agents for therapy, e.g., lymphodepleting therapy and/or combination therapy, as described herein, and optionally instructions for administering the additional agents. In some disclosures, the articles of manufacture may further contain one or more therapeutic agents. In some disclosures, the therapeutic agent is an immunomodulatory agent, a cytotoxic agent, an anti-cancer agent or a radiotherapeutic.

The articles of manufacture and/or kits may include a container and a label or package insert on or associated with the container. Suitable containers include, for example, bottles, vials, syringes, IV solution bags, etc. The containers may be formed from a variety of materials such as glass or plastic. The container in some disclosures holds a composition which is by itself or combined with another composition effective for treating, preventing and/or diagnosing the condition. In some disclosures, the container has a sterile access port. Exemplary containers include an intravenous solution bags, vials, including those with stoppers pierceable by a needle for injection, or bottles or vials for orally administered agents. The label or package insert may indicate that the composition is used for treating a disease or condition. The article of manufacture may include (a) a first container with a composition contained therein, wherein the composition includes engineered cells expressing a recombinant receptor; and (b) a second container with a composition contained therein, wherein the composition includes the second agent. In some disclosures, the article of manufacture may include (a) a first container with a first composition contained therein, wherein the composition includes a subtype of engineered cells expressing a recombinant receptor; and (b) a second container with a composition contained therein, wherein the composition includes a different subtype of engineered cells expressing a recombinant receptor. The article of manufacture may further include a package insert indicating that the compositions can be used to treat a particular condition. Alternatively, or additionally, the article of manufacture may further include another or the same container comprising a pharmaceutically-acceptable buffer. It may further include other materials such as other buffers, diluents, filters, needles, and/or syringes.

### VI. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The terms "polypeptide" and "protein" are used interchangeably to refer to a polymer of amino acid residues, and are not limited to a minimum length. Polypeptides, including the provided receptors and other polypeptides, e.g., linkers or peptides, may include amino acid residues including natural and/or non-natural amino acid residues. The terms also include post-expression modifications of the polypeptide, for example, glycosylation, sialylation, acetylation, and phosphorylation. In some aspects, the polypeptides may contain modifications with respect to a native or natural sequence, as long as the protein maintains the desired activity. These modifications may be deliberate, as through site-directed mutagenesis, or may be accidental, such as through mutations of hosts which produce the proteins or errors due to PCR amplification.

As used herein, a "subject" is a mammal, such as a human or other animal, and typically is human. In some embodiments, the subject, e.g., patient, to whom the agent or agents, cells, cell populations, or compositions are administered, is a mammal, typically a primate, such as a human. In some embodiments, the primate is a monkey or an ape. The subject can be male or female and can be any suitable age, including infant, juvenile, adolescent, adult, and geriatric subjects. In some embodiments, the subject is a non-primate mammal, such as a rodent.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The terms do not imply complete curing of a disease or complete elimination of any symptom or effect(s) on all symptoms or outcomes.

As used herein, "delaying development of a disease" means to defer, hinder, slow, retard, stabilize, suppress and/or postpone development of the disease (such as cancer). This delay can be of varying lengths of time, depending on the history of the disease and/or individual being treated. In some embodiments, sufficient or significant delay can, in effect, encompass prevention, in that the individual does not develop the disease. For example, a late stage cancer, such as development of metastasis, may be delayed.

"Preventing," as used herein, includes providing prophylaxis with respect to the occurrence or recurrence of a disease in a subject that may be predisposed to the disease but has not yet been diagnosed with the disease. In some embodiments, the provided cells and compositions are used to delay development of a disease or to slow the progression of a disease.

As used herein, to "suppress" a function or activity is to reduce the function or activity when compared to otherwise same conditions except for a condition or parameter of interest, or alternatively, as compared to another condition. For example, cells that suppress tumor growth reduce the rate of growth of the tumor compared to the rate of growth of the tumor in the absence of the cells.

An "effective amount" of an agent, e.g., a pharmaceutical formulation, cells, or composition, in the context of administration, refers to an amount effective, at dosages/amounts and for periods of time necessary, to achieve a desired result, such as a therapeutic or prophylactic result.

A "therapeutically effective amount" of an agent, e.g., a pharmaceutical formulation or cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the populations of cells administered. In some embodiments, the provided methods and uses involve administering the cells and/or compositions at effective amounts, e.g., therapeutically effective amounts.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount. In the context of lower tumor burden, the prophylactically effective amount in some aspects will be higher than the therapeutically effective amount.

The term "about" as used herein refers to the usual error range for the respective value readily known to the skilled person in this technical field. Reference to "about" a value or parameter herein includes (and describes) embodiments that are directed to that value or parameter per se.

As used herein, the singular forms "a," "an," and "the" include plural referents unless the context clearly dictates otherwise. For example, "a" or "an" means "at least one" or "one or more."

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

As used herein, a composition refers to any mixture of two or more products, substances, or compounds, including cells. It may be a solution, a suspension, liquid, powder, a paste, aqueous, nonaqueous or any combination thereof.

As used herein, "enriching" when referring to one or more particular cell type or cell population, refers to increasing the number or percentage of the cell type or population, e.g., compared to the total number of cells in or volume of the composition, or relative to other cell types, such as by positive selection based on markers expressed by the population or cell, or by negative selection based on a marker not present on the cell population or cell to be depleted. The term does not require complete removal of other cells, cell type, or populations from the composition and does not require that the cells so enriched be present at or even near 100% in the enriched composition.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control or fluorescence minus one (FMO) gating control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control or fluorescence minus one (FMO) gating control under otherwise identical conditions, and/or at a level substantially lower than that for cell known to be positive for the marker, and/or at a level substantially similar as compared to that for a cell known to be negative for the marker.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

### VIII. EXAMPLES

The following examples are included for illustrative purposes only and are not intended to limit the scope of the invention.

### Example 1: Administration of Anti-CD19 CAR-expressing Cells to Pediatric Subjects with Relapsed and Refractory B-cell Acute Lymphoblastic Leukemia (B-ALL) and B-cell Non-Hodgkin Lymphoma (NHL)

T cell compositions containing anti-CD19 CAR-expressing T cells are generated from subjects, to be treated, that are 25 years of age or younger, in some cases less than 18 years of age, with relapsed or refractory (r/r) B-cell Acute Lymphoblastic Leukemia (B-ALL) or relapsed or refractory (r/r) B-cell Non-Hodgkin Lymphoma (NHL).

The subjects with r/r B-ALL have morphological evidence of disease in bone marrow (5% or greater lymphoblast by morphology), and either of the following: first or greater marrow relapse; any marrow relapse after allogeneic hematopoietic stem cell transplant (HSCT); are primary refractory, e.g., not achieving a complete response (CR) or complete response with incomplete blood count recovery (CRi) after two (2) or more separate induction regimens (or chemo-refractory as not achieving CR/CRi after 1 cycle of standard chemotherapy for relapsed leukemia); or ineligible for allogeneic HSCT.

In one cohort, subjects with B-ALL have less than 5% lymphoblast by morphology and/or minimum residual disease (MRD+) as detected by a validated assay at a frequency of 1 x10⁻⁴ or greater in bone marrow cells after two lines of therapy.

Subjects with Philadelphia chromosome positive ALL are eligible if they are intolerant to or have failed one or more lines of tyrosine-kinase inhibitor (TKI) therapy or if TKI therapy is contraindicated.

In one cohort, the subjects with r/r B-NHL have measurable disease after one or more lines of chemotherapy and/or have failed HSCT or are ineligible for HSCT. Subjects with r/r B-NHL include subjects with diffuse large B-cell lymphoma (DLBCL); with Burkitt lymphoma (BL); or with primary mediastinal large B-cell lymphoma (PMBCL).

Treated subjects have evidence of CD19 expression as determined by flow cytometry (in peripheral blood or bone marrow) or immunohistochemistry (bone marrow biopsy); have not received prior CAR T cell or other genetically modified T cell therapy; and/or if, after having received previous CD19-targeted therapy, have CD19-positive disease confirmed since completing the prior CD19-targeted therapy.

The T cell compositions are generated from leukapheresis samples obtained from the subjects by a process including immunoaffinity-based enrichment of CD8⁺ and CD4+ cells, respectively. Cells of the enriched compositions are separately activated and subjected to lentiviral transduction with a vector encoding an anti-CD19 CAR. The anti-CD19 CAR contains an anti-CD19 scFv derived from a murine antibody (variable region derived from FMC63), an immunoglobulin-derived spacer, a transmembrane domain derived from CD28, a costimulatory region derived from 4-1BB, and a CD3-zeta intracellular signaling domain. Transduced populations are separately incubated in the presence of stimulating reagents for cell expansion. Expanded CD8+ and CD4+ cells are formulated and cryopreserved separately and stored.

Prior to administration of the CAR-expressing T cells (d=0), subjects are treated with 30 mg/m² fludarabine daily for 3 days and 300 mg/m² cyclophosphamide daily for 3 days. The cryopreserved T cell compositions are thawed prior to intravenous administration. Autologous anti-CD19 CAR-expressing T cells are administered 2-7 days after lymphodepletion as a defined cell composition by separately administering CD4+ CAR-expressing cell T cell composition and a CD8+ CAR-expressing T cell composition administered at a target ratio of 1:1 to the subject.

In one group of subjects, subjects are eighteen (18) years of age or younger and twelve (12) kilograms or more in body weight. The subjects are administered a T cell composition (by separate administration of a CAR-expressing CD4+ and CAR-expressing CD8+ T cell composition) at a target dose of 0.5 x 10⁶ CAR-expressing T cells per kilogram body weight of the subject, with a maximum target dose of 0.5 x 10⁸ total CAR-expressing T cells.

In some groups of subjects, subjects are eighteen (18) years of age or younger and six (6) kilograms or greater in body weight. In one group, such subjects are administered a T cell composition (by separate administration of a CAR-expressing CD4+ and CAR-expressing CD8+ T cell composition) at a target dose of 1.0 x 10⁶ CAR-expressing T cells per kilogram, with a maximum dose of 1.0 x 10⁸ total CAR-expressing T cells.

In a group of subjects between eighteen (18) and twenty-five (25) years of age, inclusive, the subjects are administered a T cell composition (by separate administration of a CAR-expressing CD4+ and CAR-expressing CD8+ T cell composition) at a dose of either 0.5 x 10⁸ or 1.0 x 10⁸ total CAR-expressing T cells.

In one cohort, the subjects are administered a T cell composition at a target dose of 0.05 x 10⁶ CAR+ T cells/kg, with a maximum dose of 5 x 10⁶ CAR+ T cells. In some cases, the dose in these subjects can be escalated to 0.1 x 10⁶ CAR+ T cells/kg, if the subject exhibits no response and no toxicity, approximately 28 days after administration of CAR+ T cells and is preceded by a lymphodepleting chemotherapy (LDC). In one cohort, the subjects are administered a T cell composition at a target dose of 0.15 x 10⁶ CAR+ T cells/kg, with a maximum dose of 15 x 10⁶ CAR+ T cells. In one cohort, the subjects are administered a T cell composition at a target dose of 0.3 x 10⁶ CAR+ T cells/kg, with a maximum dose of 30 x 10⁶ CAR+ T cells. In one cohort, the subjects are administered a T cell composition at a target dose of 0.50 x 10⁶ CAR+ T cells/kg, with a maximum dose of 50 x 10⁶ CAR+ T cells. In one cohort, the subjects are administered a T cell composition at a target dose of 0.75 x 10⁶ CAR+ T cells/kg, with a maximum dose of 75 x 10⁶ CAR+ T cells.

Response to treatment is assessed based on bone marrow and blood morphologic criteria, physical examination findings, along with various laboratory assessments. In some cohorts, the dose-limiting toxicity (DLT) is evaluated approximately 28 days after administration of the CAR-expressing T cells. The presence or absence of treatment-emergent adverse events (TEAE) following treatment is also assessed. Outcome is also be assessed such as by assessing overall response rate (ORR); rate of minimum residual disease (MRD) negative responses; duration of response (DOR); relapse free-survival (RFS); progression free-survival (PFS); event-free survival (EFS); overall survival (OS); MRD response rate; and rate of hematopoietic stem cell transplant (HSCT) after response to CAR T cell infusion.-Subjects also are assessed for pharmacokinetics (PK) of anti-CD19 CAR T cells, including maximum concentration (Cₘₐₓ); time to peak concentration (Tₘₐₓ); and area under the curve (AUC) of CAR+ T cells in samples (such as blood or tumor samples) from the subject, e.g., taken at various time points, post-treatment. In some cohorts, the overall response rate (ORR), including subjects achieving a complete response (CR) or complete response with incomplete blood count recovery (CRi), is assessed approximately 28 days after administration of the CAR-expressing T cells, and confirmed approximately 56 days after administration of the CAR-expressing T cells. In some cohorts, the rate of minimum residual disease (MRD) negative responses is assessed approximately 28 days after administration of the CAR-expressing T cells, and confirmed approximately 56 days after administration of the CAR-expressing T cells. In some cases, MRD response rate can be determined from the proportion of subjects achieving a CR or CRi with no MRD detected in bone marrow (e.g., <0.01% by a validated assay).

### Example 2: Processes for Generating Therapeutic Compositions of CD4+ and CD8+ Cells Expressing an Anti-CD19 CAR.

Engineered CD4+ T cells and engineered CD8+ T cells expressing an anti-CD19 chimeric antigen receptor (CAR) were produced from T cells from pediatric subjects less than 18 years of age. An anti-CD19 CAR engineered T cell composition was administered to a subject in accord with the method of administration described in Example 1.

### A. Process for Generating Therapeutic Cell Compositions

Engineered CD4+ T cells and engineered CD8+ T cells each expressing the same anti-CD19 chimeric antigen receptor (CAR) were produced by a process involving subjecting enriched CD4+ and enriched CD8+ cell populations, separately, to process steps. CD4+ and CD8+ cells were separately selected from human peripheral blood mononuclear cells (PBMCs) that had been obtained by leukapheresis, from two (2) human subjects under the age of 18, generating separate enriched CD4+ and enriched CD8+ cell compositions, which then were cryopreserved. The CD4+ and CD8+ compositions were subsequently thawed and separately underwent steps for stimulation, transduction, and expansion.

The thawed CD4+ and CD8+ cells were separately stimulated in the presence of paramagnetic polystyrene-coated beads coupled to anti-CD3 and anti-CD28 antibodies at a 1:1 bead to cell ratio. The stimulation was carried out in media containing human recombinant IL-2, human recombinant IL-15, and N-Acetyl Cysteine (NAC). The CD4+ cell media also included human recombinant IL-7.

Following the stimulation with the anti-CD3/anti-CD28 stimulatory agent, CD4+ and CD8+ cells were separately transduced with a lentiviral vector encoding the same anti-CD19 CAR. The CAR contained an anti-CD19 scFv derived from a murine antibody (variable region derived from FMC63), an immunoglobulin spacer, a transmembrane domain derived from CD28, a costimulatory region derived from 4-1BB, and a CD3-zeta intracellular signaling domain. The vector also encoded a truncated EGFR (EGFRt) that served as a surrogate marker for CAR expression that was connected to the CAR construct by a T2A sequence. The cells were transduced in the presence of 10 µg/ml protamine sulfate.

After transduction, the beads were removed from the cell compositions by exposure to a magnetic field. The CD4+ and CD8+ cell compositions were then separately cultivated for expansion with continual mixing and oxygen transfer by a bioreactor (Xuri W25 Bioreactor). Poloxamer was added to the media. Both cell compositions were cultivated in the presence of IL-2 and IL-15. The CD4+ cell media also included IL-7. The CD4+ and CD8+ cells were each cultivated, prior to harvest, to 4-fold expansion. One day after reaching the threshold, cells from each composition were separately harvested, formulated, and cryopreserved. The exemplary process is summarized in Table E1.

**Table E1: Summary of the exemplary process for generating CD4+ and CD8+ CAR-T cells**

| **Stage** | **CD4+ cells** | | **CD8+ cells** | |
|---|---|---|---|---|
| **Stimulation** (day 1-2) | | • anti-CD3/CD28 antibody conjugated beads | | • anti-CD3/CD28 antibody conjugated beads |
| | | • 1:1 bead to cell ratio | | • 1:1 bead to cell ratio |
| | | • media: IL-2, IL-7, IL-15, and NAC | | • media: IL-2, IL-15, and NAC |
| **Transduction** (day 2-5) | | • transduction adjuvant (e.g. 10 µg/ml protamine sulfate) | | • transduction adjuvant (e.g. 10 µg/ml protamine sulfate) |
| **Bead removal** (day 5*) | | • magnetic bead removal | | • magnetic bead removal |
| **Expansion (day 5* - Harvest)** | | • rocking motion bioreactor and/or continuous mixing | | • rocking motion bioreactor and/or continuous mixing |
| | | • media: IL-2, IL-7, IL15, and poloxamer | | • media: IL-2, IL15, and poloxamer |

| | | | | |
|---|---|---|---|---|
| *Approximate | | | | |

### B. Cell Phenotype Attributes of the Generated Therapeutic Cell Composition

Expression of cell surface markers associated with particular T cell subtypes, including C-C chemokine receptor type 7 (CCR7), CD27 and CD45RA in the two (2) therapeutic cell compositions generated as described above from subjects under the age of 18, were assessed by flow cytometry. Surface expression levels of CD3, CD4, CD8, CD28, and the truncated receptor used as a surrogate marker for expression of the CAR, also were assessed; the presence of activated caspase 3 was assessed as a measure of apoptotic cells.

Among the CD8+ CAR+ cells in the generated cell compositions, more than 90% of the cells were CCR7+, more than 85% of the cells were CD27+, more than 55% of the cells were CCR7+CD45RA+, and more than 75% of the cells were CD28+CD27+, consistent with an observation of a high percentage of less differentiated, naive-like cells in the CD8+CAR+ engineered cell composition. Among the CD4+ CAR+ cells in the generated cell compositions, more than 75% of the cells were CCR7+, more than 75% of the cells were CD27+, more than 40% of the cells were CCR7+CD45RA+, and more than 70% of the cells were CD28+CD27+, consistent with an observation of a high percentage of less differentiated, naive-like cells in the CD4+CAR+ engineered cell composition. Among the CD3+CD8+ T cells in the composition, less than 10% of the cells were active caspase 3-posistive (indicative of apoptotic cells) and among the CD3+CD4+ T cells in the composition, less than 25% of the cells were active caspase 3-posistive.

The results are consistent with an observation that therapeutic cell compositions generated from pediatric subjects using a process described above, exhibited phenotypic markers associated with less differentiated, naive-like cell subtypes, and a low percentage of apoptotic cells. Without wishing to be bound by theory, the high percentage of less differentiated, naive-like T cells in the engineered CD4+ CAR+ and CD8+CAR+ T cell compositions support that administration of a lower dose of cells, such as a dose as low as0.05 x 10⁶ CAR+ T cells/kg, may be feasible for pediatric subjects.

### Example 3: Administration of Varying Doses of Anti-CD19 CAR-expressing Cells to Pediatric Subjects with Relapsed and Refractory B-cell Acute Lymphoblastic Leukemia (B-ALL) and B-cell Non-Hodgkin Lymphoma (NHL)

T cell compositions containing anti-CD19 CAR-expressing T cells are generated from pediatric subjects with relapsed or refractory (r/r) B-cell Acute Lymphoblastic Leukemia (B-ALL) or relapsed or refractory (r/r) B-cell Non-Hodgkin Lymphoma (NHL), using the process described in Example 2. The subjects include pediatric subjects with CD19-positive relapsed/refractory (r/r) B-cell acute lymphoblastic leukemia (B-ALL) and B-cell non-Hodgkin lymphoma (B-NHL) (including diffuse large B-cell lymphoma (DLBCL), Burkitt lymphoma (BL) or primary mediastinal large B-cell lymphoma (PMBCL)) in pediatric subjects.

One cohort of subjects includes those less than 18 years of age, with CD19+ B-ALL who are relapsed/refractory after 1 or more lines of chemotherapy, relapsed after hematopoietic stem cell transplantation (HSCT), or are otherwise ineligible for HSCT.

One cohort of subjects includes those that are 25 years or less of age, with a CD19+ B-ALL or B-NHL who are r/r after 1 or more lines of chemotherapy, relapsed after HSCT, or are otherwise ineligible for HSCT. For subjects that are less than 18 years old, subjects include those with r/r B-ALL; minimum residual disease-positive (MRD+) B-ALL; and r/r B-NHL (DLBCL, BL, or PMBCL). For subjects that are between 18 and 25 years of age or less, subjects with B-ALL is included. Subjects are required to be 6 kg or more in body weight. Subjects with B-NHL with a secondary central nervous system (CNS) involvement are included.

In one cohort, the subjects have r/r B-ALL, with morphological evidence of disease in bone marrow (5% or greater lymphoblast by morphology), and either of the following: first or greater marrow relapse; any marrow relapse after allogeneic hematopoietic stem cell transplant (HSCT); are primary refractory, e.g., not achieving a complete response (CR) or complete response with incomplete blood count recovery (CRi) after two (2) or more separate induction regimens (or chemo-refractory as not achieving CR/CRi after 1 cycle of standard chemotherapy for relapsed leukemia); or ineligible for allogeneic HSCT. The subject is eligible regardless of MRD status in this cohort.

In one cohort, the subjects have MRD+ B-ALL, with less than 5% lymphoblast by morphology and/or minimum residual disease positive (MRD+) as detected by a validated assay at a frequency of 1 x10⁻⁴ or greater in bone marrow cells after two lines of therapy. In some cases, an optional bridging chemotherapy may convert an r/r B-ALL to MRD+ or MRD- B-ALL.

In one cohort, the subjects have r/r B-NHL, with measurable disease after one or more lines of chemotherapy and/or have failed HSCT or are ineligible for HSCT. Subjects with r/r B-NHL include subjects with diffuse large B-cell lymphoma (DLBCL); with Burkitt lymphoma (BL); or with primary mediastinal large B-cell lymphoma (PMBCL).

Subjects with Philadelphia chromosome positive ALL are eligible if they are intolerant to or have failed one or more lines of tyrosine-kinase inhibitor (TKI) therapy or if TKI therapy is contraindicated.

Treated subjects have evidence of CD19 expression as determined by flow cytometry (in peripheral blood or bone marrow) or immunohistochemistry (bone marrow biopsy). If the subject has previously received a CD19-targeted therapy, the subject must have CD19-positive disease confirmed since completing the prior CD19-targeted therapy.

Subjects have not received prior CAR T cell or other genetically modified T cell therapy, or an active CNS disease and significant neurological deterioration.

Prior to administration of the CAR-expressing T cells (d=0), subjects are treated with 30 mg/m² fludarabine daily for 3 days and 300 mg/m² cyclophosphamide daily for 3 days. The cryopreserved T cell compositions are thawed prior to intravenous administration. Autologous anti-CD19 CAR-expressing T cells are administered two to seven days after lymphodepletion as a defined cell composition by separately administering CD4+ CAR-expressing cell T cell composition and a CD8+ CAR-expressing T cell composition administered at a target ratio of 1:1 to the subject.

T cell compositions are generated from leukapheresis samples obtained from the subjects by a process including immunoaffinity-based enrichment of CD8+ and CD4+ cells and engineered to express an anti-CD19 CAR, generally as described in Example 2. Engineered CD8+ and CD4+ cells are formulated and cryopreserved separately and stored.

In one cohort, the subjects are administered a T cell composition at a target dose of 0.05 x 10⁶ CAR+ T cells/kg, with a maximum dose of 5 x 10⁶ CAR⁺ T cells. In some cases, these subjects can receive a lymphodepleting chemotherapy (LDC) followed by an additional dose of 0.1 x 10⁶ CAR+ T cells/kg (with a maximum dose of 10 x 10⁶ CAR+ T cells), if the subject exhibits no response and no toxicity, approximately 28 days after administration of CAR+ T cells. In one cohort, the subjects are administered a T cell composition at a target dose of 0.15 x 10⁶ CAR+ T cells/kg, with a maximum dose of 15 x 10⁶ CAR+ T cells. In one cohort, the subjects are administered a T cell composition at a target dose of 0.3 x 10⁶ CAR+ T cells/kg, with a maximum dose of 30 x 10⁶ CAR+ T cells. In one cohort, the subjects are administered a T cell composition at a target dose of 0.50 x 10⁶ CAR+ T cells/kg, with a maximum dose of 50 x 10⁶ CAR+ T cells. In one cohort, the subjects are administered a T cell composition at a target dose of 0.75 x 10⁶ CAR+ T cells/kg, with a maximum dose of 75 x 10⁶ CAR+ T cells. The maximum dose in each case is equivalent to a 100 kg body weight dose.

Response to treatment is assessed based on bone marrow and blood morphologic criteria, physical examination findings, along with various laboratory assessments. In some cohorts, the dose-limiting toxicity (DLT) is evaluated approximately 28 days after administration of the CAR-expressing T cells. In some cohorts, the overall response rate (ORR), including subjects achieving a complete response (CR) or complete response with incomplete blood count recovery (CRi), is assessed approximately 28 days after administration of the CAR-expressing T cells, and confirmed approximately 56 days after administration of the CAR-expressing T cells. In some cohorts, the rate of minimum residual disease (MRD) negative responses is assessed approximately 28 days after administration of the CAR-expressing T cells, and confirmed approximately 56 days after administration of the CAR-expressing T cells. In some cases, MRD negative rate can be determined from the proportion of subjects a MRD- response. In some cohorts, the overall response rate (ORR), including subjects achieving a complete response (CR) or partial response (PR) is assessed approximately 28 days after administration of the CAR-expressing T cells.

The presence or absence of treatment-emergent adverse events (TEAE) following treatment is also assessed. Outcome is also be assessed such as by assessing overall response rate (ORR); rate of minimum residual disease (MRD) negative responses; duration of response (DOR); relapse free-survival (RFS); progression free-survival (PFS); event-free survival (EFS); overall survival (OS); MRD response rate; and rate of hematopoietic stem cell transplant (HSCT) after response to CAR-expressing T cell infusion. In some cases, the percentage of r/r B-ALL subjects who achieve CR or CRi with no MRD detected in bone marrow (BM) (<0.01% by a validated assay) assessed during 24 months after the administration of CAR-expressing cells. Subjects also are assessed for pharmacokinetics (PK) of anti-CD19 CAR T cells, including maximum concentration (Cₘₐₓ); time to peak concentration (Tₘₐₓ); and area under the curve (AUC) of CAR+ T cells in samples (such as blood or tumor samples) from the subject, e.g., taken at various time points, post-treatment.

The present invention is not intended to be limited in scope to the particular disclosed embodiments, which are provided, for example, to illustrate various aspects of the invention. Various modifications to the compositions and methods described will become apparent from the description and teachings herein.

### Sequences

| # | **SEQUENCE** | **ANNOTATION** |
|---|---|---|
| 1 | ESKYGPPCPPCP | spacer (IgG4hinge) (aa) |
| 2 | GAATCTAAGTACGGACCGCCCTGCCCCCCTTGCCCT | spacer (IgG4hinge) (nt) |
| 3 | ESKYGPPCPPCPGQPREPQVYTLPPSQEEMTKNQVSLTCLVKGFYPSD IAVEWESNGQPENNYKTTPPVLDSDGSFFLYSRLTVDKSRWQEGNVFS CSVMHEALHNHYTQKSLSLSLGK | Hinge-CH3 spacer |
| 4 | ESKYGPPCPPCPAPEFLGGPSVFLFPPKPKDTLMISRTPEVTCVVVDV SQEDPEVQFNWYVDGVEVHNAKTKPREEQFNSTYRVVSVLTVLHQDWL NGKEYKCKVSNKGLPSSIEKTISKAKGQPREPQVYTLPPSQEEMTKNQ VSLTCLVKGFYPSDIAVEWESNGQPENNYKTTPPVLDSDGSFFLYSRL TVDKSRWQEGNVFSCSVMHEALHNHYTQKSLSLSLGK | Hinge-CH2-CH3 spacer |
| 5 | RWPESPKAQASSVPTAQPQAEGSLAKATTAPATTRNTGRGGEEKKKEK EKEEQEERETKTPECPSHTQPLGVYLLTPAVQDLWLRDKATFTCFVVG SDLKDAHLTWEVAGKVPTGGVEEGLLERHSNGSQSQHSRLTLPRSLWN AGTSVTCTLNHPSLPPQRLMALREPAAQAPVKLSLNLLASSDPPEAAS WLLCEVSGFSPPNILLMWLEDQREVNTSGFAPARPPPQPGSTTFWAWS VLRVPAPPSPQPATYTCVVSHEDSRTLLNASRSLEVSYVTDH | IgD-hinge-Fc |
| 6 | LEGGGEGRGSLLTCGDVEENPGPR | T2A artificial |
| 7 | MLLLVTSLLLCELPHPAFLLIPRKVCNGIGIGEFKDSLSINATNIKHF KNCTSISGDLHILPVAFRGDSFTHTPPLDPQELDILKTVKEITGFLLI QAWPENRTDLHAFENLEIIRGRTKQHGQFSLAVVSLNITSLGLRSLKE ISDGDVIISGNKNLCYANTINWKKLFGTSGQKTKIISNRGENSCKATG QVCHALCSPEGCWGPEPRDCVSCRNVSRGRECVDKCNLLEGEPREFVE NSECIQCHPECLPQAMNITCTGRGPDNCIQCAHYIDGPHCVKTCPAGV MGENNTLVWKYADAGHVCHLCHPNCTYGCTGPGLEGCPTNGPKIPSIA TGMVGALLLLLVVALGIGLFM | tEGFR artificial |
| 8 | FWVLVVVGGVLACYSLLVTVAFIIFWV | CD28 (amino acids 153-179 of Accession No. P10747) |
| | | Homo sapiens |
| 9 | IEVMYPPPYLDNEKSNGTIIHVKGKHLCPSPLFPGPSKPFWVLVVVGG VLACYSLLVTVAFIIFWV | CD28 (amino acids 114-179 of Accession No. P10747) |
| | | Homo sapiens |
| 10 | RSKRSRLLHSDYMNMTPRRPGPTRKHYQPYAPPRDFAAYRS | CD28 (amino acids 180-220 of P10747) |
| | | Homo sapiens |
| 11 | RSKRSRGGHSDYMNMTPRRPGPTRKHYQPYAPPRDFAAYRS | CD28 (LL to GG) Homo sapiens |
| 12 | KRGRKKLLYIFKQPFMRPVQTTQEEDGCSCRFPEEEEGGCEL | 4-1BB (amino acids 214-255 of Q07011.1) |
| | | Homo sapiens |
| 13 | RVKFSRSADAPAYQQGQNQLYNELNLGRREEYDVLDKRRGRDPEMGGK PRRKNPQEGLYNELQKDKMAEAYSEIGMKGERRRGKGHDGLYQGLSTA TKDTYDALHMQALPPR | CD3 zeta |
| | | Homo sapiens |
| 14 | RVKFSRSAEPPAYQQGQNQLYNELNLGRREEYDVLDKRRGRDPEMGGK PRRKNPQEGLYNELQKDKMAEAYSEIGMKGERRRGKGHDGLYQGLSTA TKDTYDALHMQALPPR | CD3 zeta |
| | | Homo sapiens |
| 15 | RVKFSRSADAPAYKQGQNQLYNELNLGRREEYDVLDKRRGRDPEMGGK PRRKNPQEGLYNELQKDKMAEAYSEIGMKGERRRGKGHDGLYQGLSTA TKDTYDALHMQALPPR | CD3 zeta |
| | | Homo sapiens |
| 16 | RKVCNGIGIGEFKDSLSINATNIKHFKNCTSISGDLHILPVAFRGDSF THTPPLDPQELDILKTVKEITGFLLIQAWPENRTDLHAFENLEIIRGR TKQHGQFSLAVVSLNITSLGLRSLKEISDGDVIISGNKNLCYANTINW KKLFGTSGQKTKIISNRGENSCKATGQVCHALCSPEGCWGPEPRDCVS CRNVSRGRECVDKCNLLEGEPREFVENSECIQCHPECLPQAMNITCTG RGPDNCIQCAHYIDGPHCVKTCPAGVMGENNTLVWKYADAGHVCHLCH PNCTYGCTGPGLEGCPTNGPKIPSIATGMVGALLLLLVVALGIGLFM | tEGFR artificial |
| 17 | EGRGSLLTCGDVEENPGP | T2A artificial |
| 18 | GSGATNFSLLKQAGDVEENPGP | P2A |
| 19 | ATNFSLLKQAGDVEENPGP | P2A |
| 20 | QCTNYALLKLAGDVESNPGP | E2A |
| 21 | VKQTLNFDLLKLAGDVESNPGP | F2A |
| 22 | PGGG- (SGGGG) 5-P- wherein P is proline, G is glycine and S is serine | linker |
| 23 | GSADDAKKDAAKKDGKS | Linker |
| 24 | GSTSGSGKPGSGEGSTKG | Linker |
| 25 | gacatccagatgacccagaccacctccagcctgagcgccagcctgggc gaccgggtgaccatcagctgccgggccagccaggacatcagcaagtac ctgaactggtatcagcagaagcccgacggcaccgtcaagctgctgatc taccacaccagccggctgcacagcggcgtgcccagccggtttagcggc agcggctccggcaccgactacagcctgaccatctccaacctggaacag gaagatatcgccacctacttttgccagcagggcaacacactgccctac acctttggcggcggaacaaagctggaaatcaccggcagcacctccggc agcggcaagcctggcagcggcgagggcagcaccaagggcgaggtgaag ctgcaggaaagcggccctggcctggtggcccccagccagagcctgagc gtgacctgcaccgtgagcggcgtgagcctgcccgactacggcgtgagc tggatccggcagccccccaggaagggcctggaatggctgggcgtgatc tggggcagcgagaccacctactacaacagcgccctgaagagccggctg accatcatcaaggacaacagcaagagccaggtgttcctgaagatgaac agcctgcagaccgacgacaccgccatctactactgcgccaagcactac tactacggcggcagctacgccatggactactggggccagggcaccagc gtgaccgtgagcagc | Sequence encoding scFv |
| 26 | X₁PPX₂P | Hinge |
| | X₁ is glycine, cysteine or arginine | |
| | X₂ is cysteine or threonine | |
| 27 | Glu Pro Lys Ser Cys Asp Lys Thr His Thr Cys Pro Pro Cys Pro | Hinge |
| 28 | Glu Arg Lys Cys Cys Val Glu Cys Pro Pro Cys Pro | Hinge |
| 29 | ELKTPLGDTHTCPRCPEPKSCDTPPPCPRCPEPKSCDTPPPCPRCPEP KSCDTPPPCPRCP | Hinge |
| 30 | Glu Ser Lys Tyr Gly Pro Pro Cys Pro Ser Cys Pro | Hinge |
| 31 | Glu Ser Lys Tyr Gly Pro Pro Cys Pro Pro Cys Pro | Hinge |
| 32 | Tyr Gly Pro Pro Cys Pro Pro Cys Pro | Hinge |
| 33 | Lys Tyr Gly Pro Pro Cys Pro Pro Cys Pro | Hinge |
| 34 | Glu Val Val Val Lys Tyr Gly Pro Pro Cys Pro Pro Cys Pro | Hinge |
| 35 | RASQDISKYLN | CDR L1 |
| 36 | SRLHSGV | CDR L2 |
| 37 | GNTLPYTFG | CDR L3 |
| 38 | DYGVS | CDR H1 |
| 39 | VIWGSETTYYNSALKS | CDR H2 |
| 40 | YAMDYWG | CDR H3 |
| 41 | EVKLQESGPGLVAPSQSLSVTCTVSGVSLPDYGVSWIRQPPRKGLEWL GVIWGSETTYYNSALKSRLTIIKDNSKSQVFLKMNSLQTDDTAIYYCA KHYYYGGSYAMDYWGQGTSVTVSS | V_{H} |
| 42 | DIQMTQTTSSLSASLGDRVTISCRASQDISKYLNWYQQKPDGTVKLLI YHTSRLHSGVPSRFSGSGSGTDYSLTISNLEQEDIATYFCQQGNTLPY TFGGGTKLEIT | V_{L} |
| 43 | DIQMTQTTSSLSASLGDRVTISCRASQDISKYLNWYQQKPDGTVKLLI YHTSRLHSGVPSRFSGSGSGTDYSLTISNLEQEDIATYFCQQGNTLPY TFGGGTKLEITGSTSGSGKPGSGEGSTKGEVKLQESGPGLVAPSQSLS VTCTVSGVSLPDYGVSWIRQPPRKGLEWLGVIWGSETTYYNSALKSRL TIIKDNSKSQVFLKMNSLQTDDTAIYYCAKHYYYGGSYAMDYWGQGTS VTVSS | scFv |
| 44 | KASQNVGTNVA | CDR L1 |
| 45 | SATYRNS | CDR L2 |
| 46 | QQYNRYPYT | CDR L3 |
| 47 | SYWMN | CDR H1 |
| 48 | QIYPGDGDTNYNGKFKG | CDR H2 |
| 49 | KTISSVVDFYFDY | CDR H3 |
| 50 | EVKLQQSGAELVRPGSSVKISCKASGYAFSSYWMNWVKQRPGQGLEWI GQIYPGDGDTNYNGKFKGQATLTADKSSSTAYMQLSGLTSEDSAVYFC ARKTISSVVDFYFDYWGQGTTVTVSS | V_{H} |
| 51 | DIELTQSPKFMSTSVGDRVSVTCKASQNVGTNVAWYQQKPGQSPKPLI YSATYRNSGVPDRFTGSGSGTDFTLTITNVQSKDLADYFCQQYNRYPY TSGGGTKLEIKR | V_{L} |
| 52 | GGGGSGGGGSGGGGS | Linker |
| 53 | EVKLQQSGAELVRPGSSVKISCKASGYAFSSYWMNWVKQRPGQGLEWI GQIYPGDGDTNYNGKFKGQATLTADKSSSTAYMQLSGLTSEDSAVYFC ARKTISSVVDFYFDYWGQGTTVTVSSGGGGSGGGGSGGGGSDIELTQS PKFMSTSVGDRVSVTCKASQNVGTNVAWYQQKPGQSPKPLIYSATYRN SGVPDRFTGSGSGTDFTLTITNVQSKDLADYFCQQYNRYPYTSGGGTK LEIKR | scFv |
| 54 | HYYYGGSYAMDY | CDR H3 |
| 55 | HTSRLHS | CDR L2 |
| 56 | QQGNTLPYT | CDR L3 |
| 57 | ACACGGCCTCGTGTATTACTGT | IGH primer |
| 58 | ACCTGAGGAGACGGTGACC | IGH Primer |

## Claims

1. A T cell composition for use in a method of treating a B cell malignancy in a subject at or younger than 25 years of age, wherein the T cell composition comprises T cells expressing an anti-CD19 chimeric antigen receptor (CAR),
wherein the T cells are autologous to the subject;
wherein the CAR comprises an scFv specific for a human CD19, a transmembrane domain, a cytoplasmic signaling domain derived from a costimulatory molecule, and a cytoplasmic signaling domain derived from a primary signaling ITAM-containing molecule, which is or comprises a human CD3zeta signaling domain;
the method comprising:
a) determining the dose of CAR+ T cells based on the age of the subject; and
b) administering, to the subject, the CAR+ T cell composition, wherein:
(i) if the subject is younger than 18 years of age, from at or about 0.05 x 10⁶ CAR+ T cells/kg body weight of the subject to at or about 1.5 x 10⁶ CAR+ T cells/kg body weight of the subject, but that does not exceed at or about 1.5 x 10⁸ total CAR+ T cells; and
(ii) if the subject is between 18 and 25 years of age, inclusive, from at or about 0.05 x 10⁸ CAR+ T cells to at or about 1.5 x 10⁸ CAR+ T cells.

2. The T cell composition for use of claim 1, wherein:
(A):
(i) if the subject is younger than 18 years of age, the T cell composition is administered in an amount from at or about 0.5 x 10⁶ CAR+ T cells/kg body weight of the subject to at or about 1.5 x 10⁶ CAR+ T cells/kg body weight of the subject, but that does not exceed at or about 1.5 x 10⁸ total CAR+ T cells; and
(ii) if the subject is between 18 and 25 years of age, inclusive, the T cell composition is administered in an amount from at or about 0.5 x 10⁸ CAR+ T cells to at or about 1.5 x 10⁸ CAR+ T cells; or
(B):
(i) if the subject is younger than 18 years of age, the T cell composition is administered in an amount from at or about 0.05 x 10⁶ CAR+ T cells/kg body weight of the subject to at or about 0.75 x 10⁶ CAR+ T cells/kg body weight of the subject, but that does not exceed at or about 0.75 x 10⁸ total CAR+ T cells; and
(ii) if the subject is between 18 and 25 years of age, inclusive, the T cell composition is administered in an amount from at or about 0.05 x 10⁸ CAR+ T cells to at or about 0.75 x 10⁸ CAR+ T cells.

3. The T cell composition for use of claim 1 or claim 2, wherein the subject is at least at or about 6 kg in body weight or is at least at or about 12 kg in body weight.

4. A T cell composition for use of any of claims 1 to 3, wherein the subject is at or younger than 25 years of age and weighing 12 kg or more, and wherein the T cell composition is administered in an amount selected from:
(i) if the subject is younger than 18 years of age, at or about 0.5 x 10⁶ CAR+ T cells/kg body weight of the subject, but not exceeding at or about 0.5 x 10⁸ total CAR+ T cells; and
(ii) if the subject is between 18 and 25 years of age, inclusive, at or about 0.5 x 10⁸ CAR+ T cells.

5. The T cell composition for use of any of claims 1-4, wherein a total volume of at least at or about 0.05 mL of the T cell composition is administered, a total volume of at least at or about 0.1 mL of the T cell composition is administered, a total volume of at least at or about 0.5 mL of the T cell composition is administered, or a total volume of at least at or about 1 mL of the T cell composition is administered.

6. The T cell composition for use of any of claims 1-5, wherein:
(a) the concentration of the T cells is at or greater than 2.5 x 10⁶ cells/mL, optionally wherein
(i) a total volume of at least at or about 0.1 mL at a concentration of at or greater than 2.5 x 10⁶ cells/mL of the T cell composition is administered,
(ii) a total volume of at least at or about 0.5 mL at a concentration of at or greater than 2.5 x 10⁶ cells/mL of the T cell composition is administered, or
(iii) a total volume of at least at or about 1 mL at a concentration of at or greater than 2.5 x 10⁶ cells/mL of the T cell composition is administered.

7. The T cell composition for use of any of claims 1-6, wherein the T cell composition comprises CD4⁺ and CD8⁺ CAR⁺ T cells.

8. The T cell composition for use of claim 7, wherein the T cell composition is administered as a plurality of compositions, wherein the composition comprises a first composition comprising one of the CD4+ T cells and the CD8+ T cells and a second composition comprising the other of the CD4+ T cells and the CD8+ T cells, optionally , wherein (a) the first composition and the second composition are administered separately,
optionally wherein (i) the first composition and the second composition are administered simultaneously, or (ii) the first composition and the second composition are administered sequentially, in either order.

9. The T cell composition for use of claim 7 or claim 8, wherein the amount of the T cell composition administered comprises a defined ratio of CD4⁺ CAR⁺ T cells to CD8⁺ CAR⁺ T cells and/or of CD4⁺ T cells to CD8⁺ T cells, that is or is approximately 1:1 or is between approximately 1:3 and approximately 3:1, optionally wherein the defined ratio is or is approximately 1:1.

10. The T cell composition for use of any of claims 1-9, wherein the B cell malignancy is:
(a) a lymphoma or a leukemia; or
(b) relapsed and/or refractory.

11. The T cell composition for use of any of claims 1-10, wherein
(a) the B cell malignancy is a B-cell acute lymphoblastic leukemia (B-ALL), optionally CD19+ B-ALL; and/or
(b) the B cell malignancy is relapsed or refractory (r/r) B-cell Acute Lymphoblastic Leukemia (B-ALL), optionally wherein
the subject with r/r B-ALL has morphological evidence of disease in bone marrow,
further optionally wherein the subject has 5% or greater lymphoblast by morphology;
and/or
(c)
(i) the subject has a B-ALL comprising any of the following: first or greater marrow relapse, any marrow relapse after allogeneic hematopoietic stem cell transplantation (HSCT); primary refractory, optionally not achieving a complete response (CR) or complete response with incomplete blood count recovery (CRi), optionally following 2 or more separate induction regimens; chemo-refractory, optionally not achieving CR/Cri, optionally after 1 cycle of chemotherapy for relapsed leukemia; or is ineligible for allogeneic HSCT; optionally wherein:
(ii) the B-ALL is minimum residual disease positive (MRD⁺), and/or
(iii) subjects with B-ALL has less than 5% lymphoblast by morphology and/or minimum residual disease positive (MRD+) disease as detected by a validated assay at a frequency of 1 x10⁻⁴ or greater in bone marrow cells after two lines of therapy.

12. The T cell composition for use of claim 11 wherein;
(a) the B cell malignancy is a B-cell acute lymphoblastic leukemia (B-ALL), optionally CD19+ B-ALLand the B-ALL is minimum residual disease positive (MRD⁺),
optionally wherein
subjects with B-ALL has less than 5% lymphoblast by morphology and/or minimum residual disease positive (MRD+) disease as detected by a validated assay at a frequency of 1 x10⁻⁴ or greater in bone marrow cells after two lines of therapy.

13. The T cell composition for use of any of claims 1-12, wherein the subject has Philadelphia chromosome positive ALL and is intolerant to or have failed one or more lines of tyrosine-kinase inhibitor (TKI) therapy, or TKI therapy is contraindicated.

14. The T cell composition for use of any of claims 1-10, wherein (a) the B cell malignancy is a B-cell non-Hodgkin lymphoma (B-NHL), optionally CD19+ B-NHL and optionally
wherein:
(i) the B cell malignancy is relapsed or refractory (r/r) B-cell non-Hodgkin lymphoma (B-NHL);
and/or
(ii) the subject has a B-NHL in which there is measurable disease after 1 or more lines of chemotherapy, has failed HSCT, or is ineligible for HSCT; and/or
(iii) the B-NHL is diffuse large B cell lymphoma (DLBCL), primary mediastinal large B cell lymphoma (PMBCL), or Burkitt's lymphoma (BL).

15. The T cell composition for use of any of claims 1-14, wherein:
(1) prior to administering the composition, the subject is or has been identified as having cells expressing CD19, optionally wherein the expression of CD19 is detected by flow cytometry, optionally in the peripheral blood or bone marrow, and/or by immunohistochemistry, optionally of a bone marrow biopsy; and/or
(2) the subject has received a prior therapy targeting CD19, optionally wherein the subject is or has been identified as having cells expressing CD19 or having a CD19-positive disease after completion of the prior therapy targeting CD19; and/or
(3) prior to the administration, the subject has been preconditioned with a lymphodepleting therapy comprising the administration of fludarabine and/or cyclophosphamide, optionally .
(a) wherein the lymphodepleting therapy comprises administration of (i) cyclophosphamide at about 200-400 mg/m², optionally at or about 300 mg/m², inclusive, and/or (ii) fludarabine at about 20-40 mg/m², optionally 30 mg/m², daily for 2-4 days, optionally for 3 days; and/or,
(b) wherein the lymphodepleting therapy comprises administration of cyclophosphamide at about 300 mg/m² daily for 3 days and fludarabine at about 30 mg/m² daily for 3 days and/or
(c) wherein the lymphodepleting therapy is administered 2-7 days prior to the administration of the T cell composition.

16. The T cell composition for use of any of claims 1-15, wherein the costimulatory molecule is or comprises a human 4-1BB, and wherein the CAR further comprises a spacer between the transmembrane domain and the scFv,
optionally wherein the spacer is (i) a polypeptide spacer that comprises or consists of all or a portion of an immunoglobulin hinge or a modified version thereof, optionally an IgG4 hinge, or a modified version thereof; and/or (ii) about 15 amino acids or less, optionally at or about 12 amino acids in length.

17. The T cell composition for use of claim 16, wherein:
(1)
(a) the scFv comprises a variable light (V_{L}) chain comprising a CDRL1 sequence of RASQDISKYLN (SEQ ID NO: 35), a CDRL2 sequence of SRLHSGV (SEQ ID NO: 36), and a CDRL3 sequence of GNTLPYTFG (SEQ ID NO: 37), and a variable heavy (V_{H}) chain comprising a CDRH1 sequence of DYGVS (SEQ ID NO: 38), a CDRH2 sequence of VIWGSETTYYNSALKS (SEQ ID NO: 39), and a CDRH3 sequence of YAMDYWG (SEQ ID NO: 40), or
(b) the scFv comprises a V_{L} comprising a CDRL1 sequence of FMC63, a CDRL2 sequence of FMC63, a CDRL3 sequence of FMC63, and a V_{H} comprising a CDRH1 sequence of FMC63, a CDRH2 sequence of FMC63, and a CDRH3 sequence of FMC63;
And optionally wherein the scFv comprises a V_{H} set forth in SEQ ID NO:41 and a V_{L} set forth in SEQ ID NO: 42 or the scFv is or comprises the sequence set forth in SEQ ID NO:43;
And/or
(2) (a) the spacer comprises or consists of the sequence of SEQ ID NO: 1, a sequence encoded by SEQ ID NO: 2, SEQ ID NO: 30, SEQ ID NO: 31, SEQ ID NO: 32, SEQ ID NO: 33, SEQ ID NO: 34, or a variant of any of the foregoing having at least 85%, 86%, 87%, 88%, 89%, 90%, 91%, 92%, 93%, 94%, 95%, 96%, 97%, 98%, 99% or more sequence identity thereto; and/or
(b) the spacer comprises the formula X₁PPX₂P, where X₁ is glycine, cysteine or arginine and X₂ is cysteine or threonine;
And/or
(3) the cytoplasmic signaling domain derived from a costimulatory molecule comprises SEQ ID NO: 12 or a variant thereof having at least 85%, 86%, 87%, 88%, 89%, 90%, 91%, 92%, 93%, 94%, 95%, 96%, 97%, 98%, 99% or more sequence identity thereto;
and/or
(4) the cytoplasmic signaling domain derived from a primary signaling ITAM-containing molecule comprises SEQ ID NO: 13, 14 or 15 having at least 85%, 86%, 87%, 88%, 89%, 90%, 91%, 92%, 93%, 94%, 95%, 96%, 97%, 98%, 99% or more sequence identity thereto.

18. The T cell composition for use of claim 16 or claim 17, wherein the transmembrane domain is a transmembrane domain of human CD28, optionally wherein the transmembrane domain comprises the sequence of amino acids set forth in SEQ ID NO: 8 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 8 or the sequence of amino acids set forth in SEQ ID NO: 9 or a sequence of amino acids having at least at or about 85%, 86%, 87%, 88%, 89%, 90%, 91%, 92%, 93%, 94%, 95%, 96%, 97%, 98%, 99% or more sequence identity to SEQ ID NO:9.

19. The T cell composition for use of any of claims 1-18, wherein the T cells are primary T cells obtained from the subject.

## Patentansprüche

1. T-Zell-Zusammensetzung zur Verwendung bei einem Verfahren zum Behandeln einer B-Zell-Malignität bei einem Individuum, das jünger als 25 Jahre oder im Alter von 25 Jahren ist, wobei die T-Zell-Zusammensetzung T-Zellen umfasst, die einen chimären Anti-CD19-Antigenrezeptor (CAR) exprimieren,
wobei die T-Zellen autolog gegenüber dem Individuum sind; wobei der CAR ein scFv, das für ein menschliches CD19 spezifisch ist, eine Transmembrandomäne, eine zytoplasmatische Signalgebungsdomäne, die von einem costimulatorischen Molekül abgeleitet ist, und eine zytoplasmatische Signalgebungsdomäne, die von einem primären signalgebenden ITAM-haltigen Molekül abgeleitet ist, bei dem es sich um eine menschliche CD3zeta-Signalgebungsdomäne handelt oder das diese umfasst, umfasst;
wobei das Verfahren Folgendes umfasst:
a) Bestimmen der Dosis von CAR+-T-Zellen auf Basis des Alters des Individuums; und
b) Verabreichen der CAR+-T-Zell-Zusammensetzung an das Individuum, wobei:
(i) genau oder etwa 0,05 x 10⁶ CAR+-T-Zellen/kg Körpergewicht des Individuums bis genau oder etwa 1,5 x 10⁶ CAR+-T-Zellen/kg Körpergewicht des Individuums, aber genau oder etwa 1,5 x 10⁸ CAR+-T-Zellen insgesamt nicht überschreitend, wenn das Individuum jünger als 18 Jahre ist; und
(ii) genau oder etwa 0,05 x 10⁸ CAR+-T-Zellen bis genau oder etwa 1,5 x 10⁸ CAR+-T-Zellen, wenn das Individuum zwischen einschließlich 18 und 25 Jahren alt ist.

2. T-Zell-Zusammensetzung zur Verwendung nach Anspruch 1, wobei:
(A) :
(i) die T-Zell-Zusammensetzung in einer Menge von genau oder etwa 0,5 x 10⁶ CAR+-T-Zellen/kg Körpergewicht des Individuums bis genau oder etwa 1,5 x 10⁶ CAR+-T-Zellen/kg Körpergewicht des Individuums, die genau oder etwa 1,5 x 10⁸ CAR+-T-Zellen insgesamt nicht überschreitet, verabreicht wird, wenn das Individuum jünger als 18 Jahre ist; und
(ii) die T-Zell-Zusammensetzung in einer Menge von genau oder etwa 0,5 x 10⁸ CAR+-T-Zellen bis genau oder etwa 1,5 x 10⁸ CAR+-T-Zellen verabreicht wird, wenn das Individuum zwischen einschließlich 18 und 25 Jahren alt ist; oder
(B) :
(i) die T-Zell-Zusammensetzung in einer Menge von genau oder etwa 0,05 x 10⁶ CAR+-T-Zellen/kg Körpergewicht des Individuums bis genau oder etwa 0,75 x 10⁶ CAR+-T-Zellen/kg Körpergewicht des Individuums, die genau oder etwa 0,75 x 10⁸ CAR+-T-Zellen insgesamt nicht überschreitet, verabreicht wird, wenn das Individuum jünger als 18 Jahre ist; und
(ii) die T-Zell-Zusammensetzung in einer Menge von genau oder etwa 0,05 x 10⁸ CAR+-T-Zellen bis genau oder etwa 0,75 x 10⁸ CAR+-T-Zellen verabreicht wird, wenn das Individuum zwischen einschließlich 18 und 25 Jahren alt ist.

3. T-Zell-Zusammensetzung zur Verwendung nach Anspruch 1 oder Anspruch 2, wobei das Individuum ein Körpergewicht von mindestens genau oder etwa 6 kg oder ein Körpergewicht von mindestens genau oder etwa 12 kg aufweist.

4. T-Zell-Zusammensetzung zur Verwendung nach einem der Ansprüche 1 bis 3, wobei das Individuum mindestens genau oder jünger als 25 Jahre alt ist und 12 kg oder mehr wiegt und wobei die T-Zell-Zusammensetzung in einer Menge verabreicht wird, die aus den Folgenden ausgewählt ist:
(i) genau oder etwa 0,5 x 10⁶ CAR+-T-Zellen/kg Körpergewicht des Individuums, aber genau oder etwa 0,5 x 10⁸ CAR+-T-Zellen insgesamt nicht überschreitend, wenn das Individuum jünger als 18 Jahre ist; und
(ii) genau oder etwa 0,5 x 10⁸ CAR+-T-Zellen, wenn das Individuum zwischen einschließlich 18 und 25 Jahren alt ist.

5. T-Zell-Zusammensetzung zur Verwendung nach einem der Ansprüche 1-4, wobei ein Gesamtvolumen von mindestens genau oder etwa 0,05 ml T-Zell-Zusammensetzung verabreicht wird, ein Gesamtvolumen von mindestens genau oder etwa 0,1 ml T-Zell-Zusammensetzung verabreicht wird, ein Gesamtvolumen von mindestens genau oder etwa 0,5 ml T-Zell-Zusammensetzung verabreicht wird oder ein Gesamtvolumen von mindestens genau oder etwa 1 ml T-Zell-Zusammensetzung verabreicht wird.

6. T-Zell-Zusammensetzung zur Verwendung nach einem der Ansprüche 1-5, wobei:
(a) die Konzentration der T-Zellen genau oder größer als 2,5 x 10⁶ Zellen/ml ist, gegebenenfalls wobei
(i) ein Gesamtvolumen von mindestens genau oder etwa 0,1 ml T-Zell-Zusammensetzung in einer Konzentration von genau oder mehr als 2,5 x 10⁶ Zellen/ml verabreicht wird,
(ii) ein Gesamtvolumen von mindestens genau oder etwa 0,5 ml T-Zell-Zusammensetzung in einer Konzentration von genau oder mehr als 2,5 x 10⁶ Zellen/ml verabreicht wird oder
(iii) ein Gesamtvolumen von mindestens genau oder etwa 1 ml T-Zell-Zusammensetzung in einer Konzentration von genau oder mehr als 2,5 x 10⁶ Zellen/ml verabreicht wird.

7. T-Zell-Zusammensetzung zur Verwendung nach einem der Ansprüche 1-6, wobei die T-Zell-Zusammensetzung CD4⁺- und CD8⁺-CAR⁺-T-Zellen umfasst.

8. T-Zell-Zusammensetzung zur Verwendung nach Anspruch 7, wobei die T-Zell-Zusammensetzung als mehrere Zusammensetzungen verabreicht wird, wobei die Zusammensetzung eine erste Zusammensetzung, die eine der CD4+-T-Zellen und CD8+-T-Zellen umfasst, und eine zweite Zusammensetzung, die die andere der CD4+-T-Zellen und CD8+-T-Zellen umfasst, umfasst, wobei (a) die erste Zusammensetzung und die zweite Zusammensetzung getrennt verabreicht werden,
gegebenenfalls wobei (i) die erste Zusammensetzung und die zweite Zusammensetzung gleichzeitig verabreicht werden oder (ii) die erste Zusammensetzung und die zweite Zusammensetzung nacheinander in einer der Reihenfolgen verabreicht werden.

9. T-Zell-Zusammensetzung zur Verwendung nach Anspruch 7 oder Anspruch 8, wobei die verabreichte Menge der T-Zell-Zusammensetzung ein definiertes Verhältnis von CD4⁺-CAR⁺-T-Zellen zu CD8⁺-CAR⁺-T-Zellen und/oder von CD4 ⁺ -T-Zellen zu CD8⁺-T-Zellen umfasst, das 1:1 oder ungefähr 1:1 beträgt oder zwischen ungefähr 1:3 und ungefähr 3:1 liegt, gegebenenfalls wobei das definierte Verhältnis 1:1 oder ungefähr 1:1 beträgt.

10. T-Zell-Zusammensetzung zur Verwendung nach einem der Ansprüche 1-9, wobei die B-Zell-Malignität Folgendes ist:
(a) ein Lymphom oder eine Leukämie; oder
(b) rezidiv und/oder refraktär.

11. T-Zell-Zusammensetzung zur Verwendung nach einem der Ansprüche 1-10, wobei
(a) es sich bei der B-Zell-Malignität um eine akute lymphoblastische B-Zell-Leukämie (B-ALL), gegebenenfalls CD19+-B-ALL handelt; und/oder
(b) es sich bei der B-Zell-Malignität um eine rezidive oder refraktäre (r/r) akute lymphoblastische B-Zell-Leukämie (B-ALL) handelt, gegebenenfalls wobei
das Individuum mit r/r B-ALL morphologische Beweise einer Erkrankung im Knochenmark aufweist,
ferner gegebenenfalls wobei das Individuum 5 % oder mehr Lymphoblast durch Morphologie aufweist;
und/oder
(c)
(i) das Individuum eine B-ALL aufweist, die eine der Folgenden umfasst: erstes oder größeres Markrezidiv, ein Markrezidiv nach allogener hämatopoetischer Stammzelltransplantation (HSCT) ; primär refraktär, gegebenenfalls kein vollständiges Ansprechen (CR) oder vollständiges Ansprechen mit unvollständiger Blutbilderholung (CRi), gegebenenfalls nach 2 oder mehr getrennten Induktionsschemata, erreicht wird; chemorefraktär, gegebenenfalls CR/CRi nicht erreicht wird, gegebenenfalls nach 1 Zyklus Chemotherapie für rezidive Leukämie; oder gegebenenfalls für eine allogene HSCT nicht in Frage kommt; gegebenenfalls wobei:
(ii) das B-ALL minimale-Resterkrankung-positiv (MRD+) ist, und/oder
(iii) Individuen mit B-ALL weniger als 5 % Lymphoblast durch Morphologie und/oder eine minimale-Resterkrankungpositive (MRD+) Erkrankung aufweisen, wie durch einen validierten Assay bei einer Häufigkeit von 1 x 10⁻⁴ oder mehr in Knochenmarkzellen nach zwei Therapielinien nachgewiesen.

12. T-Zell-Zusammensetzung zur Verwendung nach Anspruch **11,** wobei:
(a) es sich bei der B-Zell-Malignität um eine akute lymphoblastische B-Zell-Leukämie (B-ALL), gegebenenfalls CD19+-B-ALL, handelt und die B-ALL minimale-Resterkrankung-positiv ist (MRD⁺),
gegebenenfalls wobei:
Individuen mit B-ALL weniger als 5 % Lymphoblast durch Morphologie und/oder eine minimale-Resterkrankungpositive (MRD+) Erkrankung aufweisen, wie durch einen validierten Assay bei einer Häufigkeit von 1 x 10⁻⁴ oder mehr in Knochenmarkzellen nach zwei Therapielinien nachgewiesen.

13. T-Zell-Zusammensetzung zur Verwendung nach einem der Ansprüche 1-12, wobei das Individuum Philadelphia-Chromosom-positive ALL aufweist und eine oder mehrere Linien Tyrosinkinase-Inhibitor(TKI)-Therapie nicht verträgt oder solche fehlgeschlagen sind, oder TKI-Therapie kontraindiziert ist.

14. T-Zell-Zusammensetzung zur Verwendung nach einem der Ansprüche 1-10, wobei (a) es sich bei der B-Zell-Malignität um ein Non-Hodgkin-B-Zell-Lymphom (B-NHL), gegebenenfalls CD19+-B-NHL handelt und gegebenenfalls wobei:
(i) es sich bei der B-Zell-Malignität um ein rezidives oder refraktäres (r/r) Non-Hodgkin-B-Zell-Lymphom (B-NHL) handelt; und/oder
(ii) das Individuum ein B-NHL aufweist, bei dem es nach 1 oder mehreren Chemotherapielinien messbare Erkrankung gibt, HSCT versagt hat oder das für HSCT nicht in Frage kommen ist; und/oder
(iii) es sich bei dem B-NHL um ein diffuses großzelliges B-Zell-Lymphom (DLBCL), primär mediastinales großzelliges B-Zell-Lymphom (PMBCL) oder Burkitt-Lymphom (BL) handelt.

15. T-Zell-Zusammensetzung zur Verwendung nach einem der Ansprüche 1-14, wobei:
(1) das Individuum vor dem Verabreichen der Zusammensetzung als CD19 exprimierende Zellen aufweisend identifiziert wird oder wurde, gegebenenfalls wobei die CD19-Expression durch Durchflusszytometrie, gegebenenfalls im peripheren Blut oder Knochenmark, und/oder durch Immunhistochemie, gegebenenfalls eine Knochenmarkbiopsie nachgewiesen wird; und/oder
(2) das Individuum eine vorherige Therapie erhalten hat, die auf CD19 abzielt, gegebenenfalls wobei das Individuum als CD19 exprimierende Zellen aufweisend oder eine CD19-positive Erkrankung nach Abschluss der vorherigen Therapie, die auf CD19 abzielt, aufweisend identifiziert wird oder wurde; und/oder
(3) das Individuum vor der Verabreichung mit einer lymphodepletierenden Therapie vorkonditioniert wurde, die die Verabreichung von Fludarabin und/oder Cyclophosphamid umfasst.
(a) wobei die lymphodepletierende Therapie Verabreichung von (i) einschließlich etwa 200-400 mg/m², gegebenenfalls genau oder etwa 300 mg/m², Cyclophosphamid und/oder (ii) etwa 20-40 mg/m², gegebenenfalls 30 mg/m², Fludarabin täglich 2-4 Tage lang, gegebenenfalls 3 Tage lang, umfasst; und/oder
(b) wobei die lymphodepletierende Therapie die Verabreichung von etwa 300 mg/m² Cyclophosphamid täglich für 3 Tage und etwa 30 mg/m² Fludarabin täglich für 3 Tage umfasst und/oder
(c) wobei die lymphodepletierende Therapie 2-7 Tage vor der Verabreichung der T-Zell-Zusammensetzung verabreicht wird.

16. T-Zell-Zusammensetzung zur Verwendung nach einem der Ansprüche 1-15, wobei es sich bei dem costimulatorischen Molekül um ein menschliches 4-1BB handelt oder es dieses umfasst und wobei der CAR ferner einen Spacer zwischen der Transmembrandomäne und dem scFv umfasst,
gegebenenfalls wobei der Spacer (i) ein Polypeptid-Spacer ist, der ein gesamtes Immunglobulinscharnier oder eine modifizierte Version davon oder einen Teil eines Immunglobulinscharniers oder einer modifizierten Version davon, gegebenenfalls ein IgG4-Scharnier oder eine modifizierte Version davon, umfasst oder daraus besteht; und/oder (ii) etwa 15 Aminosäuren oder weniger, gegebenenfalls genau oder etwa 12 Aminosäuren lang ist.

17. T-Zell-Zusammensetzung zur Verwendung nach Anspruch 16, wobei:
(1)
(a) das scFv eine variable Leichtkette (V_{L}), die eine CDRL1-Sequenz von RASQDISKYLN (SEQ ID NO: 35), eine CDRL2-Sequenz von SRLHSGV (SEQ ID NO: 36) und eine CDRL3-Sequenz von GNTLPYTFG (SEQ ID NO: 37) umfasst, und eine variable Schwerkette (V_{H}), die eine CDRH1-Sequenz von DYGVS (SEQ ID NO: 38), eine CDRH2-Sequenz von VIWGSETTYYNSALKS (SEQ ID NO: 39) und eine CDRH3-Sequenz von YAMDYWG (SEQ ID NO: 40) umfasst, umfasst oder
(b) das scFv eine V_{L}, die eine CDRL1-Sequenz von FMC63, eine CDRL2-Sequenz von FMC63, eine CDRL3-Sequenz von FMC63 umfasst, und eine V_{H}, die eine CDRH1-Sequenz von FMC63, eine CDRH2-Sequenz von FMC63 und eine CDRH3-Sequenz von FMC63 umfasst, umfasst;
und gegebenenfalls wobei das scFv eine unter SEQ ID NO: 41 dargestellte V_{H} und eine unter SEQ ID NO: 42 dargestellte V_{L} umfasst oder es sich bei dem scFv um die unter SEQ ID NO: 43 dargestellte Sequenz handelt oder es diese umfasst;
und/oder
(2)
(a) der Spacer die Sequenz unter SEQ ID NO: 1, eine durch SEQ ID NO: 2, SEQ ID NO: 30, SEQ ID NO: 31, SEQ ID NO: 32, SEQ ID NO: 33, SEQ ID NO: 34 codierte Sequenz oder eine Variante einer der vorhergehenden mit mindestens 85 %, 86 %, 87 %, 88 %, 89 %, 90 %, 91 %, 92 %, 93 %, 94 %, 95 %, 96 %, 97 %, 98 %, 99 % oder mehr Sequenzidentität dazu umfasst oder daraus besteht; und/oder
(b) der Spacer die Formel X₁PPX₂P umfasst, wobei X₁ für Glycin, Cystein oder Arginin steht und X₂ für Cystein oder Threonin steht;
und/oder
(3) die zytoplasmatische Signalgebungsdomäne, die von einem costimulatorischen Molekül abgeleitet ist, SEQ ID NO: 12 oder eine Variante davon mit mindestens 85 %, 86 %, 87 %, 88 %, 89 %, 90 %, 91 %, 92 %, 93 %, 94 %, 95 %, 96 %, 97 %, 98 %, 99 % oder mehr Sequenzidentität dazu umfasst;
und/oder
(4) die zytoplasmatische Signalgebungsdomäne, die von einem primären signalgebenden ITAM-enthaltenden Molekül abgeleitet ist, SEQ ID NO: 13, 14 oder 15 mit mindestens 85 %, 86 %, 87 %, 88 %, 89 %, 90 %, 91 %, 92 %, 93 %, 94 %, 95 %, 96 %, 97 %, 98 %, 99 % oder mehr Sequenzidentität dazu umfasst.

18. T-Zell-Zusammensetzung zur Verwendung nach Anspruch 16 oder Anspruch 17, wobei es sich bei der Transmembrandomäne um eine Transmembrandomäne des menschlichen CD28 handelt, gegebenenfalls wobei die Transmembrandomäne die unter SEQ ID NO: 8 dargestellte Sequenz von Aminosäuren oder eine Sequenz von Aminosäuren mit mindestens 85 %, 86 %, 87 %, 88 %, 89 %, 90 %, 91 %, 92 %, 93 %, 94 %, 95 %, 96 %, 97 %, 98 %, 99 % oder mehr Sequenzidentität zu SEQ ID NO: 8 oder die unter SEQ ID NO: 9 dargestellte Sequenz von Aminosäuren oder eine Sequenz von Aminosäuren mit mindestens oder etwa 85 %, 86 %, 87 %, 88 %, 89 %, 90 %, 91 %, 92 %, 93 %, 94 %, 95 %, 96 %, 97 %, 98 %, 99 % oder mehr Sequenzidentität zu SEQ ID NO:9 umfasst.

19. T-Zell-Zusammensetzung zur Verwendung nach einem der Ansprüche 1-18, wobei es sich bei den T-Zellen um primäre T-Zellen handelt, die von dem Individuum gewonnen wurden.

## Revendications

1. Composition de lymphocytes T pour une utilisation dans une méthode de traitement d'une néoplasie à cellules B chez un sujet âgé de moins de 25 ans, la composition de lymphocytes T comprenant des lymphocytes T exprimant un récepteur d'antigène chimérique anti-CD19 (CAR), dans laquelle les lymphocytes T sont autologues au sujet ;
dans laquelle le CAR comprend un scFv spécifique d'un CD19 humain, un domaine transmembranaire, un domaine de signalisation cytoplasmique dérivé d'une molécule costimulatrice, et un domaine de signalisation cytoplasmique dérivé d'une molécule contenant un ITAM de signalisation primaire, qui est ou comprend un domaine de signalisation CD3zêta humain ;
la méthode comprenant :
a) la détermination de la dose de lymphocytes T CAR+ en fonction de l'âge du sujet ; et
b) l'administration au sujet de la composition de lymphocytes T CAR+, à raison de :
(i) si le sujet est âgé de moins de 18 ans, environ 0,05 x 10⁶ lymphocytes T CAR+/kg de poids corporel du sujet à environ 1,5 x 10⁶ lymphocytes T CAR+/kg de poids corporel du sujet, mais sans dépasser environ 1,5 x 10⁸ lymphocytes T CAR+ totaux ; et
(ii) si le sujet est âgé de 18 à 25 ans inclus, d'environ 0,05 x 10⁸ lymphocytes T CAR+ à environ 1,5 x 10⁸ lymphocytes T CAR+.

2. Composition de lymphocytes T selon la revendication 1, dans laquelle :
(A) :
(i) si le sujet est âgé de moins de 18 ans, la composition de lymphocytes T est administrée en une quantité d'environ 0,5 x 10⁶ lymphocytes T CAR+/kg de poids corporel du sujet à environ 1,5 x 10⁶ lymphocytes T CAR+/kg de poids corporel du sujet, mais qui ne dépasse pas environ 1,5 x 10⁸ lymphocytes T CAR+ totaux ; et
(ii) si le sujet est âgé de 18 à 25 ans inclus, la composition de lymphocytes T est administrée en une quantité d'environ 0,5 x 10⁸ lymphocytes T CAR+ à environ 1,5 x 10⁸ lymphocytes T CAR+ ; ou
(B) :
(i) si le sujet est âgé de moins de 18 ans, la composition de lymphocytes T est administrée en une quantité d'environ 0,05 x 10⁶ lymphocytes T CAR+/kg de poids corporel du sujet à environ 0,75 x 10⁶ lymphocytes T CAR+/kg de poids corporel du sujet, mais qui ne dépasse pas environ 0,75 x 10⁸ lymphocytes T CAR+ totaux ; et
(ii) si le sujet est âgé de 18 à 25 ans inclus, la composition de lymphocytes T est administrée en une quantité d'environ 0,05 x 10⁸ lymphocytes T CAR+ à environ 0,75 x 10⁸ lymphocytes T CAR+.

3. Composition de lymphocytes T selon la revendication 1 ou la revendication 2, le sujet ayant un poids corporel d'au moins environ 6 kg à au moins environ 12 kg.

4. Composition de lymphocytes T selon l'une quelconque des revendications 1 à 3, le sujet étant âgé d'au plus 25 ans et pesant au moins 12 kg, et la composition de lymphocytes T étant administrée en une quantité choisie parmi :
(i) si le sujet est âgé de moins de 18 ans, environ 0,5 x 10⁶ lymphocytes T CAR+/kg de poids corporel du sujet, mais pas plus d'environ 0,5 x 10⁸ lymphocytes T CAR+ totaux ; et
(ii) si le sujet est âgé de 18 à 25 ans, inclus, environ 0,5 x 10⁸ lymphocytes T CAR+.

5. Composition de lymphocytes T selon l'une quelconque des revendications 1 à 4, dans laquelle un volume total d'au moins environ 0,05 ml de la composition de lymphocytes T est administré, un volume total d'au moins environ 0,1 ml de la composition de lymphocytes T est administré, un volume total d'au moins environ 0,5 ml de la composition de lymphocytes T est administré, ou un volume total d'au moins environ 1 ml de la composition de lymphocytes T est administré.

6. Composition de lymphocytes T selon l'une quelconque des revendications 1 à 5, dans laquelle :
(a) la concentration des lymphocytes T est supérieure ou égale à 2,5 x 10⁶ cellules/ml, facultativement **caractérisée en ce que**
(i) un volume total d'au moins environ 0,1 ml à une concentration supérieure ou égale à 2,5 x 10⁶ cellules/mL de la composition de lymphocytes T est administré,
(ii) un volume total d'au moins environ 0,5 ml à une concentration supérieure ou égale à 2,5 x 10⁶ cellules/mL de la composition de lymphocytes T est administré, ou
(iii) un volume total d'au moins environ 1 ml à une concentration supérieure ou égale à 2,5 x 10⁶ cellules/mL de la composition de lymphocytes T est administré.

7. Composition de lymphocytes T selon l'une quelconque des revendications 1 à 6, la composition de lymphocytes T comprenant des lymphocytes T CAR⁺ CD4⁺ et CD8⁺.

8. Composition de lymphocytes T pour utilisation selon la revendication 7, la composition de lymphocytes T étant administrée sous la forme d'une pluralité de compositions, la composition comprenant une première composition comprenant un type de lymphocytes T parmi des lymphocytes T CD4+ ou des lymphocytes T CD8+ et une deuxième composition comprenant l'autre type de lymphocytes T parmi des lymphocytes T CD4+ et des lymphocytes T CD8+, facultativement **caractérisée en ce que** (a) la première composition et la deuxième composition sont administrées séparément,
facultativement **caractérisée en ce que** (i) la première composition et la deuxième composition sont administrées simultanément, ou (ii) la première composition et la deuxième composition sont administrées séquentiellement, dans un ordre quelconque.

9. Composition de lymphocytes T selon la revendication 7 ou la revendication 8, la quantité de la composition de lymphocytes T administrée comprenant un rapport défini des lymphocytes T CAR⁺ CD4⁺ aux lymphocytes T CAR⁺ CD8⁺ et/ou des lymphocytes T CD4⁺ aux lymphocytes T CD8 ⁺, qui est d'environ 1:1 ou est compris entre environ 1:3 et environ 3:1, facultativement, le rapport défini étant d'environ 1:1.

10. Composition de lymphocytes T selon l'une quelconque des revendications 1 à 9, dans laquelle la néoplasie à cellules B est :
(a) un lymphome ou une leucémie ; ou
(b) récidivante et/ou réfractaire.

11. Composition de lymphocytes T selon l'une quelconque des revendications 1 à 10, **caractérisée en ce que** :
(a) la néoplasie à cellules B est une leucémie lymphoblastique aiguë à cellules B (B-ALL), facultativement B-ALL CD19+ ; et/ou
(b) la malignité à lymphocytes B est une leucémie lymphoblastique aiguë à cellules B (B-ALL) récidivante ou réfractaire (r/r), facultativement où
le sujet avec B-ALL r/r présente des signes morphologiques de la maladie dans la moelle osseuse,
en outre, facultativement, le sujet présente au moins 5 % de lymphoblastes par morphologie ;
et/ou
(c)
(i) le sujet est atteint d'une B-ALL présentant l'une quelconque des caractéristiques suivantes : au moins une première récidive médullaire, récidive médullaire après une greffe de cellules souches hématopoïétiques allogéniques (HSCT) ; réfractaire primaire, facultativement sans réponse complète (CR) ou présentant une réponse complète avec récupération incomplète des numérations sanguines (CRi), facultativement après au moins 2 régimes d'induction séparés ; chimioréfractaire, facultativement n'atteignant pas CR/CRi, facultativement après 1 cycle de chimiothérapie pour une leucémie récidivante ; ou n'est pas éligible à une HSCT ; facultativement **caractérisée en ce que**
(ii) la B-ALL présente une maladie résiduelle minimale positive (MRD⁺), et/ou
(iii) les sujets atteints de B-ALL présentent moins de 5 % de lymphoblastes par morphologie et/ou présentent une maladie résiduelle minimale positive (MRD+) tel que détecté par un dosage validé à une fréquence d'au moins 1 x 10⁻⁴ dans des cellules de moelle osseuse après deux lignes de traitement.

12. Composition de lymphocytes T selon la revendication 11, **caractérisée en ce que** ;
(a) la néoplasie à cellules B est une leucémie lymphoblastique aiguë à cellules B (B-ALL), facultativement B-ALL CD19+ et la B-ALL présente une maladie résiduelle minimale positive (MRD⁺), facultativement où :
les sujets atteints de B-ALL présentent moins de 5 % de lymphoblastes par morphologie et/ou présentent une maladie résiduelle minimale positive (MRD+) tel que détecté par un dosage validé à une fréquence d'au moins 1 x 10⁻⁴ dans des cellules de moelle osseuse après deux lignes de traitement.

13. Composition de lymphocytes T selon l'une quelconque des revendications 1 à 12, **caractérisée en ce que** le sujet est atteint d'une LLA positive pour le chromosome de Philadelphie et est intolérant ou n'a pas répondu à une ou plusieurs lignes de traitement par un inhibiteur de tyrosine kinase (TKI), ou le traitement par TKI est contre-indiqué.

14. Composition de lymphocytes T selon l'une quelconque des revendications 1 à 10, **caractérisée en ce que** (a) la néoplasie à cellules B est un lymphome non hodgkinien à cellules B (LNH-B), facultativement LNH-B CD19+ et facultativement
**caractérisée en ce que** :
(i) la néoplasie à cellules B est un lymphome B non hodgkinien (LNH-B) récidivant ou réfractaire (r/r) ; et/ou
(ii) le sujet est atteint d'un LNH-B et la maladie est encore mesurable après une ou plusieurs lignes de chimiothérapie, n'a pas répondu à une HSCT ou n'est pas éligible à une HSCT ; et/ou
(iii) le LNH-B est un lymphome B diffus à grandes cellules (DLBCL), un lymphome B médiastinal primaire à grandes cellules (PMBCL) ou un lymphome de Burkitt (BL).

15. Composition de lymphocytes T selon l'une quelconque des revendications 1 à 14, dans laquelle :
(1) avant l'administration de la composition, le sujet est ou a été identifié comme ayant des cellules exprimant CD19, facultativement, l'expression de CD19 étant détectée par cytométrie en flux, facultativement dans le sang périphérique ou la moelle osseuse, et/ou par immunohistochimie, facultativement d'une biopsie de moelle osseuse ; et/ou
(2) le sujet a reçu un traitement précédent ciblant CD19, facultativement, le sujet ayant été identifié comme ayant des cellules exprimant CD19 ou ayant une maladie positive pour CD19 après la fin du traitement précédent ciblant CD19 ; et/ou
(3) avant l'administration, le sujet a été préconditionné par un traitement lymphodépléteur comprenant l'administration de fludarabine et/ou de cyclophosphamide, facultativement,
(a) le traitement lymphodépléteur comprenant l'administration (i) de cyclophosphamide à raison d'environ 200 à 400 mg/m² , facultativement d'environ 300 mg/m² inclus, et/ou (ii) de fludarabine à raison d'environ 20 à 40 mg/m² , facultativement de 30 mg m², quotidiennement pendant 2 à 4 jours, facultativement pendant 3 jours ; et/ou
(b) le traitement lymphodépléteur comprenant l'administration de cyclophosphamide à environ 300 mg/m² par jour pendant 3 jours et de fludarabine à environ 30 mg/m² par jour pendant 3 jours et/ou
(c) le traitement lymphodépléteur étant administrée 2 à 7 jours avant l'administration de la composition de lymphocytes T.

16. Composition de lymphocytes T selon l'une quelconque des revendications 1 à 15, dans laquelle la molécule costimulatrice est ou comprend un 4-1BB humain, et dans laquelle le CAR comprend en outre un espaceur entre le domaine transmembranaire et le scFv,
facultativement, dans laquelle l'espaceur (i) est un espaceur polypeptidique qui comprend ou est constitué de la totalité ou d'une partie d'une charnière d'immunoglobuline ou d'une version modifiée de celle-ci, facultativement une charnière d'IgG4, ou une version modifiée de celle-ci ; et/ou (ii) a une longueur inférieure ou égale à environ 15 acides aminés, facultativement inférieure ou égale à environ 12 acides aminés.

17. Composition de lymphocytes T selon la revendication 16, dans laquelle :
(1)
(a) le scFv comprend une chaîne légère variable (V_{L}) comprenant une séquence CDRL1 de RASQDISKYLN (SEQ ID NO : 35), une séquence CDRL2 de SRLHSGV (SEQ ID NO : 36), et une séquence CDRL3 de GNTLPYTFG (SEQ ID NO : 37), et une chaîne lourde variable (V_{H}) comprenant une séquence CDRH1 de DYGVS (SEQ ID NO : 38), une séquence CDRH2 de VIWGSETTYYNSALKS (SEQ ID NO : 39), et une séquence CDRH3 de YAMDYWG (SEQ ID NO : 40), ou
(b) le scFv comprend une V_{L} comprenant une séquence CDRL1 de FMC63, une séquence CDRL2 de FMC63, une séquence CDRL3 de FMC63, et une V_{H} comprenant une séquence CDRH1 de FMC63, une séquence CDRH2 de FMC63, et une séquence CDRH3 de FMC63 ;
et facultativement, le scFv comprend un V_{H} décrit dans SEQ ID NO : 41 et un V_{L} décrit dans SEQ ID NO : 42 ou le scFv est ou comprend la séquence décrite dans SEQ ID NO : 43 ;
et/ou
(2)
(a) l'espaceur comprend ou est constitué de la séquence de SEQ ID NO : 1, une séquence codée par SEQ ID NO : 2, SEQ ID NO : 30, SEQ ID NO : 31, SEQ ID NO : 32, SEQ ID NO : 33, SEQ ID NO : 34, ou un variant de l'une quelconque de celles-ci présentant au moins 85 %, 86 %, 87 %, 88 %, 89 %, 90 %, 91 %, 92 %, 93 %, 94 %, 95 %, 96 %, 97 %, 98 %, 99 % d'identité de séquence avec celle-ci ; et/ou
(b) l'espaceur comprend la formule X₁PPX₂P, où X₁ est la glycine, la cystéine ou l'arginine et X₂ est la cystéine ou la thréonine ;
et/ou
(3) le domaine de signalisation cytoplasmique dérivé d'une molécule costimulatrice comprend SEQ ID NO : 12 ou un variant de celle-ci présentant au moins 85 %, 86 %, 87 %, 88 %, 89 %, 90 %, 91 %, 92 %, 93 %, 94 %, 95 %, 96 %, 97 %, 98 %, 99 % d'identité de séquence avec celle-ci ;
et/ou
(4) le domaine de signalisation cytoplasmique dérivé d'une molécule contenant un ITAM de signalisation primaire comprend SEQ ID NO : 13, 14 ou 15 présentant au moins 85 %, 86 %, 87 %, 88 %, 89 %, 90 %, 91 %, 92 %, 93 %, 94 %, 95 %, 96 %, 97 %, 98 %, 99 % d'identité de séquence avec celles-ci.

18. Composition de lymphocytes T selon la revendication 16 ou la revendication 17, dans laquelle le domaine transmembranaire est un domaine transmembranaire de CD28 humain, facultativement dans laquelle le domaine transmembranaire comprend la séquence d'acides aminés décrite dans SEQ ID NO : 8 ou une séquence d'acides aminés qui présente au moins 85 %, 86 %, 87 %, 88 %, 89 %, 90 %, 91 %, 92 %, 93 %, 94 %, 95 %, 96 %, 97 %, 98 %, 99 % d'identité de séquence avec SEQ ID NO : 8 ou la séquence d'acides aminés décrite dans SEQ ID NO : 9 ou une séquence d'acides aminés présentant au moins ou environ 85 %, 86 %, 87 %, 88 %, 89 %, 90 %, 91 %, 92 %, 93 %, 94 %, 95 %, 96 %, 97 %, 98 %, 99 % d'identité de séquence avec SEQ ID NO : 9.

19. Composition de lymphocytes T selon l'une quelconque des revendications 1 à 18, dans laquelle les lymphocytes T sont des lymphocytes T primaires obtenus à partir du sujet.
